# EUROPÄISCHE PATENTSCHRIFT

(11) **EP 3 063 150 B1**
(45) Veröffentlichungstag und Bekanntmachung des Hinweises auf die Patenterteilung: **22.11.2017**
(21) Anmeldenummer: 14789580.9
(22) Anmeldetag: 28.10.2014
(51) Int. Cl.: C07D 471/04

(54) **SUBSTITUIERTE OXOPYRIDIN-DERIVATE**
SUBSTITUTED OXOPYRIDINE DERIVATIVES
DÉRIVÉS D'OXOPYRIDINE SUBSTITUÉE

(30) Priorität: 30.10.2013 EP 13190940; 24.09.2014 EP 14186078
(43) Veröffentlichungstag der Anmeldung: 07.09.2016
(73) Patentinhaber: Bayer Pharma Aktiengesellschaft, 13353 Berlin (DE)
(72) Erfinder: RÖHRIG, Susanne, 40724 Hilden (DE); HILLISCH, Alexander, 42651 Solingen (DE); STRASSBURGER, Julia, 42115 Wuppertal (DE); HEITMEIER, Stefan, 42489 Wülfrath (DE); SCHMIDT, Martina, Victoria, 51063 Köln (DE); SCHLEMMER, Karl-Heinz, 42113 Wuppertal (DE); TERSTEEGEN, Adrian, 42111 Wuppertal (DE); BUCHMÜLLER, Anja, 45259 Essen (DE); GERDES, Christoph, 51063 Köln (DE); SCHÄFER, Martina, 13465 Berlin (DE); TELLER, Henrik, 18258 Schwaan (DE); JIMENEZ NUNEZ, Eloisa, 42117 Wuppertal (DE); SCHIROK, Hartmut, 40764 Langenfeld (DE); KLAR, Jürgen, 42109 Wuppertal (DE)
(74) Vertreter: BIP Patents
(86) Internationale Anmeldenummer: PCT/EP2014/073132
(87) Internationale Veröffentlichungsnummer: WO 2015/063093

(56) Entgegenhaltungen:
- WO-A2-02/42273
- GB-A- 2 497 806

## Beschreibung

Die Erfindung betrifft substituierte Oxopyridin-Derivate und Verfahren zu ihrer Herstellung sowie ihre Verwendung zur Herstellung von Arzneimitteln zur Behandlung und/oder Prophylaxe von Krankheiten, insbesondere von Herz-Kreislauf-Erkrankungen, vorzugsweise von thrombotischen beziehungsweise thromboembolischen Erkrankungen sowie von Ödemen, als auch von ophthalmologischen Erkrankungen.

Die Blutgerinnung ist ein Schutzmechanismus des Organismus, mit dessen Hilfe Defekte in der Gefäßwand rasch und zuverlässig "abgedichtet" werden können. So kann ein Blutverlust vermieden beziehungsweise minimiert werden. Die Blutstillung nach Gefäßverletzung erfolgt im Wesentlichen durch das Gerinnungssystem, bei dem eine enzymatische Kaskade komplexer Reaktionen von Plasmaproteinen ausgelöst wird. Hierbei sind zahlreiche Blutgerinnungsfaktoren beteiligt, von denen jeder, sobald aktiviert, die jeweils nächste inaktive Vorstufe in ihre aktive Form überführt. Am Ende der Kaskade steht die Umwandlung des löslichen Fibrinogens in das unlösliche Fibrin, so dass es zu einem Blutgerinnsel kommt. Traditionell unterscheidet man bei der Blutgerinnung zwischen dem intrinsischen und dem extrinsischen System, die in einem abschließenden gemeinsamen Reaktionsweg münden. Hierbei kommen den Faktoren Xa und IIa (Thrombin) Schlüsselrollen zu: Faktor Xa bündelt die Signale der beiden Gerinnungswege, da er sowohl über Faktor VIIa/Tissue Factor (extrinsischer Weg) wie auch den Tenase Komplex (intrisischer Weg) durch Umsetzung von Faktor X entsteht. Die aktivierte Serinprotease Xa spaltet Prothrombin zu Thrombin, das über eine Reihe von Umsetzungen die Impulse aus der Kaskade auf den Gerinnungsstatus des Blutes überträgt.

In der jüngeren Vergangenheit ist die traditionelle Theorie der zwei getrennten Bereiche der Koagulationskaskade (extrinsischer beziehungsweise intrinsischer Pfad) aufgrund neuer Erkenntnisse modifiziert worden: In diesen Modellen wird die Koagulation durch Bindung von aktiviertem Faktor VIIa an Tissue Faktor (TF) initiiert. Der entstandene Komplex aktiviert Faktor X, was wiederum zur Thrombin-Generierung mit anschließender Herstellung von Fibrin und Thrombozyten-Aktivierung (via PAR-1) als verletzungsverschließende Endprodukte der Hämostase führt. Im Vergleich zur anschließenden Amplifikations-/Propagationsphase ist die Geschwindigkeit der Thrombinherstellung in dieser ersten Phase klein und durch das Auftreten von TFPI als Hemmer des TF-FVIIa-FX-Komplexes zeitlich begrenzt.

Ein zentraler Bestandteil des Übergangs von der Initiation zur Amplifikation und Propagation der Koagulation ist Faktor XIa: Thrombin aktiviert in positiven Rückkopplungsschleifen neben Faktor V und Faktor VIII auch Faktor XI zu Faktor XIa, der Faktor IX zu Faktor IXa umsetzt und über den so generierten Faktor IXa/Faktor VIIIa-Komplex die Faktor X-Aktivierung und damit wiederum die Thrombinbildung stark stimuliert, was zu starkem Thrombuswachstum führt und den Thrombus stabilisiert.

Darüber hinaus ist in den Blickpunkt gerückt, dass neben der Stimulation über Tissue Faktor die Aktivierung des Gerinnungssystems an insbesondere negativ geladenen Oberflächen erfolgen kann, zu denen neben Oberflächenstrukturen körperfremder Zellen (z.B. Bakterien) auch artifizielle Oberflächen wie Gefäßprothesen, Stents und extrakorporale Kreisläufe gehören. Auf der Oberfläche findet zunächst die Aktivierung von Faktor XII (FXII) zu Faktor XIIa statt, der im Folgenden an Zelloberflächen gebundenen Faktor XI zu Faktor XIa aktiviert. Dieser führt wie zuvor beschrieben zur weiteren Aktivierung der Gerinnungskaskade. Daneben aktiviert Faktor XIIa ebenfalls gebundenes Plasmaprokallikrein zu Plasmakallikrein (PK), das zum einen zu weiterer Faktor XII-Aktivierung im Rahmen einer Potentierungsschleife führt, was insgesamt eine Verstärkung der Initiation der Gerinnungskaskade zur Folge hat. Zusätzlich stellt PK eine wichtige Bradikinin-freisetzende Protease dar, die somit unter anderem zum Anstieg der endothelialen Permeabilität führt. Als weitere Substrate wurden Prorenin und Prourokinase beschrieben, deren Aktivierung die regulatorischen Prozesse des Renin-Angiotensin-Systems und der Fibrinolyse beeinflussen kann. Damit stellt die Aktivierung von PK ein wichtiges Bindeglied zwischen koagulativen und inflammatorischen Prozessen dar.

Eine unkontrollierte Aktivierung des Gerinnungssystems oder eine defekte Hemmung der Aktivierungsprozesse kann die Bildung von lokalen Thrombosen oder Embolien in Gefäßen (Arterien, Venen, Lymphgefäßen) oder Herzhöhlen bewirken. Darüber hinaus kann eine systemische Hyperkoagulabilität zur systemweiten Bildung von Thromben und schließlich zu einer Verbrauchskoagulopathie im Rahmen einer disseminierten intravasalen Gerinnung führen. Thromboembolische Komplikationen können ferner in extrakorporalen Blutkreisläufen, wie z. B. während einer Hämodialyse, sowie in Gefäß- beziehungsweise Herzklappenprothesen und Stents auftreten.

Im Verlauf vieler Herzkreislauf- und Stoffwechselerkrankungen kommt es infolge systemischer Faktoren, wie z.B. Hyperlipidämie, Diabetes oder Rauchen, infolge von Blutflußveränderungen mit Stase, wie z.B. beim Vorhofflimmern, oder infolge pathologischer Gefäßwandveränderungen, z.B. endothelialer Dysfunktionen oder Atherosklerose, zu einer erhöhten Neigung von Gerinnungs- und Thrombozytenaktivierung. Diese unerwünschte und überschießende Aktivierung der Gerinnung kann durch Bildung fibrin- und plättchenreicher Thromben zu thromboembolischen Erkrankungen und thrombotischen Komplikationen mit lebensbedrohlichen Zuständen führen. Hierbei können auch entzündliche Prozesse involviert sein. Thromboembolische Erkrankungen gehören daher nach wie vor zu den häufigsten Ursachen von Morbidität und Mortalität in den meisten industrialisierten Ländern.

Die aus dem Stand der Technik bekannten Antikoagulantien, d.h. Stoffe zur Hemmung oder Verhinderung der Blutgerinnung, weisen verschiedene, Nachteile auf. Eine effiziente Behandlungsmethode beziehungsweise Prophylaxe von thrombotischen/thromboembolischen Erkrankungen erweist sich in der Praxis deshalb als sehr schwierig und unbefriedigend.

In der Therapie und Prophylaxe von thromboembolischen Erkrankungen findet zum einen Heparin Verwendung, das parenteral oder subkutan appliziert wird. Aufgrund günstigerer pharmakokinetischer Eigenschaften wird zwar heutzutage zunehmend niedermolekulares Heparin bevorzugt; allerdings können auch hierdurch die im Folgenden geschilderten bekannten Nachteile nicht vermieden werden, die bei der Therapierung mit Heparin bestehen. So ist Heparin oral unwirksam und besitzt nur eine vergleichsweise geringe Halbwertszeit. Darüber hinaus besteht ein hohes Blutungsrisiko, insbesondere können Hirnblutungen und Blutungen im Gastrointestinaltrakt auftreten, und es kann zu Thrombopenie, Alopecia medicomentosa oder Osteoporose kommen. Niedermolekulare Heparine besitzen zwar eine geringere Wahrscheinlichkeit zur Ausbildung einer Heparin-induzierten Thrombocytopenie, sind aber auch nur subkutan applizierbar. Dies gilt auch für Fondaparinux, einem synthetisch hergestellten, selektiven Faktor Xa Inhibitor mit einer langen Halbwertszeit.

Eine zweite Klasse von Antikoagulantien stellen die Vitamin K-Antagonisten dar. Hierzu gehören beispielsweise 1,3-Indandione, vor allem aber Verbindungen wie Warfarin, Phenprocoumon, Dicumarol und andere Cumarin-Derivate, die unselektiv die Synthese verschiedener Produkte bestimmter Vitamin K-abhängiger Gerinnungsfaktoren in der Leber hemmen. Durch den Wirkmechanismus bedingt, setzt die Wirkung nur sehr langsam ein (Latenzzeit bis zum Wirkeintritt 36 bis 48 Stunden). Die Verbindungen können zwar oral appliziert werden, aufgrund des hohen Blutungsrisikos und des engen therapeutischen Indexes ist aber eine aufwendige individuelle Einstellung und Beobachtung des Patienten notwendig. Darüber hinaus sind weitere Nebenwirkungen wie gastrointestinale Störungen, Haarausfall und Hautnekrosen beschrieben.

Neuere Ansätze für orale Antikoagulantien befinden sich in verschiedenen Phasen der klinischen Erprobung beziehungsweise im klinischen Einsatz und haben ihre Wirksamkeit in verschiedenen Studien unter Beweis gestellt. Allerdings kann es auch unter der Einnahme dieser Arzneimittel insbesondere bei prädisponierten Patienten zu Blutungskomplikationen kommen. Daher ist bei antithrombotischen Arzneimitteln ist die therapeutische Breite von zentraler Bedeutung: Der Abstand zwischen der therapeutisch wirksamen Dosis zur Gerinnungshemmung und der Dosis, bei der Blutungen auftreten können, sollte möglichst groß sein, so dass eine maximale therapeutische Wirksamkeit bei minimalem Risikoprofil erreicht wird.

In verschiedenen in-vitro und in-vivo Modellen mit beispielsweise Antikörpern als Faktor XIa Inhibitoren, aber auch in Faktor XIa-Knock-out-Modellen, wurde der anti-thrombotische Effekt bei geringer/keiner Verlängerung der Blutungszeit oder Vergrößerung des Blutvolumens belegt. In klinischen Studien waren erhöhte Faktor XIa-Spiegel mit einer gesteigerten Ereignisrate assoziiert. Dagegen führte Faktor XI-Defizienz (Hämophilie C) nicht zu spontanen Blutungen und fiel nur im Rahmen von Operationen und Traumen auf, zeigte aber eine Protektion gegenüber bestimmten thromboembolischen Ereignissen.

Daneben ist Plasmakallikrein (PK) mit weiteren Erkrankungen assoziiert, die mit erhöhten Gefäßpermeabilitäten oder chronisch-entzündlichen Erkrankungen einhergehen, wie es z.B. bei der diabetischen Retinopathie, dem Makulaödem und dem hereditären Angioödem beziehungsweise chronisch-entzündlichen Darmerkrankungen der Fall ist. Der diabetischen Retinopathie liegt in erster Linie eine Mikrogefäßschwäche zu Grunde, in deren Folge es zu einer Basalmembranverdickung der Gefäße und zum Verlust von gefäß-ummantelnden Perizyten, später zum Gefäßverschluss mit retinaler Ischämie kommt, welche aufgrund der hervorgerufenen retinale Hypoxie zu verstärkter Gefäßpermeabilität mit nachfolgender Ausbildung eines Makulaödems und aufgrund aller vorliegender Prozesse zur Erblindung des Patienten führen kann. Beim Hereditären Angioödem (HAE) kommt es durch verminderte Bildung des physiologischen Kallikrein-Inhibitors C1-Esterase-Inhibitors zur unkontrollierten Plasmakallikrein-Aktivierung zu Entzündungen mit fulminanter Ödembildung und starken Schmerzen. Aus tierexperimentellen Ansätzen gibt es Hinweise, dass die Inhibition von Plasmakallikrein die erhöhte Gefäßpermeabilität inhibiert und somit die Ausbildung eines Makulaödems beziehungsweise der diabetischen Retinopathie verhindern beziehungsweise die akute Symptomatik des HAE verbessern kann. Orale Plasmakallikrein-Inhibitoren könnten ebenfalls zur Prophylaxe des HAE eingesetzt werden.

Bei der Progression von chronisch-entzündlichen Darmerkrankungen (CED) haben vor allem die mittels Plasmakallikrein generierten Kinine eine tragende Rolle. Deren pro-inflammatorische Wirkung über Aktivierung von Bradykinin-Rezeptoren induziert und potenziert den Krankheitsverlauf. Studien an Morbus Crohn-Patienten zeigen eine Korrelation zwischen der Kallikrein-Konzentration im Darmepithel und dem Grad der Darmentzündung. Eine Aktivierung des Kallikrein-Kinin-Systems wurde ebenfalls in tierexperimentellen Studien beobachtet. Eine Hemmung der Bradykinin-Synthese durch Kallikrein-Inhibitoren könnte demnach auch zur Prophylaxe und/oder Therapie von chronisch-entzündlichen Darmerkrankungen eingesetzt werden.

Weiterhin kann auch die Kombination von antithrombotischen und antiinflammtorischen Prinzipien für viele Erkrankungen besonders attraktiv sein, um die wechselseitige Verstärkung von Koagulation und Inflammation zu unterbinden.

Eine Aufgabe der vorliegenden Erfindung ist daher die Bereitstellung neuer Verbindungen zur Behandlung von Herz-Kreislauf-Erkrankungen, insbesondere von thrombotischen beziehungsweise thromboembolischen Erkrankungen, und/oder ödematösen Erkrankungen, und/oder ophthalmologischen Erkrankungen, insbesondere von diabetischer Retinopathie beziehungsweise des Makulaödems, bei Menschen und Tieren, die eine große therapeutische Bandbreite aufweisen. WO 2006/030032 beschreibt unter anderem substituierte Pyridinone als allosterische Modulatoren des mGluR2 Rezeptors und WO 2008/079787 beschreibt substituierte Pyridin-2-one und ihre Verwendung als Glucokinase Aktivatoren.

WO 2002/42273 beschreibt Hemmer des Faktors XIa. Gegenstand der Erfindung sind Verbindungen der Formel 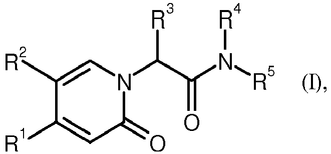 in welcher
- R¹: für eine Gruppe der Formel 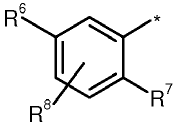 steht,
wobei * die Anknüpfstelle an den Oxopyridinring ist,
R⁶ für Brom, Chlor, Fluor, Methyl, Difluormethyl, Trifluormethyl, Methoxy, Difluormethoxy oder Trifluormethoxy steht,
R⁷ für Brom, Chlor, Fluor, Cyano, Nitro, Hydroxy, Methyl, Difluormethyl, Trifluormethyl, Methoxy, Ethoxy, Difluormethoxy, Trifluormethoxy, Ethinyl, 3,3,3-Trifluorprop-1-in-1-yl oder Cyclopropyl steht,
R⁸ für Wasserstoff, Chlor oder Fluor steht,
- R²: für Wasserstoff, Brom, Chlor, Fluor, Cyano, C₁-C₃-Alkyl, Difluormethyl, Trifluormethyl, 1,1-Difluorethyl, 2,2-Difluorethyl, 2,2,2-Trifluorethyl, C₁-C₃-Alkoxy, Difluormethoxy, Trifluormethoxy, 1,1-Difluorethoxy, 2,2-Difluorethoxy, 2,2,2-Trifluorethoxy, Hydroxycarbonyl, Methylcarbonyl oder Cyclopropyl steht,
- R³: für Wasserstoff, C₁-C₅-Alkyl, C₁-C₄-Alkoxy, Difluormethyl, Trifluormethyl, 1,1-Difluorethyl, 3,3,3-Trifluor-2-hydroxyprop-1-yl, 3,3,3-Trifluor-2-methoxyprop-1-yl, 3,3,3-Trifluor-2-ethoxyprop-1-yl, Prop-2-in-1-yl, Cyclopropyloxy oder Cyclobutyloxy steht,
wobei Alkyl substituiert sein kann mit einem Substituenten ausgewählt aus der Gruppe bestehend aus Fluor, Cyano, Hydroxy, Difluormethyl, Trifluormethyl, Methoxy, Ethoxy, Difluormethoxy, Trifluormethoxy, C₃-C₆-Cycloalkyl, 4- bis 6-gliedriges Oxo-Heterocyclyl, 1,4-Dioxanyl, Oxazolyl, Phenyl und Pyridyl,
worin Cycloalkyl substituiert sein kann mit 1 bis 2 Substituenten unabhängig voneinander ausgewählt aus der Gruppe bestehend aus Fluor, Hydroxy, Methyl, Ethyl, Methoxy, Ethoxy, Difluormethyl, Trifluormethyl, Difluormethoxy und Trifluormethoxy,
- R⁴: für Wasserstoff steht,
- R⁵: für eine Gruppe der Formel 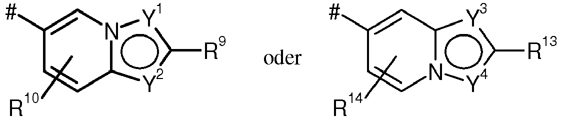 oder 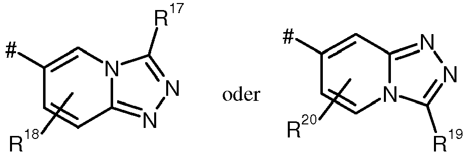 oder 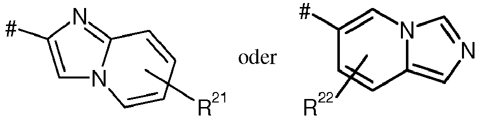 steht,
wobei # die Anknüpfstelle an das Stickstoffatom ist,
Y¹ für ein Stickstoffatom oder C-R¹¹ steht,
worin
R¹¹ für Wasserstoff, Chlor, Hydroxy, Methoxy oder C₁-C₃-Alkoxycarbonyl steht,
Y² für ein Stickstoffatom oder C-R¹² steht,
worin
R¹² für Wasserstoff, Chlor, Hydroxy oder Methoxy steht,
R⁹ für Wasserstoff, Hydroxycarbonyl, Hydroxycarbonylmethyl oder Phenyl steht, worin Phenyl substituiert sein kann mit 1 bis 2 Substituenten Fluor,
R¹⁰ für Wasserstoff, Chlor, Fluor oder Methyl steht,
Y³ für ein Stickstoffatom oder C-R¹⁵ steht,
worin
R¹⁵ für Wasserstoff, Chlor, Hydroxy oder Methoxy steht,
Y⁴ für ein Stickstoffatom oder C-R¹⁶ steht,
worin
R¹⁶ für Wasserstoff, Chlor, Hydroxy oder Methoxy steht,
R¹³ für Wasserstoff, Hydroxycarbonyl, Hydroxycarbonylmethyl, C₁-C₃-Alkoxycarbonyl oder Aminocarbonyl steht,
R¹⁴ für Wasserstoff, Chlor, Fluor oder Methyl steht,
R¹⁷ für Wasserstoff, Chlor, Hydroxy, C₁-C₄-Alkyl, Methoxy, C₁-C₃-Alkylaminomethyl oder Morpholinylmethyl steht,
R¹⁸ für Wasserstoff, Chlor, Fluor oder Methyl steht,
R¹⁹ für Wasserstoff, Chlor, Hydroxy oder Methoxy steht,
R²⁰ für Wasserstoff, Chlor, Fluor oder Methyl steht,
R²¹ für Wasserstoff, Hydroxycarbonyl oder Hydroxycarbonylmethyl steht,
R²² für Wasserstoff, Chlor, Fluor oder Methyl steht,
und ihre Salze, ihre Solvate und die Solvate ihrer Salze.

Erfindungsgemäße Verbindungen sind die Verbindungen der Formel (I) und deren Salze, Solvate und Solvate der Salze, sowie die von Formel (I) umfassten, nachfolgend als Ausführungsbeispiel(e) genannten Verbindungen und deren Salze, Solvate und Solvate der Salze, soweit es sich bei den von Formel (I) umfassten, nachfolgend genannten Verbindungen nicht bereits um Salze, Solvate und Solvate der Salze handelt.

Die erfindungsgemäßen Verbindungen können in Abhängigkeit von ihrer Struktur in unterschiedlichen stereoisomeren Formen existieren, d.h. in Gestalt von Konfigurationsisomeren oder gegebenenfalls auch als Konformationsisomere (Enantiomere und/oder Diastereomere, einschließlich solcher bei Atropisomeren). Die vorliegende Erfindung umfasst deshalb die Enantiomere und Diastereomere und ihre jeweiligen Mischungen. Aus solchen Mischungen von Enantiomeren und/ oder Diastereomeren lassen sich die stereoisomer einheitlichen Bestandteile in bekannter Weise isolieren; vorzugsweise werden hierfür chromatographische Verfahren verwendet, insbesondere die HPLC-Chromatographie an achiraler beziehungsweise chiraler Phase.

Sofern die erfindungsgemäßen Verbindungen in tautomeren Formen vorkommen können, umfasst die vorliegenden Erfindung sämtliche tautomere Formen.

Die vorliegende Erfindung umfasst auch alle geeigneten isotopischen Varianten der erfindungsgemäßen Verbindungen. Unter einer isotopischen Variante einer erfindungsgemäßen Verbindung wird hierbei eine Verbindung verstanden, in welcher mindestens ein Atom innerhalb der erfindungsgemäßen Verbindung gegen ein anderes Atom der gleichen Ordnungszahl, jedoch mit einer anderen Atommasse als der gewöhnlich oder überwiegend in der Natur vorkommenden Atommasse ausgetauscht ist. Beispiele für Isotope, die in eine erfindungsgemäße Verbindung inkorporiert werden können, sind solche von Wasserstoff, Kohlenstoff, Stickstoff, Sauerstoff, Phosphor, Schwefel, Fluor, Chlor, Brom und Iod, wie ²H (Deuterium), ³H (Tritium), ¹³C, ¹⁴C, ¹⁵N, ¹⁷O, ¹⁸O, ³²P, ³³P, ³³S, ³⁴S, ³⁵S, ³⁶S, ¹⁸F, ³⁶Cl, ⁸²Br, ¹²³I, ¹²⁴I, ¹²⁹I und ¹³¹I. Bestimmte isotopische Varianten einer erfindungsgemäßen Verbindung, wie insbesondere solche, bei denen ein oder mehrere radioaktive Isotope inkorporiert sind, können von Nutzen sein beispielsweise für die Untersuchung des Wirkmechanismus oder der Wirkstoff-Verteilung im Körper; aufgrund der vergleichsweise leichten Herstell- und Detektierbarkeit sind hierfür insbesondere mit ³H- oder ¹⁴C-Isotopen markierte Verbindungen geeignet. Darüber hinaus kann der Einbau von Isotopen, wie beispielsweise von Deuterium, zu bestimmten therapeutischen Vorteilen als Folge einer größeren metabolischen Stabilität der Verbindung führen, wie beispielsweise eine Verlängerung der Halbwertszeit im Körper oder eine Reduktion der erforderlichen Wirkdosis; solche Modifikationen der erfindungsgemäßen Verbindungen können daher gegebenenfalls auch eine bevorzugte Ausführungsform der vorliegenden Erfindung darstellen. Isotopische Varianten der erfindungsgemäßen Verbindungen können nach den dem Fachmann bekannten Verfahren hergestellt werden, so beispielsweise nach den weiter unten beschriebenen Methoden und den bei den Ausführungsbeispielen wiedergegebenen Vorschriften, indem entsprechende isotopische Modifikationen der jeweiligen Reagenzien und/oder Ausgangsverbindungen eingesetzt werden.

Als Salze sind im Rahmen der vorliegenden Erfindung physiologisch unbedenkliche Salze der erfindungsgemäßen Verbindungen bevorzugt. Umfasst sind aber auch Salze, die für pharmazeutische Anwendungen selbst nicht geeignet sind aber beispielsweise für die Isolierung oder Reinigung der erfindungsgemäßen Verbindungen verwendet werden können.

Physiologisch unbedenkliche Salze der erfindungsgemäßen Verbindungen umfassen Säureadditionssalze von Mineralsäuren, Carbonsäuren und Sulfonsäuren, z.B. Salze der Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Trifluoressigsäure, Propionsäure, Milchsäure, Weinsäure, Äpfelsäure, Zitronensäure, Fumarsäure, Maleinsäure und Benzoesäure.

Physiologisch unbedenkliche Salze der erfindungsgemäßen Verbindungen umfassen auch Salze üblicher Basen, wie beispielhaft und vorzugsweise Alkalimetallsalze (z.B. Natrium- und Kaliumsalze), Erdalkalisalze (z.B. Calcium- und Magnesiumsalze) und Ammoniumsalze, abgeleitet von Ammoniak oder organischen Aminen mit 1 bis 16 C-Atomen, wie beispielhaft und vorzugsweise Ethylamin, Diethylamin, Triethylamin, Ethyldiisopropylamin, Monoethanolamin, Diethanolamin, Triethanolamin, Dicyclohexylamin, Dimethylaminoethanol, Prokain, Dibenzylamin, *N*-Methylmorpholin, Arginin, Lysin, Ethylendiamin, N-Methylpiperidin und Cholin.

Als Solvate werden im Rahmen der Erfindung solche Formen der erfindungsgemäßen Verbindungen bezeichnet, welche in festem oder flüssigem Zustand durch Koordination mit Lösungsmittelmolekülen einen Komplex bilden. Hydrate sind eine spezielle Form der Solvate, bei denen die Koordination mit Wasser erfolgt.

Außerdem umfasst die vorliegende Erfindung auch Prodrugs der erfindungsgemäßen Verbindungen. Der Begriff "Prodrugs" umfaßt Verbindungen, welche selbst biologisch aktiv oder inaktiv sein können, jedoch während ihrer Verweilzeit im Körper zu erfindungsgemäßen Verbindungen umgesetzt werden (beispielsweise metabolisch oder hydrolytisch).

Folgende zwei Darstellungsweisen (A) und (B) eines 1,4-disubstituierten Cyclohexylderivats sind äquivalent zueinander und gleichbedeutend und in beiden Fällen deskriptiv für ein *trans-*1,4-disubsituiertes Cyclohexylderivat.

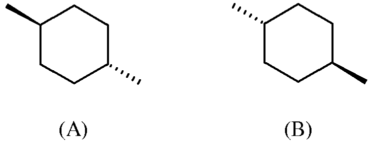

Dies gilt insbesondere für das Strukturelement des (*trans*-4-hydroxycyclohexyl)methyl in 3-(*trans-*4-hydroxycyclohexyl)propanamid.

Im Sinne der vorliegenden Erfindung umfasst der Begriff "Behandlung" oder "behandeln" ein Hemmen, Verzögern, Aufhalten, Lindern, Abschwächen, Einschränken, Verringern, Unterdrücken, Zurückdrängen oder Heilen einer Krankheit, eines Leidens, einer Erkrankung, einer Verletzung oder einer gesundheitlichen Störung, der Entfaltung, des Verlaufs oder des Fortschreitens solcher Zustände und/oder der Symptome solcher Zustände. Der Begriff "Therapie" wird hierbei als synonym mit dem Begriff "Behandlung" verstanden.

Die Begriffe "Prävention", "Prophylaxe" oder "Vorbeugung" werden im Rahmen der vorliegenden Erfindung synonym verwendet und bezeichnen das Vermeiden oder Vermindern des Risikos, eine Krankheit, ein Leiden, eine Erkrankung, eine Verletzung oder eine gesundheitliche Störung, eine Entfaltung oder ein Fortschreiten solcher Zustände und/oder die Symptome solcher Zustände zu bekommen, zu erfahren, zu erleiden oder zu haben.

Die Behandlung oder die Prävention einer Krankheit, eines Leidens, einer Erkrankung, einer Verletzung oder einer gesundheitlichen Störung können teilweise oder vollständig erfolgen.

Im Rahmen der vorliegenden Erfindung haben die Substituenten, soweit nicht anders spezifiziert, die folgende Bedeutung:
Alkyl steht für einen linearen oder verzweigten Alkylrest mit 1 bis 5 Kohlenstoffatomen, bevorzugt 1 bis 3 Kohlenstoffatomen, beispielhaft und vorzugsweise für Methyl, Ethyl, n-Propyl, iso-Propyl, 2-Methyl-prop-1-yl, n-Butyl, *tert.*-Butyl und 2,2-Dimethylprop-1-yl.

Alkoxy steht für einen linearen oder verzweigten Alkoxyrest mit 1 bis 4 Kohlenstoffatomen, bevorzugt 1 bis 3 Kohlenstoffatomen, beispielhaft und vorzugsweise für Methoxy, Ethoxy, n-Propoxy, iso-Propoxy, 2-Methyl-prop-1-oxy, n-Butoxy und *tert*.-Butoxy.

Alkoxycarbonyl steht für einen linearen oder verzweigten Alkoxyrest, der über eine Carbonylgruppe gebunden ist, mit 1 bis 3 Kohlenstoffatomen, bevorzugt 1 bis 2 Kohlenstoffatomen, beispielhaft und vorzugsweise für Methoxycarbonyl, Ethoxycarbonyl, n-Propoxycarbonyl und iso-Propoxycarbonyl.

Alkylaminomethyl steht für eine Amino-Gruppe mit einem oder zwei unabhängig voneinander gewählten gleichen oder verschiedenen geradkettigen oder verzweigten Alkylsubstituenten, die jeweils 1 bis 3 Kohlenstoffatome aufweisen, und die über eine Methylgruppe gebunden ist, beispielhaft und vorzugsweise für Methylaminomethyl, Ethylaminomethyl, n-Propylaminomethyl, iso-Propylaminomethyl, *N*,*N*-Dimethylaminomethyl, *N*,*N*-Diethylaminomethyl, *N*-Ethyl-*N-*methylaminomethyl, *N*-Methyl-*N*-*n*-propylaminomethyl, *N*-iso-Propyl-*N*-n-propylaminomethyl und *N*,*N*-Diisopropylaminomethyl. C₁-C₃-Alkylaminomethyl steht beispielsweise für einen Monoalkylaminomethylrest mit 1 bis 3 Kohlenstoffatomen oder für einen Dialkylaminomethylrest mit jeweils 1 bis 3 Kohlenstoffatomen pro Alkylsubstituent.

Cycloalkyl steht für eine monocyclische Cycloalkylgruppe mit 3 bis 6 Kohlenstoffatomen, beispielhaft und vorzugsweise für Cycloalkyl seien genannt Cyclopropyl, Cyclobutyl, Cyclopentyl und Cyclohexyl.

4- bis 6-gliedriges Oxo-Heterocyclyl in der Definition des Restes R³ steht für einen gesättigten monocyclischen Rest mit 4 bis 6 Ringatomen, in dem ein Ringatom ein Sauerstoffatom ist, beispielhaft und vorzugsweise für Oxetanyl, Tetrahydrofuranyl und Tetrahydro-2H-pyranyl.

In den Formeln der Gruppe, die für R¹ stehen kann, steht der Endpunkt der Linie, neben der jeweils ein * steht, nicht für ein Kohlenstoffatom beziehungsweise eine CH₂-Gruppe sondern ist Bestandteil der Bindung zu dem Atom, an das R¹ gebunden ist.

In den Formeln der Gruppe, die für R⁵ stehen kann, steht der Endpunkt der Linie, neben der jeweils ein # steht, nicht für ein Kohlenstoffatom beziehungsweise eine CH₂-Gruppe sondern ist Bestandteil der Bindung zu dem Atom, an das R⁵ gebunden ist.

Bevorzugt sind Verbindungen der Formel (I), in welcher
in welcher
- R¹: für eine Gruppe der Formel 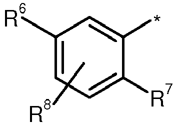 steht,
wobei * die Anknüpfstelle an den Oxopyridinring ist,
R⁶ für Brom, Chlor, Fluor, Methyl, Difluormethyl, Trifluormethyl, Methoxy, Difluormethoxy oder Trifluormethoxy steht,
R⁷ für Brom, Chlor, Fluor, Cyano, Nitro, Hydroxy, Methyl, Difluormethyl, Trifluormethyl, Methoxy, Ethoxy, Difluormethoxy, Trifluormethoxy, Ethinyl, 3,3,3-Trifluorprop-1-in-1-yl oder Cyclopropyl steht,
R⁸ für Wasserstoff, Chlor oder Fluor steht,
- R²: für Wasserstoff, Brom, Chlor, Fluor, Cyano, C₁-C₃-Alkyl, Difluormethyl, Trifluormethyl, 1,1-Difluorethyl, 2,2-Difluorethyl, 2,2,2-Trifluorethyl, C₁-C₃-Alkoxy, Difluormethoxy, Trifluormethoxy, 1,1-Difluorethoxy, 2,2-Difluorethoxy, 2,2,2-Trifluorethoxy, Methylcarbonyl oder Cyclopropyl steht,
- R³: für Wasserstoff, C₁-C₅-Alkyl, C₁-C₄-Alkoxy, Difluormethyl, Trifluormethyl, 1,1-Difluorethyl, 3,3,3-Trifluor-2-hydroxyprop-1-yl, 3,3,3-Trifluor-2-methoxyprop-1-yl, 3,3,3-Trifluor-2-ethoxyprop-1-yl, Prop-2-in-1-yl, Cyclopropyloxy oder Cyclobutyloxy steht,
wobei Alkyl substituiert sein kann mit einem Substituenten ausgewählt aus der Gruppe bestehend aus Fluor, Cyano, Hydroxy, Difluormethyl, Trifluormethyl, Methoxy, Ethoxy, Difluormethoxy, Trifluormethoxy, C₃-C₆-Cycloalkyl, 4- bis 6-gliedriges Oxo-Heterocyclyl, 1,4-Dioxanyl, Phenyl und Pyridyl,
worin Cycloalkyl substituiert sein kann mit 1 bis 2 Substituenten unabhängig voneinander ausgewählt aus der Gruppe bestehend aus Fluor, Hydroxy, Methyl, Ethyl, Methoxy, Ethoxy, Difluormethyl, Trifluormethyl, Difluormethoxy und Trifluormethoxy,
- R⁴: für Wasserstoff steht,
- R⁵: für eine Gruppe der Formel 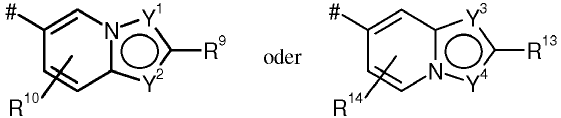 oder 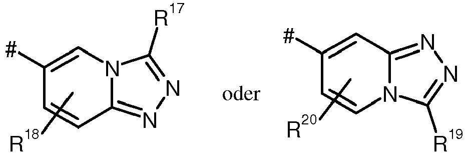 oder 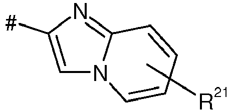 steht,
wobei # die Anknüpfstelle an das Stickstoffatom ist,
Y¹ für ein Stickstoffatom oder C-R¹¹ steht,
worin
R¹¹ für Wasserstoff, Chlor, Hydroxy oder Methoxy steht,
Y² für ein Stickstoffatom oder C-R¹² steht,
worin
R¹² für Wasserstoff, Chlor, Hydroxy oder Methoxy steht,
R⁹ für Wasserstoff, Hydroxycarbonyl oder Hydroxycarbonylmethyl steht,
R¹⁰ für Wasserstoff, Chlor, Fluor oder Methyl steht,
Y³ für ein Stickstoffatom oder C-R¹⁵ steht,
worin
R¹⁵ für Wasserstoff, Chlor, Hydroxy oder Methoxy steht,
Y⁴ für ein Stickstoffatom oder C-R¹⁶ steht,
worin
R¹⁶ für Wasserstoff, Chlor, Hydroxy oder Methoxy steht,
R¹³ für Wasserstoff, Hydroxycarbonyl oder Hydroxycarbonylmethyl steht,
R¹⁴ für Wasserstoff, Chlor, Fluor oder Methyl steht,
R¹⁷ für Wasserstoff, Chlor, Hydroxy oder Methoxy steht,
R¹⁸ für Wasserstoff, Chlor, Fluor oder Methyl steht,
R¹⁹ für Wasserstoff, Chlor, Hydroxy oder Methoxy steht,
R²⁰ für Wasserstoff, Chlor, Fluor oder Methyl steht,
R²¹ für Wasserstoff, Hydroxycarbonyl oder Hydroxycarbonylmethyl steht,
und ihre Salze, ihre Solvate und die Solvate ihrer Salze.

Bevorzugt sind auch Verbindungen der Formel (I), in welcher
- R¹: für eine Gruppe der Formel 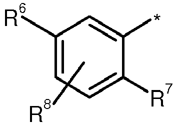 steht,
wobei * die Anknüpfstelle an den Oxopyridinring ist,
R⁶ für Chlor steht,
R⁷ für Cyano, Difluormethyl, Trifluormethyl, Difluormethoxy oder Trifluormethoxy steht,
R⁸ für Wasserstoff steht,
- R²: für Chlor, Cyano, Methoxy, Ethoxy oder Difluormethoxy steht,
- R³: für Wasserstoff, Methyl, Ethyl, n-Propyl, 2-Methyl-prop-1-yl, n-Butyl oder Ethoxy steht, wobei Methyl substituiert sein kann mit einem Substituenten ausgewählt aus der Gruppe bestehend aus Difluormethyl, Trifluormethyl, Cyclopropyl, Cyclobutyl, Cyclohexyl, Oxetanyl, Tetrahydrofuranyl, Tetrahydro-2H-pyranyl und 1,4-Dioxanyl,
worin Cyclopropyl, Cyclobutyl, Cyclohexyl und Oxetanyl substituiert sein können mit 1 bis 2 Substituenten unabhängig voneinander ausgewählt aus der Gruppe bestehend aus Fluor, Hydroxy, Methyl, Ethyl und Methoxy,
und
wobei Ethyl, n-Propyl und n-Butyl substituiert sein können mit einem Substituenten ausgewählt aus der Gruppe bestehend aus Fluor, Methoxy und Trifluormethoxy,
- R⁴: für Wasserstoff steht,
- R⁵: für eine Gruppe der Formel 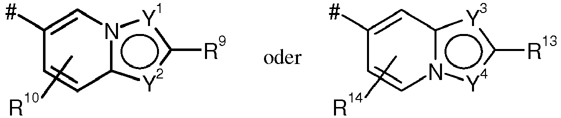 oder 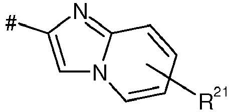 steht,
wobei # die Anknüpfstelle an das Stickstoffatom ist,
Y¹ für ein Stickstoffatom oder C-R¹¹ steht,
worin
R¹¹ für Wasserstoff, Chlor, Hydroxy oder Methoxy steht,
Y² für ein Stickstoffatom oder C-R¹² steht,
worin
R¹² für Wasserstoff, Chlor, Hydroxy oder Methoxy steht,
R⁹ für Wasserstoff oder Hydroxycarbonyl steht,
R¹⁰ für Wasserstoff oder Fluor steht,
Y³ für ein Stickstoffatom oder C-R¹⁵ steht,
worin
R¹⁵ für Wasserstoff, Chlor, Hydroxy oder Methoxy steht,
Y⁴ für ein Stickstoffatom oder C-R¹⁶ steht,
worin
R¹⁶ für Wasserstoff, Chlor, Hydroxy oder Methoxy steht,
R¹³ für Wasserstoff oder Hydroxycarbonyl steht,
R¹⁴ für Wasserstoff oder Fluor steht,
R²¹ für Wasserstoff oder Hydroxycarbonyl steht, und ihre Salze, ihre Solvate und die Solvate ihrer Salze.

Bevorzugt sind auch Verbindungen der Formel (I), in welcher
- R¹: für eine Gruppe der Formel 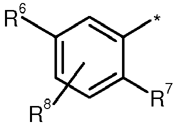 steht,
wobei * die Anknüpfstelle an den Oxopyridinring ist,
R⁶ für Chlor steht,
R⁷ für Cyano oder Difluormethoxy steht,
R⁸ für Wasserstoff steht,
- R²: für Methoxy steht,
- R³: für Wasserstoff, Methyl oder Ethyl steht, wobei Methyl substituiert sein kann mit einem Substituenten Cyclobutyl,
- R⁴: für Wasserstoff steht,
- R⁵: für eine Gruppe der Formel 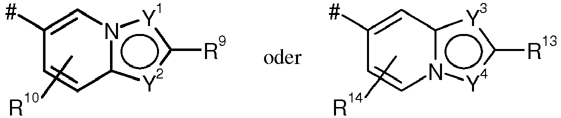 oder 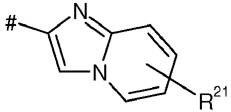 steht,
wobei # die Anknüpfstelle an das Stickstoffatom ist,
Y¹ für ein C-R¹¹ steht,
worin
R¹¹ für Wasserstoff steht,
Y² für ein Stickstoffatom steht,
R⁹ für Wasserstoff oder Hydroxycarbonyl steht,
R¹⁰ für Wasserstoff steht,
Y³ für ein Stickstoffatom steht,
Y⁴ für C-R¹⁶ steht,
worin
R¹⁶ für Wasserstoff steht,
R¹³ für Hydroxycarbonyl steht,
R¹⁴ für Wasserstoff steht,
R²¹ für Hydroxycarbonyl steht,
und ihre Salze, ihre Solvate und die Solvate ihrer Salze.

Bevorzugt sind auch Verbindungen der Formel (I), in welcher
- R¹: für eine Gruppe der Formel 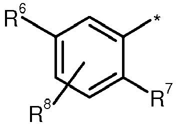 steht,
wobei * die Anknüpfstelle an den Oxopyridinring ist,
R⁶ für Chlor steht,
R⁷ für Cyano oder Difluormethoxy steht,
R⁸ für Wasserstoff steht,
- R²: für Methoxy steht,
- R³: für Methyl oder Ethyl steht,
wobei Methyl substituiert sein kann mit einem Substituenten ausgewählt aus der Gruppe bestehend aus Cyclobutyl und Tetrahydro-2H-pyranyl,
und
wobei Ethyl substituiert sein kann mit einem Substituenten Methoxy,
- R⁴: für Wasserstoff steht,
- R⁵: für eine Gruppe der Formel 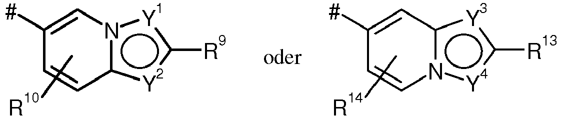
steht,
wobei # die Anknüpfstelle an das Stickstoffatom ist,
- Y¹: für ein C-R¹¹ steht,
worin
R¹¹ für Wasserstoff oder Chlor steht,
- Y²: für ein Stickstoffatom steht,
- R⁹: für Wasserstoff oder Hydroxycarbonyl steht,
- R¹⁰: für Wasserstoff steht,
- Y³: für ein Stickstoffatom steht,
und
- Y⁴: für C-R¹⁶ steht,
worin
R¹⁶ für Wasserstoff steht,
oder
- Y³: für C-R¹⁵ steht,
worin
R¹⁵ für Wasserstoff oder Chlor steht,
und
- Y⁴: für ein Stickstoffatom steht,
- R¹³: für Wasserstoff oder Hydroxycarbonyl steht,
- R¹⁴: für Wasserstoff steht,
und ihre Salze, ihre Solvate und die Solvate ihrer Salze.

Bevorzugt sind auch Verbindungen der Formel (I), in welcher
- R¹: für eine Gruppe der Formel 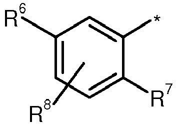 steht,
wobei * die Anknüpfstelle an den Oxopyridinring ist,
R⁶ für Chlor steht,
R⁷ für Cyano oder Difluormethoxy steht,
R⁸ für Wasserstoff steht.

Bevorzugt sind auch Verbindungen der Formel (I), in welcher R² für Chlor, Cyano, Methoxy, Ethoxy oder Difluormethoxy steht.

Bevorzugt sind auch Verbindungen der Formel (I), in welcher R² für Methoxy steht.

Bevorzugt sind auch Verbindungen der Formel (I), in welcher
- R³: für Methyl, Ethyl, n-Propyl, 2-Methyl-prop-1-yl, n-Butyl oder Ethoxy steht,
wobei Methyl substituiert sein kann mit einem Substituenten ausgewählt aus der Gruppe bestehend aus Difluormethyl, Trifluormethyl, Cyclopropyl, Cyclobutyl, Cyclohexyl, Oxetanyl, Tetrahydrofuranyl, Tetrahydro-2H-pyranyl und 1,4-Dioxanyl,
worin Cyclopropyl, Cyclobutyl, Cyclohexyl und Oxetanyl substituiert sein können mit 1 bis 2 Substituenten unabhängig voneinander ausgewählt aus der Gruppe bestehend aus Fluor, Hydroxy, Methyl, Ethyl und Methoxy,
und
wobei Ethyl, n-Propyl und n-Butyl substituiert sein können mit einem Substituenten ausgewählt aus der Gruppe bestehend aus Fluor, Methoxy und Trifluormethoxy.

Bevorzugt sind auch Verbindungen der Formel (I), in welcher
- R³: für Methyl oder Ethyl steht,
wobei Methyl substituiert sein kann mit einem Substituenten ausgewählt aus der Gruppe bestehend aus Cyclobutyl und Tetrahydro-2H-pyranyl,
und
wobei Ethyl substituiert sein kann mit einem Substituenten Methoxy.

Bevorzugt sind auch Verbindungen der Formel (I), in welcher
- R³: für Wasserstoff, Methyl oder Ethyl steht, wobei Methyl substituiert sein kann mit einem Substituenten Cyclobutyl.

Bevorzugt sind auch Verbindungen der Formel (I), in welcher
- R³: für Methyl oder Ethyl steht, wobei Methyl substituiert sein kann mit einem Substituenten Cyclobutyl.

Bevorzugt sind auch Verbindungen der Formel (I), in welcher
- R⁵: für eine Gruppe der Formel 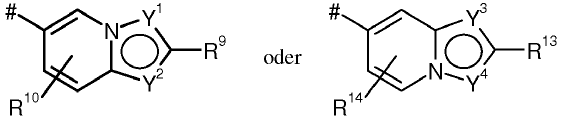 oder 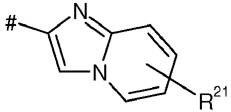 steht,
wobei # die Anknüpfstelle an das Stickstoffatom ist,
Y¹ für ein C-R¹¹ steht,
worin
R¹¹ für Wasserstoff steht,
Y² für ein Stickstoffatom steht,
R⁹ für Wasserstoff oder Hydroxycarbonyl steht,
R¹⁰ für Wasserstoff steht,
Y³ für ein Stickstoffatom steht,
Y⁴ für C-R¹⁶ steht,
worin
R¹⁶ für Wasserstoff steht,
R¹³ für Hydroxycarbonyl steht,
R¹⁴ für Wasserstoff steht,
R²¹ für Hydroxycarbonyl steht.

Bevorzugt sind auch Verbindungen der Formel (I), in welcher
- R⁵: für eine Gruppe der Formel 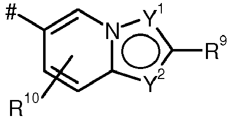 steht,
wobei # die Anknüpfstelle an das Stickstoffatom ist,
Y¹ für ein C-R¹¹ steht,
worin
R¹¹ für Wasserstoff steht,
Y² für ein Stickstoffatom steht,
R⁹ für Wasserstoff oder Hydroxycarbonyl steht,
R¹⁰ für Wasserstoff steht.

Bevorzugt sind auch Verbindungen der Formel (I), in welcher
- R⁵: für eine Gruppe der Formel 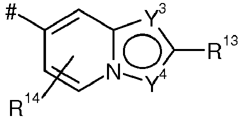 steht,
wobei # die Anknüpfstelle an das Stickstoffatom ist,
Y³ für ein Stickstoffatom steht, und
Y⁴ für C-R¹⁶ steht,
worin
R¹⁶ für Wasserstoff steht, oder
Y³ für C-R¹⁵ steht,
worin
R¹⁵ für Wasserstoff oder Chlor steht, und
Y⁴ für ein Stickstoffatom steht,
R¹³ für Wasserstoff oder Hydroxycarbonyl steht,
R¹⁴ für Wasserstoff steht.

Bevorzugt sind auch Verbindungen der Formel (I), in welcher
- R⁵: für eine Gruppe der Formel 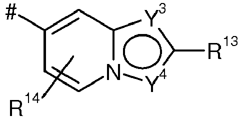 steht, wobei # die Anknüpfstelle an das Stickstoffatom ist,
Y³ für C-R¹⁵ steht,
worin
R¹⁵ für Wasserstoff steht,
Y⁴ für ein Stickstoffatom steht,
R¹³ für Wasserstoff steht,
R¹⁴ für Wasserstoff steht.

Bevorzugt sind auch Verbindungen der Formel (I), in welcher R¹³ für Wasserstoff oder Hydroxycarbonyl steht.

Bevorzugt sind auch Verbindungen, welche die Formel (Ia) aufweisen 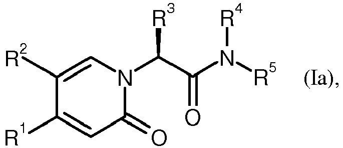 worin R¹, R², R³, R⁴ und R⁵ wie oben definiert sind.

Gegenstand der Erfindung ist weiterhin ein Verfahren zur Herstellung der Verbindungen der Formel (I), oder ihrer Salze, ihrer Solvate oder der Solvate ihrer Salze, wobei
[A] die Verbindungen der Formel 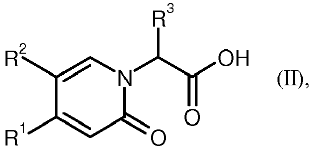 in welcher
   R¹, R² und R³ die oben angegebene Bedeutung haben, in der ersten Stufe mit Verbindungen der Formel 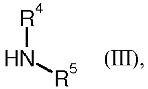 in welcher
   R⁴ und R⁵ die oben angegebene Bedeutung haben,
   in Gegenwart eines Dehydratisierungsreagenzes umgesetzt werden, und
   gegebenfalls in einer zweiten Stufe durch saure oder basische Esterspaltung zu Verbindungen der Formel (I) umgesetzt werden,
   oder
[B] die Verbindungen der Formel 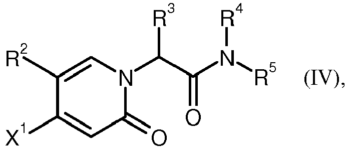 in welcher
   R², R³, R⁴ und R⁵ die oben angegebene Bedeutung haben, und
   X¹ für Chlor, Brom oder Iod steht,
   mit Verbindungen der Formel 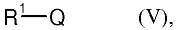 in welcher
   R¹ die oben angegebene Bedeutung hat, und
   Q für -B(OH)₂, einen Boronsäure-Ester, bevorzugt Boronsäurepinakolester, oder -BF₃⁻K⁺ steht,
   unter Suzuki-Kupplungsbedingungen zu Verbindungen der Formel (I) umgesetzt werden.

Die Umsetzung der ersten Stufe nach Verfahren [A] erfolgt im Allgemeinen in inerten Lösungsmitteln, gegebenenfalls in Gegenwart einer Base, bevorzugt in einem Temperaturbereich von 0°C bis Raumtemperatur bei Normaldruck.

Als Dehydratisierungsreagenzien eignen sich hierbei beispielsweise Carbodiimide wie z.B. *N,N'-*Diethyl-, *N*,*N'*-Dipropyl-, *N*,*N'*-Diisopropyl-, *N*,*N'*-Dicyclohexylcarbodiimid, *N*-(3-Dimethylamino-isopropyl)-*N'*-ethylcarbodiimid-Hydrochlorid (EDC) (gegebenenfalls in Gegenwart von Pentafluorphenol (PFP)), *N*-Cyclohexylcarbodiimid-*N'*-propyloxymethyl-Polystyrol (PS-Carbodiimid) oder Carbonylverbindungen wie Carbonyldiimidazol, oder 1,2-Oxazoliumverbindungen wie 2-Ethyl-5-phenyl-1,2-oxazolium-3-sulfat oder 2-tert.-Butyl-5-methyl-isoxazolium-perchlorat, oder Acylaminoverbindungen wie 2-Ethoxy-1-ethoxycarbonyl-1,2-dihydrochinolin, oder Propanphosphonsäureanhydrid, oder Isobutylchloroformat, oder Bis-(2-oxo-3-oxazolidinyl)-phosphorylchlorid oder Benzotriazolyloxy-tri(dimethylamino)phosphoniumhexafluorophosphat, oder *O*-(Benzotriazol-1-yl)-*N*,*N*,*N',N'*-tetra-methyluronium-hexafluorophosphat (HBTU), 2-(2-Oxo-1-(2H)-pyridyl)-1,1,3,3-tetramethyluroniumtetrafluoroborat (TPTU), (Benzotriazol-1-yloxy)bisdimethylamino-methyliumfluoroborat (TBTU) oder *O*-(7-Azabenzotriazol-1-yl)-*N*,*N*,*N'*,*N'*-tetramethyl-uronium-hexafluorophosphat (HATU), oder 1-Hydroxybenztriazol (HOBt), oder Benzotriazol-1-yloxy-tris(dimethylamino)-phosphoniumhexafluorophosphat (BOP), oder Ethyl-cyan(hydroxy-imino)acetat (Oxyma), oder (1-Cyano-2-ethoxy-2-oxoethylidenaminooxy)dimethylamino-morpholino-carbenium hexafluorophosphat (COMU), oder N-[(Dimethylamino)(3*H-*[1,2,3]triazolo[4,5-b]pyridin-3-yloxy)methyliden]-N-methylmethanaminium-hexafluorophosphat, oder 2,4,6-Tripropyl-1,3,5,2,4,6-trioxatriphosphinan-2,4,6-trioxid (T3P), oder Mischungen aus diesen, mit Basen. Vorzugsweise wird die Kondensation mit HATU durchgeführt.

Basen sind beispielsweise Alkalicarbonate, wie z.B. Natrium- oder Kaliumcarbonat, oder -hydrogencarbonat, oder organische Basen wie Trialkylamine, z.B. Triethylamin, *N*-Methylmorpholin, *N*-Methylpiperidin, 4-Dimethylaminopyridin oder Diisopropylethylamin, oder Pyridin. Vorzugsweise wird die Kondensation mit Diisopropylethylamin durchgeführt.

Inerte Lösungsmittel sind beispielsweise Halogenkohlenwasserstoffe wie Dichlormethan oder Trichlormethan, Kohlenwasserstoffe wie Benzol, oder andere Lösungsmittel wie Nitromethan, Dioxan, Dimethylformamid, Dimethylsulfoxid oder Acetonitril. Ebenso ist es möglich, Gemische der Lösemittel einzusetzen. Besonders bevorzugt ist Dimethylformamid.

Die Verbindungen der Formel (III) sind bekannt, lassen sich nach bekannten Verfahren aus den entsprechenden Ausgangsverbindungen synthetisieren oder können analog den im Beispielteil beschriebenen Verfahren hergestellt werden.

Die Umsetzung der zweiten Stufe nach Verfahren [A] erfolgt bei einer sauren Esterspaltung im Allgemeinen in inerten Lösungsmitteln, bevorzugt in einem Temperaturbereich von Raumtemperatur bis 60°C bei Normaldruck.

Inerte Lösungsmittel sind beispielsweise Halogenkohlenwasserstoffe wie Dichlormethan, Trichlormethan, Tetrachlormethan oder 1,2-Dichlorethan, oder Ether wie Tetrahydrofuran oder Dioxan, bevorzugt ist Dichlormethan.

Säuren sind beispielsweise Trifluoressigsäure oder Chlorwasserstoff in Dioxan, bevorzugt ist Trifluoressigsäure.

Die Umsetzung der zweiten Stufe nach Verfahren [A] erfolgt bei einer basischen Esterspaltung im Allgemeinen in inerten Lösungsmitteln, bevorzugt in einem Temperaturbereich von Raumtemperatur bis zum Rückfluss der Lösungsmittel bei Normaldruck.

Inerte Lösungsmittel sind beispielsweise Halogenkohlenwasserstoffe wie Dichlormethan, Trichlormethan, Tetrachlormethan oder 1,2-Dichlorethan, Alkohole wie Methanol oder Ethanol, Ether wie Diethylether, Methyl-tert-butylether, 1,2-Dimethoxyethan, Dioxan oder Tetrahydrofuran, oder andere Lösungsmittel wie Dimethylformamid, Dimethylacetamid, Acetonitril oder Pyridin, oder Gemische von Lösungsmitteln, oder Gemische von Lösungsmittel mit Wasser, bevorzugt ist ein Gemisch aus Tetrahydrofuran und Wasser.

Basen sind beispielsweise Alkalihydroxide wie Natrium-, Lithium- oder Kaliumhydroxid, oder Alkalicarbonate wie Cäsiumcarbonat, Natrium- oder Kaliumcarbonat, oder Alkoholate wie Kalium- oder Natrium-tert-butylat, bevorzugt ist Lithiumhydroxid.

Die Umsetzung nach Verfahren [B] erfolgt im Allgemeinen in inerten Lösungsmitteln, in Gegenwart eines Katalysators, gegebenenfalls in Gegenwart eines Zusatzreagenzes, gegebenenfalls in einer Mikrowelle, bevorzugt in einem Temperaturbereich von Raumtemperatur bis 150°C bei Normaldruck bis 3 bar.

Katalysatoren sind beispielsweise für Suzuki-Reaktionsbedingungen übliche Palladium-Katalysatoren, bevorzugt sind Katalysatoren wie z.B. Dichlorbis(triphenylphosphin)-palladium, Tetrakistriphenylphosphinpalladium(0), Palladium(II)acetat/Triscyclohexylphosphin, Tris(dibenzylidenaceton)dipalladium, Bis-(diphenylphosphanferrocenyl)-palladium-(II)-chlorid, 1,3-Bis(2,6-diisopropylphenyl)imidazol-2-yliden(1,4-napthtochinon)palladiumdimer, Allyl(chlor)-(1,3-dimesityl-1,3-dihydro-2H-imidazol-2-yliden)palladium, Palladium(II)acetat/Dicyclohexyl-(2',4',6'-triisopropyl-biphenyl-2-yl)-phosphin, [1,1-Bis-(diphenylphosphino)-ferrocen]-palladium(II)chlorid-Monodichlormethan-addukt oder XPhos Präkatalysator [(2'-Aminobiphenyl-2-yl)(chlor)palladium-Dicyclohexyl(2',4',6'-triisopropylbiphenyl-2-yl)phosphan (1:1)], bevorzugt ist Tetrakistriphenylphosphin-palladium(0), [1,1-Bis-(diphenylphosphino)-ferrocen]-palladium(II)chlorid-Monodichlormethan-addukt oder XPhos Präkatalysator [(2'-Aminobiphenyl-2-yl)(chlor)palladium-Dicyclohexyl(2',4',6'-triisopropylbiphenyl-2-yl)phosphan (1:1)].

Zusatzreagenzien sind beispielsweise Kaliumacetat, Cäsium-, Kalium- oder Natriumcarbonat, Kalium-tert.-butylat, Cäsiumfluorid oder Kaliumphosphat, wobei diese in wässriger Lösung vorliegen können, bevorzugt sind Zusatzreagenzien wie Kaliumcarbonat oder wässrige Kaliumphosphat-Lösung.

Inerte Lösungsmittel sind beispielsweise Ether wie Dioxan, Tetrahydrofuran oder 1,2-Dimethoxyethan, Kohlenwasserstoffe wie Benzol, Xylol oder Toluol, oder Carbonsäureamide wie Dimethylformamid oder Dimethylacetamid, Alkylsulfoxide wie Dimethylsulfoxid, oder N-Methylpyrrolidon oder Acetonitril, oder Gemische der Lösungsmittel mit Alkoholen wie Methanol oder Ethanol und/oder Wasser, bevorzugt ist Tetrahydrofuran, Dioxan oder Acetonitril.

Die Verbindungen der Formel (V) sind bekannt oder lassen sich nach bekannten Verfahren aus den entsprechenden Ausgangsverbindungen synthetisieren.

Die Verbindungen der Formel (II) sind bekannt oder können hergestellt werden, indem
[C] Verbindungen der Formel 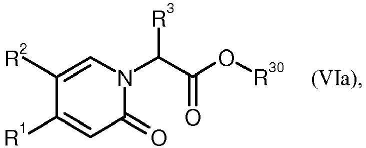 in welcher
   R¹, R² und R³ die oben angegebene Bedeutung haben, und
   R³⁰ für tert-Butyl steht,
   mit einer Säure umgesetzt werden,
   oder
[D] Verbindungen der Formel 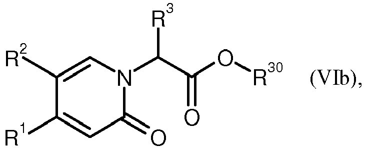 in welcher
   R¹, R² und R³ die oben angegebene Bedeutung haben, und
   R³⁰ für Methyl oder Ethyl steht,
   mit einer Base umgesetzt werden.

Die Verbindungen der Formeln (VIa) und (VIb) bilden zusammen die Menge der Verbindungen der Formel (VI).

Die Umsetzung nach Verfahren [C] erfolgt im Allgemeinen in inerten Lösungsmitteln, bevorzugt in einem Temperaturbereich von Raumtemperatur bis 60°C bei Normaldruck.

Inerte Lösungsmittel sind beispielsweise Halogenkohlenwasserstoffe wie Dichlormethan, Trichlormethan, Tetrachlormethan oder 1,2-Dichlorethan, oder Ether wie Tetrahydrofuran oder Dioxan, bevorzugt ist Dichlormethan.

Säuren sind beispielsweise Trifluoressigsäure oder Chlorwasserstoff in Dioxan, bevorzugt ist Trifluoressigsäure.

Die Umsetzung nach Verfahren [D] erfolgt im Allgemeinen in inerten Lösungsmitteln, bevorzugt in einem Temperaturbereich von Raumtemperatur bis zum Rückfluss der Lösungsmittel bei Normaldruck.

Inerte Lösungsmittel sind beispielsweise Halogenkohlenwasserstoffe wie Dichlormethan, Trichlormethan, Tetrachlormethan oder 1,2-Dichlorethan, Alkohole wie Methanol oder Ethanol, Ether wie Diethylether, Methyl-tert-butylether, 1,2-Dimethoxyethan, Dioxan oder Tetrahydrofuran, oder andere Lösungsmittel wie Dimethylformamid, Dimethylacetamid, Acetonitril oder Pyridin, oder Gemische von Lösungsmitteln, oder Gemische von Lösungsmittel mit Wasser, bevorzugt ist ein Gemisch aus Tetrahydrofuran und Wasser.

Basen sind beispielsweise Alkalihydroxide wie Natrium-, Lithium- oder Kaliumhydroxid, oder Alkalicarbonate wie Cäsiumcarbonat, Natrium- oder Kaliumcarbonat, oder Alkoholate wie Kalium- oder Natrium-tert-butylat, bevorzugt ist Lithiumhydroxid.

Die Verbindungen der Formel (VI) sind bekannt oder können hergestellt werden, indem
[E] Verbindungen der Formel 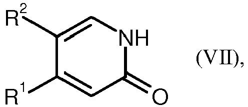 in welcher
   R¹ und R² die oben angegebene Bedeutung haben,
   mit Verbindungen der Formel 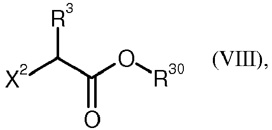 in welcher
   R³ die oben angegebene Bedeutung hat,
   R³⁰ für Methyl, Ethyl oder tert-Butyl steht, und
   X² für Chlor, Brom, Iod, Methansulfonyloxy oder Trifluormethansulfonyloxy steht, umgesetzt werden,
   oder
[F] Verbindungen der Formel 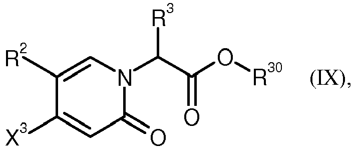 in welcher
   R² und R³ die oben angegebene Bedeutung haben,
   R³⁰ für Methyl, Ethyl oder tert-Butyl steht, und
   X³ für Chlor, Brom oder Iod steht,
   mit Verbindungen der Formel (V) unter Suzuki-Kupplungsbedingungen umgesetzt werden.

Die Umsetzung nach Verfahren [E] erfolgt im Allgemeinen in inerten Lösungsmitteln, gegebenenfalls in Gegenwart einer Base, bevorzugt in einem Temperaturbereich von Raumtemperatur bis zum Rückfluss der Lösungsmittel bei Normaldruck.

Inerte Lösungsmittel sind beispielsweise Halogenkohlenwasserstoffe wie Dichlormethan, Trichlormethan, Tetrachlormethan oder 1,2-Dichlorethan, Alkohole wie Methanol oder Ethanol, Ether wie Diethylether, Methyl-tert-butylether, 1,2-Dimethoxyethan, Dioxan oder Tetrahydrofuran, oder andere Lösungsmittel wie Dimethylformamid, Dimethylacetamid, Acetonitril oder Pyridin, oder Gemische von Lösungsmitteln, oder Gemische von Lösungsmittel mit Wasser, bevorzugt ist Dimethylformamid.

Basen sind beispielsweise Alkalihydroxide wie Natrium-, Lithium- oder Kaliumhydroxid, oder Alkalicarbonate wie Cäsiumcarbonat, Natrium- oder Kaliumcarbonat, oder Kalium- oder Natrium-tert-butylat, Natriumhydrid oder eine Mischung aus diesen Basen oder eine Mischung aus Natriumhydrid und Lithiumbromid, bevorzugt ist Kaliumcarbonat oder Natriumhydrid.

Die Verbindungen der Formel (VIII) sind bekannt oder lassen sich nach bekannten Verfahren aus den entsprechenden Ausgangsverbindungen synthetisieren.

Die Umsetzung nach Verfahren [F] erfolgt wie für Verfahren [B] beschrieben.

Die Verbindungen der Formel (VII) sind bekannt oder können hergestellt werden, indem Verbindungen der Formel 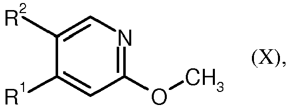 in welcher
R¹ und R² die oben angegebene Bedeutung haben,
mit Pyridinium-Hydrochlorid oder Pyridinium-Hydrobromid umgesetzt werden.

Die Umsetzung erfolgt im Allgemeinen in inerten Lösungsmitteln, bevorzugt in einem Temperaturbereich von 80°C bis 120°C bei Normaldruck.

Inerte Lösungsmittel sind beispielsweise Kohlenwasserstoffe wie Benzol, oder andere Lösungsmittel wie Nitromethan, Dioxan, Dimethylformamid, Dimethylsulfoxid oder Acetonitril. Ebenso ist es möglich, Gemische der Lösemittel einzusetzen. Besonders bevorzugt ist Dimethylformamid.

Die Verbindungen der Formel (X) sind bekannt oder können hergestellt werden, indem Verbindungen der Formel 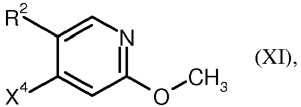 in welcher
R² die oben angegebene Bedeutung hat, und
X⁴ für Chlor, Brom oder Iod steht,
mit Verbindungen der Formel (V) unter Suzuki-Kupplungsbedingungen umgesetzt werden.

Die Umsetzung erfolgt wie für Verfahren [B] beschrieben.

Die Verbindungen der Formel (XI) sind bekannt oder lassen sich nach bekannten Verfahren aus den entsprechenden Ausgangsverbindungen synthetisieren.

Die Verbindungen der Formel (IX) sind bekannt oder können hergestellt werden, indem Verbindungen der Formel 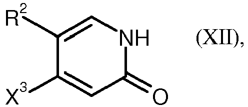 in welcher
R² die oben angegebene Bedeutung hat, und
X³ für Chlor, Brom oder Iod steht,
mit Verbindungen der Formel (VIII) umgesetzt werden.

Die Umsetzung erfolgt wie für Verfahren [E] beschrieben.

Die Verbindungen der Formel (XII) sind bekannt oder lassen sich nach bekannten Verfahren aus den entsprechenden Ausgangsverbindungen synthetisieren.

Die Verbindungen der Formel (IV) sind bekannt oder können hergestellt werden, indem Verbindungen der Formel 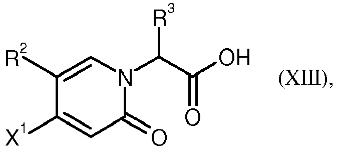 in welcher
R² und R³ die oben angegebene Bedeutung haben, und
X¹ für Chlor, Brom oder Iod steht,
mit Verbindungen der Formel (III) in Gegenwart eines Dehydratisierungsreagenzes umgesetzt werden.

Die Umsetzung erfolgt wie für Verfahren [A] beschrieben.

Die Verbindungen der Formel (XIII) sind bekannt oder können hergestellt werden, indem
[G] Verbindungen der Formel 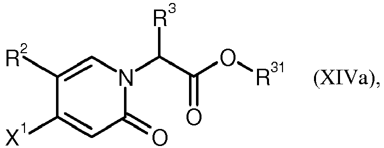 in welcher
   R² und R³ die oben angegebene Bedeutung haben,
   R³¹ für tert-Butyl steht, und
   X¹ für Chlor, Brom oder Iod steht,
   mit einer Säure umgesetzt werden,
   oder
[H] Verbindungen der Formel 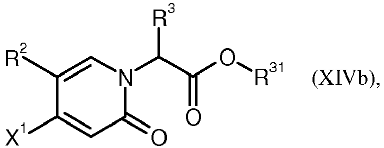 in welcher
   R² und R³ die oben angegebene Bedeutung haben,
   R³¹ für Methyl oder Ethyl steht, und
   X¹ für Chlor, Brom oder Iod steht,
   mit einer Base umgesetzt werden.

Die Verbindungen der Formeln (XIVa) und (XIVb) bilden zusammen die Menge der Verbindungen der Formel (XIV).

Die Umsetzung nach Verfahren [G] erfolgt wie für Verfahren [C] beschrieben.

Die Umsetzung nach Verfahren [H] erfolgt wie für Verfahren [D] beschrieben.

Die Verbindungen der Formel (XIV) sind bekannt oder können hergestellt werden, indem Verbindungen der Formel 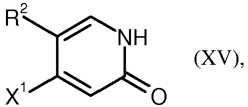 in welcher
R² die oben angegebene Bedeutung habt, und
X¹ für Chlor, Brom oder Iod steht,
mit Verbindungen der Formel 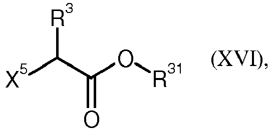 in welcher
R³ die oben angegebene Bedeutung hat,
R³¹ für Methyl, Ethyl oder tert-Butyl steht, und
X⁵ für Chlor, Brom, Iod, Methansulfonyloxy oder Trifluormethansulfonyloxy steht,
umgesetzt werden.

Die Umsetzung erfolgt wie für Verfahren [E] beschrieben.

Die Verbindungen der Formel (XV) und (XVI) sind bekannt oder lassen sich nach bekannten Verfahren aus den entsprechenden Ausgangsverbindungen synthetisieren.

In einem alternativen Verfahren können die Verbindungen der Formel (VI) hergestellt werden, indem Verbindungen der Formel 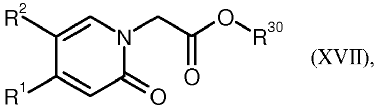 in welcher
R¹ und R² die oben angegebene Bedeutung haben, und
R³⁰ für Methyl, Ethyl oder tert-Butyl steht,
mit Verbindungen der Formel

R³-X⁶ (XVIII),

in welcher
R³ die oben angegebene Bedeutung hat, und
X⁶ für Chlor, Brom, Iod, Methansulfonyloxy, Trifluormethansulfonyloxy oder para-Toluolsulfonyloxy steht,
umgesetzt werden.

Die Umsetzung erfolgt im Allgemeinen in inerten Lösungsmitteln, gegebenenfalls in Gegenwart einer Base, bevorzugt in einem Temperaturbereich von -78°C bis Raumtemperatur bei Normaldruck.

Inerte Lösungsmittel sind beispielsweise Halogenkohlenwasserstoffe wie Dichlormethan, Trichlormethan, Tetrachlormethan oder 1,2-Dichlorethan, Alkohole wie Methanol oder Ethanol, Ether wie Diethylether, Methyl-tert-butylether, 1,2-Dimethoxyethan, Dioxan oder Tetrahydrofuran, oder andere Lösungsmittel wie Dimethylformamid, Dimethylacetamid, Acetonitril oder Pyridin, oder Gemische von Lösungsmitteln, oder Gemische von Lösungsmittel mit Wasser, bevorzugt ist Tetrahydrofuran.

Basen sind beispielsweise Kalium- oder Natrium-tert-butylat, Natriumhydrid, N-Butyllithium oder Bis-(trimethylsilyl)-lithiumamid, bevorzugt ist Bis-(trimethylsilyl)-lithiumamid.

Die Verbindungen der Formel (XVII) sind bekannt oder lassen sich nach den oben beschriebenen Verfahren, z.B. Verfahren [E], aus den entsprechenden Ausgangsverbindungen synthetisieren.

Die Verbindungen der Formel (XVIII) sind bekannt oder lassen sich nach bekannten Verfahren aus den entsprechenden Ausgangsverbindungen synthetisieren.

In einem alternativen Verfahren können die Verbindungen der Formel (II) hergestellt werden, indem Verbindungen der Formel 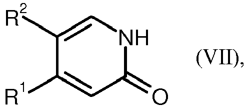 in welcher
R¹ und R² die oben angegebene Bedeutung haben,
mit Verbindungen der Formel 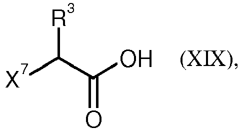 in welcher
R³ die oben angegebene Bedeutung hat, und
X⁷ für Chlor, Brom oder Iod steht,
umgesetzt werden.

Die Umsetzung erfolgt im Allgemeinen in inerten Lösungsmitteln, gegebenenfalls in Gegenwart einer Base, bevorzugt in einem Temperaturbereich von -10°C bis 90°C bei Normaldruck.

Inerte Lösungsmittel sind beispielsweise Halogenkohlenwasserstoffe wie Dichlormethan, Trichlormethan, Tetrachlormethan oder 1,2-Dichlorethan, Alkohole wie Methanol oder Ethanol, Ether wie Diethylether, Methyl-tert-butylether, 1,2-Dimethoxyethan, Dioxan oder Tetrahydrofuran, oder andere Lösungsmittel wie Dimethylformamid, Dimethylacetamid, Acetonitril oder Pyridin, oder Gemische von Lösungsmitteln, oder Gemische von Lösungsmittel mit Wasser, bevorzugt ist Tetrahydrofuran.

Basen sind beispielsweise Kalium- oder Natrium-tert-butylat, Natriumhydrid oder Bis-(trimethylsilyl)-lithiumamid oder ein Gemisch aus Magnesiumdi-tert-butylat und Kalium-tert-butylat, bevorzugt ist ein Gemisch aus Magnesiumdi-tert-butylat und Kalium-tert-butylat.

Die Verbindungen der Formel (XIX) sind bekannt oder lassen sich nach bekannten Verfahren aus den entsprechenden Ausgangsverbindungen synthetisieren.

In einem alternativen Verfahren können die Verbindungen der Formel (XIII) hergestellt werden, indem Verbindungen der Formel 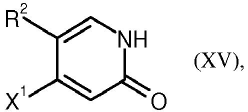 in welcher
R² die oben angegebene Bedeutung habt, und
X¹ für Chlor, Brom oder Iod steht,
mit Verbindungen der Formel 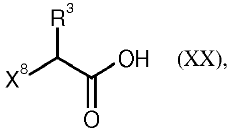 in welcher
R³ die oben angegebene Bedeutung hat, und
X⁸ für Chlor, Brom oder Iod steht,
umgesetzt werden.

Die Umsetzung erfolgt wie für die Umsetzung von Verbindungen der Formel (VII) mit Verbindungen der Formel (XIX) beschrieben.

Die Verbindungen der Formel (XX) sind bekannt oder lassen sich nach bekannten Verfahren aus den entsprechenden Ausgangsverbindungen synthetisieren.

Die Herstellung der Ausgangsverbindungen und der Verbindungen der Formel (I) kann durch das folgende Syntheseschema verdeutlicht werden.

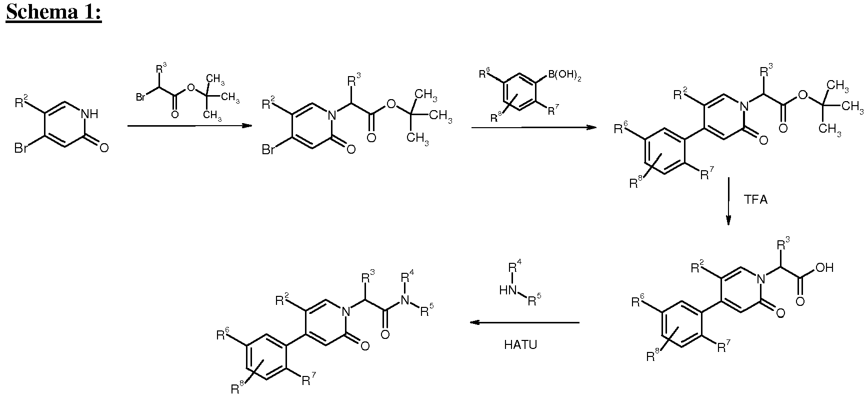

Die erfindungsgemäßen Verbindungen zeigen ein nicht vorhersehbares, wertvolles pharmakologisches Wirkspektrum und ein gutes pharmakokinetisches Verhalten. Es handelt sich dabei um Verbindungen, die die proteolytische Aktivität der Serinprotease Faktor XIa (FXIa) und/oder der Serinprotease Plasmakallikrein (PK) beeinflussen. Die erfindungsgemäßen Verbindungen hemmen die von FXIa und/oder PK katalysierte enzymatische Spaltung von Substraten, die wesentliche Rollen in der Aktivierung der Blutgerinnung, in der Aggregation von Blutplättchen via Reduktion des für die PAR-1-Aktivierung der Plättchen notwendigen Thrombins, und in inflammatorischen Prozessen einnehmen, die insbesondere eine Steigerung der Gefäßpermeabilität miteinschließen.

Sie eigenen sich daher zur Verwendung als Arzneimittel zur Behandlung und/oder Prophylaxe von Krankheiten bei Menschen und Tieren.

Weiterer Gegenstand der vorliegenden Erfindung ist der Einsatz der erfindungsgemäßen Verbindungen zur Behandlung und/oder Prophylaxe von Erkrankungen, insbesondere von Herz-Kreislauf-Erkrankungen, vorzugsweise von thrombotischen beziehungsweise thromboembolischen Erkrankungen und/oder thrombotischen beziehungsweise thromboembolischen Komplikationen, und/oder ophthalmologischen Erkrankungen, insbesondere von diabetischer Retinopathie beziehungsweise des Makulaödems, und/oder entzündlichen Erkrankungen, insbesondere diejenigen, die mit übermäßiger Plasmakallikrein-Aktivität in Zusammenhang stehen, wie z.B. das hereditäre Angioödem (HAE) oder chronisch-entzündliche Erkrankungen, insbesondere des Darms, wie z. B. Morbus Crohn.

Faktor XIa (FXIa) ist ein wichtiges Enzym im Rahmen der Koagulation, das sowohl durch Thrombin als auch Faktor XIIa (FXIIa) aktiviert werden kann und somit in zwei wesentlichen Prozessen der Koagulation involviert ist: Es ist ein zentraler Bestandteil des Übergangs von der Initiation zur Amplifikation und Propagation der Koagulation: Thrombin aktiviert in positiven Rückkopplungsschleifen neben Faktor V und Faktor VIII auch Faktor XI zu Faktor XIa, der Faktor IX zu Faktor IXa umsetzt und über den so generierten Faktor IXa/ Faktor VIIIa-Komplex die Faktor X-Aktivierung und damit wiederum die Thrombinbildung stark stimuliert, was zu starkem Thrombuswachstum führt und den Thrombus stabilisiert.

Darüber hinaus ist Faktor XIa wichtiger Bestandteil der intrinsischen Initiation der Gerinnung: Neben der Stimulation über Gewebefaktor (tissue factor, TF) kann die Aktivierung des Gerinnungssystems auch auf insbesondere negativ geladenen Oberflächen erfolgen, zu denen neben Oberflächenstrukturen körperfremder Zellen (z.B. Bakterien) auch artifizielle Oberflächen wie Gefäßprothesen, Stents und extrakorporale Kreisläufe gehören. Auf der Oberfläche findet zunächst die Aktivierung von Faktor XII (FXII) zu Faktor XIIa (FXIIa) statt, der im Folgenden an Zelloberflächen gebundenen FXI zu FXIa aktiviert. Dieser führt wie zuvor beschrieben zur weiteren Aktivierung der Gerinnungskaskade.

Dagegen bleibt die Thrombingenerierung in der Initiationsphase über TF/Faktor VIIa und Faktor X-Aktivierung und letztlich Thrombinbildung, die physiologische Reaktion auf Gefäßverletzungen, unbeeinflußt. Dies könnte erklären, warum keine Verlängerungen der Blutungszeiten in FXIa-Knock- out-Mäusen, wie auch in Kaninchen und anderen Spezies unter FXIa-Inhibitor-Gabe gefunden wurde. Diese niedrige durch die Substanz hervorgerufene Blutungsneigung ist für die Anwendung am Menschen insbesondere bei Patienten mit erhöhtem Blutungsrisiko von großem Vorteil.

Daneben aktiviert Faktor XIIa im Rahmen der intrinsischen Aktivierung ebenfalls Plasmaprokallikrein zu Plasmakallikrein (PK), das unter anderem zu weiterer Faktor XII-Aktivierung im Rahmen einer Potentierungsschleife führt, was insgesamt eine Verstärkung der Initiation der Gerinnungskaskade an Oberflächen zur Folge hat. Eine PK-hemmende Aktivität einer erfindungsgemäßen Verbindung wird also die Gerinnung via Oberflächenaktivierung reduzieren und somit antikoagulatorisch wirken. Ein Vorteil könnte in der Kombination von Faktor XIa- und PK-inhibitorischer Aktivität liegen, die eine balancierte antithrombotische Wirkung zuläßt.

Die erfindungsgemäßen Verbindungen eignen sich daher zur Behandlung und/oder Prophylaxe von Erkrankungen oder Komplikationen, die durch Gerinnselbildung entstehen können.

Zu den "thrombotischen beziehungsweise thromboembolischen Erkrankungen" im Sinne der vorliegenden Erfindung zählen Erkrankungen, die sowohl im arteriellen als auch im venösen Gefäßbett auftreten und mit den erfindungsgemäßen Verbindungen behandelt werden können, insbesondere Erkrankungen in den Koronararterien des Herzens, wie das akute Koronarsyndrom (ACS), Herzinfarkt mit ST-Segment-Erhöhung (STEMI) und ohne ST-Segment-Erhöhung (non-STEMI), stabile Angina Pectoris, instabile Angina Pectoris, Reokklusionen und Restenosen nach Koronarinterventionen wie Angioplastie, Stentimplantation oder aortokoronarem Bypass, aber auch thrombotische beziehungsweise thromboembolische Erkrankungen in weiteren Gefäßen, die zu peripheren arteriellen Verschlusskrankheiten, Lungenembolien, venösen Thromboembolien, venösen Thrombosen, insbesondere in tiefen Beinvenen und Nierenvenen, transitorische ischämische Attacken sowie thrombotischer Hirnschlag und thromboembolischer Hirnschlag führen.

Die Stimulation des Gerinnungssystems kann durch verschiedene Ursachen beziehungsweise Begleiterkrankungen erfolgen. Unter anderem kann im Rahmen von chirurgischen Eingriffen, Immobilität, Bettlägerigkeit, Infektionen, Inflammation oder einer Krebserkrankung beziehungsweise Krebstherapie das Gerinnungssystem stark angeregt sein und es zu thrombotischen Komplikationen, insbesondere venösen Thrombosen, kommen. Die erfindungsgemäßen Verbindungen eignen sich daher zur Thromboseprophylaxe im Rahmen von chirurgischen Eingriffen bei Patienten, die eine Krebserkrankung haben. Die erfindungsgemäßen Verbindungen eignen sich daher auch zur Thromboseprophylaxe in Patienten mit aktiviertem Gerinnungssystem, beispielsweise unter den beschriebenen Stimulationssituationen.

Die erfindungsgemäßen Verbindungen eignen sich daher auch zur Prävention und Behandlung von kardiogenen Thromboembolien, wie beispielsweise Hirn-Ischämien, Schlaganfall und systemischen Thromboembolien und Ischämien, bei Patienten mit akuten, intermittierenden oder persistierenden Herzarrhythmien, wie beispielsweise Vorhofflimmern, und bei Patienten, die sich einer Kardioversion unterziehen, ferner bei Patienten mit Herzklappen-Erkrankungen oder mit künstlichen Herzklappen.

Darüber hinaus sind die erfindungsgemäßen Verbindungen zur Behandlung und Prävention einer disseminierten intravasalen Gerinnung (DIC) geeignet, die unter anderem im Rahmen einer Sepsis, aber auch infolge von Operationen, Tumorerkrankungen, Verbrennungen oder anderen Verletzungen auftreten und durch Mikrothrombosierungen zu schweren Organschäden führen kann.

Thromboembolische Komplikationen treten ferner auf bei mikroangiopathischen hämolytischen Anämien und durch Kontakt des Blutes mit körperfremden Oberflächen im Rahmen von extrakorporalen Blutkreisläufen, wie zum Beispiel Hämodialyse, ECMO ("extracorporal membrane oxygenation"), LVAD ("left ventricular assist device") und ähnlichen Verfahren, AV-Fisteln, Gefäß- sowie Herzklappenprothesen.

Außerdem kommen die erfindungsgemäßen Verbindungen zur Behandlung und/oder Prophylaxe von Erkrankungen in Betracht, bei denen Mikrogerinnselbildungen beziehungsweise Fibrinablagerungen in Gehirngefäßen auftreten, die zu Demenzerkrankungen, wie beispielsweise vaskulärer Demenz oder Morbus Alzheimer führen können. Hierbei kann das Gerinnsel sowohl über Okklusionen als auch über die Bindung weiterer krankheitsrelevanter Faktoren zur Erkrankung beitragen.

Außerdem kommen die erfindungsgemäßen Verbindungen insbesondere zur Behandlung und/oder Prophylaxe von Erkrankungen in Betracht, bei denen neben der prokoagulanten auch die proinflammatorische Komponente eine wesentliche Rolle spielt. Insbesondere die gegenseitige Verstärkung von Koagulation und Inflammation kann durch die erfindungsgemäßen Verbindungen unterbunden und daher die Wahrscheinlichkeit für eine thrombotische Komplikation entscheidend gesenkt werden. Dabei können sowohl die Faktor XIa-inhibitorische Komponente (über Hemmung der Thrombinproduktion) als auch die PK-inhibitorische Komponente zur antikoagulanten und antiinflammatorischen Wirkung (z.B. via Bradikinin) beitragen. Daher kommen unter anderem die Behandlung und/oder Prophylaxe im Rahmen von atherosklerotischen Gefäßerkrankungen, Entzündungen im Rahmen von rheumatischen Erkrankungen des Bewegungsapparates, entzündlichen Erkrankungen der Lunge, wie beispielsweise Lungenfibrosen, entzündliche Erkrankungen der Niere, wie beispielsweise Glomerulonephritiden, entzündliche Erkrankungen des Darms, wie beispielsweise Morbus Crohn oder Colitis ulcerosa, oder Erkrankungen, die im Rahmen einer diabetischen Grunderkrankung vorliegen können, wie beispielsweise diabetische Retinopathie beziehungsweise Nephropathie in Betracht.

Bei der Progression von chronisch-entzündlichen Darmerkrankungen (CED) haben unter anderem mittels Plasmakallikrein generierten Kinine eine tragende Rolle. Deren pro-inflammatorische Wirkung über Aktivierung von Bradykinin-Rezeptoren induziert und potenziert den Krankheitsverlauf. Studien an Morbus Crohn-Patienten zeigen eine Korrelation zwischen der Kallikrein-Konzentration im Darmepithel und dem Grad der Darmentzündung. Eine Aktivierung des Kallikrein-Kinin-Systems wurde ebenfalls in tierexperimentellen Studien beobachtet. Eine Hemmung der Bradykinin-Synthese durch Kallikrein-Inhibitoren könnte demnach auch zur Prophylaxe und/oder Therapie von chronisch-entzündlichen Darmerkrankungen eingesetzt werden.

Außerdem können die erfindungsgemäßen Verbindungen zur Inhibition des Tumorwachstums und der Metastasenbildung, sowie zur Prophylaxe und/oder Behandlung thromboembolischer Komplikationen, wie beispielsweise venöser Thromboembolien, bei Tumorpatienten, insbesondere solchen, die sich größeren chirurgischen Eingriffen oder einer Chemo- oder Radiotherapie unterziehen, eingesetzt werden.

Außerdem kommen die erfindungsgemäßen Verbindungen auch für die Prophylaxe und/oder Behandlung von pulmonaler Hypertonie in Betracht.

Der Begriff "pulmonale Hypertonie" umfasst im Rahmen der vorliegenden Erfindung die pulmonale arterielle Hypertonie, die pulmonale Hypertonie bei Erkrankungen des linken Herzens, die pulmonale Hypertonie bei Lungenerkrankung und/oder Hypoxie und die Pulmonale Hypertonie aufgrund chronischer Thrombembolien (CTEPH).

Die "pulmonale arterielle Hypertonie" beinhaltet die Idiopathische Pulmonale Arterielle Hypertonie (IPAH, früher auch als primäre pulmonale Hypertonie bezeichnet), die Familiär bedingte Pulmonale Arterielle Hypertonie (FPAH) und die Assoziierte Pulmonal-Arterielle Hypertonie (APAH), die assoziiert ist mit Kollagenosen, kongenitalen systemisch-pulmonalen Shuntvitien, portaler Hypertension, HIV-Infektionen, der Einnahme bestimmter Drogen und Medikamente, mit anderen Erkrankungen (Schilddrüsenerkrankungen, Glykogenspeicherkrankheiten, Morbus Gaucher, hereditäre Teleangiektasie, Hämoglobinopathien, myeloproliferative Erkrankungen, Splenektomie), mit Erkrankungen mit einer signifikanten venösen/kapillären Beteiligung wie der pulmonal-venookklusiven Erkrankung und der pulmonal-kapillären Hämangiomatose, sowie die persistierende pulmonale Hypertonie der Neugeborenen.

Die pulmonale Hypertonie bei Erkrankungen des linken Herzens beinhaltet die Erkrankung des linken Vorhofes oder Ventrikels und Mitral- oder Aortenklappenfehler.

Die pulmonale Hypertonie bei Lungenerkrankung und/oder Hypoxie beinhaltet chronisch obstruktive Lungenerkrankungen, interstitielle Lungenerkrankung, Schlafapnoe-Syndrom, alveolärer Hypoventilation, chronische Höhenkrankheit und anlagebedingte Fehlbildungen.

Die Pulmonale Hypertonie aufgrund chronischer Thrombembolien (CTEPH) beinhaltet den thrombembolischen Verschluss proximaler Lungenarterien, den thrombembolischen Verschluss distaler Lungenarterien und nicht-thrombotische Lungenembolien (Tumor, Parasiten, Fremdkörper).

Weiterer Gegenstand der vorliegenden Erfindung ist die Verwendung der erfindungsgemäßen Verbindungen zur Herstellung von Arzneimitteln zur Behandlung und/oder Prophylaxe von pulmonaler Hypertonie bei Sarkoidose, Histiozytose X und Lymphangiomatosis.

Außerdem kommen die erfindungsgemäßen Substanzen auch zur Behandlung von pulmonalen und hepatischen Fibrosen in Betracht.

Außerdem kommen die erfindungsgemäßen Verbindungen auch für die Behandlung und/oder Prophylaxe einer Disseminierten intravasalen Koagulation im Rahmen einer Infektionserkrankung und/oder von Systemic Inflammatory Syndrome (SIRS), septischer Organdysfunktion, septischem Organversagen und Multiorganversagen, Acute Respiratory Distress Syndrome (ARDS), Acute Lung Injury (ALI), Septischer Schock und/oder des septischen Organversagens in Betracht.

Im Verlauf einer Infektion kann es zur generalisierten Aktivierung des Gerinnungssystems kommen ("Disseminated Intravascular coagulation", oder "Verbrauchskoagulopathie", nachfolgend als "DIC" bezeichnet) mit Mikrothrombosierung in verschiedenen Organen und sekundärer Blutungskomplikationen. Außerdem kann es zur endothelialen Schädigung mit Erhöhung der Gefäßpermeabilität und Austritt von Flüssigkeit und Proteinen in den Extravasalraum kommen. Im weiteren Verlauf kann ein Versagen eines Organs (z.B. Nierenversagen, Leberversagen, Atemversagen, zentralnervöse Defizite und Herz-/Kreislaufversagen) oder zum Multiorganversagen kommen.

Bei der DIC kommt es an der Oberfläche von geschädigten Endothelzellen, Fremdkörperoberflächen oder vernetztem extravaskulärem Gewebe zur massiven Aktivierung des Gerinnungssystems. Als Folge kommt es zur Gerinnung in kleinen Gefäßen verschiedener Organe mit Hypoxie und anschließender Organdysfunktion. Sekundär kommt es zum Verbrauch von Gerinnungsfaktoren (z.B. Faktor X, Prothrombin und Fibrinogen) und Plättchen, wodurch die Gerinnungsfähigkeit des Blutes herabgesetzt wird und schwere Blutungen auftreten können.

Erfindungsgemäße Verbindungen, die Plasmakallikrein alleine oder in Kombination mit Faktor XIa hemmen, kommen zusätzlich zur Behandlung und/oder Prophylaxe von Erkrankungen in Betracht, in deren Verlauf Plasmakallikrein involviert ist. Zusätzlich zur antikoagulanten Aktivität stellt Plasmakallikrein eine wichtige Bradikinin-freisetzende Protease dar, die somit unter anderem zum Anstieg der endothelialen Permeabilität führt. Die Verbindungen können somit zur Behandlung und/oder Prophylaxe von Erkrankungen eingesetzt werden, die mit Ödembildungen einhergehen, wie zum Beispiel ophthalmologische Erkrankungen, insbesondere die diabetische Retinopathie oder das Makulaödem, oder das hereditäre Angioödem.

Zu den "ophthalmologischen Erkrankungen" im Sinne der vorliegenden Erfindung zählen insbesondere Erkrankungen wie diabetische Retinopathie, diabetisches Makulaödem (diabetic macular edema, DME), Makulaödem, Makulaödem assoziiert mit retinalem Venenverschluss, altersbedingte Makula-Degeneration (AMD), choroidale Neovaskularisierung (CNV), choroidale neovaskuläre Membranen (CNVM), zystoides Makulaödem (cystoid macula edema, CME), epiretinale Membranen (ERM) und Makula-Perforationen, Myopie-assoziierte choroidale Neovaskularisierung, angioide beziehungsweise vaskuläre Streifen (angioid streaks, vascular streaks), Retina-Ablösung, atrophische Veränderungen des retinalen Pigmentepithels, hypertrophische Veränderungen des retinalen Pigmentepithels, retinaler Venenverschluss, choroidaler retinaler Venenverschluss, Retinitis pigmentosa, Stargardt'sche Erkrankung, Frühgeborenen-Retinopathie, Glaukom, entzündliche Erkrankungen des Auges wie z.B. Uveitis, Skleritis oder Endophthalmitis, Katarakt, Refraktionsanomalien wie z.B. Myopie, Hyperopie oder Astigmatismus und Keratokonus, Erkrankungen des vorderen Auges wie z.B. korneale Angiogenese als Folge von z.B. Keratitis, Hornhaut-Transplantation oder Keratoplastie, korneale Angiogenese als Folge von Hypoxie (z.B. durch zu langes Tragen von Kontaktlinsen), Pterygium conjunctivae, subkorneales Ödem und intrakorneales Ödem.

Weiterhin kommen die erfindungsgemäßen Verbindungen zur Primärprophylaxe thrombotischer oder thromboembolischer Erkrankungen und/oder inflammatorischer Erkrankungen und/oder Erkrankungen mit erhöhter Gefäßpermeabilität in Patienten in Frage, in denen Genmutationen zu verstärkter Aktivität der Enzyme oder erhöhten Spiegeln der Zymogene führen und diese durch entsprechende Tests/Messungen der Enzymaktivität bzw. Zymogenkonzentrationen festgestellt werden.

Weiterer Gegenstand der vorliegenden Erfindung ist die Verwendung der erfindungsgemäßen Verbindungen zur Behandlung und/oder Prophylaxe von Erkrankungen, insbesondere der zuvor genannten Erkrankungen.

Weiterer Gegenstand der vorliegenden Erfindung ist die Verwendung der erfindungsgemäßen Verbindungen zur Herstellung eines Arzneimittels zur Behandlung und/oder Prophylaxe von Erkrankungen, insbesondere der zuvor genannten Erkrankungen.

Weiterer Gegenstand der vorliegenden Erfindung ist ein Verfahren zur Behandlung und/oder Prophylaxe von Erkrankungen, insbesondere der zuvor genannten Erkrankungen, unter Verwendung einer therapeutisch wirksamen Menge einer erfindungsgemäßen Verbindung.

Weiterer Gegenstand der vorliegenden Erfindung sind die erfindungsgemäßen Verbindungen zur Verwendung in einem Verfahren zur Behandlung und/oder Prophylaxe von Erkrankungen, insbesondere der zuvor genannten Erkrankungen, unter Verwendung einer therapeutisch wirksamen Menge einer erfindungsgemäßen Verbindung.

Weiterer Gegenstand der vorliegenden Erfindung sind Arzneimittel, enthaltend eine erfindungsgemäße Verbindung und einen oder mehrere weitere Wirkstoffe.

Die erfindungsgemäßen Verbindungen können darüber hinaus auch zur Verhinderung von Koagulation *ex vivo* eingesetzt werden, z.B. zum Schutz von zu transplantierenden Organen vor durch Gerinnselbildung verursachten Organschäden und zum Schutz des Organ-Empfängers vor Thromboemboli aus dem transplantierten Organ, zur Konservierung von Blut- und Plasmaprodukten, zur Reinigung/Vorbehandlung von Kathetern und anderen medizinischen Hilfsmitteln und Geräten, zur Beschichtung künstlicher Oberflächen von *in vivo* oder *ex vivo* eingesetzten medizinischen Hilfsmitteln und Geräten oder bei biologischen Proben, die Faktor XIa oder Plasmakallikrein enthalten könnten.

Weiterer Gegenstand der vorliegenden Erfindung ist ein Verfahren zur Verhinderung der Blutkoagulation *in vitro,* insbesondere bei Blutkonserven oder biologischen Proben, die Faktor XIa oder Plasmakallikrein oder beide Enzyme enthalten könnten, das dadurch gekennzeichnet ist, dass eine antikoagulatorisch wirksame Menge der erfindungsgemäßen Verbindung zugegeben wird.

Weiterer Gegenstand der vorliegenden Erfindung sind Arzneimittel, enthaltend eine erfindungsgemäße Verbindung und einen oder mehrere weitere Wirkstoffe, insbesondere zur Behandlung und/oder Prophylaxe der zuvor genannten Erkrankungen. Als geeignete Kombinationswirkstoffe seien beispielhaft und vorzugsweise genannt:
- Lipidsenker, insbesondere HMG-CoA-(3-Hydroxy-3-methylglutaryl-Coenzym A)-Reduktase-Inhibitoren wie beispielsweise Lovastatin (Mevacor), Simvastatin (Zocor), Pravastatin (Pravachol), Fluvastatin (Lescol) und Atorvastatin (Lipitor);
- Koronartherapeutika/Vasodilatatoren, insbesondere ACE-(Angiotensin-Converting-Enzyme)-Inhibitoren wie beispielsweise Captopril, Lisinopril, Enalapril, Ramipril, Cilazapril, Benazepril, Fosinopril, Quinapril und Perindopril, oder AII-(Angiotensin II)-Rezeptor-Antagonisten wie beispielsweise Embusartan, Losartan, Valsartan, Irbesartan, Candesartan, Eprosartan und Temisarta, oder β-Adrenozeptor-Antagonisten wie beispielsweise Carvedilol, Alprenolol, Bisoprolol, Acebutolol, Atenolol, Betaxolol, Carteolol, Metoprolol, Nadolol, Penbutolol, Pindolol, Propanolol und Timolol, oder alpha-1-Adrenozeptor-Antagonisten wie beispielsweise Prazosin, Bunazosin, Doxazosin und Terazosin, oder Diuretika wie beispielsweise Hydrochlorothiazid, Furosemid, Bumetanid, Piretanid, Torasemid, Amilorid und Dihydralazin, oder Calciumkanal-Blocker wie beispielsweise Verapamil und Diltiazem, oder Dihydropyridin-Derivate wie beispielsweise Nifedipin (Adalat) und Nitrendipin (Bayotensin), oder Nitropräparate wie beispielsweise Isosorbid-5-mononitrat, Isosorbiddinitrat und Glyceroltrinitrat, oder Substanzen, die eine Erhöhung von cyclischem Guanosinmonophosphat (cGMP) bewirken, wie beispielsweise Stimulatoren der löslichen Guanylatcyclase, wie beispielsweise Riociguat;
- Plasminogen-Aktivatoren (Thrombolytika/Fibrinolytika) und die Thrombolyse/Fibrinolyse steigernde Verbindungen wie Inhibitoren des Plasminogen-Aktivator-Inhibitors (PAI-Inhibitoren) oder Inhibitoren des Thrombin-aktivierten Fibrinolyse-Inhibitors (TAFI-Inhibitoren) wie beispielsweise Gewebsplasminogen-Aktivator (t-PA, beispielsweise Actilyse®), Streptokinase, Reteplase und Urokinase oder Plasminogen-modulierende Substanzen, die zu verstärkter Plasmin-Bildung führen;
- antikoagulatorisch wirksame Substanzen (Antikoagulantien) wie beispielsweise Heparin (UFH), niedermolekulare Heparine (NMH) wie beispielsweise Tinzaparin, Certoparin, Parnaparin, Nadroparin, Ardeparin, Enoxaparin, Reviparin, Dalteparin, Danaparoid, Semuloparin (AVE 5026), Adomiparin (M118) und EP-42675/ORG42675;
- direkte Thrombin Inhibitoren (DTI) wie beispielsweise Pradaxa (Dabigatran), Atecegatran (AZD-0837), DP-4088, SSR-182289A, Argatroban, Bivalirudin und Tanogitran (BIBT-986 und prodrug BIBT-1011), Hirudin;
- direkte Faktor Xa-Inhibitoren wie beispielsweise Rivaroxaban, Apixaban, Edoxaban (DU-176b), Betrixaban (PRT-54021), R-1663, Darexaban (YM-150), Otamixaban (FXV-673/RPR-130673), Letaxaban (TAK-442), Razaxaban (DPC-906), DX-9065a, LY-517717, Tanogitran (BIBT-986, prodrug: BIBT-1011), Idraparinux und Fondaparinux,
- plättchenaggregationshemmende Substanzen (Plättchenaggregationshemmer, Thrombozytenaggregationshemmer) wie beispielsweise Acetylsalicylsäure (wie beispielsweise Aspirin), P2Y12-Antagonisten wie beispielsweise Ticlopidin (Ticlid), Clopidogrel (Plavix), Prasugrel, Ticagrelor, Cangrelor, Elinogrel, PAR-1-Antagonisten wie beispielsweise Vorapaxar, PAR-4 Antagonisten, EP3-Antagonisten wie beispielsweise DG041;
- Plättchenadhäsionshemmern wie GPVI- und/oder GPIb-Antagonisten wie beispielsweise Revacept oder Caplacizumab;
- Fibrinogen-Rezeptor-Antagonisten (Glycoprotein-IIb/IIIa-Antagonisten) wie beispielsweise Abciximab, Eptifibatide, Tirofiban, Lamifiban, Lefradafiban und Fradafiban;
- rekombinantes humanes aktiviertes Protein C wie beispielsweise Xigris oder rekombinantes Thrombomodulin;
- sowie Antiarrhythmika;
- Inhibitoren der VEGF- und/oder PDGF Signalwege wie beispielsweise Aflibercept, Ranibizumab, Bevacizumab, KH-902, Pegaptanib, Ramucirumab, Squalamin oder Bevasiranib, Apatinib, Axitinib, Brivanib, Cediranib, Dovitinib, Lenvatinib, Linifanib, Motesanib, Pazopanib, Regorafenib, Sorafenib, Sunitinib, Tivozanib, Vandetanib, Vatalanib, Vargatef und E-10030;
- Hemmer der Angiopoietin-Tie Signalwege wie beispielsweise AMG386;
- Inhibitoren der Tie2 Rezeptortyrosinkinase;
- Hemmer der Integrin-Signalwege wie beispielsweise Volociximab, Cilengitid und ALG1001;
- Hemmer der PI3K-Akt-mTor Signalwege wie beispielsweise XL-147, Perifosin, MK2206, Sirolimus, Temsirolimus und Everolimus;
- Corticosteroid wie beispielsweise Anecortave, Betamethason, Dexamethason, Triamcinolon, Fluocinolon und Fluocinolonacetonid;
- Inhibitoren des ALK1-Smad1/5 Signalweges wie beispielsweise ACE041;
- Cyclooxygenaseinhibitoren wie beispielsweise Bromfenac und Nepafenac;
- Hemmer des Kallikrein-Kinin-Systems wie beispielsweise Safotibant und Ecallantid;
- Inhibitoren der Sphingosin-1-phosphat-Signalwege wie beispielsweise Sonepcizumab;
- Inhibitoren des Komplement-C5a Rezeptors wie beispielsweise Eculizumab;
- Inhibitoren des 5HT1a Rezeptors wie beispielsweise Tandospiron;
- Hemmer des Ras-Raf-Mek-Erk Signalwegs; Hemmer der MAPK Signalwege; Hemmer der FGF-Signalwege; Hemmer der Endothelzellproliferation; Apoptose-induzierende Wirkstoffe;
- Photodynamische Therapie, bestehend aus einem Wirkstoff und der Einwirkung von Licht, wobei der Wirkstoff beispielsweise Verteporfin ist.

Unter "Kombinationen" im Sinne der Erfindung werden nicht nur Darreichungsformen, die alle Komponenten enthalten (sog. Fixkombinationen) und Kombinationspackungen, die die Komponenten voneinander getrennt enthalten, verstanden, sondern auch gleichzeitig oder zeitlich versetzt applizierte Komponenten, sofern sie zur Prophylaxe und/oder Behandlung derselben Krankheit eingesetzt werden. Ebenso ist es möglich, zwei oder mehr Wirkstoffe miteinander zu kombinieren, es handelt sich dabei also jeweils um zwei- oder mehrfach-Kombinationen.

Die erfindungsgemäßen Verbindungen können systemisch und/oder lokal wirken. Zu diesem Zweck können sie auf geeignete Weise appliziert werden, wie z.B. oral, parenteral, pulmonal, nasal, sublingual, lingual, buccal, rectal, dermal, transdermal, conjunctival, otisch oder als Implantat beziehungsweise Stent.

Für diese Applikationswege können die erfindungsgemäßen Verbindungen in geeigneten Applikationsformen verabreicht werden.

Für die orale Applikation eignen sich nach dem Stand der Technik funktionierende schnell und/oder modifiziert die erfindungsgemäßen Verbindungen abgebende Applikationsformen, die die erfindungsgemäßen Verbindungen in kristalliner und/ oder amorphisierter und/oder gelöster Form enthalten, wie z.B. Tabletten (nicht überzogene oder überzogene Tabletten, beispielsweise mit magensaftresistenten oder sich verzögert auflösenden oder unlöslichen Überzügen, die die Freisetzung der erfindungsgemäßen Verbindung kontrollieren), in der Mundhöhle schnell zerfallende Tabletten oder Filme/Oblaten, Filme/Lyophylisate, Kapseln (beispielsweise Hart- oder Weichgelatinekapseln), Dragees, Granulate, Pellets, Pulver, Emulsionen, Suspensionen, Aerosole oder Lösungen.

Die parenterale Applikation kann unter Umgehung eines Resorptionsschrittes geschehen (z.B. intravenös, intraarteriell, intrakardial, intraspinal oder intralumbal) oder unter Einschaltung einer Resorption (z.B. intramuskulär, subcutan, intracutan, percutan oder intraperitoneal). Für die parenterale Applikation eignen sich als Applikationsformen u.a. Injektions- und Infusionszubereitungen in Form von Lösungen, Suspensionen, Emulsionen, Lyophilisaten oder sterilen Pulvern.

Für die extraoculare (topische) Applikation eignen sich nach dem Stand der Technik funktionierende schnell und/oder modifiziert beziehungsweise kontrolliert den Wirkstoff abgebende Applikationsformen, die den Wirkstoff in kristalliner und/oder amorphisierter und/oder gelöster Form enthalten, wie z.B. Augentropfen, Sprays und Lotionen (z.B. Lösungen, Suspensionen, vesikuläre/kolloidale Systeme, Emulsionen, Aerosole), Pulver für Augentropfen, Sprays und Lotionen (z.B. gemahlener Wirkstoff, Mischungen, Lyophilisate, gefällter Wirkstoff), halbfeste Augenzubereitungen (z.B. Hydrogele, in-situ Hydrogele, Cremes und Salben), Augeninserte (feste und halbfeste Zubereitungen, z.B. Bioadhesives, Filme/Wafer, Tabletten, Kontaktlinsen).

Die intraoculare Applikation umfasst z.B. die intravitreale subretinale, subsclerale, intrachoroidale, subconjunctivale, retrobulbare und subtenone Applikation. Für die intraoculare Applikation eignen sich nach dem Stand der Technik funktionierende schnell und/oder modifiziert beziehungsweise kontrolliert den Wirkstoff abgebende Applikationsformen, die den Wirkstoff in kristalliner und/oder amorphisierter und/oder gelöster Form enthalten, wie z.B. Injektionen und Konzentrate für Injektionen (z.B. Lösungen, Suspensionen, vesikulärelkolloidale Systeme, Emulsionen, Pulver für Injektionen (z.B. gemahlener Wirkstoff, Mischungen, Lyophilisate, gefällter Wirkstoff), Gele für Injektionen (halbfeste Zubereitungen, z.B. Hydrogele, in-situ Hydrogele) und Implantate (feste Zubereitungen, z.B. bioabbaubare und nicht-bioabbaubare Implantate, implantierbare Pumpen).

Bevorzugt ist die orale Applikation beziehungsweise bei ophthalmologischen Erkrankungen die extraokulare und die intraokulare Applikation.

Für die sonstigen Applikationswege eignen sich z.B. Inhalationsarzneiformen (u.a. Pulverinhalatoren, Nebulizer), Nasentropfen, -lösungen, -sprays; lingual, sublingual oder buccal zu applizierende Tabletten, Filme/Oblaten oder Kapseln, Suppositorien, Ohren- oder Augenpräparationen, Vaginalkapseln, wässrige Suspensionen (Lotionen, Schüttelmixturen), lipophile Suspensionen, Salben, Cremes, transdermale therapeutische Systeme (wie beispielsweise Pflaster), Milch, Pasten, Schäume, Streupuder, Implantate oder Stents.

Die erfindungsgemäßen Verbindungen können in die angeführten Applikationsformen überführt werden. Dies kann in an sich bekannter Weise durch Mischen mit inerten, nichttoxischen, pharmazeutisch geeigneten Hilfsstoffen geschehen. Zu diesen Hilfsstoffen zählen u.a. Trägerstoffe (beispielsweise mikrokristalline Cellulose, Laktose, Mannitol), Lösungsmittel (z.B. flüssige Polyethylenglycole), Emulgatoren und Dispergier- oder Netzmittel (beispielsweise Natriumdodecylsulfat, Polyoxysorbitanoleat), Bindemittel (beispielsweise Polyvinylpyrrolidon), synthetische und natürliche Polymere (beispielsweise Albumin), Stabilisatoren (z.B. Antioxidantien wie beispielsweise Ascorbinsäure), Farbstoffe (z.B. anorganische Pigmente wie beispielsweise Eisenoxide) und Geschmacks- und / oder Geruchskorrigentien.

Weiterer Gegenstand der vorliegenden Erfindung sind Arzneimittel, die mindestens eine erfindungsgemäße Verbindung, vorzugsweise zusammen mit einem oder mehreren inerten nichttoxischen, pharmazeutisch geeigneten Hilfsstoff enthalten, sowie deren Verwendung zu den zuvor genannten Zwecken.

Im Allgemeinen hat es sich als vorteilhaft erwiesen, bei parenteraler Applikation Mengen von etwa 5 bis 250 mg je 24 Stunden zur Erzielung wirksamer Ergebnisse zu verabreichen. Bei oraler Applikation beträgt die Menge etwa 5 bis 500 mg je 24 Stunden.

Trotzdem kann es gegebenenfalls erforderlich sein, von den genannten Mengen abzuweichen, und zwar in Abhängigkeit von Körpergewicht, Applikationsweg, individuellem Verhalten gegenüber dem Wirkstoff, Art der Zubereitung und Zeitpunkt beziehungsweise Intervall, zu welchem die Applikation erfolgt.

Die Prozentangaben in den folgenden Tests und Beispielen sind, sofern nicht anders angegeben, Gewichtsprozente; Teile sind Gewichtsteile. Lösungsmittelverhältnisse, Verdünnungsverhältnisse und Konzentrationsangaben von flüssig/flüssig-Lösungen beziehen sich jeweils auf das Volumen. Die Angabe "w/v" bedeutet "weight/volume" (Gewicht/Volumen). So bedeutet beispielsweise "10% w/v": 100 ml Lösung oder Suspension enthalten 10 g Substanz.

### A) Beispiele

### Abkürzungen:

- Boc: *tert.*-Butyloxycarbonyl
- ca.: circa
- d: Tag(e), Dublett (bei NMR)
- DABCO: 1,4-Diazabicyclo[2.2.2]octan
- DC: Dünnschicht-Chromatographie
- DCM: Dichlormethan
- DCI: direkte chemische Ionisation (bei MS)
- dd: Doppeltes Dublett (bei NMR)
- DIC: *N*,*N'*-Diisopropylcarbodiimid
- DIEA: *N*,*N*-Diisopropylethylamin
- DMAP: 4-Dimethylaminopyridin
- DMF: *N*,*N*-Dimethylformamid
- DMSO: Dimethylsulfoxid
- d. Th.: der Theorie (bei Ausbeute)
- eq.: Äquivalent(e)
- ESI: Elektrospray-Ionisation (bei MS)
- h: Stunde(n)
- HATU: *O*-(7-Azabenzotriazol-1-yl)-*N*,*N*,*N'*,*N'*-tetramethyluronium-Hexafluorphosphat
- HPLC: Hochdruck-, Hochleistungsflüssigchromatographie
- HV: Hochvakuum
- LC-MS: Flüssigchromatographie-gekoppelte Massenspektroskopie
- LDA: Lithiumdiisopropylamid
- m: Multiplett (bei NMR)
- min: Minute(n)
- MS: Massenspektroskopie
- NMR: Kernresonanzspektroskopie
- Oxima: Hydroxyiminocyanoessigsaeureethylester
- q: Quartett oder Quadruplett (bei NMR)
- quant.: quantitativ
- quin: Quintett (bei NMR)
- RP: reverse phase (bei HPLC)
- RT: Raumtemperatur
- Rt: Retentionszeit (bei HPLC)
- s: Singulett (bei NMR)
- sxt: Sextett (bei NMR)
- SFC: superkritische Flüssigkeitschromatographie (mit überkritischem Kohlendioxid als mobile Phase)
- t: Triplett (bei NMR)
- THF: Tetrahydrofuran
- TFA: Trifluoressigsäure
- T3P: 2,4,6-Tripropyl-1,3,5,2,4,6-trioxatriphosphinan-2,4,6-trioxid
- Xantphos: 4,5-Bis(diphenylphosphino)-9,9-dimethylxanthen
- XPhos Präkatalysator: [(2'-Aminobiphenyl-2-yl)(chlor)palladium-dicyclohexyl(2',4',6'-triisopropylbiphenyl-2-yl)phosphan (1:1)], J. Am. Chem. Soc. 2010, 132, 14073-14075
- CATAXCium A Präkatalysator: (2'-Aminobiphenyl-2-yl)(methansulfonat)palladium-butyl[di-(3S,5S,7S)-tricyclo[3.3.1.13,7]dec-1-yl]phosphan (1:1)

### HPLC-, LC/MS- und GC-Methoden:

Methode 1: Instrument: Waters ACQUITY SQD UPLC System; Säule: Waters Acquity UPLC HSS T3 1.8 µ 50 mm x 1 mm; Eluent A: 11 Wasser + 0.25 ml 99%ige Ameisensäure, Eluent B: 11 Acetonitril + 0.25 ml 99%ige Ameisensäure; Gradient: 0.0 min 90% A → 1.2 min 5% A → 2.0 min 5% A; Ofen: 50°C; Fluss: 0.40 ml/min; UV-Detektion: 208-400 nm.

Methode 2: Instrument: Waters ACQUITY SQD UPLC System; Säule: Waters Acquity UPLC HSS T3 1.8 µ 50 mm x 1 mm; Eluent A: 11 Wasser + 0.25 ml 99%ige Ameisensäure, Eluent B: 11 Acetonitril + 0.25 ml 99%ige Ameisensäure; Gradient: 0.0 min 95% A → 6.0 min 5% A → 7.5 min 5% A; Ofen: 50°C; Fluss: 0.35 ml/min; UV-Detektion: 210-400 nm.

Methode 3: Instrument: Micromass Quattro Premier mit Waters UPLC Acquity; Säule: Thermo Hypersil GOLD 1.9 µ 50 mm x 1 mm; Eluent A: 11 Wasser + 0.5 ml 50%ige Ameisensäure, Eluent B: 11 Acetonitril + 0.5 ml 50%ige Ameisensäure; Gradient: 0.0 min 97% A → 0.5 min 97% A → 3.2 min 5% A → 4.0 min 5% A; Ofen: 50°C; Fluss: 0.3 ml/min; UV-Detektion: 210 nm.

Methode 4: Instrument MS: Waters (Micromass) Quattro Micro; Instrument HPLC: Agilent 1100 Serie; Säule: YMC-Triart C18 3µ 50 mm x 3 mm; Eluent A: 1 1 Wasser + 0.01 mol Ammoniumcarbonat, Eluent B: 11 Acetonitril; Gradient: 0.0 min 100% A → 2.75 min 5% A → 4.5 min 5% A; Ofen: 40°C; Fluss: 1.25 ml/min; UV-Detektion: 210 nm.

Methode 5: Instrument MS: Waters (Micromass) QM; Instrument HPLC: Agilent 1100 Serie; Säule: Agient ZORBAX Extend-C18 3.0 mm x 50 mm 3.5-Micron; Eluent A: 1 l Wasser + 0.01 mol Ammoniumcarbonat, Eluent B: 1 l Acetonitril; Gradient: 0.0 min 98% A → 0.2 min 98% A → 3.0 min 5% A→ 4.5 min 5% A; Ofen: 40°C; Fluss: 1.75 ml/min; UV-Detektion: 210 nm.

Methode 6: Instrument MS: Waters (Micromass) ZQ; Instrument HPLC: Agilent 1100 Serie; Säule: Agient ZORBAX Extend-C18 3.0 mm x 50 mm 3.5-Micron; Eluent A: 1 l Wasser + 0.01 mol Ammoniumcarbonat, Eluent B: 1 l Acetonitril; Gradient: 0.0 min 98% A → 0.2 min 98% A → 3.0 min 5% A→ 4.5 min 5% A; Ofen: 40°C; Fluss: 1.75 ml/min; UV-Detektion: 210 nm.

Methode 7: Instrument: Thermo DFS, Trace GC Ultra; Säule: Restek RTX-35, 15 m x 200 µm x 0.33 µm; konstanter Fluss mit Helium: 1.20 ml/min; Ofen: 60°C; Inlet: 220°C; Gradient: 60°C, 30°C/min → 300°C (3.33 min halten).

Methode 8: Instrument: Agilent MS Quad 6150; HPLC: Agilent 1290; Säule: Waters Acquity UPLC HSS T3 1.8 µ 50 mm x 2.1 mm; Eluent A: 1 l Wasser + 0.25 ml 99%ige Ameisensäure, Eluent B: 1 l Acetonitril + 0.25 ml 99%ige Ameisensäure; Gradient: 0.0 min 90% A → 0.3 min 90% A → 1.7 min 5% A → 3.0 min 5% A; Ofen: 50°C; Fluss: 1.20 ml/min; UV-Detektion: 205-305 nm.

Methode 9: Instrument: Thermo Scientific DSQII, Thermo Scientific Trace GC Ultra; Säule: Restek RTX-35MS, 15 m x 200 µm x 0.33 µm; konstanter Fluss mit Helium: 1.20 ml/min; Ofen: 60°C; Inlet: 220°C; Gradient: 60°C, 30°C/min → 300°C (3.33 min halten).

Mikrowelle: Als Mikrowellenreaktor wurde ein "single mode" Gerät vom Typ Emrys™ Optimizer verwendet.

Bei Aufreinigungen von erfindungsgemäßen Verbindungen per präparativer HPLC nach den oben beschriebenen Methoden, in denen die Elutionsmittel Zusatzstoffe wie beispielsweise Trifluoressigsäure, Ameisensäure oder Ammoniak enthalten, können die erfindungsgemäßen Verbindungen in Salz-Form, beispielsweise als Trifluoracetat, Formiat oder Ammonium-Salz anfallen, sofern die erfindungsgemäßen Verbindungen eine ausreichend basische beziehungsweise saure Funktionalität enthalten. Ein solches Salz kann durch verschiedene dem Fachmann bekannte Methoden in die entsprechende freie Base beziehungsweise Säure überführt werden.

Wenn bei den im Folgenden beschriebenen Synthese-Intermediaten und Ausführungsbeispielen der Erfindung eine Verbindung in der Form eines Salzes der korrespondierenden Base beziehungsweise Säure aufgeführt ist, so ist die exakte stöchiometrische Zusammensetzung eines solchen Salzes, wie es nach dem jeweiligen Herstell- und/oder Reinigungsverfahren erhalten wurde, in der Regel nicht bekannt. Sofern nicht genauer spezifiziert, sind daher Namens- und Strukturformel-Zusätze wie beispielsweise "Hydrochlorid", "Trifluoracetat", "Natrium-Salz" bzw. "x HCl", "x CF₃COOH", "x Na⁺" bei solchen Salzen nicht stöchiometrisch zu verstehen, sondern haben allein deskriptiven Charakter bezüglich der enthaltenen salzbildenden Komponenten.

Sinngemäß gleiches gilt für den Fall, dass Synthese-Intermediate oder Ausführungsbeispiele oder Salze hiervon nach den beschriebenen Herstell- und/oder Reinigungsverfahren in Form von Solvaten, wie beispielsweise Hydraten, erhalten wurden, deren stöchiometrische Zusammensetzung (sofern definierter Art) nicht bekannt ist.

### Ausgangsverbindungen

### Allgemeine Methode 1A: Darstellung einer Boronsäure

Eine Lösung des entsprechenden Pyridinderivates in Tetrahydrofuran (ca. 3 ml/mmol) wurde bei -78°C mit Lithiumdiisopropylamid (2M in Tetrahydrofuran/Heptan/Ethylbenzol) versetzt, 2-4 h gerührt und anschließend zügig mit Triisopropylborat versetzt. Das Reaktionsgemisch wurde für weitere 2-3 h bei -78°C gehalten und anschließend langsam über Nacht auf RT aufgetaut. Nach Zugabe von Wasser wurde das Tetrahydrofuran im Vakuum entfernt und die wässrige Phase zweimal mit Essigsäureethylester extrahiert. Die wässrige Phase wurde mit wässriger Salzsäure (2M) angesäuert, wobei in der Regel ein Niederschlag ausfiel, der filtriert, mit Wasser gewaschen und getrocknet wurde. Die wässrige Phase wurde dreimal mit Essigsäureethylester extrahiert. Die vereinigten, organischen Phasen wurden getrocknet (Natrium- oder Magnesiumsulfat), filtriert und im Vakuum eingeengt.

### Allgemeine Methode 2A: Suzuki-Kupplung

In einem ausgeheizten, mit Argon gespülten Kolben wurden 1.0 eq. der entsprechenden Boronsäuren, 1.0 eq. des Arylbromides oder Aryliodides, 3.0 eq. Kaliumcarbonat und 0.1 eq. [1,1-Bis-(diphenylphosphino)-ferrocen]-palladium(II)chlorid-Monodichlormethan-addukt oder Tetrakis(triphenylphosphin)palladium(0) vorgelegt. Der Kolben wurde anschließend dreimal evakuiert und immer wieder mit Argon belüftet. Das Reaktionsgemisch wurde mit Dioxan (ca. 6 ml/mmol) versetzt und für mehrere Stunden bis zur weitgehend vollständigen Umsetzung bei 110°C gerührt. Das Reaktionsgemisch wurde anschließend über Celite filtriert, das Filtrat im Vakuum eingeengt. Der Rückstand wurde mit Wasser versetzt. Nach Zugabe von Essigsäureethylester und Phasentrennung wurde die organische Phase einmal mit Wasser und einmal mit gesättigter, wässriger Natriumchlorid-Lösung gewaschen, getrocknet (Natrium- oder Magnesiumsulfat), filtriert und im Vakuum eingeengt. Das Rohprodukt wurde anschließend entweder mittels Normalphasen-Chromatographie (Eluent: Cyclohexan-Essigsäureethylester-Gemische oder Dichlormethan-Methanol-Gemische) oder präparativer RP-HPLC (Wasser-Acetonitril-Gradient oder Wasser-Methanol-Gradient) aufgereinigt.

### Allgemeine Methode 3A: Methoxypyridin Spaltung

Eine Lösung des entsprechenden Methoxypyridins in Dimethylformamid (10-12.5 ml/mmol) wurde mit 20 eq. Pyridinium-Hydrochlorid oder Pyridinium-Hydrobromid versetzt und bei 100°C für mehrere Stunden bis Tage gerührt, eventuell mit weiterem Pyridinium-Hydrochlorid oder Pyridinium-Hydrobromid versetzt, bis zur weitgehend vollständigen Umsetzung. Anschließend wurde die Reaktionslösung im Vakuum eingeengt und der Rückstand mit Wasser verrührt. Der anfallende Niederschlag wurde filtriert, mit Wasser gewaschen und im Vakuum getrocknet.

### Allgemeine Methode 4A: N-Alkylierung von 2-Pyridinon-Derivaten mit den entsprechenden 2-Brom- oder 2-Chlorpropansäurederivaten

Eine Suspension von 1.0 eq. des entsprechenden 2-Pyridinon-Derivates, 2.0 eq. Magnesiumdi-*tert*.-butylat und 1.05 eq. Kalium-*tert.*-butylat in Tetrahydrofuran (5-10 ml/mmol) wurde unter Argon 10-20 min bei RT gerührt. Das Reaktionsgemisch wurde im Eisbad gekühlt und mit 1.5 eq. des entsprechenden 2-Brom- oder 2-Chlorpropansäurederivates versetzt. Anschließend wurde das Reaktionsgemisch zunächst 2.5 h bei RT und anschließend über Nacht bei 35-90°C nachgerührt und mit wässriger Salzsäure (6N) versetzt. Nach Zugabe von Essigsäureethylester und Phasentrennung wurde die organische Phase einmal mit Wasser und einmal mit gesättigter, wässriger Natriumchlorid-Lösung gewaschen, getrocknet (Natrium- oder Magnesiumsulfat), filtriert und im Vakuum eingeengt. Das Rohprodukt wurde anschließend entweder mittels Normalphasen-Chromatographie (Eluent: Cyclohexan-Essigsäureethylester-Gemische oder Dichlormethan-Methanol-Gemische) oder präparativer RP-HPLC (Wasser-Acetonitril-Gradient oder Wasser-Methanol-Gradient) aufgereinigt.

### Allgemeine Methode 4B: N-Alkylierung von 2-Pyridinon-Derivaten mit den entsprechenden 2-Brom- oder 2-Chlorpropansäureesterderivaten in Gegenwart von Kaliumcarbonat

Eine Lösung von 1.0 eq. des entsprechenden 2-Pyridinon-Derivates in Dimethylformamid (5-10 ml/mmol) wurde unter Argon bei RT mit 1.2 eq. des entsprechenden 2-Brom- oder 2-Chlorpropansäureesterderivates und 1.5 eq. Kaliumcarbonat versetzt und bei 100°C gerührt. Nach Entfernen des Dimethylformamids und Zugabe von Wasser/Essigsäureethylester und Phasentrennung wurde die organische Phase mit Wasser und mit gesättigter, wässriger Natriumchlorid-Lösung gewaschen, getrocknet (Natrium- oder Magnesiumsulfat), filtriert und im Vakuum eingeengt. Das Rohprodukt wurde anschließend entweder mittels Normalphasen-Chromatographie (Eluent: Cyclohexan-Essigsäureethylester-Gemische oder Dichlormethan-Methanol-Gemische) oder präparativer RP-HPLC (Wasser-Acetonitril-Gradient oder Wasser-Methanol-Gradient) aufgereinigt.

### Allgemeine Methode 4C: N-Alkylierung von 2-Pyridinon-Derivaten mit den entsprechenden Triflaten in Gegenwart von Natriumhydrid

Eine Lösung des entsprechenden 2-Pyridinon-Derivates (1 eq.) in Tetrahydrofuran (0.05-0.2M) wurde unter Argon bei RT mit Natriumhydrid (1.1-1.5 eq.) versetzt und 30-90 min gerührt.

Anschließend wurde das entsprechende Triflat (1.0-2.0 eq.) als Reinsubstanz oder Lösung in Tetrahydrofuran hinzugegeben. Die resultierende Reaktionsmischung wurde 1-5 h bei RT nachgerührt. Das Reaktionsgemisch wurde mit gesättigter, wässriger Ammoniumchlorid-Lösung versetzt. Nach Phasentrennung wurde die wässrige Phase mit Essigsäureethylester extrahiert. Die vereinigten, organischen Phasen wurden getrocknet (Natriumsulfat oder Magnesiumsulfat), filtriert und unter reduziertem Druck eingeengt. Das Rohprodukt wurde anschließend entweder mittels Normalphasen-Chromatographie (Eluent: Cyclohexan-Essigsäureethylester-Gemische oder Dichlormethan-Methanol-Gemische) oder präparativer RP-HPLC (Wasser-Acetonitril-Gradient oder Wasser-Methanol-Gradient) aufgereinigt.

### Allgemeine Methode 5A: Amid-Kupplung mit HATU/DIEA

Eine Lösung der entsprechenden Carbonsäure (1.0 eq.) in Dimethylformamid (7-15 ml/mmol) wurde unter Argon bei RT mit dem Amin (1.1 eq.), mit *N,N*-Dïsopropylethylamin (2.2 eq.) und einer Lösung von HATU (1.2 eq.) in etwas Dimethylformamid versetzt. Das Reaktionsgemisch wurde bei RT gerührt. Nach Zugabe von Wasser/Essigsäureethylester und Phasentrennung wurde die organische Phase mit Wasser und mit gesättigter, wässriger Natriumchlorid-Lösung gewaschen, getrocknet (Natriumsulfat oder Magnesiumsulfat), filtriert und im Vakuum eingeengt. Das Rohprodukt wurde anschließend entweder mittels Normalphasen-Chromatographie (Eluent: Cyclohexan-Essigsäureethylester-Gemische oder Dichlormethan-Methanol-Gemische) oder präparativer RP-HPLC (Wasser-Acetonitril-Gradient oder Wasser-Methanol-Gradient) aufgereinigt.

### Allgemeine Methode 5B: Amid-Kupplung mit OXIMA/DIC

Zu einer entgasten Lösung der entsprechenden Carbonsäure (1 eq.), Anilin (1 eq.) und Hydroxyiminocyanoessigsäureethylester (Oxima) (1 eq.) in Dimethylformamid (0.1M) wurde tropfenweise *N,N'*-Dïsopropylcarbodiimid (DIC) (1 eq.) gegeben und die resultierende Reaktionslösung für 8-24 h bei RT bis 40°C gerührt. Das Lösungsmittel wurde unter reduziertem Druck entfernt. Der Rückstand wurde entweder mit Wasser versetzt und das gewünschte Produkt filtriert oder mittels Normalphasen-Chromatographie (Cyclohexan/Essigsäureethylester-Gradient) oder präparativer RP-HPLC (Wasser-Acetonitril-Gradient oder Wasser-Methanol-Gradient) aufgereinigt.

### Allgemeine Methode 5C: Amid-Kupplung mit T3P/DIEA

Eine Lösung der Carbonsäure und des entsprechenden Amins (1.1-1.5 eq.) in Dimethylformamid (0.15-0.05 mmol) wurde unter Argon bei 0°C tropfenweise mit *N,N*-Diisopropylethylamin (3 eq.) und Propylphosphonsäureanhydrid (T3P, 50%ig in Dimethylformamid, 3 eq.) versetzt. Das Reaktionsgemisch wurde bei RT gerührt und anschließend im Vakuum eingeengt. Nach Zugabe von Wasser/Essigsäureethylester und Phasentrennung wurde die wässrige Phase zweimal mit Essigsäureethylester extrahiert. Die vereinigten, organischen Phasen wurden getrocknet (Natriumsulfat oder Magnesiumsulfat), filtriert und im Vakuum eingeengt. Das Rohprodukt wurde anschließend entweder mittels Flash-Chromatographie (Kieselgel-60, Eluent: Cyclohexan-Essigsäureethylester-Gemische oder Dichlormethan-Methanol-Gemische) oder präparativer HPLC (Reprosil C18, Wasser-Acetonitril-Gradient oder Wasser-Methanol-Gradient) aufgereinigt.

### Allgemeine Methode 5D: Amid-Kupplung mit T3P/Pyridin

Eine Lösung der entsprechenden Carbonsäure (1 eq.) und des entsprechenden Amins (1.1-1.5 eq.) in Pyridin (ca. 0.1M) wurde auf 60°C erwärmt und tropfenweise mit T3P (50%ig in Essigsäureethylester, 15 eq.) versetzt. Alternativ wurde T3P bei RT hinzugefügt und danach bei RT gerührt oder auf 60 bis 90°C erhitzt. Nach 1-20 h wurde das Reaktionsgemisch auf RT gekühlt und mit Wasser und Essigsäureethylester versetzt. Die wässrige Phase wurde mit Essigsäureethylester extrahiert. Die vereinigten organischen Phasen wurden mit wässriger PufferLösung (pH=5), mit gesättigter, wässriger Natriumhydrogencarbonat-Lösung und mit gesättigter, wässriger Natriumchlorid-Lösung gewaschen, über Natriumsulfat getrocknet und im Vakuum eingeengt. Das Rohprodukt wurde gegebenenfalls anschließend entweder mittels Normalphasen-Chromatographie (Eluent: Cyclohexan-Essigsäureethylester-Gemische oder Dichlormethan-Methanol-Gemische) oder präparativer RP-HPLC (Wasser-Acetonitril-Gradient oder Wasser-Methanol-Gradient) aufgereinigt.

### Allgemeine Methode 6A: Verseifung eines tert.-Butylesters oder eines Boc-geschützten Amins mittels TFA

Eine Lösung von 1.0 eq. des entsprechenden *tert*.-Butylesterderivates in Dichlormethan (ca. 5-10 ml/mmol) wurde bei RT mit 20 eq. TFA versetzt und bei RT für 1-8 h gerührt. Anschließend wurde das Reaktionsgemisch im Vakuum eingeengt, der Rückstand mehrmals mit Dichlormethan und Toluol coevaporiert und im Vakuum getrocknet. Das Rohprodukt wurde gegebenenfalls anschließend entweder mittels Normalphasen-Chromatographie (Eluent: Cyclohexan-Essigsäureethylester-Gemische oder Dichlormethan-Methanol-Gemische) oder präparativer RP-HPLC (Wasser-Acetonitril-Gradient oder Wasser-Methanol-Gradient) aufgereinigt.

### Allgemeine Methode 6B: Verseifung eines Methyl-/Ethyl- oder Benzylesters mit Lithiumhydroxid

Eine Lösung von 1.0 eq. des entsprechenden Methyl- oder Ethylesters in Tetrahydrofuran/Wasser (3:1, ca. 7-15 ml/mmol) wurde bei RT mit Lithiumhydroxid (2-4 eq.) versetzt. Das Reaktionsgemisch wurde bei RT bis 60°C gerührt und anschließend mit wässriger Salzsäure (1N) auf pH 1 gestellt. Nach Zugabe von Wasser/Essigsäureethylester und Phasentrennung wurde die wässrige Phase dreimal mit Essigsäureethylester extrahiert. Die vereinigten, organischen Phasen wurden getrocknet (Natriumsulfat oder Magnesiumsulfat), filtriert und im Vakuum eingeengt. Das Rohprodukt wurde anschließend entweder mittels Normalphasen-Chromatographie (Eluent: Cyclohexan-Essigsäureethylester-Gemische oder Dichlormethan-Methanol-Gemische) oder präparativer RP-HPLC (Wasser-Acetonitril-Gradient oder Wasser-Methanol-Gradient) aufgereinigt.

### Allgemeine Methode 7A: Darstellung von Triflaten

Eine Lösung des entsprechenden Alkohols (1 eq.) wurde in Dichlormethan (0.1M) vorgelegt und bei -20°C nacheinander mit Lutidin (1.1-1.5 eq.) oder Triethylamin (1.1-1.5 eq.) und mit Trifluormethansulfonsäureanhydrid (1.05-1.5 eq.) versetzt. Das Reaktionsgemisch wurde für 1h bei -20°C nachgerührt und anschließend mit der dreifachen Menge (bezogen aufs Reaktionsvolumen) Methyl-*tert*.-butylether verdünnt. Die organische Phase wurde dreimal mit einer 3:1-Mischung aus gesättigter, wässriger Natriumchlorid-Lösung/1N Salzsäure und abschließend mit gesättigter, wässriger Natriumhydrogencarbonat-Lösung gewaschen, getrocknet (Natrium- oder Magnesiumsulfat), filtriert und das Lösungsmittel unter reduziertem Druck entfernt. Das Rohprodukt wurde ohne weitere Aufreinigung im nächsten Schritt eingesetzt.

### Allgemeine Methode 8A: Alkylierung von Essigsäureestern mit Triflaten

Eine Lösung des entsprechenden Essigsäureesters (1 eq.) in Tetrahydrofuran (0.1-0.2M) wurde unter Argon bei -78°C tropfenweise mit Bis-(trimethylsilyl)-lithiumamid (1.0M in THF, 1.1-1.3 eq.) versetzt und 15 min gerührt. Anschließend wurde das entsprechende Alkyltriflat (1.5-2.0 eq.) als Reinsubstanz oder Lösung in THF hinzugegeben. Die resultierende Reaktionsmischung wurde 15 min bei -78°C und 1h bei RT nachgerührt. Das Reaktionsgemisch wurde mit gesättigter, wässriger Ammoniumchlorid-Lösung versetzt. Nach Phasentrennung wurde die wässrige Phase mit Essigsäureethylester extrahiert. Die vereinigten, organischen Phasen wurden getrocknet (Natriumsulfat oder Magnesiumsulfat), filtriert und unter reduziertem Druck eingeengt. Das Rohprodukt wurde anschließend entweder mittels Normalphasen-Chromatographie (Eluent: Cyclohexan-Essigsäureethylester-Gemische oder Dichlormethan-Methanol-Gemische) oder präparativer RP-HPLC (Wasser-Acetonitril-Gradient oder Wasser-Methanol-Gradient) aufgereinigt.

### Allgemeine Methode 8B: Alkylierung von Essigsäureestern mit Halogeniden

Eine Lösung des entsprechenden Essigsäureesters in THF (ca. 10 ml/mmol) wurde unter Argon bei -78°C mit 1.1 eq. Bis-(trimethylsilyl)-lithiumamid (1.0M in THF) versetzt und 10 min bei -78°C gerührt. Anschließend wurde das Reaktionsgemisch mit einer Lösung des entsprechenden Iodids/Bromids/Chlorids in THF versetzt, 10 min bei -78°C und weiter im Eisbad gerührt und anschließend mit Wasser gequencht. Nach Zugabe von Essigsäureethylester und Phasentrennung wurde die wässrige Phase zweimal mit Essigsäureethylester extrahiert. Die vereinigten, organischen Phasen wurden getrocknet (Natriumsulfat), filtriert und im Vakuum eingeengt. Das Rohprodukt wurde anschließend entweder mittels Flash-Chromatographie (Kieselgel-60, Eluent: Cyclohexan-Essigsäureethylester-Gemische oder Dichlormethan-Methanol-Gemische) oder präparativer HPLC (Reprosil C18, Wasser-Acetonitril-Gradient oder Wasser-Methanol-Gradient) aufgereinigt.

### Beispiel 11A

### 6-Aminoimidazo[1,2-a]pyridin-2-carbonsäureethylester

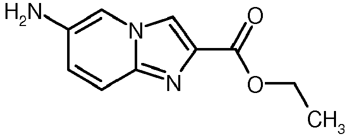

Eine Lösung von 250 mg (1.01 mmol) 6-Nitroimidazo[1,2-a]pyridin-2-carbonsäureethylester in 20 ml Ethanol wurde in Gegenwart von 30 mg Palladium (10%ig auf Aktivkohle) 5 h bei RT und Normaldruck hydriert. Anschließend wurde das Reaktionsgemisch über Celite filtriert und der Rückstand mit Ethanol gewaschen. Die vereinigten Filtrate wurden im Vakuum eingeengt und getrocknet. Ausbeute: 215 mg (quant.)
LC/MS [Methode 5]: Rₜ = 1.40 min; MS (ESIpos): m/z = 206 (M+H)⁺,
¹H-NMR (400 MHz, DMSO-d₆): δ [ppm] = 8.33 (s, 1H), 7.66 (s, 1H), 7.37 (d, 1H), 6.94 (dd, 1H), 5.11 (s, 2H), 4.26 (q, 2H), 1.29 (t, 3H).

### Beispiel 12A

### 7-Nitroimidazo[1,2-a]pyridin-2-carbonsäureethylester

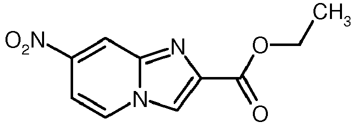

Eine Suspension von 500 mg (2.41 mmol) 7-Nitroimidazo[1,2-a]pyridin-2-carbonsäure in 20 ml Dimethylformamid wurde unter Argon bei RT mit 434 mg (3.14 mmol, 1.1 eq.) Kaliumcarbonat, 212 µl (2.66 mmol, 1.1 eq.) Iodethan und 5 ml Tetrahydrofuran (zur Verbesserung der Rührbarkeit) versetzt und über Nacht bei RT gerührt. Nach Zugabe von weiteren 35 µl (0.48 mmol, 0.2 eq.) Iodethan und Rühren bei RT für weitere 2 d wurde das Reaktionsgemisch im Vakuum eingeengt. Der Rückstand wurde mit Wasser versetzt, filtriert und im Vakuum getrocknet. Ausbeute: 273 mg (48% d. Th.)
LC/MS [Methode 1]: Rₜ = 0.71 min; MS (ESIpos): m/z = 236 (M+H)⁺.

### Beispiel 1.2B

### 7-Aminoimidazo[1,2-a]pyridin-2-carbonsäureethylester

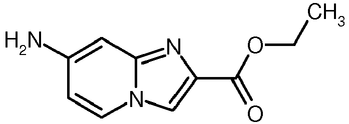

Eine Lösung von 273 mg (1.16 mmol) 7-Nitroimidazo[1,2-a]pyridin-2-carbonsäureethylester in 10 ml Ethanol wurde in Gegenwart von 30 mg Palladium (10%ig auf Aktivkohle) über Nacht bei RT und Normaldruck hydriert. Anschließend wurde das Reaktionsgemisch über Celite filtriert und der Rückstand mit Ethanol gewaschen. Die vereinigten Filtrate wurden im Vakuum eingeengt und getrocknet. Ausbeute: 214 mg (90% d. Th.)
LC/MS [Methode 5]: Rₜ = 1.45 min; MS (ESIpos): m/z = 206 (M+H)⁺,
¹H-NMR (400 MHz, DMSO-d₆): δ [ppm] = 8.16 (d, 1H), 8.14 (s, 1H), 6.46 (dd, 1H), 6.32 (d, 1H), 5.84 (s, 2H), 4.24 (q, 2H), 1.28 (t, 3H).

### Beispiel 13A

### Imidazo [1,2-a]pyridin-6-amin

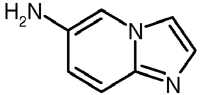

Eine Lösung von 600 mg (3.68 mmol) 6-Nitroimidazo[1,2-a]pyridin in 30 ml Ethanol wurde in Gegenwart von 60 mg Palladium (10%ig auf Aktivkohle) über Nacht bei RT und Normaldruck hydriert. Anschließend wurde das Reaktionsgemisch über Celite filtriert und der Rückstand mit Ethanol gewaschen. Die vereinigten Filtrate wurden im Vakuum eingeengt und getrocknet. Das Rohprodukt wurde ohne weitere Reinigung in der nächsten Stufe eingesetzt. Ausbeute: 512 mg (quant.)
LC/MS [Methode 5]: Rₜ = 0.89 min; MS (ESIpos): m/z = 134 (M+H)⁺,
¹H-NMR (400 MHz, DMSO-d₆): δ [ppm] = 7.72-7.62 (m, 2H), 7.33 (d, 1H), 7.30 (d, 1H), 6.80 (dd, 1H), 4.83 (s, 2H).

### Beispiel 1.4A

### 6-Nitroimidazo[1,2-a]pyridin-3-carbonsäureethylester

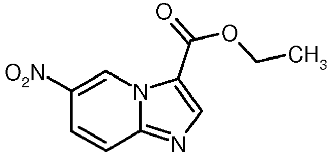

3.00 g (21.6 mmol) 2-Amino-5-nitropyridin und 13.4 g (71.2 mmol, 3.3 eq.) Kalium-(1*E*)-2-chlor-3-ethoxy-3-oxoprop-1-en-1-olat (T. Ikemoto et al., Tetrahedron 2000, 56, 7915-7921) wurden in 136 ml Ethanol gelöst und 1.91 ml Schwefelsäure vorsichtig zugesetzt. Man erhitzte 12 h auf Rückfluss, filtrierte vom Niederschlag ab und wusch mit Ethanol nach. Das Filtrat wurde im Vakuum eingeengt. Man nahm in Essigsäureethylester und Wasser auf und säuerte mit 1M Salzsäure leicht an. Die wässrige Phase wurde dann dreimal mit Essigsäureethylester extrahiert. Die vereinten organischen Phasen wurden über Magnesiumsulfat getrocknet und das Lösungsmittel im Vakuum entfernt. Man reinigte 3 g des Rohproduktes durch Flash-Chromatographie (Kieselgel-50, Eluent: Cyclohexan-Essigsäureethylester-Gemische) und erhielt 720 mg Produkt (93% Reinheit). Der Rest wurde durch präparative HPLC (XBridge C18, 5 µM, 100 mm x 30 mm, Eluent: Acetonitril/Wasser 2:3) gereinigt und ergab weitere 690 mg Produkt. Ausbeute: 720 mg (93% Reinheit, 13% d. Th.) und 690 mg (14% d. Th.)
LC/MS [Methode 5]: Rₜ = 2.12 min; MS (ESIpos): m/z = 236 (M+H)⁺,
¹H-NMR (400 MHz, DMSO-*d*₆): δ [ppm] = 10.14 (dd, 1H), 8.51 (s, 1H), 8.25 (dd, 1H), 7.98 (dd, 1H), 4.43 (q, 2H), 1.38 (t, 3H).

### Beispiel 1.4B

### 6-Aminoimidazo[1,2-a]pyridin-3-carbonsäureethylester

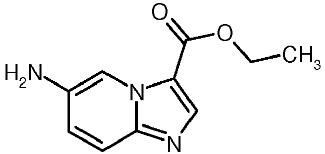

250 mg (1.06 mmol) 6-Nitroimidazo[1,2-*a*]pyridin-3-carbonsäureethylester wurden in 10 ml Ethanol vorgelegt. Man setzte 68 mg (64 µmol, 0.06 eq.) 10%iges Palladium auf Aktivkohle zu und hydrierte über Nacht unter normalem Druck. Die Reaktionslösung wurde über Kieselgur abfiltriert und im Vakuum eingeengt. Ausbeute: 217 mg (99% d. Th.)
LC/MS [Methode 1]: Rₜ = 0.33 min; MS (ESIpos): m/z = 206 (M+H)⁺,
¹H-NMR (400 MHz, DMSO-*d*₆): δ [ppm] = 8.62 (d, 1H), 8.04 (s, 1H), 7.53 (d, 1H), 7.11 (dd, 1H), 5.35 (s, 2H), 4.32 (q, 2H), 1.33 (t, 3H).

### Beispiel 1.5A

### 7-Nitroimidazo[1,2-a]pyridin-2-carboxamid

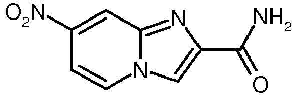

670 mg (2.85 mmol) 7-Nitroimidazo[1,2-a]pyridin-2-carbonsäureethylester wurden mit 16 ml einer methanolischen Ammoniaklösung (7N) und 30 ml Ammoniaklösung (35%ig in Wasser) versetzt. Man aliquotierte in vier Portionen und erhitzte diese in geschlossenen Gefäßen 1.5 h auf 80°C in der Mikrowelle. Danach wurden die Reaktionslösungen vereinigt, in Essigsäureethylester/Wasser aufgenommen und die wässrige Phase mit Salzsäure (1N) neutralisiert. Man extrahierte zweimal mit Essigsäureethylester, wusch die vereinten organischen Phasen mit gesättigter, wässriger Natriumchlorid-Lösung und trocknete sie über Magnesiumsulfat. Das Lösungsmittel wurde im Vakuum entfernt und das Rohprodukt durch Flash-Chromatographie (Kieselgel-50, Dichlormethan-Methanol-Gemische) gereinigt. Ausbeute 81 mg (91% Reinheit, 12% d. Th.)
LC/MS [Methode 5]: Rₜ = 1.40 min; MS (ESIpos): m/z = 207 (M+H)⁺,
¹H-NMR (400 MHz, DMSO-d₆): δ [ppm] = 9.92 (dd, 1H), 8.57 (d, 1H), 8.01 (dd, 1H), 7.87 (br. s, 1H), 7.75 (dt, 1H), 7.59 (br. s, 1H).

### Beispiel 1.5B

### 7-Aminoimidazo[1,2-a]pyridin-2-carboxamid

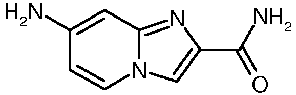

80 mg (91% Reinheit, 0.35 mmol) 7-Nitroimidazo[1,2-*a*]pyridin-2-carboxamid wurden in 15 ml Ethanol vorgelegt. Man gab 19 mg Palladium (10%ig auf Aktivkohle) zu und hydrierte 3 h bei RT und Normaldruck. Man filtrierte die Reaktionslösung durch Kieselgur und entfernte das Lösungsmittel im Vakuum. Ausbeute 50 mg (90% Reinheit, 73% d. Th.)
LC/MS [Methode 5]: Rₜ = 0.95 min; MS (ESIpos): m/z = 177 (M+H)⁺,
¹H-NMR (400 MHz, DMSO-d₆): δ [ppm] = 8.10 (d, 1H), 7.68 (dd, 1H), 7.48 (br. s, 1H), 7.33 (d, 1H), 7.18 (br. s, 1H), 6.92 (dd, 1H), 5.02 (s, 2H).

### Beispiel 1_{.}6A

### 2-(4-Fluorphenyl)-6-nitroimidazo[1,2-a]pyridin

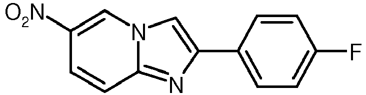

1.00 g (7.19 mmol) 2-Amino-5-nitropyridin und 1.56 g (7.19 mmol) 2-Brom-1-(4-fluorphenyl)ethanon wurden mit 80.6 mg (0.72 mmol, 0.1 eq.) DABCO und 36 ml Wasser versetzt. Man rührte 2 h bei 65°C und nach Rühren über Nacht bei RT weitere 6 h bei 65°C. Nach 48 h bei RT wurde der ausgefallene Niederschlag abgesaugt und mit Methyl-*tert*.-butylether verrührt und abgesaugt. Ausbeute: 576 mg (92% Reinheit, 29% d. Th.)
LC/MS [Methode 1]: Rₜ = 0.93 min; MS (ESIpos): m/z = 258 (M+H)⁺,
¹H-NMR (400 MHz, DMSO-*d*₆): δ [ppm] = 9.84 (d, 1H), 8.60 (s, 1H), 8.07-8.01 (m, 2H), 7.96 (dd, 1H), 7.74 (d, 1H), 7.36-7.29 (m, 2H).

### Beispiel 1.6B

### 2-(4-Fluorphenyl)imidazo[1,2-a]pyridin-6-amin

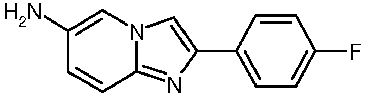

450 mg (92% Reinheit, 1.61 mmol) 2-(4-Fluorphenyl)-6-nitroimidazo[1,2-*a*]pyridin wurden in 20 ml Ethanol vorgelegt. Man setzte 171 mg (161 µmol, 0.1 eq.) 10%iges Palladium auf Aktivkohle zu und hydrierte über Nacht unter normalem Druck. Die Reaktionslösung wurde über Kieselgur abfiltriert und im Vakuum eingeengt. Ein analoger Ansatz wurde mit 100 mg Ausgangsmaterial durchgeführt. Die Produkte wurden vereinigt und mit Methyl-*tert*.-butylether verrührt und abgesaugt. Ausbeute: 395 mg (80% Reinheit, 71% d. Th.)
LC/MS [Methode 1]: Rₜ = 0.46 min; MS (ESIpos): m/z = 228 (M+H)⁺,
¹H-NMR (400 MHz, DMSO-*d*₆): δ [ppm] = 8.16 (s, 1H), 7.91 (dd, 2H), 7.67 (d, 1H), 7.33 (d, 1H), 7.22 (t, 2H), 6.85 (dd, 1H), 4.95 (br. s, 2H).

### Beispiel 1.7A

### 6-Nitro[1,2,4]triazolo[4,3-a]pyridin

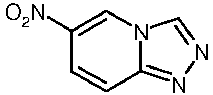

2.00 g (13.0 mmol) 2-Hydrazino-5-nitropyridin wurden in 80 ml Dichlormethan vorgelegt und 5.51 g (51.9 mmol) Trimethylorthoformiat zugesetzt. Man ließ 15 min bei RT rühren. Anschließend wurde 1.00 ml (13.0 mmol) Trifluoressigsäure zugegeben und weitere 30 min gerührt. Danach entfernte man flüchtige Bestandteile im Vakuum und reinigte durch Flash-Chromatographie (Kieselgel-50, Cyclohexan-Essigsäureethylester-Gemische). Ausbeute: 896 mg (42% d. Th.)
LC/MS [Methode 1]: Rₜ = 0.41 min; MS (ESIpos): m/z = 165 (M+H)⁺,
¹H-NMR (400 MHz, DMSO-d₆): δ [ppm] = 10.2 (dd, 1H), 8.82 (s, 1H), 8.39 (d, 1H), 8.37 (d, 1H), 8.04 (dd, 2H).

### Beispiel 1.7B

### [1,2,4]Triazolo[4,3-a]pyridin-6-amin

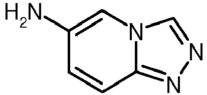

Eine Lösung von 890 mg (5.42 mmol) 6-Nitro[1,2,4]triazolo[4,3-*a*]pyridin in 60 ml Ethanol wurde in Gegenwart von 577 mg Palladium (10%ig auf Aktivkohle) 6 h bei RT und Normaldruck hydriert. Anschließend wurde das Reaktionsgemisch über Celite filtriert, nochmals mit der gleichen Menge Palladium-Katalysator versetzt und weitere 2 h hydriert. Das Reaktionsgemisch wurde nach Filtration über Celite eingeengt, der Rückstand mit Pentan / Methyl-*tert*.-butylether kristallisiert und der Feststoff abgesaugt. Ausbeute: 469 mg (65% d. Th.)
LC/MS [Methode 5]: Rₜ = 0.62 min; MS (ESIpos): m/z = 135 (M+H)⁺,
¹H-NMR (400 MHz, DMSO-d₆): δ [ppm] = 8.17 (s, 1H), 8.02 (dd, 1H), 7.56 (dd, 1H), 7.19 (dd, 1H), 5.24 (br. s, 2H).

### Beispiel 1.8A

### 3-Methyl-6-nitro[1,2,4]triazolo[4,3-a]pyridin

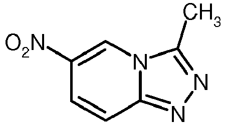

2.00 g (13.0 mmol) 2-Hydrazino-5-nitropyridin wurden in 50 ml Ethanol vorgelegt und 25 ml (195 mmol, 15 eq.) Trimethylorthoacetat zugesetzt. Man erhitzte 1h auf Rückfluss. Die Reaktionsmischung wurde dann im Vakuum eingeengt. Man reinigte das Rohprodukt durch Flash-Chromatographie (Kieselgel-50, Cyclohexan-Essigsäureethylester-Gemische). Ausbeute: 1.81 g (78% d. Th.)
LC/MS [Methode 5]: Rₜ = 1.30 min; MS (ESIpos): m/z = 179 (M+H)⁺,
¹H-NMR (400 MHz, DMSO-*d*₆): δ [ppm] = 9.57 (dd, 1H), 7.98 (dd, 1H), 7.87 (d, 1H), 2.81 (s, 3H).

### Beispiel 1.8B

### 3-Methyl[1,2,4]triazolo[4,3-a]pyridin-6-amin

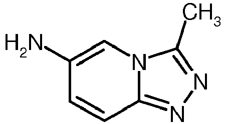

Eine Suspension von 200 mg (1.12 mmol) 3-Methyl-6-nitro[1,2,4]triazolo[4,3-*a*]pyridin in 10 ml Ethanol wurde mit 1.27 g (5.61 mmol, 5.0 eq.) Zinn(II)-chlorid-Dihydrat versetzt und 12 h auf Rückfluss erhitzt. Man versetzte die Reaktionslösung dann mit gesättigter, wässriger Natriumhydrogencarbonat-Lösung und extrahierte dreimal mit Essigsäureethylester. Die vereinten organischen Phasen wurden über Magnesiumsulfat getrocknet und im Vakuum eingeengt. Ausbeute: 83 mg (74% Reinheit, 37% d. Th.)
LC/MS [Methode 5]: Rₜ = 0.94 min; MS (ESIpos): m/z = 149 (M+H)⁺.

### Beispiel 1.9A

### 3-Ethyl-6-nitro[1,2,4]triazolo[4,3-a]pyridin

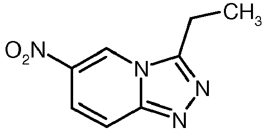

2.00 g (13.0 mmol) 2-Hydrazino-5-nitropyridin wurden in 50 ml Ethanol vorgelegt und 27 ml (195 mmol, 15 eq.) Trimethylorthopropionat zugesetzt. Man erhitzte 1h auf Rückfluss. Die Reaktionsmischung wurde dann im Vakuum eingeengt. Man reinigte das Rohprodukt durch Flash-Chromatographie (Kieselgel-50, Cyclohexan-Essigsäureethylester-, dann Essigsäureethylester-Propanol-Gemische). Ausbeute: 2.37 g (95% d. Th.)
LC/MS [Methode 5]: Rₜ = 1.42 min; MS (ESIpos): m/z = 193 (M+H)⁺,
¹H-NMR (400 MHz, DMSO-*d*₆): δ [ppm] = 9.58 (dd, 1H), 7.98 (dd, 1H), 7.88 (dd, 1H), 3.22 (q, 3H), 1.40 (t, 4H).

### Beispiel 1.9B

### 3-Ethyl[1,2,4]triazolo[4,3-a]pyridin-6-amin

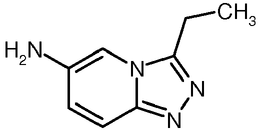

1.00 g (5.20 mmol) 3-Ethyl-6-nitro[1,2,4]triazolo[4,3-*a*]pyridin wurde in 60 ml Ethanol vorgelegt. Man setzte 554 mg (0.52 mmol) 10%iges Palladium auf Aktivkohle zu und hydrierte 4 h unter normalem Druck. Die Reaktionslösung wurde über Kieselgur abfiltriert und im Vakuum eingeengt. Dann reinigte man das Rohprodukt durch Flash-Chromatographie (Kieselgel-50, Dichlormethan-Methanol-Gemische). Ausbeute: 600 mg (69% d. Th.)
LC/MS [Methode 5]: Rₜ = 1.07 min; MS (ESIpos): m/z = 163 (M+H)⁺,
¹H-NMR (400 MHz, DMSO-*d*₆): δ [ppm] = 7.50 (dd, 1H), 7.35 (dd, 1H), 6.96 (dd, 1H), 5.11 (s, 2H), 2.92 (d, 2H), 1.32 (t, 3H).

### Beispiel 1.10A

### 3-Butyl-6-nitro[1,2,4]triazolo[4,3-a]pyridin

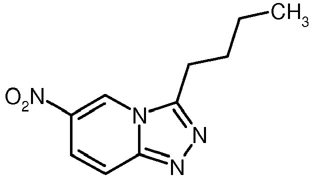

1.00 g (6.49 mmol) 2-Hydrazino-5-nitropyridin wurden in 13 ml Ethanol vorgelegt und 2.2 ml (13 mmol, 2 eq.) Trimethylorthovalerat zugesetzt. Man erhitzte 1h auf Rückfluss. Die Reaktionsmischung wurde dann im Vakuum eingeengt. Man reinigte das Rohprodukt durch Flash-Chromatographie (Kieselgel-50, Cyclohexan-Essigsäureethylester-, dann Essigsäureethylester-2-Propanol-Gemische). Ausbeute: 1.47 g (99% d. Th.)
LC/MS [Methode 5]: Rₜ = 1.86 min; MS (ESIpos): m/z = 221 (M+H)⁺,
¹H-NMR (400 MHz, DMSO-*d*₆): δ [ppm] = 9.62 (dd, 1H), 7.98 (dd, 1H), 7.87 (dd, 1H), 3.22 (t, 2H), 1.81 (quin, 2H), 1.44 (tq, 2H), 0.95 (t, 3H).

### Beispiel 1.10B

### 3-Butyl[1,2,4]triazolo[4,3-a]pyridin-6-amin

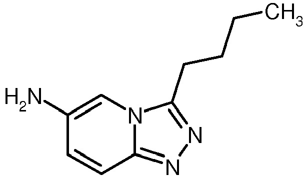

1.20 g (5.45 mmol) 3-Butyl-6-nitro[1,2,4]triazolo[4,3-*a*]pyridin wurden in 65 ml Ethanol vorgelegt. Man setzte 580 mg (0.55 mmol) 10%iges Palladium auf Aktivkohle zu und hydrierte über Nacht unter normalem Druck. Die Reaktionslösung wurde über Kieselgur abfiltriert und im Vakuum eingeengt. Dann reinigte man das Rohprodukt durch Flash-Chromatographie (Kieselgel-50, Dichlormethan-Methanol-Gemische). Ausbeute: 86 mg (85% Reinheit, 7% d. Th.)
LC/MS [Methode 5]: Rₜ = 1.64 min; MS (ESIpos): m/z = 191 (M+H)⁺,
¹H-NMR (400 MHz, DMSO-*d*₆): δ [ppm] = 7.93 (d, 1H), 7.42 (d, 1H), 7.12 (dd, 1H), 5.12 (s, 2H), 2.69 (t, 2H), 1.69 (quin, 2H), 1.34 (tq, 2H), 0.89 (t, 3H).

### Beispiel 1.11A

### 3-(Chlormethyl)-6-nitro [1,2,4] triazolo [4,3-a]pyridin

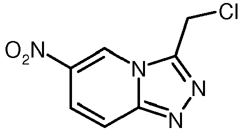

10.0 g (64.9 mmol) 2-Hydrazino-5-nitropyridin wurden in 125 ml Ethanol vorgelegt und 17.5 ml (130 mmol, 2 eq.) 2-Chlor-1,1,1-trimethoxyethan zugesetzt. Man erhitzte 1h auf Rückfluss. Die Reaktionsmischung wurde dann im Vakuum eingeengt. Man reinigte das Rohprodukt durch Flash-Chromatographie (Kieselgel-50, Cyclohexan-Essigsäureethylester-, dann Essigsäureethylester-2-Propanol-Gemische). Ausbeute: 13.1 g (95% d. Th.)
LC/MS [Methode 1]: Rₜ = 0.52 min; MS (ESIpos): m/z = 213 (M+H)⁺,
¹H-NMR (400 MHz, DMSO-*d*₆): δ [ppm] = 9.84 (dd, 1H), 8.12 (dd, 1H), 8.03 (dd, 1H), 5.57 (s, 2H).

### Beispiel 1.11B

### N,N-Dimethyl-1-(6-nitro[1,2,4]triazolo[4,3-a]pyridin-3-yl)methanamin

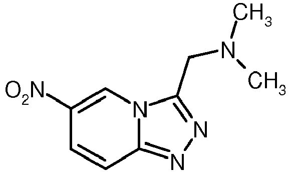

200 mg (0.94 mmol) 3-(Chlormethyl)-6-nitro[1,2,4]triazolo[4,3-*a*]pyridin wurden in 2.9 ml 33%iger Dimethylamin-Lösung in Ethanol gelöst und 4 h bei RT gerührt. Man filtrierte dann den ausgefallenen Feststoff ab und trocknete ihn im Vakuum. Ausbeute: 148 mg (61% d. Th.)
LC/MS [Methode 5]: Rₜ = 1.54 min; MS (ESIpos): m/z = 222 (M+H)⁺,
¹H-NMR (400 MHz, DMSO-*d*₆): δ [ppm] = 10.10 (dd, 1H), 8.35 (dd, 1H), 7.95 (dd, 1H), 3.74 (s, 3H), 2.27 (s, 6H).

### Beispiel 1.11C

### 3-[(Dimethylamino)methyl][1,2,4]triazolo[4,3-a]pyridin-6-amin

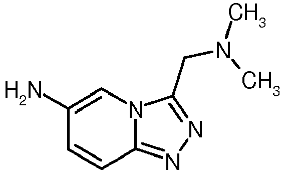

145 mg (0.66 mmol) N,N-Dimethyl-1-(6-nitro[1,2,4]triazolo[4,3-*a*]pyridin-3-yl)methanamin wurden in 10.7 ml Ethanol vorgelegt. Man fügte 15 mg (56 µmol, 0.1 eq., 83%ig) Platin(IV)dioxid hinzu und hydrierte 4 h unter normalem Druck. Danach wurde über Kieselgur abfiltriert und das Filtrat im Vakuum vorsichtig eingeengt. Ausbeute: 115 mg (92% d. Th.)
LC/MS [Methode 5]: Rₜ = 1.19 min; MS (ESIpos): m/z = 192 (M+H)⁺,
¹H-NMR (400 MHz, DMSO-*d*₆): δ [ppm] = 7.96 (d, 1H), 7.48 (d, 1H), 7.15 (dd, 1H), 5.18 (s, 2H), 3.55 (s, 2H), 2.22 (s, 6H).

### Beispiel 1.12A

### 3-(Morpholin-4-ylmethyl)-6-nitro[1,2,4]triazolo[4,3-a]pyridin

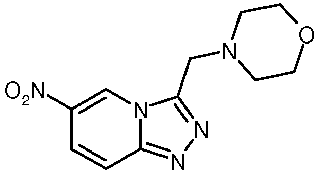

300 mg (1.41 mmol) 3-(Chlormethyl)-6-nitro[1,2,4]triazolo[4,3-*a*]pyridin wurden in 2.0 ml Ethanol gelöst und 0.37 ml (4.23 mmol, 3.0 eq.) Morpholin zugesetzt. Man ließ 4 h bei RT rühren, erwärmte dann für weitere 4 h auf 50°C und setzte Essigsäureethylester und gesättigte, wässrige Natriumhydrogencarbonat-Lösung hinzu. Die wässrige Phase wurde zweimal mit Essigsäureethylester extrahiert. Die vereinten organischen Phasen wurden mit gesättigter wässriger Natriumchlorid-Lösung gewaschen und im Vakuum eingeengt. Ausbeute: 150 mg (40% d. Th.)
LC/MS [Methode 5]: Rₜ = 1.51 min; MS (ESIpos): m/z = 264 (M+H)⁺,
¹H-NMR (400 MHz, DMSO-*d*₆): δ [ppm] = 10.11 (dd, 1H), 8.35 (dd, 1H), 7.95 (dd, 1H), 3.80 (s, 2H), 3.60-3.55 (m, 4H).

### Beispiel 1.12B

### 3-(Morpholin-4-ylmethyl)[1,2,4]triazolo[4,3-a]pyridin-6-amin

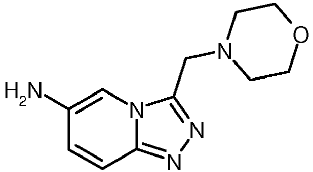

140 mg (0.53 mmol) 3-(Morpholin-4-ylmethyl)-6-nitro[1,2,4]triazolo[4,3-*a*]pyridin wurden in 9.3 ml Ethanol vorgelegt. Man fügte 12 mg (53 µmol, 0.1 eq., 83%ig) Platin(IV)dioxid hinzu und hydrierte 4 h unter normalem Druck. Danach wurde über Kieselgur abfiltriert und das Filtrat im Vakuum vorsichtig eingeengt. Ausbeute: 93 mg (77% Reinheit, 58% d. Th.)
LC/MS [Methode 5]: Rₜ = 1.20 min; MS (ESIpos): m/z = 234 (M+H)⁺,
¹H-NMR (400 MHz, DMSO-*d*₆): δ [ppm] = 7.97 (d, 1H), 7.47 (d, 1H), 7.16 (dd, 1H), 5.20 (br. s, 2H), 3.60 (s, 2H), 3.59-3.51 (m, 8H).

### Beispiel 1.13A

### tert. -Butyl-imidazo [1,5-a]pyridin-6-ylcarbamat

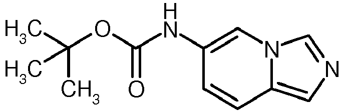

Ein Mikrowellengefäß wurde unter Argon mit 200 mg (1.02 mmol) 6-Bromimidazo[1,5-a]pyridin, 428 mg (3.63 mmol, 3.6 eq.) *tert*.-Butylcarbamat, 16.6 mg (0.07 mmol) Palladium(II)acetat, 58.7 mg (0.10 mmol) Xantphos, 496 mg (1.52 mmol, 1.5 eq.) Cäsiumcarbonat und mit 10 ml 1,4-Dioxan beladen. Ein Argonstrom wurde 2 min lang durch die Suspension geleitet. Das Reaktionsgemisch wurde 4 h in der Mikrowelle bei 140°C erhitzt. Nach Filtrieren über Kieselgur wurde das Filtrat im Vakuum eingeengt. Das Rohprodukt wurde mittels Normalphasen-Chromatographie (Eluent: Dichlormethan-Methanol (2-5%) Gemische) aufgereinigt. Ausbeute: 31.5 mg (13% d. Th.)
LC/MS [Methode 1]: Rₜ = 0.52 min; MS (ESIpos): m/z = 234 (M+H)⁺,
¹H-NMR (400 MHz, DMSO-d₆): δ [ppm] = 9.35 (br. s, 1H), 8.71 (br. s, 1H), 8.33 (s, 1H), 7.46 (d, 1H), 7.26 (s, 1H), 6.70 (dd, 1H), 1.49 (s, 9H).

### Beispiel 1.13B

### Imidazo [1,5-a]pyridin-6-amin

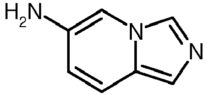

Eine Lösung von 66 mg (0.28 mmol) *tert*.-Butyl-imidazo[1,5-a]pyridin-6-ylcarbamat in Dichlormethan (2 ml) wurde bei RT mit 1ml (12.98 mmol) TFA versetzt und bei RT für 1h gerührt. Anschließend wurde das Reaktionsgemisch im Vakuum eingeengt. Der Rückstand wurde mit Essigsäureethylester gelöst und mit gesättigter, wässriger Natriumhydrogencarbonat-Lösung gewaschen. Nach Phasentrennung wurde die wässrige Phase über Natriumsulfat getrocknet und im Vakuum eingeengt. Ausbeute: 38.9 mg (69% Reinheit, 72% d. Th.).
LC/MS [Methode 5]: Rₜ = 1.08 min, MS (ESIpos): m/z = 134 (M+H)⁺.

### Beispiel 2.1A

### 2,5-Dimethoxypyridin-4-ylboronsäure

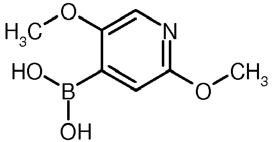

Nach der allgemeinen Methode 1A wurden 11.53 g (82.9 mmol) 2,5-Dimethoxypyridin umgesetzt. Nach Ansäuern der wässrigen Phase fiel das gewünschte Produkt als Niederschlag aus. Ausbeute: 9.53 g (61% d. Th.)
LC/MS [Methode 1]: Rₜ = 0.47 min; MS (ESIpos): m/z = 184 (M+H)⁺.

### Beispiel 2.1B

### 4-Chlor-2-(2,5-dimethoxypyridin-4-yl)benzonitril

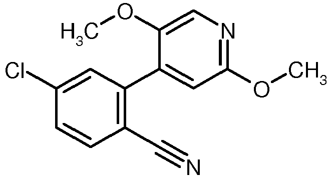

Nach der allgemeinen Methode 2A wurden 7.87 g (95% Reinheit, 40.86 mmol) 2,5-Dimethoxypyridin-4-ylboronsäure mit 8.85 g (40.86 mmol) 2-Brom-4-chlorbenzonitril in Gegenwart von [1,1-Bis-(diphenylphosphino)-ferrocen]-palladium(II)chlorid-dichlormethan-Monoaddukt umgesetzt. Ausbeute: 6.23 g (92% Reinheit, 51% d. Th.)
LC/MS [Methode 1]: Rₜ = 1.08 min; MS (ESIpos): m/z = 275 (M+H)⁺.

### Beispiel 2.1C

### 4-Chlor-2-(5-methoxy-2-oxo-1,2-dihydropyridin-4-yl)benzonitril

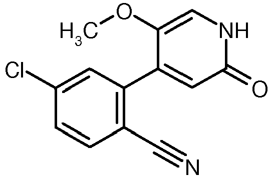

Nach der allgemeinen Methode 3A wurden 7.23 g (92% Reinheit, 24.21 mmol) 4-Chlor-2-(2,5-dimethoxypyridin-4-yl)benzonitril mit Pyridinium-Hydrochlorid umgesetzt. Ausbeute: 6.66 g (91% Reinheit, 96% d. Th.)
LC/MS [Methode 1]: Rₜ = 0.76 min; MS (ESIpos): m/z = 261 (M+H)⁺,
¹H-NMR (400 MHz, DMSO-d₆): δ [ppm] = 11.45 (br. s, 1H), 7.98 (d, 1H), 7.75-7.67 (m, 2H), 7.29 (br. s, 1H), 6.43 (s, 1H), 3.64 (s, 3H).

### Beispiel 2.1D

### 2-[4-(5-Chlor-2-cyanophenyl)-5-methoxy-2-oxopyridin-1(2H)-yl]butansäure-tert.-butylester (Racemat)

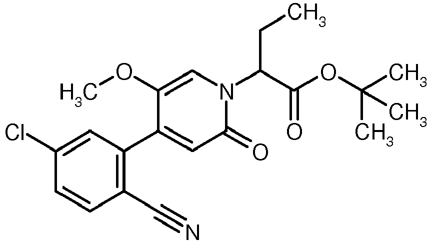

Eine Lösung von 5.0 g (13.3 mmol) [4-(5-Chlor-2-cyanophenyl)-5-methoxy-2-oxopyridin-1(2*H*) yl]essigsäure-*tert*.-butylester in 100 ml Tetrahydrofuran wurde unter Argon bei -78°C tropfenweise mit 14.0 ml (1.0M in THF, 14.0 mmol, 1.05 eq.) Bis-(trimethylsilyl)-lithiumamid versetzt und 15 min bei -78°C gerührt. Anschließend wurden tropfenweise 2.6 g (14.7 mmol, 1.1 eq.) Ethyltrifluormethansulfonat als Reinsubstanz hinzugegeben. Das Kältebad wurde entfernt und die Reaktionsmischung 1h bei RT nachgerührt. Das Reaktionsgemisch wurde auf 0°C gekühlt und mit gesättigter, wässriger Ammoniumchlorid-Lösung versetzt. Nach Phasentrennung wurde die wässrige Phase zweimal mit Methyl-*tert*.-butylether extrahiert. Die vereinigten, organischen Phasen wurden getrocknet (Natriumsulfat), filtriert und im Vakuum eingeengt. Das Rohprodukt wurde anschließend mittels Flash-Chromatographie (340 g Kieselgel, Eluent: Cyclohexan-Essigsäureethylester-Gemische 8:1, 4:1) aufgereinigt. Die produkthaltigen Fraktionen wurden vereinigt und im Vakuum eingeengt. Der Rückstand wurde in der Wärme in Methyl-*tert*.-butylether gelöst, die Lösung offen stehen gelassen, wobei nach 10 min das Gemisch fast vollständig durchkristallisierte. Der Kristallisat wurde filtriert und zweimal mit Methyl-*tert*.-butylether gewaschen. Die vereinigten Filtrate wurden im Vakuum eingeengt und der Rückstand wie beschrieben nochmals auskristallisiert. Beide Kristallisate wurden vereinigt und im Vakuum getrocknet. Ausbeute: 4.2 g (78% d. Th.)
LC/MS [Methode 1]: Rₜ = 1.05 min; MS (ESIpos): m/z = 403 (M+H)⁺,
¹H-NMR (400 MHz, DMSO-d₆): δ [ppm] = 7.99 (d, 1H), 7.77-7.70 (m, 2H), 7.36 (s, 1H), 6.50 (s, 1H), 5.03 (dd, 1H), 3.64 (s, 3H), 2.19-2.06 (m, 2H), 1.40 (s, 9H), 0.85 (t, 3H).

### Beispiel 2.1E

### 2-[4-(5-Chlor-2-cyanophenyl)-5-methoxy-2-oxopyridin-1(2H)-yl]butansäure (Racemat)

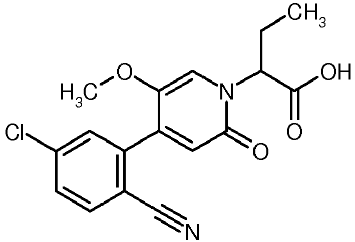

Nach der allgemeinen Methode 4A wurden 159 mg (82% Reinheit, 0.5 mmol) 4-Chlor-2-(5-methoxy-2-oxo-l,2-dihydropyridin-4-yl)benzonitril mit 1.5 eq. 2-Brombutansäure (Racemat) bei 50°C umgesetzt. Ausbeute: 55 mg (32% d. Th.)
LC/MS [Methode 1]: Rₜ = 0.85 min; MS (ESIpos): m/z = 347 (M+H)⁺.

### Alternativsynthese:

Eine Lösung von 4.1 g (10.2 mmol) 2-[4-(5-Chlor-2-cyanophenyl)-5-methoxy-2-oxopyridin-1(2*H*)-yl]butansäure-*tert*.-butylester (Racemat) in 40 ml Dichlormethan wurde unter Argon bei RT mit 7.8 ml (101.8 mmol, 10 eq.) Trifluoressigsäure versetzt, 1h bei RT gerührt, mit weiteren 7.8 ml (101.8 mmol, 10 eq.) Trifluoressigsäure versetzt, 1h bei RT gerührt, mit weiteren 7.8 ml (101.8 mmol, 10 eq.) Trifluoressigsäure versetzt und 1h bei RT gerührt. Nach vollständiger Umsetzung wurde das Reaktionsgemisch im Vakuum eingeengt, der Rückstand jeweils dreimal mit Dichlormethan und einmal mit Toluol coevaporiert und im Vakuum getrocknet. Der Rückstand wurde in 100 ml Essigsäureethylester aufgenommen und mehrfach mit einer stark verdünnten, wässrigen Natriumhydrogencarbonat-Lösung gewaschen (wobei der pH-Wert des Waschwassers pH 3-4 nicht überschreiten sollte, da das Produkt ansonsten gut in Wasser löslich ist). Die organische Phase wurde anschließend getrocknet (Natriumsulfat), filtriert und im Vakuum eingeengt. Der Rückstand wurde in Methyl-*tert*.-butylether verrührt, filtriert, zweimal mit Methyl-*tert*.-butylether gewaschen und im Vakuum getrocknet. Ausbeute: 2.9 g (83% d. Th.)
LC/MS [Methode 1]: Rₜ = 0.81 min; MS (ESIpos): m/z = 347 (M+H)⁺,
¹H-NMR (400 MHz, DMSO-d₆): δ [ppm] = 12.97 (s, 1H), 7.99 (d, 1H), 7.77-7.70 (m, 2H), 7.41 (s, 1H), 6.49 (s, 1H), 5.09 (dd, 1H), 3.64 (s, 3H), 2.21-2.09 (m, 2H), 0.84 (t, 3H).

### Beispiel 2.1F

### 2-({2-[4-(5-Chlor-2-cyanophenyl)-5-methoxy-2-oxopyridin-1(2H)-yl]butanoyl}amino)imidazo[1,2-a]pyridin-6-carbonsäuremethylester (Racemat)

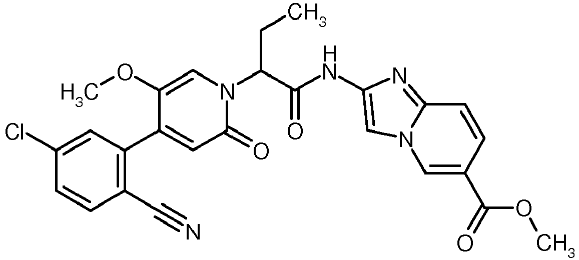

Nach der allgemeinen Methode 5A wurden 72 mg (0.20 mmol) 2-[4-(5-Chlor-2-cyanophenyl)-5-methoxy-2-oxopyridin-1(2*H*)-yl]butansäure (Racemat) mit 43 mg (0.22 mmol, 1.1 eq.) 2-Aminoimidazo[1,2-a]pyridin-6-carbonsäuremethylester umgesetzt. Das Rohprodukt wurde mittels präparativer HPLC (Reprosil C18, Wasser-Acetonitril-Gradient) aufgereinigt. Ausbeute: 59 mg (56% d. Th.)
LC/MS [Methode 1]: Rₜ = 1.01 min; MS (ESIpos): m/z = 520 (M+H)⁺.

### Beispiel 2.2A

### 6-({2-[4-(5-Chlor-2-cyanophenyl)-5-methoxy-2-oxopyridin-1(2H)-yl]butanoyl}amino)imidazo[1,2-a]pyridin-2-carbonsäureethylester (Racemat)

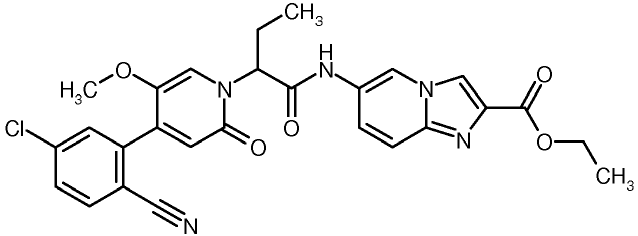

Nach der allgemeinen Methode 5A wurden 87 mg (0.25 mmol) 2-[4-(5-Chlor-2-cyanophenyl)-5-methoxy-2-oxopyridin-1(2*H*)-yl]butansäure (Racemat) mit 59 mg (0.28 mmol, 1.1 eq.) 6-Aminoimidazo[1,2-a]pyridin-2-carbonsäureethylester umgesetzt. Das Rohprodukt wurde mittels präparativer HPLC (Reprosil C18, Wasser-Acetonitril-Gradient) aufgereinigt. Ausbeute: 86 mg (64% d. Th.)
LC/MS [Methode 1]: Rₜ = 0.96 min; MS (ESIpos): m/z = 534 (M+H)⁺,
¹H-NMR (400 MHz, DMSO-d₆): δ [ppm] = 10.76 (s, 1H), 9.32 (s, 1H), 8.61 (s, 1H), 8.00 (d, 1H), 7.78-7.71 (m, 2H), 7.64 (d, 1H), 7.51 (s, 1H), 7.34 (dd, 1H), 6.55 (s, 1H), 5.65 (dd, 1H), 4.30 (q, 2H), 3.70 (s, 3H), 2.28-2.10 (m, 2H), 1.30 (t, 3H), 0.92 (t, 3H).

### Beispiel 2.3A

### 7-({2-[4-(5-Chlor-2-cyanophenyl)-5-methoxy-2-oxopyridin-1(2H)-yl]butanoyl}amino)imidazo[1,2-a]pyridin-2-carbonsäureethylester (Racemat)

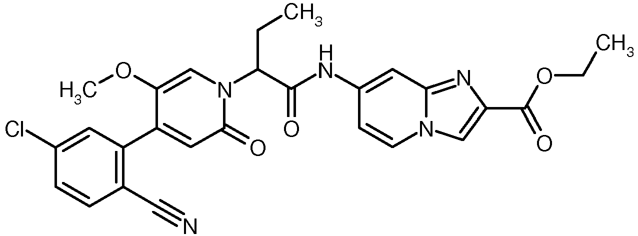

Nach der allgemeinen Methode 5A wurden 87 mg (0.25 mmol) 2-[4-(5-Chlor-2-cyanophenyl)-5-methoxy-2-oxopyridin-1(2*H*)-yl]butansäure (Racemat) mit 56 mg (0.28 mmol, 1.1 eq.) 7-Aminoimidazo[1,2-a]pyridin-2-carbonsäureethylester umgesetzt. Das Rohprodukt wurde mittels präparativer HPLC (Reprosil C18, Wasser-Acetonitril-Gradient) aufgereinigt. Ausbeute: 18 mg (13% d. Th.)
LC/MS [Methode 8]: Rₜ = 1.11 min; MS (ESIpos): m/z = 534 (M+H)⁺.

### Beispiel 3.1A

### [4-(5-Chlor-2-cyanophenyl)-5-methoxy-2-oxopyridin-1(2H)-yl]essigsäure-tert.-butylester

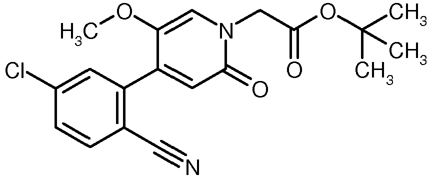

Nach der allgemeinen Methode 4B wurden 516 mg (91% Reinheit, 1.8 mmol) 4-Chlor-2-(5-methoxy-2-oxo-1,2-dihydropyridin-4-yl)benzonitril mit 1.2 eq. Bromessigsäure-*tert*.-butylester bei 100°C umgesetzt. Ausbeute: 464 mg (68% d. Th.)
LC/MS [Methode 1]: Rₜ = 1.00 min; MS (ESIpos): m/z = 375 (M+H)⁺.

### Beispiel 3.1B

### [4-(5-Chlor-2-cyanophenyl)-5-methoxy-2-oxopyridin-1(2H)-yl]essigsäure

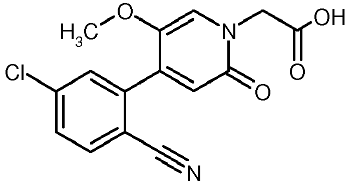

Gemäß allgemeiner Methode 6A wurden 187 mg (500 µmol) [4-(5-Chlor-2-cyanophenyl)-5-methoxy-2-oxopyridin-1(2*H*)-yl]essigsäure-*tert*-butylester mit 770 µl (10.0 mmol) TFA umgesetzt. Ausbeute: 159 mg (93% d. Th.)
LC/MS [Methode 1]: Rₜ = 0.72 min; MS (ESIneg): m/z = 317 (M-H)⁻,
¹H-NMR (400 MHz, DMSO-d₆): δ [ppm] = 13.1 (s, 1H), 8.00 (d, 1H), 7.74 (dd, 1H), 7.72 (s, 1H), 7.58 (s, 1H), 6.51 (s, 1H), 4.64 (s, 2H), 3.62 (s, 3H).

### Beispiel 3.1C

### 6-({[4-(5-Chlor-2-cyanophenyl)-5-methoxy-2-oxopyridin-1(2H)-yl]acetyl}amino)imidazo[1,2-a]pyridin-2-carbonsäureethylester

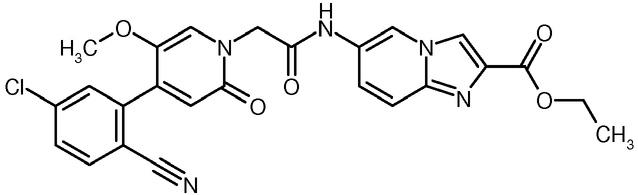

Nach der allgemeinen Methode 5A wurden 130 mg (0.25 mmol) [4-(5-Chlor-2-cyanophenyl)-5-methoxy-2-oxopyridin-1(2*H*)-yl]essigsäure mit 56 mg (0.28 mmol, 1.1 eq.) 6-Aminoimidazo[1,2-a]pyridin-2-carbonsäureethylester umgesetzt. Das Rohprodukt wurde mittels Flash-Chromatographie (Kieselgel (40-60 µm), Dichlormethan-Methanol 10:1) aufgereinigt. Ausbeute: 99 mg (48% d. Th.)
LC/MS [Methode 1]: Rₜ = 0.83 min; MS (ESIpos): m/z = 506 (M+H)⁺,
¹H-NMR (400 MHz, DMSO-d₆): δ [ppm] = 10.65 (s, 1H), 9.30 (s, 1H), 8.62 (s, 1H), 8.01 (d, 1H), 7.70-7.71 (m, 2H), 7.65 (d, 1H), 7.61 (s, 1H), 7.33 (dd, 1H), 6.52 (s, 1H), 4.84 (s, 2H), 4.30 (q, 2H), 3.64 (s, 3H), 1.31 (t, 3H).

### Beispiel 4.1A

### 3-Cyclobutyl-2-hydroxypropansäureethylester (Racemat)

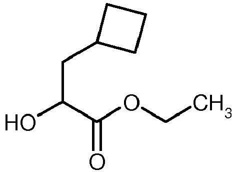

359 mg (14.8 mmol, 1.1 eq.) Magnesiumspäne wurden mit Diethylether überschichtet und durch Zugabe eines Krümels Iod 3-4 min lang angeätzt. Dieses Gemisch wurde unter Argon bei RT mit 5 ml einer Lösung von 2.0 g (13.4 mmol) (Brommethyl)cyclobutan in 30 ml Diethylether unter Rühren versetzt, 5 min lang gerührt (bis zum Anspringen der Reaktion) und innerhalb von weiteren 10 min tropfenweise mit der restlichen (Brommethyl)cyclobutan/Diethylether-Lösung versetzt. Das Reaktionsgemisch wurde 1h unter Rückfluß gerührt, unter Argonstrom abgekühlt und unter Eiswasser-Kühlung tropfenweise zu einer Lösung von 2.4 ml (12.1 mmol, 0.9 eq.) Glyoxylsäureethylester (50%ig in Toluol) gegeben. Das Reaktionsgemisch wurde 1h bei RT gerührt, mit 20 ml einer Kaliumcitrat/Citronensäurelösung (pH 5) vorsichtig auf pH 7 gequencht und anschließend mit wässriger Salzsäure (1N) auf pH 4-5 gebracht. Nach Phasentrennung wurde die wässrige Phase mit Diethylether extrahiert. Die vereinigten, organischen Phasen wurden getrocknet (Natriumsulfat), filtriert und im Vakuum eingeengt. Der Rückstand wurde mittels Flash-Chromatograhpie (Kieselgel-50, Eluent: Cyclohexan-Essigsäureethylester 20-33%) aufgereinigt. Ausbeute: 110 mg (94% Reinheit, 5% d. Th.)
LC/MS [Methode 8]: Rₜ = 3.37 min; MS (ESIpos): m/z = 172 (M)⁺.

### Beispiel 4.1B

### 3-Cyclobutyl-2-{[(trifluormethyl)sulfonyl]oxy}propansäureethylester (Racemat)

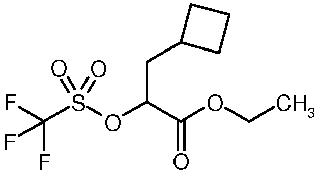

Nach der allgemeinen Methode 7A wurden 110 mg (94% Reinheit, 0.60 mmol) 3-Cyclobutyl-2-hydroxypropansäureethylester (Racemat) mit 142 µl (0.84 mmol, 1.4 eq.) Trifluormethansulfonsäureanhydrid in Gegenwart von 105 µl (0.90 mmol, 1.5 eq.) 2,6-Dimethylpyridin umgesetzt. Das Rohprodukt wurde ohne weitere Reinigung in der nächsten Stufe umgesetzt.

### Beispiel 4.1C

### 2-[4-(5-Chlor-2-cyanophenyl)-5-methoxy-2-oxopyridin-1(2H)-yl]-3-cyclobutylpropansäure-ethylester (Racemat)

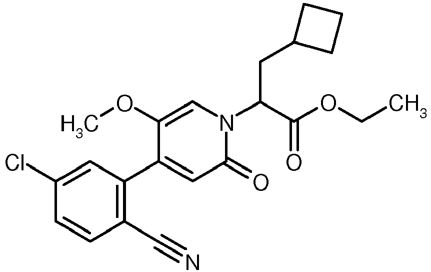

Nach der allgemeinen Methode 4C wurden 122 mg (87% Reinheit, 0.41 mmol) 4-Chlor-2-(5-methoxy-2-oxo-l,2-dihydropyridin-4-yl)benzonitril in Gegenwart von 1.3 eq. Natriumhydrid mit 161 mg (0.53 mmol, 1.3 eq.) 3-Cyclobutyl-2-{[(trifluormethyl)sulfonyl]oxy}propansäureethylester (Racemat) bei RT umgesetzt. Das Rohprodukt wurde mittels Flash-Chromatographie (KP-SIL, Cyclohexan/Essigsäureethylester 15-33%) aufgereinigt. Ausbeute: 140 mg (82% d. Th.)
LC/MS [Methode 1]: Rₜ = 1.15 min; MS (ESIpos): m/z = 415 (M+H)⁺,
¹H-NMR (400 MHz, DMSO-d₆): δ [ppm] = 7.99 (d, 1H), 7.78-7.69 (m, 2H), 7.42 (s, 1H), 6.48 (s, 1H), 5.12 (dd, 1H), 4.21-4.07 (m, 2H), 3.64 (s, 3H), 2.38-2.24 (m, 1H), 2.23-2.11 (m, 2H), 2.05-1.93 (m, 1H), 1.89-1.61 (m, 4H), 1.60-1.47 (m, 1H), 1.18 (t, 3H).

### Beispiel 4.1D

### 2-[4-(5-Chlor-2-cyanophenyl)-5-methoxy-2-oxopyridin-1(2H-yl]-3-cyclobutylpropansäure (Racemat)

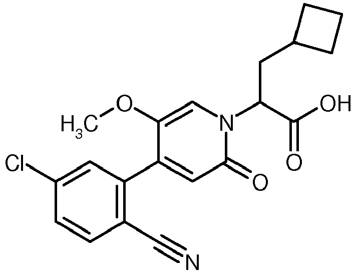

Nach der allgemeinen Methode 6B wurden 138 mg (0.33 mmol) 2-[4-(5-Chlor-2-cyanophenyl)-5-methoxy-2-oxopyridin-1(2*H*)-yl]-3-cyclobutylpropansäureethylester (Racemat) mit Lithiumhydroxid verseift. Ausbeute: 104 mg (82% d. Th.)
LC/MS [Methode 1]: Rₜ = 0.95 min; MS (ESIpos): m/z = 387 (M+H)⁺.

### Beispiel 4.1E

### 6-({2-[4-(5-Chlor-2-cyanophenyl)-5-methoxy-2-oxopyridin-1(2H)-yl]-3-cyclobutylpropanoyl}-amino)imidazo[1,2-a]pyridin-2-carbonsäureethylester (Racemat)

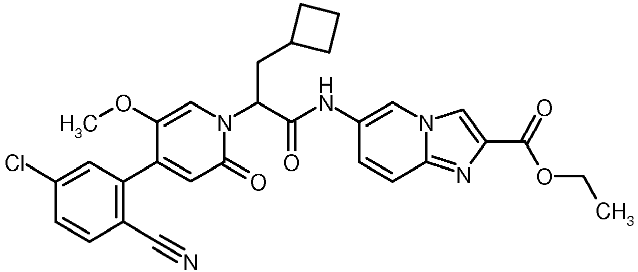

Nach der allgemeinen Methode 5A wurden 109 mg (0.28 mmol) 2-[4-(5-Chlor-2-cyanophenyl)-5-methoxy-2-oxopyridin-1(2*H*)-yl]-3-cyclobutylpropansäure (Racemat) mit 64 mg (0.31 mmol, 1.1 eq.) 6-Aminoimidazo[1,2-a]pyridin-2-carbonsäureethylester umgesetzt. Das Rohprodukt wurde mittels präparativer HPLC (Reprosil C18, Wasser-Acetonitril-Gradient) aufgereinigt. Ausbeute: 69 mg (43% d. Th.)
LC/MS [Methode 1]: Rₜ = 1.08 min; MS (ESIpos): m/z = 574 (M+H)⁺.

### Beispiel 5.1A

### 2-Brom-4-chlorphenyl-difluormethylether

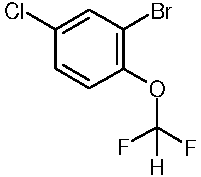

Eine Lösung von 3.5 g (16.9 mmol) 2-Brom-4-chlorphenol in 36 ml Acetonitril wurde mit 36 ml wässriger Kaliumhydroxid-Lösung (6M) versetzt, im Eisbad gekühlt und unter starkem Rühren tropfenweise mit 6.5 ml (26.9 mmol, 1.6 eq.) Difluormethyltrifluormethansulfonat [Angew. Chem. Int. Ed. 2013, 52, 1-5; Journal of Fluorine Chemistry 2009, 130, 667-670] versetzt. Das Reaktionsgemisch wurde 5 min gerührt und mit 200 ml Wasser verdünnt. Die wässrige Phase wurde zweimal mit je 150 ml Diethylether extrahiert. Die vereinigten, organischen Phasen wurden getrocknet (Natriumsulfat), filtriert, im Vakuum eingeengt und getrocknet. Die wässrige Phase wurde nochmals mit Diethylether extrahiert. Die organische Phase wurde getrocknet (Natriumsulfat), filtriert, im Vakuum eingeengt und getrocknet. Ausbeute beider vereinigter Rückstände: 3.4 g (80% d.Th.)
LC/MS [Methode 9]: Rₜ = 3.51 min; MS (ESIpos): m/z = 256 (M+H)⁺,
¹H-NMR (400 MHz, DMSO-d₆): δ [ppm] = 7.91 (d, 1H), 7.55 (dd, 1H), 7.37 (d, 1H), 7.30 (t, 1H).

### Beispiel 5.1B

### 4-[5-Chlor-2-(difluormethoxy)phenyl]-2,5-dimethoxypyridin

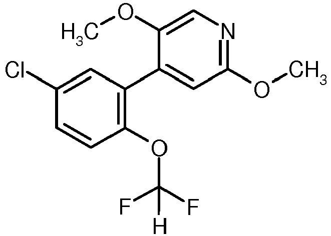

Nach der allgemeinen Methode 2A wurden 417 mg (2.19 mmol, 1.2 eq.) 2,5-Dimethoxypyridin-4-ylboronsäure mit 494 mg (1.82 mmol) 2-Brom-4-chlorphenyl-difluormethylether in Gegenwart von [1,1-Bis-(diphenylphosphino)-ferrocen]-palladium(II)chlorid-Dichlormethan-Monoaddukt umgesetzt. Das Rohprodukt wurde mittels Flash-Chromatographie (KP-SIL, Petrolether/Essigsäureethylester 15-20%) aufgereinigt. Ausbeute: 170 mg (90% Reinheit, 27% d. Th.)
LC/MS [Methode 1]: Rₜ = 1.16 min; MS (ESIpos): m/z = 316 (M+H)⁺,
¹H-NMR (400 MHz, DMSO-d₆): δ [ppm] = 7.96 (s, 1H), 7.57 (dd, 1H), 7.45 (d, 1H), 7.30 (d, 1H), 7.11 (t, 1H), 6.74 (s, 1H), 3.83 (s, 3H), 3.75 (s, 3H).

### Beispiel 5.1C

### 4-[5-Chlor-2-(difluormethoxy)phenyl]-5-methoxypyridin-2(1H-on

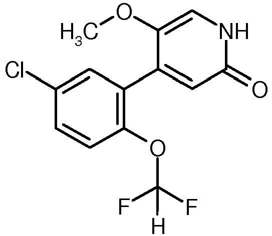

Nach der allgemeinen Methode 3A wurden 170 mg (90% Reinheit, 0.49 mmol) 4-[5-Chlor-2-(difluormethoxy)phenyl]-2,5-dimethoxypyridin mit Pyridinium-Hydrobromid umgesetzt. Ausbeute: 127 mg (87% d. Th.)
LC/MS [Methode 1]: Rₜ = 0.84 min; MS (ESIpos): m/z = 302 (M+H)⁺.

### Beispiel 5.1D

### 2-{4-[5-Chlor-2-(difluormethoxy)phenyl]-5-methoxy-2-oxopyridin-1(2H)-yl}butansäureethylester (Racemat)

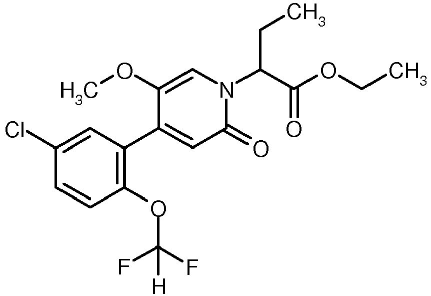

Eine Lösung von 618 mg (2.03 mmol) 4-[5-Chlor-2-(difluormethoxy)phenyl]-5-methoxypyridin-2(1*H*)*-*on in 25 ml Tetrahydrofuran wurde unter Argon bei RT mit 105 mg (2.64 mmol, 1.3 eq.) Natriumhydrid (60%ig in Mineralöl) versetzt, 60 min bei RT gerührt, anschließend tropfenweise mit 871 mg (2.64 mmol, 1.3 eq.) 2-{[(Trifluormethyl)sulfonyl]oxy}butansäureethylester (Racemat) [J. Castells et al. Tetrahedron, 1994, 50, 13765-13774] versetzt und 1h bei RT gerührt. Das Reaktionsgemisch wurde mit weiteren 38 mg (0.96 mmol) Natriumhydrid (60%ig in Mineralöl) versetzt, 5 min bei RT gerührt, tropfenweise mit weiteren 871 mg (2.64 mmol, 1.3 eq.) 2-{[(Trifluormethyl)sulfonyl]oxy}butansäureethylester (Racemat) versetzt, 15 min bei RT gerührt und anschließend mit Wasser gequencht. Nach Phasentrennung wurde die wässrige Phase zweimal mit Essigsäureethylester extrahiert. Die vereinigten, organischen Phasen wurden mit gesättigter, wässriger Natriumchchlorid-Lösung gewaschen, getrocknet (Natriumsulfat), filtriert und im Vakuum eingeengt. Der Rückstand wurde mittels Flash-Chromatographie (Kieselgel-50, Cyclohexan/Essigsäureethylester-Gradient) aufgereinigt. Ausbeute: 415 mg (48% d. Th.)
LC/MS [Methode 1]: Rₜ = 1.08 min; MS (ESIpos): m/z = 416 (M+H)⁺.

### Beispiel 5.1E

### 2-{4-[5-Chlor-2-(difluormethoxy)phenyl]-5-methoxy-2-oxopyridin-1(2H)-yl}butansäure (Racemat)

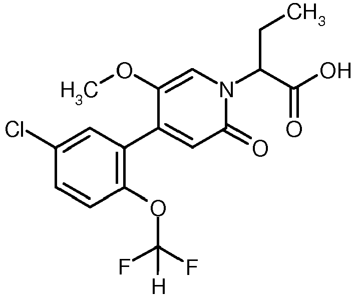

Nach der allgemeinen Methode 6B wurden 415 mg (0.97 mmol) 2-{4-[5-Chlor-2-(difluormethoxy)phenyl]-5-methoxy-2-oxopyridin-1(2*H*)-yl}butansäureethylester (Racemat) mit Lithiumhydroxid verseift. Ausbeute: 348 mg (93% d. Th.)
LC/MS [Methode 1]: Rₜ = 0.91 min; MS (ESIpos): m/z = 388 (M+H)⁺,
¹H-NMR (400 MHz, DMSO-d₆): δ [ppm] = 12.96 (br. s, 1H), 7.57 (dd, 1H), 7.50 (d, 1H), 7.34-7.25 (m, 2H), 7.12 (t, 1H), 6.35 (s, 1H), 5.06 (dd, 1H), 3.58 (s, 3H), 2.20-2.06 (m, 2H), 0.82 (t, 3H).

### Beispiel 5.1F

### 6-[(2-{4-[5-Chlor-2-(difluormethoxy)phenyl]-5-methoxy-2-oxopyridin-1(2H)-yl}butanoyl)amino]-imidazo[1,2-a]pyridin-2-carbonsäureethylester (Racemat)

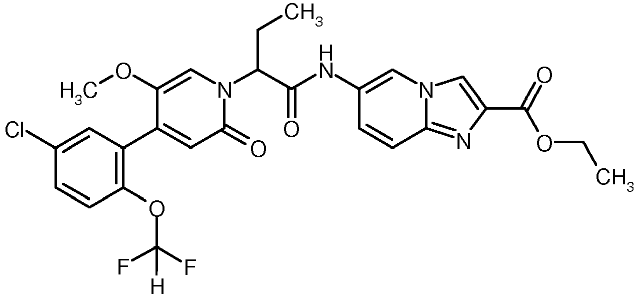

Nach der allgemeinen Methode 5A wurden 116 mg (0.30 mmol) 2-{4-[5-Chlor-2-(difluormethoxy)phenyl]-5-methoxy-2-oxopyridin-1(2*H*)-yl}butansäure (Racemat) mit 69 mg (0.33 mmol, 1.1 eq.) 6-Aminoimidazo[1,2-a]pyridin-2-carbonsäureethylester umgesetzt. Ausbeute: 198 mg (quant.)
LC/MS [Methode 1]: Rₜ = 1.03 min; MS (ESIpos): m/z = 575 (M+H)⁺.

### Beispiel 6.1A

### (5-Chlor-2-methoxypyridin-4-yl)boronsäure

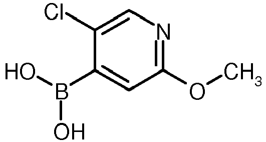

10.0 g (5-Chlor-2-methoxypyridin) wurden in 225 ml THF vorgelegt und bei -78°C mit 41.8 ml (83.6 mmol) Lithiumdiisopropylamid (2M in THF/Heptan/Ethylbenzol) versetzt. Es wurde 4 h bei -78°C nachgerührt und anschließend 32.6 ml (141 mmol) Triisopropylborat zügig zugegeben. Es wurde für 3 h bei -78°C nachgerührt und die Reaktionsmischung über Nacht auf Raumtemperatur erwärmt. Anschließend wurde die Prozedur wiederholt und weitere 20.9 ml (41.8 mmol) Lithiumdiisopropylamid (2M in THF/Heptan/Ethylbenzol) und 16.1 ml (69.7 mmol) Triisopropylborat zugegeben. Die Reaktionsmischung wurde auf 500 ml Wasser gegossen und THF unter reduziertem Druck entfernt. Die wässrige Phase wurde dreimal mit Essigsäureethylester extrahiert. Die wässrige Phase wurde mit Salzsäure (2N) angesäuert und der Niederschlag abfiltriert. Das Filtrat wurde zweimal mit Essigsäureethylester extrahiert, die organische Phase getrocknet, filtriert und das Lösungsmittel unter reduziertem Druck entfernt und der Rückstand zusammen mit dem Niederschlag im Hochvakuum getrocknet. Ausbeute: 10.4 g (91% Reinheit, 73% d. Th.)
LC/MS [Methode 1]: Rₜ = 0.50 min; MS (ESIpos): m/z = 188 (M+H)⁺,
¹H-NMR (400 MHz, DMSO-d₆): δ [ppm] = 8.64 (br. s, 2H), 8.12 (s, 1H), 6.81 (s, 1H), 3.82 (s, 3H).

### Beispiel 6.1B

### 5-Chlor-4-[5-chlor-2-(difluormethoxy)phenyl]-2-methoxypyridin

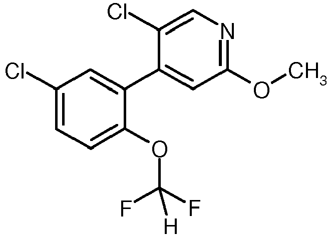

4.17 g (16.2 mmol) 2-Brom-4-chlor-1-(difluormethoxy)benzol, 3.04 g (16.2 mmol) (5-Chlor-2-methoxypyridin-4-yl)boronsäure, 561 mg (486 µmol) CATAXCium A Präkatalysator und 133 ml wässrige Kaliumphosphat-Lösung (0.5N) wurden in 73 ml THF für 1h bei 60°C gerührt. Anschließend wurde die Reaktionsmischung mit 125 ml Wasser und 125 ml Essigsäureethylester verdünnt. Die Phasen wurden getrennt und die wässrige Phase mit 125 ml Essigsäureethylester extrahiert. Die vereinigten, organischen Phasen wurden mit gesättigter, wässriger Natriumchlorid-Lösung gewaschen, über Natriumsulfat getrocknet, filtriert und das Lösungsmittel unter reduziertem Druck entfernt. Nach säulenchromatographischer Reinigung des Rohproduktes (100 g Silica Kartusche, Fluss: 50 ml/min, Cyclohexan/Essigsäureethylester-Gradient) wurde die Titelverbindung erhalten. Ausbeute: 2.80 g (86% Reinheit, 46% d. Th.)
LC/MS [Methode 1]: Rₜ = 1.20 min; MS (ESIpos): m/z = 320 (M+H)⁺.

### Beispiel 6.1C

### 5-Chlor-4-[5-chlor-2-(difluormethoxy)phenyl]pyridin-2(1H)-on

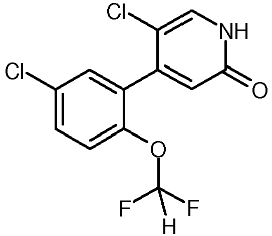

2.80 g (8.75 mmol) 5-Chlor-4-[5-chlor-2-(difluormethoxy)phenyl]-2-methoxypyridin und 28.0 g (175 mmol) Pyridiniumhydrobromid wurden in 93.5 ml Dimethylformamid gelöst und für 6 h bei 100°C gerührt. Das Lösungsmittel wurde unter reduziertem Druck entfernt und der Rückstand mit 253 ml Wasser verrührt. Der Niederschlag wurde abgesaugt, mit Wasser gewaschen und anschließend getrocknet. Ausbeute: 2.60 g (81% Reinheit, 79% d. Th.)
LC/MS [Methode 1]: Rₜ = 0.85 min; MS (ESIpos): m/z = 306 (M+H)⁺,
¹H-NMR (400 MHz, DMSO-d₆): δ [ppm] = 11.99 (br. s, 1H), 7.81 (s, 1H), 7.61 (dd, 1H), 7.49 (d, 1H), 7.34 (d, 1H), 7.20 (t, 1H), 6.44 (s, 1H).

### Beispiel 6.1D

### {5-Chlor-4-[5-chlor-2-(difluormethoxy)phenyl]-2-oxopyridin-1(2H)-yl-essigsäure-tert.-butylester

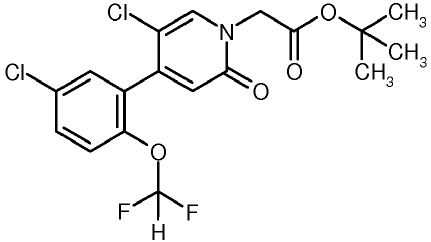

Nach der allgemeinen Methode 4B wurden 2.60 g (81% Reinheit, 6.88 mmol) 5-Chlor-4-[5-chlor-2-(difluormethoxy)phenyl]pyridin-2(1*H*)-on mit 1.2 eq. Bromessigsäure-*tert*.-butylester in Gegenwart von 1.5 eq. Kaliumcarbonat bei 100°C umgesetzt. Ausbeute: 2.44 g (84% d. Th.)
LC/MS [Methode 8]: Rₜ = 1.41 min; MS (ESIneg): m/z = 418 (M-H)⁻,
¹H-NMR (400 MHz, DMSO-d₆): δ [ppm] = 8.09 (s, 1H), 7.63 (dd, 1H), 7.51 (d, 1H), 7.35 (d, 1H), 7.23 (t, 1H), 6.50 (s, 1H), 4.62 (s, 2H), 1.44 (s, 9H).

### Beispiel 6.1E

### {5-Chlor-4-[5-chlor-2-(difluormethoxy)phenyl]-2-oxopyridin-1(2H)-yl}essigsäure

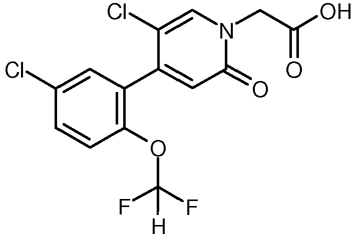

Nach der allgemeinen Methode 6A wurden 126 mg (0.30 mmol) {5-Chlor-4-[5-chlor-2-(difluormethoxy)phenyl]-2-oxopyridin-1(2*H*)-yl-essigsäure-*tert*.-butylester mit 0.46 ml (6.0 mmol) TFA umgesetzt. Ausbeute: 101 mg (92% d. Th.)
LC/MS [Methode 1]: Rₜ = 0.85 min; MS (ESIpos): m/z = 364 (M+H)⁺.

### Beispiel 6.1F

### 6-[({5-Chlor-4-[5-chlor-2-(difluormethoxy)phenyl]-2-oxopyridin-1(2H)-yl}acetyl)amino]-imidazo[1,2-a]pyridin-2-carbonsäureethylester

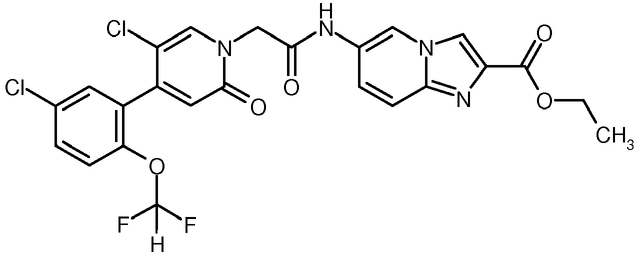

Nach der allgemeinen Methode 5A wurden 101 mg (0.28 mmol) {5-Chlor-4-[5-chlor-2-(difluormethoxy)phenyl]-2-oxopyridin-1(2*H*)-yl}essigsaure mit 63 mg (0.31 mmol, 1.1 eq.) 6-Aminoimidazo[1,2-a]pyridin-2-carbonsäureethylester umgesetzt. Ausbeute: 99 mg (65% d. Th.)
LC/MS [Methode 1]: Rₜ = 0.93 min; MS (ESIpos): m/z = 551 (M+H)⁺.

### Beispiel 7.1A

### 2-[(Benzyloxy)methyl]tetrahydro-2H-pyran (Racemat)

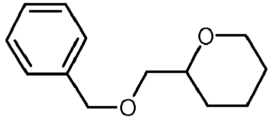

Zu einer Suspension von 9.47 g (237 mmol, 60% in Mineralöl) Natriumhydrid in 500 ml THF wurde bei 0°C eine Lösung von 25.0 g (215 mmol) Tetrahydro-2H-pyran-2-ylmethanol (Racemat) in 500 ml THF langsam zugetropft und nach vollständiger Zugabe 30 min bei 0°C nachgerührt. Anschließend wurden 25.7 ml (215 mmol) Benzylbromid zugegeben und für 30 min bei 0°C und 1h bei Raumtemperatur nachgerührt. Die Reaktion wurde durch Zugabe von 200 ml gesättigter, wässriger Ammoniumchlorid-Lösung beendet und die Phasen getrennt. Die wässrige Phase wurde zweimal mit 200 ml Methyl-*tert*.-butylether extrahiert. Die vereinigten organischen Phasen wurden über Magnesiumsulfat getrocknet, filtriert und das Lösungsmittel unter reduziertem Druck entfernt. Das Rohprodukt wurde säulenchromatographisch gereinigt (Essigsäureethylester-Cyclohexan-Gradient, 340 g Silica Kartusche, Fluss: 100 ml/min) und die Titelverbindung erhalten. Ausbeute: 41.9 g (94% d. Th.)
LC/MS [Methode 3]: Rₜ = 2.18 min; MS (ESIpos): m/z = 207 (M+H)⁺,
¹H-NMR (400 MHz, DMSO-d₆): δ [ppm] = 7.37-7.25 (m, 5H), 4.47 (s, 2H), 3.87-3.81 (m, 1H), 3.47-3.28 (m, 4H), 1.80-1.72 (m, 1H), 1.58-1.37 (m, 4H), 1.25-1.13 (m, 1H).

### Beispiel 7.1B

### (R)-2-[(Benzyloxy)methyl]tetrahydro-2H-pyran

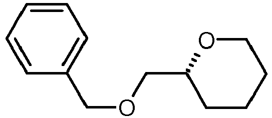

Enantiomerentrennung von 41.9 g des Racemates aus Beispiel 7.1A ergab 16.7 g der Titelverbindung Beispiel 7.1B (Enantiomer 1): Chirale HPLC: Rₜ = 5.28 min; 99% ee, 93% Reinheit.
Drehwert: [α]₅₈₉^{20.0} = +14.9° (c 0.43 g/100 cm³, Chloroform)
Trennmethode: Säule: OD-H 5 µm 250 mm x 20 mm; Eluent: 95% iso-Hexan, 5% 2-Propanol; Temperatur: 25°C; Fluss: 25 ml/min; UV-Detektion: 210 nm.
Analytik: Säule: OD-H 5 µm 250 mm x 4.6 mm; Eluent: 95% iso-Hexan, 5% 2-Propanol; Fluss: 1 ml/min; UV-Detektion: 220 nm.

### Beispiel 7.2B

### (S)-2-[(Benzyloxy)methyl]tetrahydro-2H-pyran

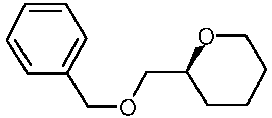

Enantiomerentrennung von 41.9 g des Racemates aus Beispiel 7.1A ergab 17.0 g der Titelverbindung Beispiel 7.2B (Enantiomer 2): Chirale HPLC: Rₜ = 7.36 min; 96% ee, 96% Reinheit.
Drehwert: [α]₅₈₉^{20.0} = -13.9° (c 0.61 g/100 cm³, Chloroform)
Trennmethode: Säule: OD-H 5 µm 250 mm x 20 mm; Eluent: 95% iso-Hexan, 5% 2-Propanol; Temperatur: 25°C; Fluss: 25 ml/min; UV-Detektion: 210 nm.
Analytik: Säule: OD-H 5 µm 250 mm x 4.6 mm; Eluent: 95% iso-Hexan, 5% 2-Propanol; Fluss: 1 ml/min; UV-Detektion: 220 nm.

### Beispiel 7.1C

### (2S)-Tetrahydro-2H-pyran-2-ylmethanol

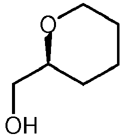

Zu einer Lösung 17.0 g (82.4 mmol) (*S*)-2-[(Benzyloxy)methyl]tetrahydro-2*H*-pyran (96% ee, 96% Reinheit) in 120 ml Ethanol wurde 3.51 g (3.30 mmol) Palladium auf Kohle (10%ig) gegeben und über Nacht bei Raumtemperatur und Normaldruck hydriert. Anschließend wurde noch einmal 1.75 g (1.65 mmol) Palladium auf Kohle (10%ig) hinzugegeben und für weitere 72 h bei Raumtemperatur hydriert. Anschließend wurde die Reaktionsmischung über Celite filtriert und das Filtrat eingeengt. Der Rückstand wurde chromatographisch (Silica, Dichlormethan/Methanol-Gradient) gereinigt und die Produktfraktionen bei < 25°C und > 50 mbar vom Lösungsmittel befreit. Ausbeute: 8.23 g (86% d. Th.)
Drehwert: [α]₅₈₉^{20.0} = + 9.1° (c 0.36 g/100 cm³, Chloroform), vgl. A. Aponick, B. Biannic, *Org. Lett.* **2011**, *13*, 1330-1333.
GC/MS [Methode 7]: Rₜ = 1.82 min; MS: m/z = 116 (M)⁺,
¹H-NMR (400 MHz, DMSO-d₆): δ [ppm] = 4.51 (t, 1H), 3.87-3.81 (m, 1H), 3.37-3.18 (m, 4H), 1.80-1.71 (m, 1H), 1.59-1.50 (m, 1H), 1.49-1.36 (m, 3H), 1.19-1.05 (m, 1H).

### Beispiel 7.1D

### (2S)-Tetrahydro-2H-pyran-2-ylmethyltrifluormethansulfonat

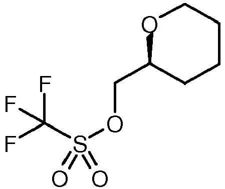

Nach der allgemeinen Methode 7A wurden 330 mg (2.84 mmol) (2*S*)-Tetrahydro-2*H*-pyran-2-ylmethanol mit 0.57 ml (3.41 mmol, 1.2 eq.) Trifluormethansulfonsäureanhydrid in Gegenwart von 0.48 ml (3.41 mmol, 1.2 eq.) Triethylamin umgesetzt. Das Rohprodukt wurde ohne weitere Reinigung in der nächsten Stufe umgesetzt.
¹H-NMR (400 MHz, DMSO-d₆): δ [ppm] = 4.32 (dd, 1H), 4.18 (dd, 1H), 4.00-3.92 (m, 1H), 3.60-3.52 (m, 1H), 3.48-3.39 (m, 1H), 1.85-1.74 (m, 1H), 1.56-1.41 (m, 4H), 1.28-1.14 (m, 1H).

### Beispiel 7.1E

### 2-[4-(5-Chlor-2-cyanophenyl)-5-methoxy-2-oxopyridin-1(2H)-yl]-3-[(2S)-tetrahydro-2H-pyran-2-yl]propansäure-tert.-butylester (Gemisch enantiomerenreiner Diastereomere)

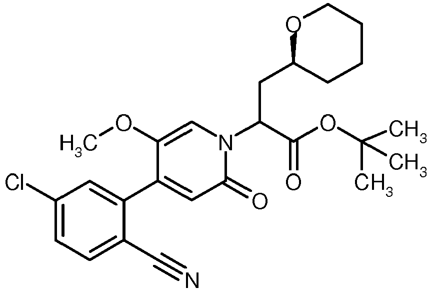

Gemäß allgemeiner Methode 8A wurden 4.10 g (10.9 mmol) [4-(5-Chlor-2-cyanophenyl)-5-methoxy-2-oxopyridin-1(2*H*)-yl]essigsäure-*tert*.-butylester, 4.07 g (16.4 mmol) (2*S*)-Tetrahydro-2H-pyran-2-ylmethyltrifluormethansulfonat und 12.0 ml (12.0 mmol) Bis-(trimethylsilyl)-lithiumamid (1M in THF) in 90 ml THF zur Reaktion gebracht. Nach wässriger Aufarbeitung wurde das Rohprodukt mittels Flash-Chromatographie (340 g Silica Kartusche, Fluss: 100 ml/min, Cyclohexan/Essigsäureethylester-Gradient) aufgereinigt. Ausbeute: 4.2 g (81% d. Th.)
LC/MS [Methode 1]: Rₜ = 1.15 min; MS (ESIpos): m/z = 473 (M+H)⁺.

### Beispiel 7.1F

### 2-[4-(5-Chlor-2-cyanophenyl)-5-methoxy-2-oxopyridin-1(2H)-yl]-3-[(2S)-tetrahydro-2H-pyran-2-yl]propansäure (Gemisch enantiomerenreiner Diastereomere)

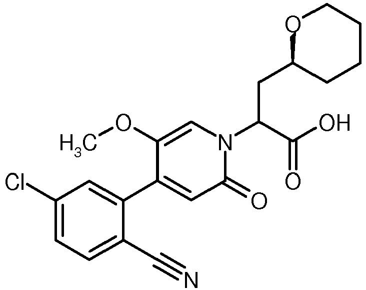

Gemäß allgemeiner Methode 6A wurden 9.8 g (20.7 mmol) 2-[4-(5-Chlor-2-cyanophenyl)-5-methoxy-2-oxopyridin-1(2*H*)-yl]-3-[(2*S*)-tetrahydro-2*H*-pyran-2-yl]propansäure-*tert*. -butylester (Gemisch enantiomerenreiner Diastereomere) in 245 ml Dichlormethan mit 59.9 ml (777 mmol) TFA umgesetzt. Ausbeute: 8.7 g (73% Reinheit, 74% der Th.)
LC/MS [Methode 1]: Rₜ = 0.92 min; MS (ESIpos): m/z = 417 (M+H)⁺.

### Beispiel 7.1G

### 6-({2-[4-(5-Chlor-2-cyanophenyl)-5-methoxy-2-oxopyridin-1(2H)-yl]-3-[(2S)-tetrahydro-2H-pyran-2-yl]propanoyl}amino)imidazo[1,2-a]pyridin-2-carbonsäureethylester (Gemisch enantiomerenreiner Diastereomere)

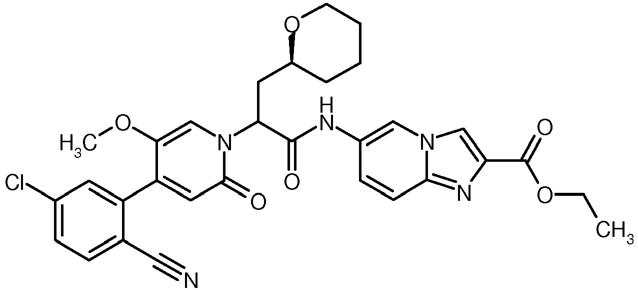

Nach der allgemeinen Methode 5A wurden 126 mg (0.30 mmol) 2-[4-(5-Chlor-2-cyanophenyl)-5-methoxy-2-oxopyridin-1(2*H*)-yl]-3-[(2*S*)-tetrahydro-2*H*-pyran-2-yl]propansäure (Gemisch enantiomerenreiner Diastereomere) mit 68 mg (0.33 mmol, 1.1 eq.) 6-Aminoimidazo[1,2-a]pyridin-2-carbonsäureethylester umgesetzt. Nach Entfernen des Dimethylformamids im Vakuum konnte die Titelverbindung aus dem Rückstand mit Wasser kristallisiert werden. Der Niederschlag wurde filtriert, mit Wasser gewaschen und im Vakuum getrocknet. Ausbeute: 162 mg (89% d. Th.)
LC/MS [Methode 1]: Rₜ = 0.99 min; MS (ESIpos): m/z = 604 (M+H)⁺.

### Beispiel 8.1A

### 2-Methoxyethyl-trifluormethansulfonat

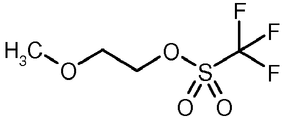

Man legte 16.3 g (57.8 mmol) Trifluormethansulfonsäureanhydrid in 20 ml Dichlormethan bei -78°C vor und tropfte eine Lösung von 4.00 g (52.6 mmol) 2-Methoxyethanol und 5.85 g (57.8 mmol) Triethylamin in 20 ml Dichlormethan so langsam hinzu, dass die interne Temperatur -50°C nicht überstieg. Man ließ 15 min bei -78°C nachrühren und erwärmte dann auf RT. Man verdünnte mit 100 ml Methyl-*tert*.-butylether und wusch dreimal mit je 50 ml einer 3:1-Mischung aus gesättigter, wässriger Natriumchlorid-Lösung und 1N Salzsäure. Die organische Phase wurde über Natriumsulfat getrocknet und im Vakuum bei RT eingeengt. Man erhielt 13 g des Rohproduktes, das direkt weiter umgesetzt wurde.

### Beispiel 8.1B

### 2-[4-(5-Chlor-2-cyanophenyl)-5-methoxy-2-oxopyridin-1(2H)-yl]-4-methoxybutansäure-tert.-butylester (Racemat)

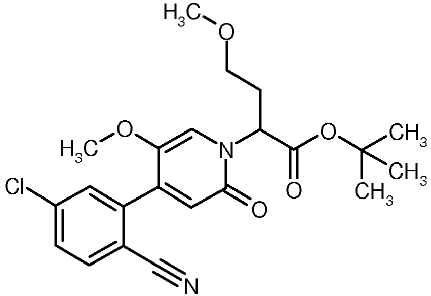

8.09 g (21.6 mmol) [4-(5-Chlor-2-cyanophenyl)-5-methoxy-2-oxopyridin-1(2*H*)-yl]essigsäure-*tert*.-butylester wurden in 180 ml THF vorgelegt und auf -78°C gekühlt. Tropfenweise wurden 23.7 ml Bis-(trimethylsilyl)-lithiumamid (1M in THF) zugegeben, und man ließ 15 min weiter rühren. Dann tropfte man 8.99 g (43.2 mmol) 2-Methoxyethyl-trifluormethansulfonat zu und ließ 15 min bei -78°C und weitere 45 min bei RT rühren. Man fügte dann gesättigte, wässrige Ammoniumchlorid-Lösung zu und extrahierte mehrfach mit Essigsäureethylester. Die vereinten organischen Phasen wurden über Natriumsulfat getrocknet und im Vakuum eingeengt. Man reinigte den Rückstand mittels Flash-Chromatographie (Kieselgel-50, Cyclohexan-Essigsäureethylester-Gradient). Ausbeute: 7.87 g (95% Reinheit, 80% d. Th.)
LC/MS [Methode 1]: Rₜ = 1.02 min; MS (ESIpos): m/z = 433 (M+H)⁺,
1H-NMR (400 MHz, DMSO-d₆): δ [ppm] = 8.01-7.96 (m, 1H), 7.76-7.69 (m, 2H), 7.37 (s, 1H), 6.48 (s, 1H), 5.11 (dd, 1H), 3.64 (s, 3H), 3.43-3.35 (m, 1H), 3.20 (s, 3H), 3.19-3.13 (m, 1H), 2.39-2.28 (m, 2H), 1.40 (s, 9H).

### Beispiel 8.1C

### 2-[4-(5-Chlor-2-cyanophenyl)-5-methoxy-2-oxopyridin-1(2H)-yl]-4-methoxybutansäure (Racemat)

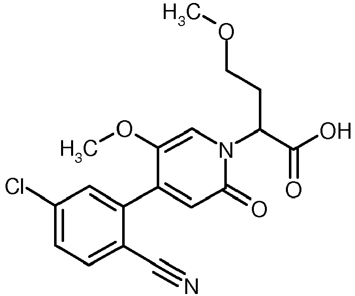

7.87 g (95% Reinheit, 17.3 mmol) 2-[4-(5-Chlor-2-cyanophenyl)-5-methoxy-2-oxopyridin-1(2*H*)-yl]-4-methoxybutansäure-*tert*.-butylester (Racemat) wurden in 175 ml Dichlormethan vorgelegt. Man setzte 42 ml (545 mmol) Trifluoressigsäure hinzu und ließ 3 h bei RT rühren. Man engte die Reaktionsmischung im Vakuum ein, nahm den Rückstand mehrmals in Dichlormethan auf und engte wieder ein. Zweimal wurde dann Toluol hinzugefügt und wieder eingeengt. Der Rückstand wurde mit Acetonitril verrührt und abgesaugt. Ausbeute 5.81 g (95% Reinheit, 84% d. Th.)
LC/MS [Methode 1]: Rₜ = 0.78 min; MS (ESIpos): m/z = 377 (M+H)⁺,
¹H-NMR (500 MHz, DMSO-d₆): δ [ppm] = 13.40-12.67 (m, 1H), 7.99 (d, 1H), 7.75 (d, 1H), 7.73 (dd, 1H), 7.43 (s, 1H), 6.48 (s, 1H), 5.14 (t, 1H), 3.64 (s, 3H), 3.41-3.36 (m, 1H), 3.19 (s, 3H), 3.13 (dt, 1H), 2.40-2.31 (m, 2H).

### Beispiel 9.1A

### trans-4-Hydroxycyclohexancarbonsäureethylester

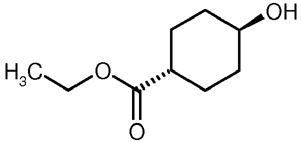

4.00 g (27.7 mmol) *trans*-4-Hydroxycyclohexancarbonsäure wurden in 50.2 ml Ethanol vorgelegt und bei Raumtemperatur 2 ml konzentrierte Schwefelsäure zugegeben. Anschließend wurde die Reaktionslösung für 10 h bei 80°C gerührt. Es wurde auf Raumtemperatur abgekühlt und die Reaktionslösung mit gesättigter, wässriger Natriumhydrogencarbonat-Lösung versetzt. Es wurde mit 200 ml Essigsäureethylester extrahiert und die organische Phase getrocknet, filtriert und das Lösungsmittel unter reduziertem Druck entfernt. Ausbeute: 4.3 g (90% d. Th.)
GC/MS [Methode 9]: Rₜ = 4.17 min; MS: m/z = 172 (M)⁺,
¹H-NMR (400 MHz, DMSO-d₆): δ [ppm] = 4.56 (d, 1H), 4.03 (q, 2H), 3.39-3.29 (m, 1H), 2.22-2.13 (m, 1H), 1.88-1.78 (m, 4H), 1.40-1.27 (m, 2H), 1.21-1.09 (m, 2H), 1.16 (t, 3H).

### Beispiel 9.1B

### trans-4-{[tert.-Butyl(dimethyl)silyl]oxy}cyclohexancarbonsäureethylester

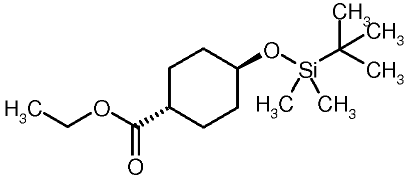

4.3 g (25 mmol) trans-4-Hydroxycyclohexancarbonsäureethylester wurden in 20 ml Dimethylformamid vorgelegt. Anschließend wurden 4.5 g (30 mmol) *tert.-*Butyldimethylsilylchlorid und 4.2 g (62 mmol) Imidazol hinzugegeben und 12 h bei 35°C nachgerührt. Die Reaktionslösung wurde mit 200 ml Essigsäureethylester versetzt und dreimal mit 100 ml Wasser extrahiert. Die organische Phase wurde getrocknet, filtriert und das Lösungsmittel unter reduziertem Druck entfernt. Ausbeute: 7.8 g (quantitativ)
GC/MS [Methode 9]: Rₜ = 5.04 min; MS: m/z = 286 (M)⁺,
¹H-NMR (400 MHz, DMSO-d₆): δ [ppm] = 4.00 (q, 2H), 3.59-3.50 (m, 1H), 2.24-2.14 (m, 1H), 1.86-1.71 (m, 4H), 1.41-1.29 (m, 2H), 1.27-1.16 (m, 2H), 1.13 (t, 3H), 0.82 (s, 9H), 0.00 (s, 6H).

### Beispiel 9.1C

### (trans-4-{[tert.-Butyl(dimethyl)silyl]oxy}cyclohexyl)methanol

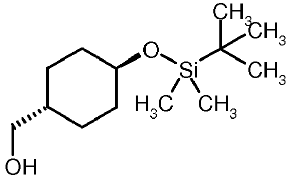

12.5 ml (29.9 mmol) Lithiumaluminiumhydrid (2.4M in THF) wurden in 90 ml Methyl-*tert*.-butylether vorgelegt und bei Raumtemperatur mit einer Lösung aus 7.8 g (27.2 mmol) *trans-*4-{[*tert*.-Butyl(dimethyl)silyl]oxy}cyclohexancarbonsäureethylester in 90 ml Methyl-*tert*.-butylether versetzt. Es wurde 4 h bei 40°C nachgerührt. Die Reaktion wurde durch Zugabe von 7 ml Wasser und 7 ml 15%iger, wässriger Kaliumhydroxid-Lösung beendet. Die organische Phase wurde abdekantiert, über Magnesiumsulfat getrocknet, filtriert und das Lösungsmittel unter reduziertem Druck entfernt. Ausbeute: 6.3 g (95% d. Th.)
GC/MS [Methode 9]: Rₜ = 4.74 min; MS: m/z = 244 (M)⁺,
¹H-NMR (400 MHz, DMSO-d₆): δ [ppm] = 4.35 (t, 1H), 3.52-3.44 (m, 1H), 3.15 (t, 2H), 1.80-1.72 (m, 2H), 1.71-1.62 (m, 2H), 1.29-1.09 (m, 3H), 0.92-0.80 (m, 2H), 0.82 (s, 9H), 0.00 (s, 6H).

### Beispiel 9.1D

### (trans-4-{[tert.-Butyl(dimethyl)silyl]oxy}cyclohexyl)methyl-trifluormethansulfonat

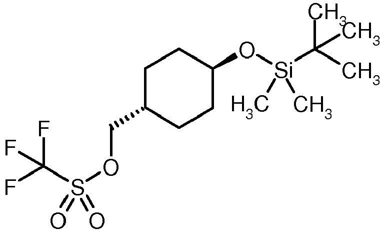

6.30 g (25.8 mmol) (*trans*-4-{[*tert*.-Butyl(dimethyl)silyl]oxy}cyclohexyl)methanol wurden in 90 ml Dichlormethan vorgelegt und bei 0°C mit 4.50 ml (38.7 mmol) Lutidin und 6.54 ml (38.7 mmol) Trifluormethansulfonsäureanhydrid umgesetzt, wobei die interne Temperatur 5°C nicht übersteigen sollte. Es wurde für 1 h nachgerührt. Anschließend wurde die Reaktionslösung mit 630 ml Methyl-*tert*.-butylether verdünnt und nacheinander dreimal mit einem Gemisch aus wässriger Salzsäure (1N)/gesättigter, wässriger Natriumchlorid-Lösung (1:3) und gesättigter, wässriger Natriumhydrogencarbonat-Lösung gewaschen. Die organische Phase wurde getrocknet, filtriert und das Lösungsmittel unter reduziertem Druck entfernt. Das Rohprodukt wurde ohne weitere Aufreinigung im nächsten Schritt eingesetzt. Ausbeute: 9.7 g (quantitativ)

### Beispiel 9.1E

### 3-(trans-4-{[tert.-Butyl(dimethyl)silyl]oxy}cyclohexyl)-2-[4-(5-chlor-2-cyanophenyl)-5-methoxy-2-oxopyridin-1(2H)-yl]propansäure-tert.-butylester (Racemat)

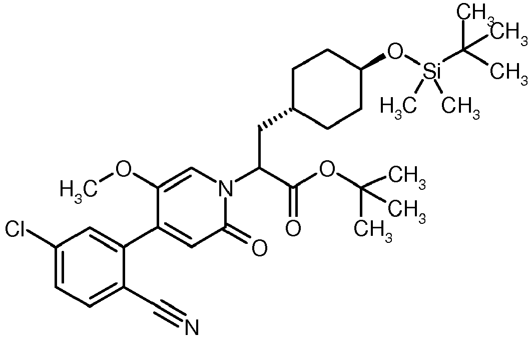

4.90 g (12.3 mmol) [4-(5-Chlor-2-cyanophenyl)-5-methoxy-2-oxopyridin-1(2*H*)-yl]essigsäure-*tert*.-butylester wurden in 98 ml THF vorgelegt und bei -78°C mit 13.6 ml (13.6 mmol) Bis-(trimethylsilyl)-lithiumamid (1M in THF) versetzt. Es wurde für 15 min bei -78°C nachgerührt und anschließend 6.97 g (18.5 mmol) (*trans*-4-{[*tert*.-Butyl(dimethyl)silyl]oxy}cyclohexyl)methyl-trifluormethansulfonat zugegeben. Es wurde für 15 min bei -78°C und 2 h bei Raumtemperatur gerührt. Die Reaktion wurde durch Zugabe von gesättigter, wässriger Ammoniumchlorid-Lösung beendet und die Phasen getrennt. Die wässrige Phase wurde dreimal mit 174 ml Methyl-*tert*.-butylether extrahiert. Die vereinigten, organischen Phasen wurden getrocknet, filtriert und das Lösungsmittel unter reduziertem Druck entfernt. Nach säulenchromatographischer Reinigung des Rohproduktes (100 g Silica Kartusche, Fluss: 50 ml/min, Cyclohexan-Essigsäureethylester-Gradient) wurde die Titelverbindung erhalten. Ausbeute: 3.10 g (42% d. Th.)
LC/MS [Methode 1]: Rₜ = 1.59 min; MS (ESIpos): m/z = 601 (M+H)⁺.

### Beispiel 9.1F

### 3-(trans-4-{[tert.-Butyl(dimethyl)silyl]oxy}cyclohexyl)-2-[4-(5-chlor-2-cyanophenyl)-5-methoxy-2-oxopyridin-1(2H)-yl]propansäure (Racemat)

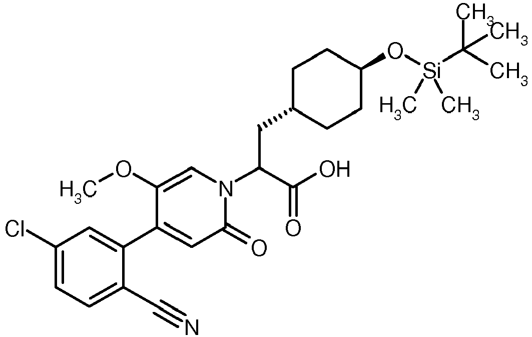

3.10 g (5.16 mmol) 3-(*trans*-4-{[*tert*.-Butyl(dimethyl)silyl]oxy}cyclohexyl)-2-[4-(5-chlor-2-cyanophenyl)-5-methoxy-2-oxopyridin-1(2*H*)-yl]propansäure-*tert*.-butylester (Racemat) wurden in 32.4 ml THF, 16.2 ml Ethanol und 16.2 ml Wasser vorgelegt und mit 1.08 g (25.8 mmol) Lithiumhydroxid-Monohydrat versetzt. Es wurde für 6 h bei Raumtemperatur nachgerührt und anschließend mit wässriger Salzsäure (1N) angesäuert (pH = 4-5). Es wurde dreimal mit 129 ml Essigsäureethylester extrahiert. Die vereinigten organischen Phasen wurden getrocknet, filtriert und das Lösungsmittel unter reduziertem Druck entfernt. Das Rohprodukt wurde ohne weitere Aufreinigung im nächsten Schritt eingesetzt. Ausbeute: 2.8 g (75% Reinheit, quantitativ)
LC/MS [Methode 1]: Rₜ = 1.37 min; MS (ESIpos): m/z = 545 (M+H)⁺.

### Beispiel 9.1G

### 3-(trans-4-{[tert.-Butyl(dimethyl)silyl]oxy}cyclohexyl)-2-[4-(5-chlor-2-cyanophenyl)-5-methoxy-2-oxopyridin-1(2H)-yl]-N-(imidazo[1,2-a]pyridin-6-yl)propanamid (Racemat)

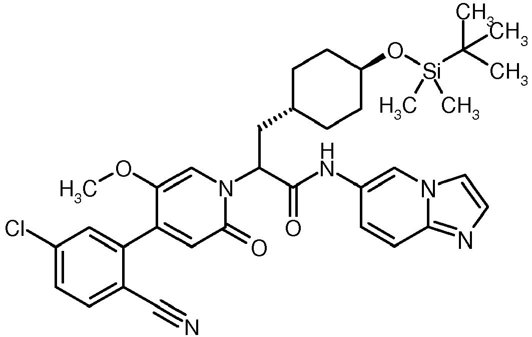

100 mg (183 µmol, 75% Reinheit) 3-(*trans*-4-{[*tert*.-Butyl(dimethyl)silyl]oxy}cyclohexyl)-2-[4-(5-chlor-2-cyanophenyl)-5-methoxy-2-oxopyridin-1(2*H*)-yl]propansäure (Racemat), 24.4 mg (183 µmol) Imidazo[1,2-a]pyridin-6-amin und 26.1 mg (183 mmol) Cyano(hydroxyimino)-ethansäureethylester wurden in 1.84 ml Dimethylformamid vorgelegt und die Lösung für 10 min entgast. Anschließend wurden 29.0 µ (183 µmol) *N*,*N*'-Diisopropylcarbodiimid zugetropft und die resultierende Reaktionslösung über Nacht bei 40°C geschüttelt. Das Lösungsmittel wurde unter reduziertem Druck entfernt, der Rückstand in wenig Dichlormethan aufgenommen und nach säulenchromatographischer Reinigung (24 g Silica Kartusche, Fluss: 35 ml/min, Dichlormethan-Methanol-Gradient) die Titelverbindung erhalten. Ausbeute: 63.1 mg (57% Reinheit, 52% d. Th.)
LC/MS [Methode 1]: Rₜ = 1.11 min; MS (ESIpos): m/z = 660 (M+H)⁺.

### Beispiel 10.1A

### 4-Chlor-2-(5-chlor-2-methoxypyridin-4-yl)benzonitril

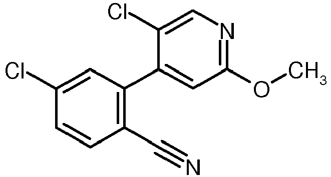

Nach der allgemeinen Methode 2A wurden 5.36 g (91% Reinheit, 26.03 mmol) 5-Chlor-2-methoxypyridin-4-ylboronsäure mit 5.12 g (23.66 mmol) 2-Brom-4-chlorbenzonitril in Gegenwart von [1,1-Bis-(diphenylphosphino)-ferrocen]-palladium(II)chlorid-dichlormethan-Monoaddukt umgesetzt. Nach Aufarbeitung wurde das Rohprodukt anschließend mittels Flash-Chromatographie (Kieselgel-60, Cyclohexan-Dichlormethan-Gemische) aufgereinigt. Ausbeute: 4.11 g (91% Reinheit, 52% d. Th.)
LC/MS [Methode 1]: Rₜ = 1.17 min; MS (ESIpos): m/z = 279 (M+H)⁺.

### Beispiel 10.1B

### 4-Chlor-2-(5-chlor-2-oxo-1,2-dihydropyridin-4-yl)benzonitril

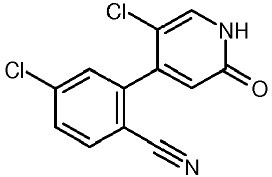

Nach der allgemeinen Methode 3A wurden 6.34 g (93% Reinheit, 21.12 mmol) 4-Chlor-2-(5-chlor-2-methoxypyridin-4-yl)benzonitril mit Pyridinium-Hydrochlorid umgesetzt. Ausbeute: 4.23 g (76% d. Th.)
LC/MS [Methode 1]: Rₜ = 0.82 min; MS (ESIpos): m/z = 265 (M+H)⁺.

### Beispiel 10.1C

### [5-Chlor-4-(5-chlor-2-cyanophenyl)-2-oxopyridin-1(2H)-yl]essigsäure-tert.-butylester

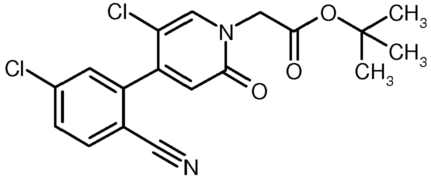

Nach der allgemeinen Methode 4B wurden 3.1 g (11.46 mmol) 4-Chlor-2-(5-chlor-2-oxo-1,2-dihydropyridin-4-yl)benzonitril mit 1.2 eq. Bromessigsäure-*tert*.-butylester bei 100°C umgesetzt. Ausbeute: 3.65 g (84% d. Th.)
LC/MS [Methode 8]: Rₜ = 1.34 min, MS (ESIneg): m/z = 377 (M-H)⁻,
¹H-NMR (400 MHz, DMSO-d₆): δ [ppm] = 8.20 (s, 1H), 8.09-8.20 (m, 1H), 7.85-7.72 (m, 2H), 6.67 (s, 1H), 4.65 (s, 2H), 1.44 (s, 9H).

### Beispiel 10.1D

### 2-[5-Chlor-4-(5-chlor-2-cyanophenyl)-2-oxopyridin-1(2H)-yl]-4-methoxybutansäure-tert.-butylester (Racemat)

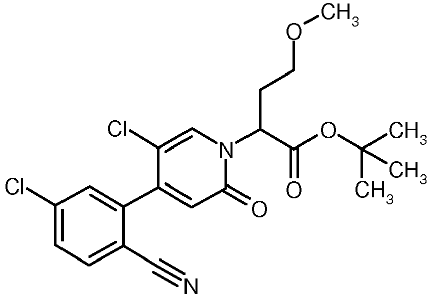

Nach der allgemeinen Methode 8A wurden 2.0 g (5.27 mmol) [5-Chlor-4-(5-chlor-2-cyanophenyl)-2-oxopyridin-1(2*H*)-yl]essigsäure-*tert*.-butylester in Gegenwart von 7.12 ml (7.12 mmol, 1.35 eq.) Bis-(trimethylsilyl)-lithiumamid (1M in THF) mit 1.33 g (95% Reinheit, 6.06 mmol, 1.15 eq.) 2-Methoxyethyl-trifluormethansulfonat umgesetzt. Ausbeute: 2.10 g (94% Reinheit, 86% d. Th.)
LC/MS [Methode 1]: Rₜ = 1.14 min; MS (ESIpos): m/z = 437 (M+H)⁺,
¹H-NMR (400 MHz, DMSO-d₆): δ [ppm] = 8.16-8.10 (m, 1H), 8.09-8.02 (m, 1H), 7.73-7.84 (m, 2H), 6.64 (s, 1H), 5.25-5.07 (m, 1H), 3.44-3.36 (m, 1H), 3.22-3.12 (m, 4H), 2.41-2.27 (m, 2H).

### Beispiel 10.1E

### 2-[5-Chlor-4-(5-chlor-2-cyanophenyl)-2-oxopyridin-1(2H)-yl]-4-methoxybutansäure (Racemat)

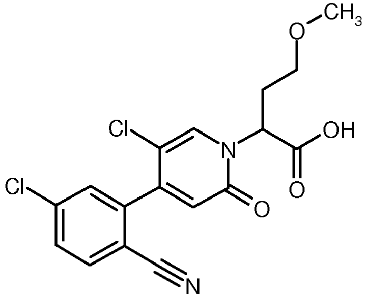

Nach der allgemeinen Methode 6A wurden 2.1 g (94% Reinheit, 4.51 mmol) 2-[5-Chlor-4-(5-chlor-2-cyanophenyl)-2-oxopyridin-1(2*H*)-yl]-4-methoxybutansäure-*tert*. -butylester (Racemat) umgesetzt. Ausbeute: 1.89 g (quant.)
LC/MS [Methode 1]: Rₜ = 0.85 min; MS (ESIpos): m/z = 381 (M+H)⁺,
¹H-NMR (400 MHz, DMSO-d₆): δ [ppm] = 13.19 (br. s, 1H), 8.15 (s, 1H), 8.05 (d, 1H), 7.82 (d, 1H), 7.81-7.76 (m, 1H), 6.63 (s, 1H), 5.31-5.13 (m, 1H), 3.46-3.35 (m, 1H), 3.22-3.08 (m, 4H), 2.43-2.27 (m, 2H).

### Beispiel 11.1A

### Pyridin-2-ylmethyl-methansulfonat

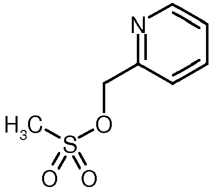

Eine Lösung von 4.00 g (36.65 mmol) Pyridin-2-ylmethanol und 11.24 ml (80.64 mmol, 2.2 eq.) Triethylamin in 122 ml Tetrahydrofuran wurde unter Argon bei 0°C tropfenweise mit einer Lösung von 2.84 ml (36.65 mmol, 1eq.) Methansulfonsäurechlorid in 24 ml Tetrahydrofuran versetzt und 3 h gerührt. Tetrahydrofuran wurde unter reduziertem Druck entfernt. Das Rohprodukt wurde anschließend in Dichlormethan gelöst und die resultierende Mischung mit gesättigter, wässriger Natriumchlorid-Lösung gewaschen. Die organische Phase wurde getrocknet (Natriumsulfat), filtriert und unter reduziertem Druck eingeengt. Das Rohprodukt wurde anschließend mittels Normalphasen-Chromatographie (Eluent: Cyclohexan-Essigsäureethylester (20-50%) Gemische) aufgereinigt. Ausbeute 4.72 g (68% d. Th.)
LC/MS [Methode 3]: Rₜ = 0.98 min; MS (ESIpos): m/z = 188 (M+H)⁺,
¹H-NMR (400 MHz, DMSO-d₆): δ [ppm] = 8.67-8.48 (m, 1H), 7.89 (td, 1H), 7.54 (d, 1H), 7.42 (ddd, 1H), 5.30 (s, 2H), 3.28 (s, 3H).

### Beispiel 11.1B

### 2-[4-(5-Chlor-2-cyanophenyl)-5-methoxy-2-oxopyridin-1(2H)-yl]-3-(pyridin-2-yl)propansäure-tert.-butylester (Racemat)

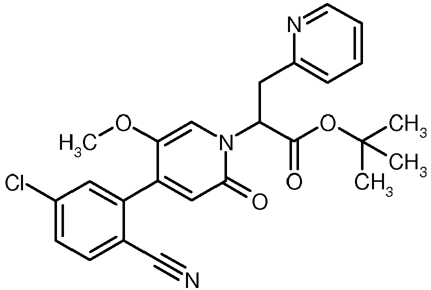

Eine Lösung von 1.50 g (4.00 mmol) [4-(5-Chlor-2-cyanophenyl)-5-methoxy-2-oxopyridin-1(2*H*)-yl]essigsäure-*tert*.-butylester in 30 ml Tetrahydrofuran wurde unter Argon bei -78°C tropfenweise mit 4.60 ml (1.0M in THF, 1.15 eq.) Bis-(trimethylsilyl)-lithiumamid versetzt und 15 min gerührt. Anschließend wurden 1.06 g (5.6 mmol, 1.4 eq.) Pyridin-2-ylmethyl-methansulfonat als Reinsubstanz hinzugegeben. Die resultierende Reaktionsmischung wurde 30 min bei -78°C und 1.5 h bei RT nachgerührt. Das Reaktionsgemisch wurde mit gesättigter, wässriger Ammoniumchlorid-Lösung versetzt. Nach Phasentrennung wurde die wässrige Phase mit Essigsäureethylester extrahiert. Die vereinigten, organischen Phasen wurden mit gesättigter, wässriger Natriumchlorid-Lösung gewaschen. Die organische Phase wurde getrocknet (Natriumsulfat), filtriert und unter reduziertem Druck eingeengt. Das Rohprodukt wurde anschließend mittels Normalphasen-Chromatographie (Eluent: Dichlormethan-Methanol (2-5%) Gemische) aufgereinigt. Ausbeute 1.99 g (93% Reinheit, 99% d. Th.)
LC/MS [Methode 1]: Rₜ = 0.97 min; MS (ESIpos): m/z = 466 (M+H)⁺.

### Beispiel 11.1C

### 2-[4-(5-Chlor-2-cyanophenyl)-5-methoxy-2-oxopyridin-1(2H)-yl]-3-(pyridin-2-yl)propansäure (Racemat)

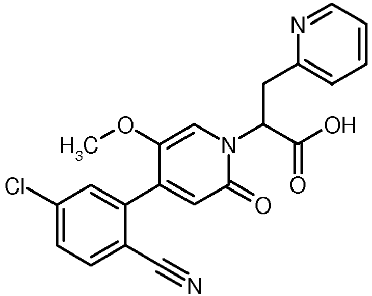

Gemäß allgemeiner Methode 6A wurden 1.99 g (93% Reinheit, 3.98 mmol) 2-[4-(5-Chlor-2-cyanophenyl)-5-methoxy-2-oxopyridin-1(2*H*)-yl]-3-(pyridin-2-yl)propansäure-*tert*.-butylester (Racemat) in 40 ml Dichlormethan mit 20 ml (259.6 mmol) TFA umgesetzt. Ausbeute: 220 mg (93% Reinheit, 13% d. Th.)
LC/MS [Methode 1]: Rₜ = 0.64 min; MS (ESIpos): m/z = 410 (M+H)⁺,
¹H-NMR (400 MHz, DMSO-d₆): δ [ppm] = 13.08 (br. s, 1H), 8.48 (d, 1H), 7.95 (d, 1H), 7.73-7.60 (m, 3H), 7.27 (s, 1H), 7.24-7.11 (m, 2H), 6.40 (s, 1H), 5.55 (t, 1H), 3.66-3.57 (m, 2H), 3.49 (s, 3H).

### Beispiel 12.1A

### 5-(Brommethyl)-1,3-oxazol

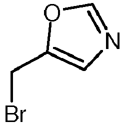

Eine Lösung von 1.83 ml (13.12 mmol, 1.3 eq.) Triethylamin und 1.0 g (10.09 mmol, 1 eq.) 1,3-Oxazol-5-ylmethanol in 14 ml *N*,*N*-Dimethylformamid wurde unter Argon bei 0°C tropfenweise mit 1.02 ml (13.12 mmol, 1.3 eq.) Methansulfonsäurechlorid versetzt und 1 h bei 0°C gerührt. Anschließend wurden 2.45 g (28.26 mmol, 2.8 eq.) Lithiumbromid zugegeben, und diese Reaktionsmischung wurde 1 h bei 0°C gerührt. Nach Zugabe von Wasser wurde die Mischung mit Essigsäureethylester extrahiert. Die vereinigten organischen Phasen wurden mit gesättigter, wässriger Natriumchlorid-Lösung gewaschen, über Natriumsulfat getrocknet und im Vakuum eingeengt. Das Rohprodukt wurde anschließend mittels Normalphasen-Chromatographie (Eluent: Dichlormethan) aufgereinigt. Ausbeute 1.23 g (80% Reinheit, 60% d. Th.)
¹H-NMR (400 MHz, DMSO-d₆): δ [ppm] = 8.42 (s, 1H), 7.26 (s, 1H), 4.93 (s, 2H).

### Beispiel 12.1B

### 2-[4-(5-Chlor-2-cyanophenyl)-5-methoxy-2-oxopyridin-1(2H)-yl]-3-(1,3-oxazol-5-yl)propansäure-tert.-butylester (Racemat)

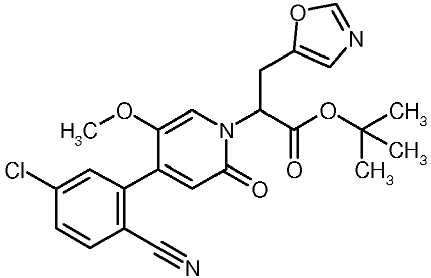

Nach der allgemeinen Methode 8B wurden 1.5 g (4.00 mmol) [4-(5-Chlor-2-cyanophenyl)-5-methoxy-2-oxopyridin-1(2*H*)-yl]essigsäure-*tert*.-butylester mit 1.78 g (51% Reinheit, 5.60 mmol, 1.4 eq.) 5-(Brommethyl)-1,3-oxazol umgesetzt. Ausbeute: 1.89 g (60% Reinheit, 62% d. Th.)
LC/MS [Methode 1]: Rₜ = 0.98 min; MS (ESIpos): m/z = 456 (M+H)⁺.

### Beispiel 12.1C

### 2-[4-(5-Chlor-2-cyanophenyl)-5-methoxy-2-oxopyridin-1(2H)-yl]-3-(1,3-oxazol-5-yl)propansäure (Racemat)

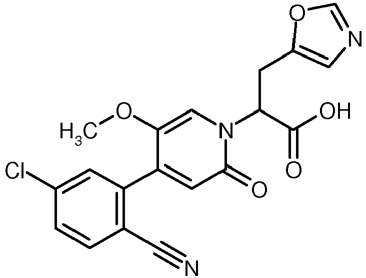

Gemäß allgemeiner Methode 6A wurden 1.89 g (60% Reinheit, 2.48 mmol) 2-[4-(5-Chlor-2-cyanophenyl)-5-methoxy-2-oxopyridin-1(2*H*)-yl]-3-(1,3-oxazol-5-yl)propansäure-*tert*.-butylester (Racemat) in 28 ml Dichlormethan mit 14 ml (435 mmol) TFA umgesetzt. Ausbeute: 597 mg (80% Reinheit, 48% d. Th.)
LC/MS [Methode 1]: Rₜ = 0.70 min; MS (ESIpos): m/z = 400 (M+H)⁺,
¹H-NMR (400 MHz, DMSO-d₆): δ [ppm] = 13.24 (br. s, 1H), 8.17 (s, 1H), 8.02-7.93 (m, 1H), 7.77-7.66 (m, 2H), 7.35 (s, 1H), 6.85 (s, 1H), 6.47 (s, 1H), 5.32 (dd, 1H), 3.63-3.72 (m, 1H), 3.58-3.47 (m, 4H).

### Beispiel 13.1A

### 2-[5-Chlor-4-(5-chlor-2-cyanophenyl)-2-oxopyridin-1(2H)-yl]-3-(1,3-oxazol-5-yl)propansäure-tert.-butylester (Racemat)

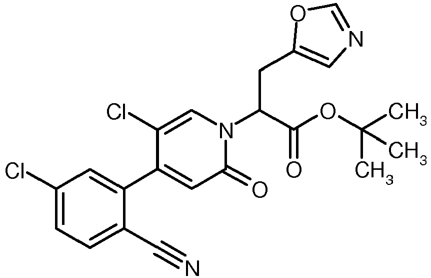

Nach der allgemeinen Methode 8B wurden 610 mg (1.61 mmol) [5-Chlor-4-(5-chlor-2-cyanophenyl)-2-oxopyridin-1(2*H*)-yl]essigsäure-*tert*.-butylester mit 1.57 g (23% Reinheit, 2.25 mmol, 1.4 eq.) 5-(Brommethyl)-1,3-oxazol umgesetzt. Ausbeute: 468 mg (83% Reinheit, 52% d. Th.)
LC/MS [Methode 1]: Rₜ = 1.05 min; MS (ESIpos): m/z = 460 (M+H)⁺.

### Beispiel 13.1B

### 2-[5-Chlor-4-(5-chlor-2-cyanophenyl)-2-oxopyridin-1(2H)-yl]-3-(1,3-oxazol-5-yl)propansäure (Racemat)

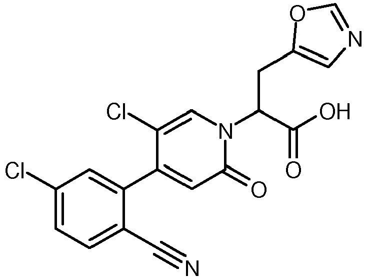

Gemäß allgemeiner Methode 6A wurden 468 mg (83% Reinheit, 0.84 mmol) 2-[5-Chlor-4-(5-chlor-2-cyanophenyl)-2-oxopyridin-1(2*H*)-yl]-3-(1,3-oxazol-5-yl)propansäure-*tert*.-butylester (Racemat) in 9 ml Dichlormethan mit 4.5 ml (58.4 mmol) TFA umgesetzt. Ausbeute: 290 mg (85% Reinheit, 72% d. Th.)
LC/MS [Methode 1]: Rₜ = 0.76 min; MS (ESIpos): m/z = 404 (M+H)⁺,
¹H-NMR (400 MHz, DMSO-d₆): δ [ppm] = 13.48 (br. s, 1H), 8.17 (s, 1H), 8.10 (s, 1H), 8.08-8.01 (m, 1H), 7.81-7.75 (m, 2H), 6.87 (s, 1H), 6.64 (s, 1H), 5.39 (br. s, 1H), 3.65 (dd, 1H), 3.56 (dd, 1H).

### Beispiel 14.1A

### 6-Methoxypyridin-3-ol

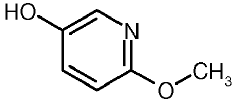

Eine Lösung von 46.0 g (392 mmol) *N*-Methylmorpholin-*N*-Oxid in 500 ml Dichlormethan wurde bei RT mit 50 g (327 mmol) 6-Methoxypyridin-3-ylboronsäure versetzt und 14 h bei 50°C gerührt. Es wurde noch zusätzliches *N*-Methylmorpholin-*N*-Oxid bis zur vollständigen Umsetzung zugegeben. Das Reaktionsgemisch wurde im Vakuum eingeengt und das Rohprodukt mittels Flash-Chromatographie (Kieselgel-60, Cyclohexan-Essigsäureethylester-Gemische) aufgereinigt. Ausbeute: 32.9 g (80% d. Th.)
LC/MS [Methode 1]: Rₜ = 0.37 min; MS (ESIpos): m/z = 126 (M+H)⁺,
¹H-NMR (400 MHz, DMSO-d₆): δ [ppm] = 9.27 (s, 1H), 7.67 (d, 1H), 7.16 (dd, 1H), 6.66 (d, 1H), 3.74 (s, 3H).

### Beispiel 14.1B

### 2-Methoxy-5-(tetrahydro-2H-pyran-2-yloxy)pyridin

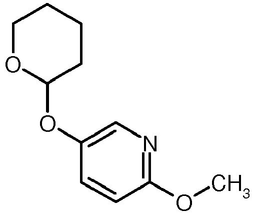

Eine Lösung von 10.0 g (79.9 mmol) 6-Methoxypyridin-3-ol in 150 ml Dichlormethan wurde mit 10.1 g (119.9 mmol, 1.5 eq.) 3,4-Dihydro-2*H*-pyran und 1.4 g (8.0 mmol, 0.1 eq.) 4-Toluolsulfonsäure versetzt und 5 Tage bei RT gerührt. Nach Zugabe von Wasser/Dichlormethan und Phasentrennung wurde die wässrige Phase mit Dichlormethan extrahiert. Die vereinigten, organischen Phasen wurden getrocknet (Natriumsulfat), filtriert und im Vakuum eingeengt. Ausbeute: 17.3 g (100% d. Th.)
LC/MS [Methode 1]: Rₜ = 0.95 min; MS (ESIpos): m/z = 210 (M+H)⁺.

### Beispiel 14.1C

### 4-Iod-2-methoxy-5-(tetrahydro-2H-pyran-2-yloxy)pyridin

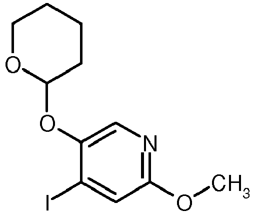

Eine Lösung von 16.2 g (75.1 mmol) 2-Methoxy-5-(tetrahydro-2*H*-pyran-2-yloxy)pyridin in 250 ml THF wurde bei -78°C mit 13.6 ml (90.1 mmol, 1.2 eq.) 1,2-Bis(dimethylamino)ethan und 54.0 ml (86.4 mmol, 1.15 eq.) *n*-Butyllithium versetzt und 1h bei -78°C gerührt. Anschließend wurde das Reaktionsgemisch mit 24.8 g (97.6 mmol, 1.3 eq.) Iod versetzt, 1 h bei -78°C gerührt und dann über Nacht auf RT kommen gelassen. Das Reaktionsgemisch wurde mit Wasser gequencht und dreimal mit Essigsäureethylester extrahiert. Die vereinigten, organischen Phasen wurden mit gesättigter Natriumthiosulfat-Lösung gewaschen, getrocknet (Natriumsulfat), filtriert und im Vakuum eingeengt. Ausbeute: 25.1 g (82% Reinheit, 82% d. Th.)
LC/MS [Methode 1]: Rₜ = 1.18 min; MS (ESIpos): m/z = 336 (M+H)⁺.

### Beispiel 14.1D

### 4-Iod-6-methoxypyridin-3-ol

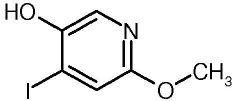

Eine Lösung von 25.1 g (82% Reinheit, 61.3 mmol) 4-Iod-2-methoxy-5-(tetrahydro-2*H*-pyran-2-yloxy)pyridin in 50 ml Dioxan und 50 ml Wasser wurde mit 50 ml (3 molar, 150 mmol) Salzsäure versetzt und 2 h bei RT gerührt. Anschließend wurde das Reaktionsgemisch filtriert, der Niederschlag mit Wasser nachgewachsen und am Hochvakuum getrocknet. Ausbeute: 13.5 g (93% Reinheit, 81% d. Th.)
LC/MS [Methode 1]: Rₜ = 0.76 min; MS (ESIpos): m/z = 252 (M+H)⁺,
¹H-NMR (400 MHz, DMSO-d₆): δ [ppm] = 7.70 (s, 1H), 7.22 (s, 1H), 3.74 (s, 3H).

### Beispiel 14.1E

### 5-(Difluormethoxy)-4-iod-2-methoxypyridin

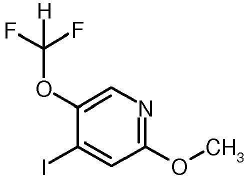

Eine Lösung von 600 mg (93% Reinheit, 2.22 mmol) 4-Iod-6-methoxypyridin-3-ol in 4.8 ml Acetonitril wurde mit 4.8 ml wässriger Kaliumhydroxid-Lösung (6M) versetzt, im Eisbad gekühlt und unter starkem Rühren mit 863 µl (75% Reinheit, 3.56 mmol, 1.6 eq.) Difluormethyltrifluormethansulfonat [Angew. Chem. Int. Ed. 2013, 52, 1-5; Journal of Fluorine Chemistry 2009, 130, 667-670] versetzt. Das Reaktionsgemisch wurde 2 min gerührt und mit 33 ml Wasser verdünnt. Die wässrige Phase wurde zweimal mit je 40 ml Diethylether extrahiert. Die vereinigten, organischen Phasen wurden getrocknet (Natriumsulfat), filtriert, im Vakuum eingeengt und getrocknet. Das Rohprodukt wurde mittels Flash-Chromatographie (Kieselgel, Petrolether-Essigsäureethylester (12-20%) Gemische) aufgereinigt. Ausbeute: 407 mg (90% Reinheit, 55% d.Th.)
¹H-NMR (400 MHz, DMSO-d₆): δ [ppm] = 8.1 (s, 1H), 7.45 (s, 1H), 7.16 (t, 1H), 3.84 (s, 3H).

### Beispiel 14.1F

### 4-Chlor-2-[5-(difluormethoxy)-2-methoxypyridin-4-yl]benzonitril

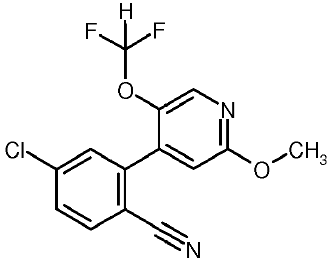

Nach der allgemeinen Methode 2A wurden 460 mg (90% Reinheit, 1.38 mmol) 5-(Difluormethoxy)-4-iod-2-methoxypyridin mit 299 mg (1.65 mmol, 1.2 eq.) 5-Chlor-2-cyanophenylboronsäure in Gegenwart von [1,1-Bis-(diphenylphosphino)-ferrocen]-palladium(II)chlorid-dichlormethan-Monoaddukt umgesetzt. Das Rohprodukt wurde mittels Flash-Chromatographie (Kieselgel, Petrolether-Essigsäureethylester (10-15%) Gemische) aufgereinigt. Ausbeute: 230 mg (80% Reinheit, 43% d. Th.)
LC/MS [Methode 1]: Rₜ = 1.12 min; MS (ESIpos): m/z = 311 (M+H)⁺,
¹H-NMR (400 MHz, DMSO-d₆): δ [ppm] = 8.26 (s, 1H), 8.06 (d, 1H), 7.82-7.74 (m, 2H), 7.09 (s, 1H), 7.06 (t, 1H), 3.91 (s, 3H).

### Beispiel 14.1G

### 4-Chlor-2-[5-(difluormethoxy)-2-oxo-1,2-dihydropyridin-4-yl]benzonitril

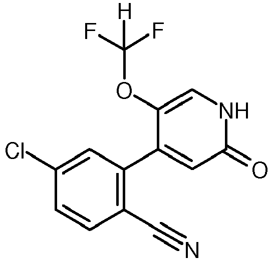

Nach der allgemeinen Methode 3A wurden 230 mg (80% Reinheit, 0.59 mmol) 4-Chlor-2-[5-(difluormethoxy)-2-methoxypyridin-4-yl]benzonitril mit Pyridinium-Hydrobromid umgesetzt. Das Rohprodukt wurde mittels Flash-Chromatographie (Kieselgel, Dichlormethan-Methanol (3-25%) Gemische) aufgereinigt. Ausbeute: 167 mg (95% d. Th.)
LC/MS [Methode 1]: Rₜ = 0.79 min; MS (ESIpos): m/z = 297 (M+H)⁺,
¹H-NMR (400 MHz, DMSO-d₆): δ [ppm] = 11.88 (br. s, 1H), 8.03 (d, 1H), 7.80-7.65 (m, 3H), 6.87 (t, 1H), 6.56 (s, 1H).

### Beispiel 14.1H

### [4-(5-Chlor-2-cyanophenyl)-5-(difluormethoxy)-2-oxopyridin-1(2H)-yllessigsäure-tert.-butylester

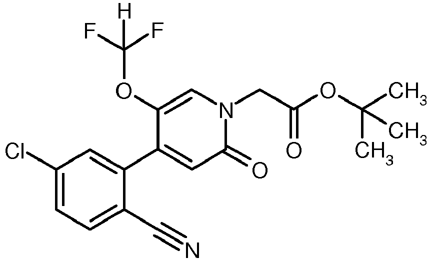

Nach der allgemeinen Methode 4B wurden 1.19 g (92% Reinheit, 3.69 mmol) 4-Chlor-2-[5-(difluormethoxy)-2-oxo-1,2-dihydropyridin-4-yl]benzonitril mit 1.2 eq. Bromessigsäure-*tert*.-butylester bei 100°C umgesetzt. Ausbeute: 1.30 g (95% Reinheit, 81% d. Th.)
LC/MS [Methode 1]: Rₜ = 0.97 min; MS (ESIpos): m/z = 411 (M+H)⁺,
¹H-NMR (400 MHz, DMSO-d₆): δ [ppm] = 8.09-7.97 (m, 2H), 7.81-7.70 (m, 2H), 6.81 (t, 1H), 6.63 (s, 1H), 4.66 (s, 2H), 1.44 (s, 9H).

### Beispiel 14.11

### 2-[4-(5-Chlor-2-cyanophenyl)-5-(difluormethoxy)-2-oxopyridin-1(2H)-yl]-3-(1,3-oxazol-5-yl)propansäure-tert.-butylester (Racemat)

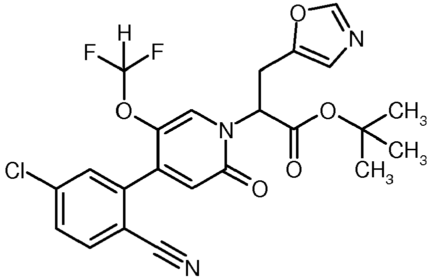

Nach der allgemeinen Methode 8B wurden 600 mg (1.39 mmol) [4-(5-Chlor-2-cyanophenyl)-5-(difluormethoxy)-2-oxopyridin-1(2*H*)-yl]essigsäure-*tert*.-butylester mit 421mg (80% Reinheit, 2.08 mmol, 1.5 eq.) 5-(Brommethyl)-1,3-oxazol umgesetzt. Ausbeute: 320 mg (47% d. Th.)
LC/MS [Methode 1]: Rₜ = 0.97 min; MS (ESIpos): m/z = 492 (M+H)⁺,
¹H-NMR (400 MHz, DMSO-d₆): δ [ppm] = 8.18 (s, 1H), 8.03 (d, 1H), 7.86 (s, 1H), 7.82-7.71 (m, 2H), 6.90 (s, 1H), 6.72 (t, 1H), 6.62 (s, 1H), 5.35 (dd, 1H), 3.68-3.48 (m, 2H), 1.40 (s, 9H).

### Beispiel 14.1J

### 2-[4-(5-Chlor-2-cyanophenyl)-5-(difluormethoxy)-2-oxopyridin-1(2H)-yl]-3-(1,3-oxazol-5-yl)propansäure (Racemat)

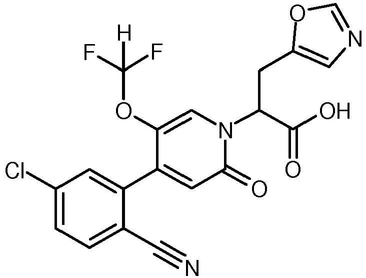

Gemäß allgemeiner Methode 6A wurden 320 mg (0.65 mmol) 2-[4-(5-Chlor-2-cyanophenyl)-5-(difluormethoxy)-2-oxopyridin-1(2*H*)-yl]-3-(1,3-oxazol-5-yl)propansäure-*tert*.-butylester (Racemat) in 10 ml Dichlormethan mit 5 ml (64.9 mmol) TFA umgesetzt. Ausbeute: 290 mg (quant.)
LC/MS [Methode 1]: Rₜ = 0.74 min; MS (ESIpos): m/z = 436 (M+H)⁺,
¹H-NMR (400 MHz, DMSO-d₆): δ [ppm] = 13.42 (br. s, 1H), 8.15 (s, 1H), 8.03 (d, 1H), 7.87 (s, 1H), 7.81-7.69 (m, 2H), 6.86 (s, 1H), 6.72 (t, 1H), 6.60 (s, 1H), 5.37 (dd, 1H), 3.64 (dd, 2H), 3.53 (dd, 1H).

### Beispiel 15.1A

### 4-(Brommethyl)-1,3-oxazol

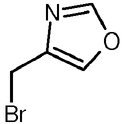

Eine Lösung von 1.91 ml (13.72 mmol, 1.3 eq.) Triethylamin und 1.05 g (10.56 mmol) 1,3-Oxazol-4-ylmethanol in 15 ml *N*,*N*-Dimethylformamid wurde unter Argon bei 0°C tropfenweise mit 1.06 ml (13.72 mmol, 1.3 eq.) Methansulfonsäurechlorid versetzt und 1 h bei 0°C gerührt. Anschließend wurden 2.57 g (29.56 mmol, 2.8 eq.) Lithiumbromid zugegeben, und die Reaktionsmischung wurde 1 h bei 0°C gerührt. Nach Zugabe von Wasser wurde die Mischung mit Essigsäureethylester extrahiert. Die vereinigten organischen Phasen wurden mit gesättigter, wässriger Natriumchlorid-Lösung gewaschen, über Natriumsulfat getrocknet und im Vakuum eingeengt. Das Rohprodukt wurde ohne weitere Aufarbeitung umgesetzt. Ausbeute 1.97 g (50% Reinheit, 58% d. Th.)
¹H-NMR (400 MHz, DMSO-d₆): δ [ppm] = 8.40 (s, 1H), 8.18 (s, 1H), 4.59 (s, 2H).

### Beispiel 15.1B

### 2-[4-(5-Chlor-2-cyanophenyl)-5-methoxy-2-oxopyridin-1(2H)-yl]-3-(1,3-oxazol-4-yl)propansäure-tert.-butylester (Racemat)

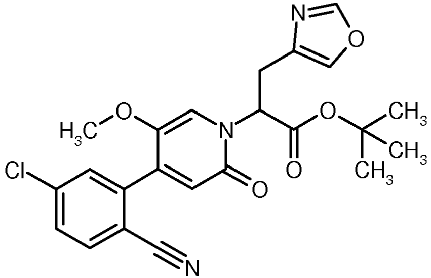

Nach der allgemeinen Methode 8B wurden 813 mg (2.17 mmol) [4-(5-Chlor-2-cyanophenyl)-5-methoxy-2-oxopyridin-1(2*H*)-yl]essigsäure-*tert*.-butylester mit 983.8 mg (50% Reinheit, 3.04 mmol, 1.4 eq.) 4-(Brommethyl)-1,3-oxazol umgesetzt. Ausbeute: 655 mg (65% d. Th.)
LC/MS [Methode 1]: Rₜ = 0.98 min; MS (ESIpos): m/z = 456 (M+H)⁺,
¹H-NMR (400 MHz, DMSO-d₆): δ [ppm] = 8.28 (s, 1H), 7.97 (d, 1H), 7.78 (s, 1H), 7.75-7.61 (m, 2H), 7.31 (s, 1H), 6.45 (s, 1H), 5.34 (dd, 1H), 3.56 (s, 3H), 3.50-3.39 (m, 1H), 3.36-3.26 (m, 1H), 1.41 (s, 9H).

### Beispiel 15.1C

### 2-[4-(5-Chlor-2-cyanophenyl)-5-methoxy-2-oxopyridin-1(2H)-yl]-3-(1,3-oxazol-4-yl)propansäure (Racemat)

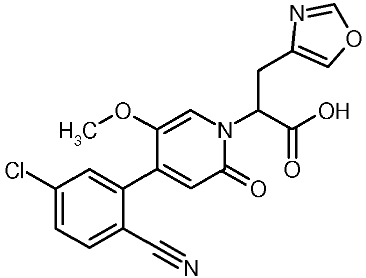

Gemäß allgemeiner Methode 6A wurden 655 mg (1.41 mmol) 2-[4-(5-Chlor-2-cyanophenyl)-5-methoxy-2-oxopyridin-1(2*H*)-yl]-3-(1,3-oxazol-4-yl)propansäure-*tert*.-butylester (Racemat) in 14 ml Dichlormethan mit 7 ml (90.86 mmol) TFA umgesetzt. Ausbeute: 403 mg (70% d. Th.)
LC/MS [Methode 1]: Rₜ = 0.73 min; MS (ESIpos): m/z = 400 (M+H)⁺,
¹H-NMR (400 MHz, DMSO-d₆): δ [ppm] = 13.14 (br. s, 1H), 8.26 (s, 1H), 7.97 (d, 1H), 7.78-7.65 (m, 3H), 7.33 (s, 1H), 6.43 (s, 1H), 5.36 (dd, 1H), 3.55 (s, 3H), 3.53-3.43 (m, 1H), 3.38-3.25 (m, 1H).

### Beispiel 16.1A

### 2-[5-Chlor-4-(5-chlor-2-cyanophenyl)-2-oxopyridin-1(2H)-yl]-3-(1,3-oxazol-4-yl)propansäure-tert.-butylester (Racemat)

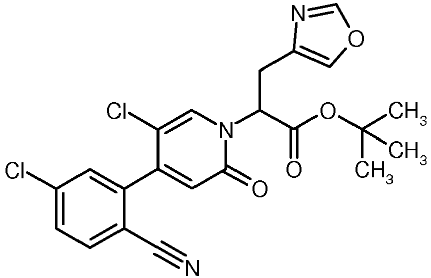

Nach der allgemeinen Methode 8B wurden 600 mg (1.58 mmol) [5-Chlor-4-(5-chlor-2-cyanophenyl)-2-oxopyridin-1(2*H*)-yl]essigsäure-*tert*.-butylester mit 717.6 mg (50% Reinheit, 2.22 mmol, 1.4 eq.) 4-(Brommethyl)-1,3-oxazol umgesetzt. Ausbeute: 530 mg (73% d. Th.)
LC/MS [Methode 1]: Rₜ = 1.07 min; MS (ESIpos): m/z = 460 (M+H)⁺,
¹H-NMR (400 MHz, DMSO-d₆): δ [ppm] = 8.29 (s, 1H), 8.11-7.97 (m, 2H), 7.87-7.69 (m, 3H), 6.62 (s, 1H), 5.45-5.25 (m, 1H), 3.55-3.38 (m, 1H), 3.38-3.25 (m, 1H), 1.41 (s, 9H).

### Beispiel 16.1B

### 2-[5-Chlor-4-(5-chlor-2-cyanophenyl)-2-oxopyridin-1(2H)-yl]-3-(1,3-oxazol-4-yl)propansäure (Racemat)

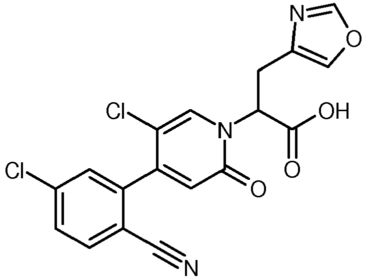

Gemäß allgemeiner Methode 6A wurden 530 mg (1.15 mmol) 2-[5-Chlor-4-(5-chlor-2-cyanophenyl)-2-oxopyridin-1(2*H*)-yl]-3-(1,3-oxazol-4-yl)propansäure-*tert*.-butylester (Racemat) in 12 ml Dichlormethan mit 6 ml (77.9 mmol) TFA umgesetzt. Ausbeute: 359 mg (77% d. Th.)
LC/MS [Methode 1]: Rₜ = 0.78 min; MS (ESIpos): m/z = 404 (M+H)⁺,
¹H-NMR (400 MHz, DMSO-d₆): δ [ppm] = 13.36 (br. s, 1H), 8.26 (s, 1H), 8.11-7.98 (m, 2H), 7.87-7.67 (m, 3H), 6.59 (s, 1H), 5.42 (dd, 1H), 3.59-3.41 (m, 1H), 3.38-3.28 (m, 1 H).

### Beispiel 17.1A

### 1,3-Acetondicarbonsäuredibenzylester

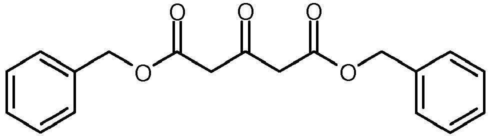

14.1 g (81.0 mmol) 1,3-Acetondicarbonsäuredimethylester und 16.8 ml (162 mmol) Benzylalkohol wurden bei Raumtemperatur zusammen gegeben. Es wurde bei 170-180°C gerührt und entstandenes Methanol abdestilliert. Anschließend wurde erst auf Raumtemperatur abgekühlt und dann überschüßiges Methanol und Benzylalkohol bei 1 mbar und maximal 150°C abdestilliert. Der Rückstand wurde mittels Flashchromatographie (500 g Silica Kartusche, Cyclohexan-Essigsäureethylester-Gradient) getrennt und die Titelverbindung erhalten. Ausbeute 9.0 g (74% Reinheit, 25% d. Th.)
LC/MS [Methode 3]: Rₜ = 2.38 min; MS (ESIneg): m/z = 325 (M-H)⁻.

### Beispiel 17.1B

### 1-(1-tert.-Butoxy-1-oxobutan-2-yl)-4-hydroxy-6-oxo-1,6-dihydropyridin-3-carbonsäurebenzylester

### (Racemat)

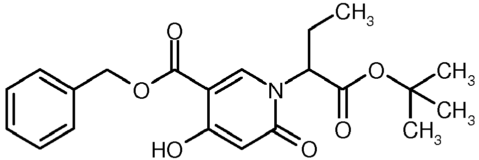

1.00 g (74% Reinheit, 2.27 mmol) 1,3-Acetondicarbonsäuredibenzylester und 515 mg (3.17 mmol) Diethoxymethylacetat wurden für 2.5 h bei 100°C refluxiert. Es wurde auf Raumtemperatur abgekühlt und die Reaktionsmischung dreimal mit Toluol codestilliert. Der Rückstand wurde in 8 ml Ethanol gelöst und bei 0°C mit einer Lösung von 387 mg (2.38 mmol) 2-Aminobutansäure-*tert*.-butylester in 2 ml Ethanol versetzt. Es wurde für 1 h bei Raumtemperatur nachgerührt und anschließend 0.53 ml (2.3 mmol) Natriumethylat (21% in Ethanol) zugetropft. Nach 30 min bei Raumtemperatur wurden weitere 0.26 ml (1.2 mmol) Natriumethylat (21% in Ethanol) zugegeben und es wurde für 30 min nachgerührt. Die Reaktion wurde durch Zugabe von 50 ml gesättigter, wässriger Ammoniumchlorid-Lösung und 25 ml Essigsäureethylester beendet. Die Phasen wurden getrennt und die wässrige Phase dreimal mit 50 ml Essigsäureethylester extrahiert. Die vereinigten organischen Phasen wurden mit gesättigter, wässriger Natriumchlorid-Lösung gewaschen, über Magnesiumsulfat getrocknet, filtriert und das Lösungsmittel unter reduziertem Druck entfernt. Das Rohprodukt wurde mittels Flashchromatographie (100 g Silica Kartusche, Cyclohexan-Essigsäureethylester-Gradient) getrennt und die Titelverbindung erhalten. Ausbeute 0.46 g (75% Reinheit, 39% d. Th.)
LC/MS [Methode 1]: Rₜ = 1.13 min; MS (ESIpos): m/z = 388 (M+H)⁺.

### Beispiel 17.1C

### 2-{5-[(Benzyloxy)carbonyl]-4-hydroxy-2-oxopyridin-1(2H)-yl}butansäure (Racemat)

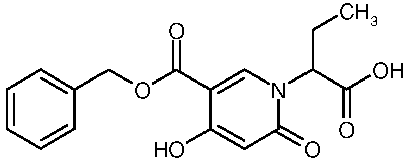

Eine Lösung von 460 mg (1.19 mmol) 1-(1-*tert*.-Butoxy-1-oxobutan-2-yl)-4-hydroxy-6-oxo-1,6-dihydropyridin-3-carbonsäurebenzylester (Racemat) in 1.2 ml Dichlormethan wurde bei 0°C (Eisbadkühlung) mit 0.92 ml (12 mmol) Trifluoressigsäure versetzt. Es wurde auf Raumtemperatur erwärmt und anschließend für 3 h nachgerührt. Das Lösungsmittel wurde unter reduziertem Druck entfernt und der Rückstand dann dreimal mit 10 ml Toluol codestilliert. Das Rohprodukt wurde mittels präparativer HPLC [Säule: Chromatorex C18, 10 µm, 125 mm x 30 mm, Eluent: Acetonitril/0.05%-Ameisensäure-Gradient (0 bis 3 min 10% Acetonitril, bis 35 min 90% Acetonitril und weitere 3 min 90% Acetonitril)] gereinigt und die Titelverbindung erhalten. Ausbeute 193 mg (49% d. Th.)
LC/MS [Methode 1]: Rₜ = 0.85 min; MS (ESIpos): m/z = 332 (M+H)⁺,
¹H-NMR (400 MHz, DMSO-d₆): δ [ppm] = 13.0 (br. s, 1H), 10.9 (s, 1H), 8.38 (s, 1H), 7.48-7.32 (m, 5H), 5.70 (s, 1H), 5.37-5.28 (m, 2H), 5.07 (dd, 1H), 2.16-1.93 (m, 2H), 0.78 (t, 3H).

### Beispiel 17.1D

### 4-Hydroxy-6-oxo-1-[1-oxo-1-(pyrazol[1,5-a]pyridin-5-ylamino)butan-2-yl]-1,6-dihydropyridin-3-carbonsäurebenzylester (Racemat)

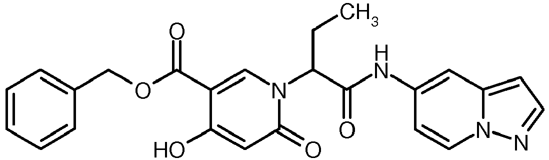

Nach der allgemeinen Methode 5D wurden 193 mg (583 µmol) 2-{5-[(Benzyloxy)carbonyl]-4-hydroxy-2-oxopyridin-1(2*H*)-yl}butansäure (Racemat) und 116 mg (874 µmol, 1.5 eq.) Pyrazol[1,5-a]pyridin-5-amin umgesetzt. Ausbeute: 233 mg (94% Reinheit, 84% d. Th.)
LC/MS [Methode 1]: Rₜ = 0.95 min; MS (ESIpos): m/z = 447 (M+H)⁺.

### Beispiel 17.1E

### 6-Oxo-1-[1-oxo-1-(pyrazol[1,5-a]pyridin-5-ylamino)butan-2-yl]-4-{[(trifluormethyl)sulfonyl]-oxy}-1,6-dihydropyridin-3-carbonsäurebenzylester (Racemat)

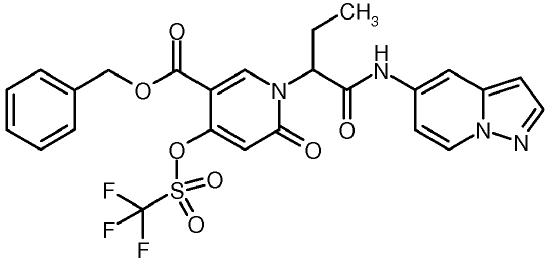

233 mg (94% Reinheit, 491 µmol) 4-Hydroxy-6-oxo-1-[1-oxo-1-(pyrazol[1,5-*a*]pyridin-5-ylamino)butan-2-yl]-1,6-dihydropyridin-3-carbonsäurebenzylester (Racemat) wurden in 5 ml Dichlormethan gelöst und die Reaktionslösung auf -78°C abgekühlt. Bei -78°C wurden 171 µl (1.23 mmol) Triethylamin und 303 mg (736 µmol) 1-{Bis[(trifluormethyl)sulfonyl]methyl}-4-*tert*.-butylbenzol hinzugegeben und es wurde über Nacht bei Raumtemperatur nachgerührt. Anschließend wurden 3 ml Dimethylformamid zugetropft und für 1 h bei Raumtemperatur nachgerührt. Das Lösungsmittel wurde unter reduziertem Druck entfernt. Der Rückstand mittels Flashchromatographie (Cyclohexan-Essigsäureethylester-Gradient) aufgereinigt und die Titelverbindung erhalten. Ausbeute: 185 mg (65% d. Th.)
LC/MS [Methode 1]: Rₜ = 1.15 min; MS (ESIpos): m/z = 579 (M+H)⁺.

### Beispiel 17.1F

### 4-(5-Chlor-2-cyanophenyl)-6-oxo-1-[1-oxo-1-(pyrazol[1,5-a]pyridin-5-ylamino)butan-2-yl]-1,6-dihydropyridin-3-carbonsäurebenzylester (Racemat)

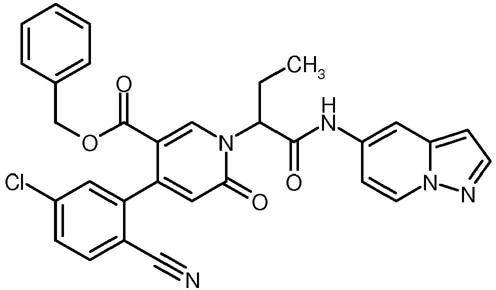

133 mg (959 µmol) Kaliumcarbonat wurden im Reaktionsgefäß getrocknet und anschließend 185 mg (320 µmol) 6-Oxo-1-[1-oxo-1-(pyrazol[1,5-*a*]pyridin-5-ylamino)butan-2-yl]-4-{[(trifluor-methyl)sulfonyl]-oxy}-1,6-dihydropyridin-3-carbonsäurebenzylester (Racemat), 67 mg (0.37 mmol) 5-Chlor-2-cyanophenylboronsäure und 4 ml Dioxan hinzugegeben. Die Suspension wurde entgast, mit 37 mg (32 µmol) Tetrakis(triphenylphosphin)palladium(0) versetzt und für 1 h bei 110°C geschüttelt. Die Reaktion wurde durch Zugabe von Wasser und Essigsäureethylester beendet. Es wurde mit 1N Salzsäure auf pH 6 angesäuert und die Phasen getrennt. Die wässrige Phase wurde dreimal mit Essigsäureethylester extrahiert, die vereinigten organischen Phasen über Magnesiumsulfat getrocknet, filtriert und das Lösungsmittel unter reduziertem Druck entfernt. Das Rohprodukt wurde mittels Flashchromatographie (Cyclohexan-Essigsäureethylester-Gradient) gereinigt und die Titelverbindung erhalten. Ausbeute: 147 mg (80% d. Th.)
LC/MS [Methode 1]: Rₜ = 1.11 min; MS (ESIpos): m/z = 566 (M+H)⁺.

### Ausführungsbeispiele

### Allgemeine Methode 1: Amidkupplung mit HATU/DIEA

Eine Lösung der entsprechenden Carbonsäure (1.0 eq.) in Dimethylformamid (ca. 7-15 ml/mmol) wurde unter Argon bei RT mit dem entsprechenden Amin (1.1 eq.), *N*,*N*-Diisopropylethylamin (DIEA) (2.2 eq.) und einer Lösung aus HATU (1.2 eq.) in wenig Dimethylformamid versetzt. Das Reaktionsgemisch wurde bei RT gerührt. Nach Zugabe von Wasser/Essigsäureethylester und Phasentrennung wurde die organische Phase mit Wasser und mit gesättigter, wässriger Natriumchlorid-Lösung gewaschen, getrocknet (Natrium- oder Magnesiumsulfat), filtriert und im Vakuum eingeengt. Das Rohprodukt wurde anschließend entweder mittels Normalphasen-Chromatographie (Eluent: Cyclohexan-Essigsäureethylester-Gemische oder Dichlormethan-Methanol-Gemische) oder präparativer RP-HPLC (Wasser-Acetonitril-Gradient oder Wasser-Methanol-Gradient) aufgereinigt.

### Allgemeine Methode 2: Verseifung eines Methyl- oder Ethylesters mit Lithiumhydroxid

Eine Lösung des entsprechenden Esters (1.0 eq.) in einem Gemisch aus Tetrahydrofuran/Wasser (3:1, ca. 7-15 ml/mmol) wurde bei RT mit Lithiumhydroxid (2-4 eq.) versetzt und bei RT gerührt. Anschließend wurde das Reaktionsgemisch mit wässriger Salzsäure-Lösung (1N) auf pH 1 gestellt. Nach Zugabe von Wasser/Essigsäureethylester wurde die wässrige Phase dreimal mit Essigsäureethylester extrahiert. Die vereinigten, organischen Phasen wurden getrocknet (Natrium- oder Magnesiumsulfat), filtriert und im Vakuum eingeengt. Das Rohprodukt wurde anschließend entweder mittels Normalphasen-Chromatographie (Eluent: Cyclohexan-Essigsäureethylester-Gemische oder Dichlormethan-Methanol-Gemische) oder präparativer RP-HPLC (Wasser-Acetonitril-Gradient oder Wasser-Methanol-Gradient) aufgereinigt.

### Allgemeine Methode 3: Verseifung eines tert.-Butylesters oder eines Boc-geschützten Amins mittels TFA

Eine Lösung des entsprechenden *tert*.-Butylestersderivates oder eines Boc-geschützten Amins (1.0 eq.) in Dichlormethan (ca. 25 ml/mmol) wurde bei RT mit TFA (20 eq.) versetzt und bei RT für 1-8 h gerührt. Anschließend wurde das Reaktionsgemisch im Vakuum eingeengt. Der Rückstand wurde mehrmals mit Dichlormethan und/oder Toluol coevaporiert. Die Aufreinigung des Rohproduktes erfolgte anschließend mittels präparativer RP-HPLC (Eluent: Acetonitril-Wasser-Gradient oder Wasser-Methanol-Gradient).

### Allgemeine Methode 4: Amid-Kupplung mit OXIMA/DIC

Zu einer entgasten Lösung der entsprechenden Carbonsäure (1 eq.), Anilin (1 eq.) und Hydroxyiminocyanoessigsäureethylester (Oxima) (0.1-1 eq.) in Dimethylformamid (0.1M) wurde tropfenweise *N,N*'-Diisopropylcarbodiimid (DIC) (1 eq.) gegeben und die resultierende Reaktionslösung für 8-24 h bei RT bis 40°C gerührt. Das Lösungsmittel wurde unter reduziertem Druck entfernt. Der Rückstand wurde entweder mit Wasser versetzt und das gewünschte Produkt filtriert oder mittels Normalphasen-Chromatographie (Cyclohexan/Essigsäureethylester-Gradient) oder präparativer RP-HPLC (Wasser-Acetonitril-Gradient oder Wasser-Methanol-Gradient) aufgereinigt.

### Allgemeine Methode 5: Amid-Kupplung mit T3P/DIEA

Eine Lösung der Carbonsäure und des entsprechenden Amins (1.1-1.5 eq.) in Dimethylformamid (0.15-0.05 mmol) wurde unter Argon bei 0°C tropfenweise mit *N*,*N*-Diisopropylethylamin (3 eq.) und Propylphosphonsäureanhydrid (T3P, 50%ig in Dimethylformamid, 3 eq.) versetzt. Das Reaktionsgemisch wurde bei RT gerührt und anschließend im Vakuum eingeengt. Nach Zugabe von Wasser/Essigsäureethylester und Phasentrennung wurde die wässrige Phase zweimal mit Essigsäureethylester extrahiert. Die vereinigten, organischen Phasen wurden getrocknet (Natriumsulfat oder Magnesiumsulfat), filtriert und im Vakuum eingeengt. Das Rohprodukt wurde anschließend entweder mittels Flash-Chromatographie (Kieselgel-60, Eluent: Cyclohexan-Essigsäureethylester-Gemische oder Dichlormethan-Methanol-Gemische) oder präparativer HPLC (Reprosil C18, Wasser-Acetonitril-Gradient oder Wasser-Methanol-Gradient) aufgereinigt.

### Allgemeine Methode 6: Amid-Kupplung mit T3P/Pyridin

Eine Lösung der entsprechenden Carbonsäure (1 eq.) und des entsprechenden Amins (1.1-1.5 eq.) in Pyridin (ca. 0.1M) wurde auf 60°C erwärmt und tropfenweise mit T3P (50%ig in Essigsäureethylester, 15 eq.) versetzt. Alternativ wurde T3P bei RT hinzugefügt und danach bei RT gerührt oder auf 60 bis 90°C erhitzt. Nach 1-20 h wurde das Reaktionsgemisch auf RT gekühlt und mit Wasser und Essigsäureethylester versetzt. Die wässrige Phase wurde mit Essigsäureethylester extrahiert. Die vereinigten organischen Phasen wurden mit wässriger PufferLösung (pH=5), mit gesättigter, wässriger Natriumhydrogencarbonat-Lösung und mit gesättigter, wässriger Natriumchlorid-Lösung gewaschen, über Natriumsulfat getrocknet und im Vakuum eingeengt. Das Rohprodukt wurde gegebenenfalls anschließend entweder mittels Normalphasen-Chromatographie (Eluent: Cyclohexan-Essigsäureethylester-Gemische oder Dichlormethan-Methanol-Gemische) oder präparativer RP-HPLC (Wasser-Acetonitril-Gradient oder Wasser-Methanol-Gradient) aufgereinigt.

### Beispiel 1

### 2-({2-[4-(5-Chlor-2-cyanophenyl)-5-methoxy-2-oxopyridin-1(2H)-yl]butanoyl}amino)imidazo[1,2-a]pyridin-6-carbonsäure (Racemat)

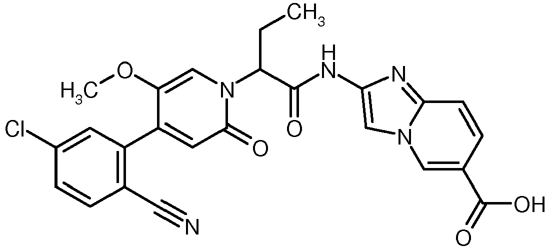

Nach der allgemeinen Methode 2 wurden 59 mg (0.11 mmol) 2-({2-[4-(5-Chlor-2-cyanophenyl)-5-methoxy-2-oxopyridin-1(2*H*)-yl]butanoyl }amino)imidazo [1,2-a]pyridin-6-carbonsäuremethylester (Racemat) mit Lithiumhydroxid verseift. Nach dem Ansäuern mit wässriger Salzsäure (1N) konnte das gewünschte Produkt als Niederschlag isoliert werden. Ausbeute: 45 mg (75% d. Th.)
LC/MS [Methode 1]: Rₜ = 0.88 min; MS (ESIpos): m/z = 506 (M+H)⁺,
¹H-NMR (400 MHz, DMSO-d₆): δ [ppm] = 13.19 (s, 1H), 11.34 (s, 1H), 9.25 (s, 1H), 8.29 (s, 1H), 8.00 (d, 1H), 7.79-7.70 (m, 2H), 7.63 (dd, 1H), 7.54-7.46 (m, 2H), 6.53 (s, 1H), 5.75 (dd, 1H), 3.70 (s, 3H), 2.28-2.10 (m, 2H), 0.89 (t, 3H).

### Beispiel 2

### 6-({2-[4-(5-Chlor-2-cyanophenyl)-5-methoxy-2-oxopyridin-1(2H)-yl]butanoyl}amino)imidazo [1,2-a]pyridin-2-carbonsäure (Racemat)

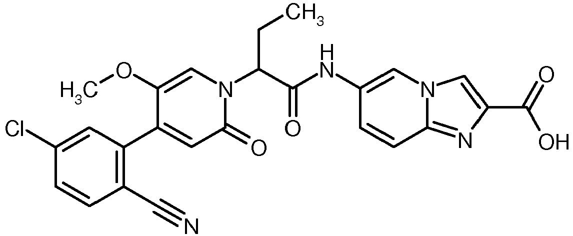

Nach der allgemeinen Methode 2 wurden 86 mg (0.16 mmol) 6-({2-[4-(5-Chlor-2-cyanophenyl)-5-methoxy-2-oxopyridin-1(2*H*)-yl]butanoyl}amino)imidazo[1,2-a]pyridin-2-carbonsäureethylester (Racemat) mit Lithiumhydroxid verseift. Nach wässriger Aufarbeitung wurde das Rohprodukt mittels präparativer RP-HPLC (Reprosil C18, Wasser-Acetonitril-Gradient) aufgereinigt. Ausbeute: 16 mg (19% d. Th.)
LC/MS [Methode 1]: Rₜ = 0.74 min; MS (ESIpos): m/z = 506 (M+H)⁺,
¹H-NMR (400 MHz, DMSO-d₆): δ [ppm] = 10.75 (s, 1H), 9.32 (s, 1H), 8.54 (s, 1H), 8.01 (d, 1H), 7.78-7.70 (m, 2H), 7.62 (d, 1H), 7.52 (s, 1H), 7.32 (dd, 1H), 6.55 (s, 1H), 5.66 (dd, 1H), 3.70 (s, 3H), 2.28-2.10 (m, 2H), 0.92 (s, 3H).

### Beispiel 3

### 7-({2-[4-(5-Chlor-2-cyanophenyl)-5-methoxy-2-oxopyridin-1(2H)-yl]butanoyl}amino)imidazo [1,2-a]pyridin-2-carbonsäure (Racemat)

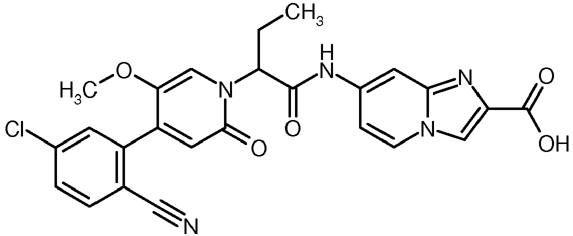

Nach der allgemeinen Methode 2 wurden 18 mg (0.03 mmol) 7-({2-[4-(5-Chlor-2-cyanophenyl)-5-methoxy-2-oxopyridin-1(2*H*)-yl]butanoyl}amino)imidazo[1,2-a]pyridin-2-carbonsäureethylester (Racemat) mit Lithiumhydroxid verseift. Nach wässriger Aufarbeitung wurde das Rohprodukt mittels präparativer RP-HPLC (Reprosil C18, Wasser-Acetonitril-Gradient) aufgereinigt. Ausbeute: 8 mg (45% d. Th.)
LC/MS [Methode 8]: Rₜ = 0.95 min; MS (ESIpos): m/z = 506 (M+H)⁺,
¹H-NMR (400 MHz, DMSO-d₆): δ [ppm] = 10.82 (s, 1H), 8.48 (d, 1H), 8.32 (s, 1H), 8.04-7.97 (m, 2H), 7.78-7.70 (m, 2H), 7.51 (s, 1H), 7.11 (dd, 1H), 6.55 (s, 1H), 5.62 (dd, 1H), 3.70 (s, 3H), 2.31-2.13 (m, 2H), 0.92 (s, 3H).

### Beispiel 4

### 2-[4-(5-Chlor-2-cyanophenyl)-5-methoxy-2-oxopyridin-1(2H)-yl]-N-(imidazo[1,2-a]pyridin-6-yl)butanamid (Racemat)

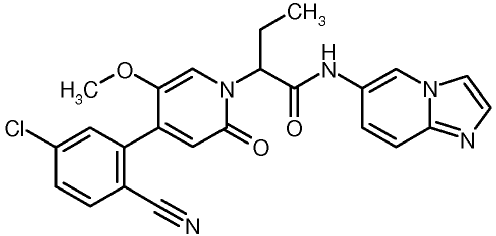

Nach der allgemeinen Methode 1 wurden 87 mg (0.25 mmol) 2-[4-(5-Chlor-2-cyanophenyl)-5-methoxy-2-oxopyridin-1(2*H*)-yl]butansäure (Racemat) mit 44 mg (0.30 mmol, 1.2 eq.) Imidazo[1,2-a]pyridin-6-amin umgesetzt. Nach wässriger Aufarbeitung wurde das Rohprodukt mittels präparativer RP-HPLC (Reprosil C18, Wasser-Acetonitril-Gradient) aufgereinigt. Ausbeute: 8 mg (7% d. Th.)
LC/MS [Methode 8]: Rₜ = 0.93 min; MS (ESIpos): m/z = 462 (M+H)⁺,
¹H-NMR (400 MHz, DMSO-d₆): δ [ppm] = 10.64 (s, 1H), 9.25 (s, 1H), 8.03-7.97 (m, 2H), 7.77-7.71 (m, 2H), 7.57 (d, 1H), 7.54 (d, 2H), 7.23 (dd, 1H), 6.55 (s, 1H), 5.66 (dd, 1H), 3.70 (s, 3H), 2.28-2.19 (m, 2H), 0.92 (t, 3H).

### Beispiel 5

### 6-({2-[4-(5-Chlor-2-cyanophenyl)-5-methoxy-2-oxopyridin-1(2H)-yl]-3-cyclobutylpropanoyl}-amino)imidazo[1,2-a]pyridin-2-carbonsäure (Racemat)

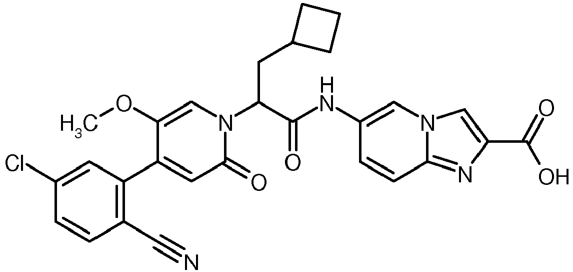

Nach der allgemeinen Methode 2 wurden 69 mg (0.12 mmol) 6-({2-[4-(5-Chlor-2-cyanophenyl)-5-methoxy-2-oxopyridin-1(2*H*)-yl]-3-cyclobutylpropanoyl}amino)imidazo[1,2-a]pyridin-2-carbonsäureethylester (Racemat) mit Lithiumhydroxid verseift. Nach wässriger Aufarbeitung wurde das Rohprodukt mittels präparativer RP-HPLC (Reprosil C18, Wasser-Acetonitril-Gradient) aufgereinigt. Ausbeute: 38 mg (58% d. Th.)
LC/MS [Methode 1]: Rₜ = 0.88 min; MS (ESIpos): m/z = 546 (M+H)⁺,
¹H-NMR (400 MHz, DMSO-d₆): δ [ppm] = 10.74 (s, 1H), 9.32 (s, 1H), 8.54 (s, 1H), 8.00 (d, 1H), 7.78-7.70 (m, 2H), 7.62 (d, 1H), 7.53 (s, 1H), 7.34 (dd, 1H), 6.53 (s, 1H), 5.75-5.66 (m, 1H), 3.69 (s, 3H), 2.35-2.18 (m, 3H), 2.02-1.90 (m, 2H), 1.86-1.61 (m, 4H).

### Beispiel 6

### 6-({[4-(5-Chlor-2-cyanophenyl)-5-methoxy-2-oxopyridin-1(2H)-yl]acetyl}amino)imidazo[1,2-a]pyridin-2-carbonsäure

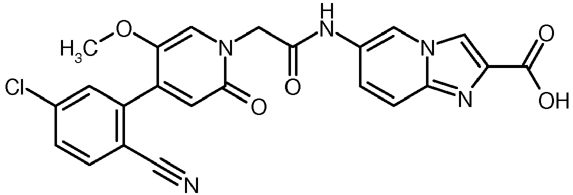

Nach der allgemeinen Methode 2 wurden 99 mg (0.20 mmol) 6-({[4-(5-Chlor-2-cyanophenyl)-5-methoxy-2-oxopyridin-1(2*H*)-yl]acetyl}amino)imidazo[1,2-a]pyridin-2-carbonsäureethylester mit Lithiumhydroxid verseift. Nach dem Ansäuern mit wässriger Salzsäure (1N) konnte das gewünschte Produkt als Niederschlag isoliert und weiter durch Verrühren mit Acetonitril/Wasser (2:1) aufgereinigt werden. Ausbeute: 42 mg (45% d. Th.)
LC/MS [Methode 1]: Rₜ = 0.69 min; MS (ESIpos): m/z = 478 (M+H)⁺,
¹H-NMR (400 MHz, DMSO-d₆): δ [ppm] = 10.87 (s, 1H), 9.42 (s, 1H), 8.70 (s, 1H), 8.00 (d, 1H), 7.79-7.68 (m, 3H), 7.62 (s, 1H), 7.56 (d, 1H), 6.52 (s, 1H), 4.86 (s, 2H), 3.64 (s, 3H).

### Beispiel 7

### 6-[(2-{4-[5-Chlor-2-(difluormethoxy)phenyl]-5-methoxy-2-oxopyridin-1(2H)-yl}butanoyl)-amino]imidazo[1,2-a]pyridin-2-carbonsäure (Racemat)

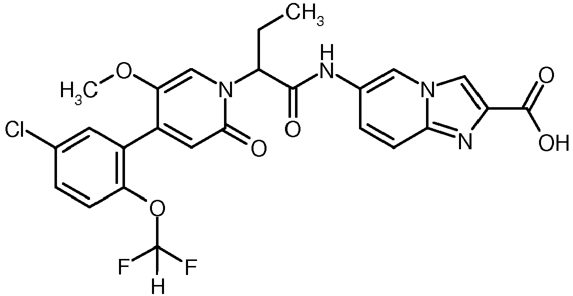

Nach der allgemeinen Methode 2 wurden 198 mg (0.28 mmol) 6-[(2-{4-[5-Chlor-2-(difluormethoxy)phenyl]-5-methoxy-2-oxopyridin-1(2*H*)-yl}butanoyl)amino]imidazo[1,2-a]pyridin-2-carbonsäureethylester (Racemat) mit Lithiumhydroxid verseift. Nach wässriger Aufarbeitung wurde das Rohprodukt mittels präparativer RP-HPLC (Reprosil C18, Wasser-Acetonitril-Gradient) aufgereinigt. Ausbeute: 85 mg (56% d. Th.)
LC/MS [Methode 1]: Rₜ = 0.85 min; MS (ESIpos): m/z = 547 (M+H)⁺,
¹H-NMR (400 MHz, DMSO-d₆): δ [ppm] = 10.74 (s, 1H), 9.32 (s, 1H), 8.55 (s, 1H), 7.63 (d, 1H), 7.58 (dd, 1H), 7.50 (dd, 1H), 7.41 (s, 1H), 7.37-7.26 (m, 2H), 7.14 (t, 1H), 6.41 (s, 1H), 5.64 (dd, 1H), 3.64 (s, 3H), 2.27-2.05 (m, 2H), 0.91 (t, 3H).

### Beispiel 8

### 6-[({5-Chlor-4-[5-chlor-2-(difluormethoxy)phenyl]-2-oxopyridin-1(2H)-yl}acetyl)amino]imidazo-[1,2-a]pyridin-2-carbonsäure

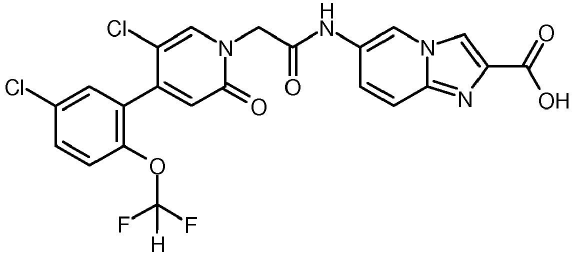

Nach der allgemeinen Methode 2 wurden 99 mg (0.18 mmol) 6-[({5-Chlor-4-[5-chlor-2-(difluormethoxy)phenyl]-2-oxopyridin-1(2*H*)-yl}acetyl)amino]imidazo[1,2-a]pyridin-2-carbonsäureethylester mit Lithiumhydroxid verseift. Nach wässriger Aufarbeitung wurde das Rohprodukt mittels präparativer RP-HPLC (Reprosil C18, Wasser-Acetonitril-Gradient) aufgereinigt. Ausbeute: 26 mg (28% d. Th.)
LC/MS [Methode 1]: Rₜ = 0.77 min; MS (ESIpos): m/z = 523 (M+H)⁺,
¹H-NMR (400 MHz, DMSO-d₆): δ [ppm] = 10.73 (s, 1H), 9.31 (s, 1H), 8.60 (s, 1H), 8.13 (s, 1H), 7.68 (d, 1H), 7.64 (dd, 1H), 7.52 (d, 1H), 7.42-7.34 (m, 2H), 7.26 (t, 1H), 6.52 (s, 1H), 4.85 (s, 2H).

### Beispiel 9

### 6-({2-[4-(5-Chlor-2-cyanophenyl)-5-methoxy-2-oxopyridin-1(2H)-yl]-3-[(2S)-tetrahydro-2H-pyran-2-yl]propanoyl}amino)imidazo[1,2-a]pyridin-2-carbonsäure (Gemisch enantiomerenreiner Diastereomere)

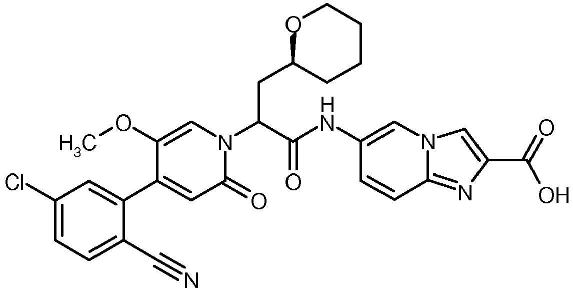

Nach der allgemeinen Methode 2 wurden 162 mg (0.27 mmol) 6-({2-[4-(5-Chlor-2-cyanophenyl)-5-methoxy-2-oxopyridin-1(2*H*)-yl]-3-[(2*S*)-tetrahydro-2*H*-pyran-2-yl]propanoyl}amino)-imidazo[1,2-a]pyridin-2-carbonsäureethylester (Gemisch enantiomerenreiner Diastereomere) mit Lithiumhydroxid verseift. Nach wässriger Aufarbeitung wurde das Rohprodukt mittels präparativer RP-HPLC (Reprosil C18, Wasser-Acetonitril-Gradient) aufgereinigt. Ausbeute: 61 mg (40% d. Th.)
LC/MS [Methode 1]: Rₜ = 0.81 min; MS (ESIpos): m/z = 576 (M+H)⁺,
¹H-NMR (400 MHz, DMSO-d₆): δ [ppm] = 10.69/10.58 (2x s, 1H), 9.31/9.28 (2x s, 1H), 8.56-8.51 (m, 1H), 8.03-7.97 (m, 1H), 7.77-7.70 (m, 2H), 7.63-7.57 (m, 1H), 7.54/7.50 (2x s, 1H), 7.42-7.33 (m, 1H), 6.53/6.52 (2x s, 1H), 5.85/5.77 (t/dd, 1H), 3.93-3.79 (m, 1H), 3.69 (s, 3H), 3.25-3.15 (m, 1H), 3.14-3.05 (m, 1H), 2.40-2.09 (m, 2H), 1.80-1.71 (m, 1H), 1.68-1.56 (m, 1H), 1.48-1.35 (m, 3H), 1.34-1.20 (m, 1H).

### Beispiel 10

### 6-({2-[4-(5-Chlor-2-cyanophenyl)-5-methoxy-2-oxopyridin-1(2H)-yl]-4-methoxybutanoyl}-amino)imidazo[1,2-a]pyridin-3-carbonsäureethylester (Racemat)

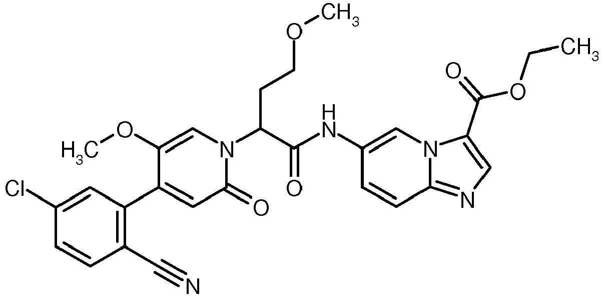

Nach der allgemeinen Methode 6 wurden 80 mg (0.21 mmol) 2-[4-(5-Chlor-2-cyanophenyl)-5-methoxy-2-oxopyridin-1(2*H*)-yl]-4-methoxybutansäure (Racemat) und 65 mg (0.32 mmol, 1.5 eq.) 6-Aminoimidazo[1,2-a]pyridin-3-carbonsäureethylester in Pyridin bei 60°C vorgelegt und durch Zugabe von T3P miteinander umgesetzt. Das Rohprodukt wurde mittels präparativer HPLC (Chromatorex 125 mm x 30 mm, 10 µm, Eluent: Wasser/Acetonitril, Gradient: 10-90% Acetonitril) aufgereinigt. Ausbeute: 66 mg (55% d. Th.)
LC/MS [Methode 1]: Rₜ = 0.96 min; MS (ESIpos): m/z = 564 (M+H)⁺,
¹H-NMR (400 MHz, DMSO-*d₆*): δ [ppm] = 10.81 (s, 1H), 10.09 (d, 1H), 8.25 (s, 1H), 8.00 (d, 1H), 7.80 (d, 1H), 7.76-7.71 (m, 2H), 7.66 (dd, 1H), 7.54 (s, 1H), 6.54 (s, 1H), 5.78 (dd, 1H), 4.35 (q, 2H), 3.71 (s, 3H), 3.46-3.39 (m, 1H), 3.30-3.25 (m, 1H), 3.22 (s, 3H), 2.49-2.36 (m, 2H), 1.34 (t, 3H).

### Beispiel 11

### 7-({2-[4-(5-Chlor-2-cyanophenyl)-5-methoxy-2-oxopyridin-1(2H)-yl]-4-methoxybutanoyl}-amino)imidazo[1,2-a]pyridin-2-carbonsäureethylester (Racemat)

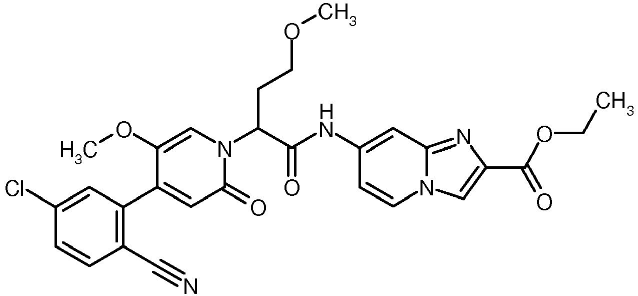

Nach der allgemeinen Methode 6 wurden 75 mg (0.20 mmol) 2-[4-(5-Chlor-2-cyanophenyl)-5-methoxy-2-oxopyridin-1(2*H*)-yl]-4-methoxybutansäure (Racemat) und 53 mg (0.26 mmol, 1.3 eq.) 7-Aminoimidazo[1,2-a]pyridin-2-carbonsäureethylester in Pyridin bei 60°C vorgelegt und durch Zugabe von T3P miteinander umgesetzt. Das Rohprodukt wurde mittels präparativer HPLC (Chromatorex 125 mm x 30 mm, 10 µm, Eluent: Gradient Wasser/Acetonitril: 10-90% Acetonitril) aufgereinigt. Ausbeute: 83 mg (74% d. Th.)
LC/MS [Methode 1]: Rₜ = 0.91 min; MS (ESIpos): m/z = 564 (M+H)⁺,
¹H-NMR (400 MHz, DMSO-d₆): δ [ppm] = 10.69 (s, 1H), 9.32-9.29 (m, 1H), 8.60 (s, 1H), 8.02-7.98 (m, 1H), 7.76-7.72 (m, 2H), 7.64-7.60 (m, 1H), 7.52 (s, 1H), 7.38 (dd, 1H), 6.54 (s, 1H), 5.78 (dd, 1H), 4.30 (q, 2H), 3.69 (s, 3H), 3.45-3.38 (m, 1H), 3.30-3.25 (m, 1H), 3.22 (s, 3H), 2.48-2.36 (m, 2H), 1.31 (t, 3H).

### Beispiel 12

### 7-({2-[4-(5-Chlor-2-cyanophenyl)-5-methoxy-2-oxopyridin-1(2H)-yl]-4-methoxybutanoyl}-amino)imidazo[1,2-a]pyridin-2-carboxamid (Racemat)

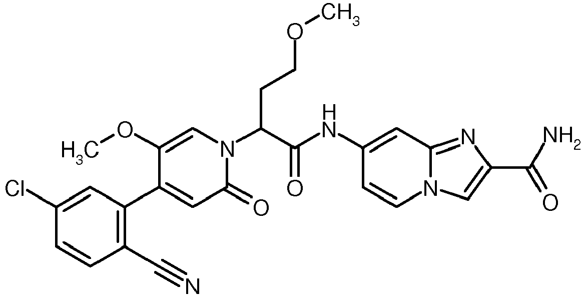

Nach der allgemeinen Methode 6 wurden 65 mg (0.17 mmol) 2-[4-(5-Chlor-2-cyanophenyl)-5-methoxy-2-oxopyridin-1(2*H*)-yl]-4-methoxybutansäure (Racemat) und 44 mg (90% Reinheit, 0.22 mmol, 1.3 eq.) 7-Aminoimidazo[1,2-a]pyridin-2-carboxamid in Pyridin bei 60°C vorgelegt und durch Zugabe von T3P miteinander umgesetzt. Das Rohprodukt wurde mittels präparativer HPLC (Chromatorex 125 mm x 30 mm, 10 µm, Eluent: Gradient Wasser/Acetonitril: 10%-90% Acetonitril) und im Anschluss durch weitere präparative HPLC (Kinetex 5 µm C18 150 mm x 21.2 mm, Gradient Wasser/Acetonitril: 5%-50% Acetonitril) aufgereingt. Ausbeute: 8 mg (9% d. Th.)
LC/MS [Methode 1]: Rₜ = 0.77 min; MS (ESIpos): m/z = 535 (M+H)⁺,
¹H-NMR (400 MHz, DMSO-d₆): δ [ppm] = 10.66 (s, 1H), 9.31-9.29 (m, 1H), 8.39 (s, 1H), 8.02-7.98 (m, 1H), 7.76-7.71 (m, 2H), 7.64-7.61 (m, 1H), 7.58 (d, 1H), 7.53 (s, 1H), 7.38-7.32 (m, 2H), 6.54 (s, 1H), 5.79 (dd, 1H), 3.69 (s, 3H), 3.45-3.38 (m, 1H), 3.28-3.24 (m, 1H), 3.22 (s, 3H), 2.48-2.38 (m, 2H).

### Beispiel 13

### 2-[4-(5-Chlor-2-cyanophenyl)-5-methoxy-2-oxopyridin-1(2H)-yl]-N-(imidazo[1,2-a]pyridin-6-yl)-4-methoxybutanamid (Racemat)

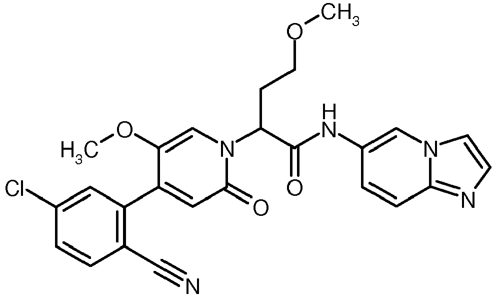

Nach der allgemeinen Methode 1 wurden 200 mg (0.53 mmol) 2-[4-(5-Chlor-2-cyanophenyl)-5-methoxy-2-oxopyridin-1(2*H*)-yl]-4-methoxybutansäure (Racemat) mit 78 mg (0.58 mmol, 1.1 eq.) Imidazo[1,2-*a*]pyridin-6-amin umgesetzt. Das Rohprodukt wurde mittels Flash-Chromatographie (Kieselgel-50, Dichlormethan-Methanol-Gradient) und anschließender Dickschicht-Chromatographie (Dichlormethan / Methanol 10:1) aufgereinigt. Ausbeute: 47 mg (90% Reinheit, 16% d. Th.)
LC/MS [Methode 2]: Rₜ = 1.80 min; MS (ESIpos): m/z = 492 (M+H)⁺,
¹H-NMR (400 MHz, DMSO-d₆): δ [ppm] = 10.59 (s, 1H), 9.23 (s, 1H), 8.03-7.95 (m, 2H), 7.76-7.67 (m, 2H), 7.58-7.50 (m, 3H), 7.25 (dd, 1H), 6.54 (s, 1H), 5.79 (dd, 1H), 3.69 (s, 3H), 3.45-3.37 (m, 1H), 3.31-3.26 (m, 1H), 3.22 (s, 3H), 2.48-2.35 (m, 2H).

### Beispiel 14

### 2-[5-Chlor-4-(5-chlor-2-cyanophenyl)-2-oxopyridin-1(2H)-yl]-N-(imidazo[1,2-a]pyridin-6-yl)-4-methoxybutanamid (Racemat)

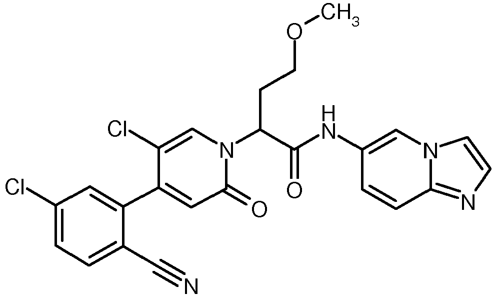

Nach der allgemeinen Methode 6 wurden 50 mg (0.13 mmol) 2-[5-Chlor-4-(5-chlor-2-cyanophenyl)-2-oxopyridin-1(2*H*)-yl]-4-methoxybutansäure (Racemat) und 28 mg (0.19 mmol, 1.5 eq.) Imidazo[1,2-a]pyridin-6-amin umgesetzt. Das Rohprodukt wurde mittels präparativer HPLC (Wasser-Acetonitril-0.1% Ameisensäure-Gradient) aufgereinigt. Ausbeute: 20mg (32% d. Th.)
LC/MS [Methode 1]: Rₜ = 0.75 min; MS (ESIpos): m/z = 496 (M+H)⁺,
¹H-NMR (400 MHz, DMSO-d₆): δ [ppm] = 10.66 (s, 1H), 9.23 (s, 1H), 8.23 (s, 1H), 8.09-8.04 (m, 1H), 7.98 (s, 1H), 7.84-7.76 (m, 2H), 7.60-7.50 (m, 2H), 7.24 (dd, 1H), 6.68 (s, 1H), 5.85-5.73 (m, 1H), 3.42 (dt, 1H), 3.29-3.24 (m, 1H), 3.21 (s, 3H), 2.46-2.38 (m, 2 H).

### Beispiel 15

### 2-[4-(5-Chlor-2-cyanophenyl)-5-methoxy-2-oxopyridin-1(2H)-yl]-3-cyclobutyl-N-(imidazo[1,2-a]pyridin-6-yl)propanamid (Racemat)

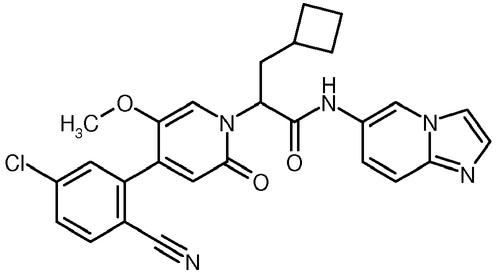

Nach der allgemeinen Methode 1 wurden 123 mg (94% Reinheit, 0.30 mmol) 2-[4-(5-Chlor-2-cyanophenyl)-5-methoxy-2-oxopyridin-1(2*H*)-yl]-3-cyclobutylpropansäure (Racemat) mit 47 mg (0.33 mmol, 1.1 eq.) Imidazo[1,2-a]pyridin-6-amin umgesetzt. Nach wässriger Aufarbeitung wurde das Rohprodukt mittels präparativer RP-HPLC (Reprosil C18, Wasser-Acetonitril-Gradient) aufgereinigt. Ausbeute: 45 mg (30% d. Th.)
LC/MS [Methode 1]: Rₜ = 0.81 min; MS (ESIpos): m/z = 502 (M+H)⁺,
¹H-NMR (400 MHz, DMSO-d₆): δ [ppm] = 10.63 (s, 1H), 9.24 (s, 1H), 8.03-7.97 (m, 2H), 7.77-7.70 (m, 2H), 7.59-7.50 (m, 3H), 7.24 (dd, 1H), 6.53 (s, 1H), 5.71 (t, 1H), 3.69 (s, 3H), 2.31-2.19 (m, 3H), 2.02-1.91 (m, 2H), 1.85-1.62 (m, 4H).

### Beispiel 16

### 2-[4-(5-Chlor-2-cyanophenyl)-5-methoxy-2-oxopyridin-1(2H)-yl]-3-(trans-4-hydroxycyclohexyl)-N-(imidazo[1,2-a]pyridin-6-yl)propanamid (Racemat)

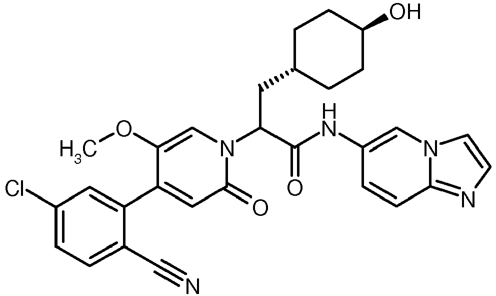

63 mg (96 µmol) 3-(*trans*-4-{[*tert*.-Butyl(dimethyl)silyl]oxy}cyclohexyl)-2-[4-(5-chlor-2-cyanophenyl)-5-methoxy-2-oxopyridin-1(2*H*)-yl]-*N*-(imidazo[1,2-*a*]pyridin-6-yl)propanamid (Racemat) wurden in 5 ml Dimethylformamid vorgelegt und mit 0.5 ml wässriger Salzsäure (1N) versetzt. Es wurde für 1 h bei Raumtemperatur nachgerührt, anschließend die Reaktionslösung mittels präparativer HPLC getrennt (Säule: Chromatorex C18, 10 µm, 125 mm x 30 mm, Lösungsmittel: Acetonitril/0.1% Ameisensäure-Gradient (0 bis 3 min 10% Acetonitril, bis 35 min 90% Acetonitril und weitere 3 min 90% Acetonitril) und die Titelverbindung erhalten. Ausbeute: 25.3 mg (48% d. Th.)
LC/MS [Methode 1]: Rₜ = 0.69 min; MS (ESIpos): m/z = 546 (M+H)⁺,
¹H-NMR (400 MHz, DMSO-d₆): δ [ppm] = 10.6 (s, 1H), 9.24-9.22 (m, 1H), 8.02-7.97 (m, 2H), 7.77-7.71 (m, 2H), 7.58-7.50 (m, 3H), 7.23 (dd, 1H), 6.55 (s, 1H), 5.85 (dd, 1H), 4.44 (d, 1H), 3.68 (s, 3H), 2.19-2.10 (m, 1H), 1.96-1.87 (m, 1H), 1.83-1.71 (m, 4H), 1.12-0.95 (m, 5H).

### Beispiel 17

### 2-[4-(5-Chlor-2-cyanophenyl)-5-methoxy-2-oxopyridin-1(2H)-yl]-N-(3-chlorimidazo[1,2-a]pyridin-6-yl)butanamid (Racemat)

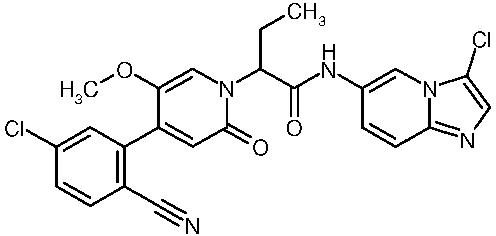

Eine Lösung von 46 mg (0.25 mmol) 2-[4-(5-Chlor-2-cyanophenyl)-5-methoxy-2-oxopyridin-1(2H)-yl]-N-(imidazo[1,2-a]pyridin-6-yl)butanamid (Racemat) in 2 ml Ethanol wurde bei RT mit 13 mg (0.10 mmol, 1.0 eq.) N-Chlorsuccinimid versetzt und über Nacht bei RT gerührt. Nach Zugabe von Wasser/Essigsäureethylester und Phasentrennung wurde die wässrige Phase mit Essigsäureethylester extrahiert. Die vereinigten, organischen Phasen wurden mit gesättigter, wässriger Natriumchlorid-Lösung gewaschen, getrocknet (Natriumsulfat), filtriert und im Vakuum eingeengt. Der Rückstand wurde mittels präparativer RP-HPLC (Reprosil C18, Wasser-Acetonitril-Gradient) aufgereinigt. Ausbeute: 18 mg (36% d. Th.)
LC/MS [Methode 1]: Rₜ = 0.91 min; MS (ESIpos): m/z = 496 (M+H)⁺,
¹H-NMR (400 MHz, DMSO-d₆): δ [ppm] = 10.84 (s, 1H), 9.09 (s, 1H), 8.00 (d, 1H), 7.77-7.71 (m, 2H), 7.70-7.65 (m, 2H), 7.52 (s, 1H), 7.36 (dd, 1H), 6.56 (s, 1H), 5.65 (dd, 1H), 3.70 (s, 3H), 2.30-2.12 (m, 2H), 0.93 (t, 3H).

### Beispiel 18

### 2-[4-(5-Chlor-2-cyanophenyl)-5-methoxy-2-oxopyridin-1(2H)-yl]-N-[2-(4-fluorphenyl)-imidazo[1,2-a]pyridin-6-yl]butanamid (Racemat)

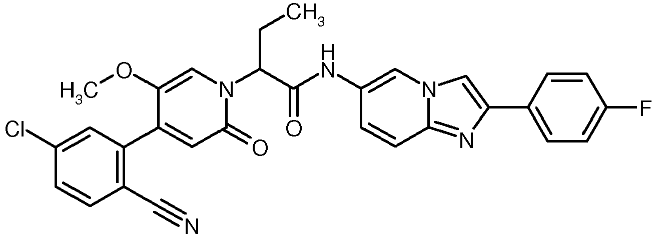

Nach der allgemeinen Methode 6 wurden 80 mg (0.23 mmol) 2-[4-(5-Chlor-2-cyanophenyl)-5-methoxy-2-oxopyridin-1(2*H*)-yl]butansäure (Racemat) und 96 mg (82% Reinheit, 0.35 mmol, 1.5 eq.) 2-(4-Fluorphenyl)imidazo[1,2-a]pyridin-6-amin in Pyridin bei 60°C vorgelegt und durch Zugabe von T3P miteinander umgesetzt. Das Rohprodukt wurde mittels präparativer HPLC (Chromatorex 125 mm x 30 mm, 10 µm, Eluent: Wasser/Acetonitril, Gradient: 10-90% Acetonitril) aufgereinigt. Ausbeute: 80 mg (62% d. Th.)
LC/MS [Methode 1]: Rₜ = 0.88 min; MS (ESIpos): m/z = 556 (M+H)⁺,
¹H-NMR (400 MHz, DMSO-d₆): δ [ppm] = 10.68 (s, 1H), 9.23 (d, 1H), 8.43 (s, 1H), 8.00 (d, 1H), 7.98-7.93 (m, 2H), 7.76-7.72 (m, 2H), 7.59 (d, 1H), 7.53 (s, 1H), 7.30-7.23 (m, 3H), 6.56 (s, 1H), 5.67 (dd, 1H), 3.70 (s, 3H), 2.30-2.09 (m, 2H), 0.93 (t, 3H).

### Beispiel 19

### 2-[4-(5-Chlor-2-cyanophenyl)-5-methoxy-2-oxopyridin-1(2H)-yl]-N-[2-(4-fluorphenyl)-imidazo[1,2-a]pyridin-6-yl]-4-methoxybutanamid (Racemat)

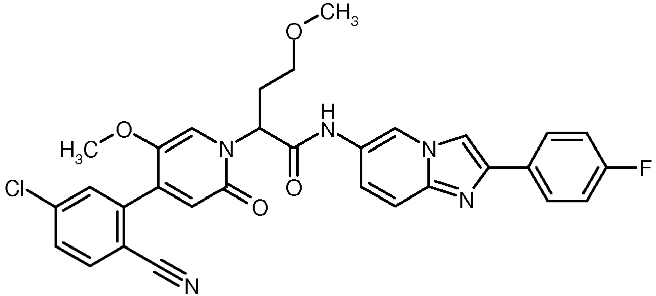

Nach der allgemeinen Methode 6 wurden 75 mg (0.20 mmol) 2-[4-(5-Chlor-2-cyanophenyl)-5-methoxy-2-oxopyridin-1(2*H*)-yl]-4-methoxybutansäure (Racemat) und 83 mg (82% Reinheit, 0.30 mmol, 1.5 eq.) 2-(4-Fluorphenyl)imidazo[1,2-*a*]pyridin-6-amin in Pyridin bei 60°C vorgelegt und durch Zugabe von T3P miteinander umgesetzt. Das Rohprodukt wurde mittels präparativer HPLC (Chromatorex 125 mm x 30 mm, 10 µm, Eluent: Wasser/Acetonitril, Gradient: 10-90% Acetonitril) aufgereinigt. Ausbeute: 53 mg (45% d. Th.)
LC/MS [Methode 1]: Rₜ = 0.86 min; MS (ESIpos): m/z = 586 (M+H)⁺,
¹H-NMR (400 MHz, DMSO-*d₆*): δ [ppm] = 10.63 (s, 1H), 9.21 (d, 1H), 8.43 (s, 1H), 8.02-7.98 (m, 1H), 7.98-7.93 (m, 2H), 7.76-7.72 (m, 2H), 7.58 (d, 1H), 7.54 (s, 1H), 7.30 (dd, 1H), 7.28-7.23 (m, 2H), 6.55 (s, 1H), 5.80 (dd, 1H), 3.70 (s, 3H), 3.46-3.39 (m, 1H), 3.30-3.26 (m, 1H), 3.23 (s, 3H), 2.48-2.35 (m, 2H).

### Beispiel 20

### 2-[4-(5-Chlor-2-cyanophenyl)-5-methoxy-2-oxopyridin-1(2H)-yl]-N-([1,2,4]triazolo[4,3-a]pyridin-6-yl)butanamid (Racemat)

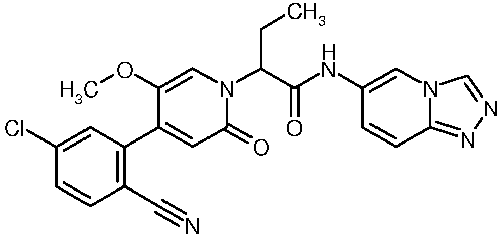

Nach der allgemeinen Methode 1 wurden 69 mg (0.19 mmol) 2-[4-(5-Chlor-2-cyanophenyl)-5-methoxy-2-oxopyridin-1(2*H*)-yl]butansäure (Racemat) mit 29 mg (0.21 mmol, 1.1 eq.) [1,2,4]Triazolo[4,3-a]pyridin-6-amin umgesetzt. Nach wässriger Aufarbeitung wurde das Rohprodukt mittels präparativer RP-HPLC (Reprosil C18, Wasser-Acetonitril-Gradient) aufgereinigt. Ausbeute: 58 mg (65% d. Th.)
LC/MS [Methode 1]: Rₜ = 0.87 min; MS (ESIpos): m/z = 463 (M+H)⁺,
¹H-NMR (400 MHz, DMSO-d₆): δ [ppm] = 10.90 (s, 1H), 9.45 (s, 1H), 8.45 (s, 1H), 8.00 (d, 1H), 7.87 (d, 1H), 7.77-7.70 (m, 2H), 7.68 (dd, 1H), 7.52 (s, 1H), 6.56 (s, 1H), 5.64 (dd, 1H), 3.70 (s, 3H), 2.30-2.11 (m, 2H), 0.92 (t, 3H).

### Beispiel 21

### 2-[4-(5-Chlor-2-cyanophenyl)-5-methoxy-2-oxopyridin-1(2H)-yl]-4-methoxy-N-([1,2,4]triazolo[4,3-a]pyridin-6-yl)butanamid (Racemat)

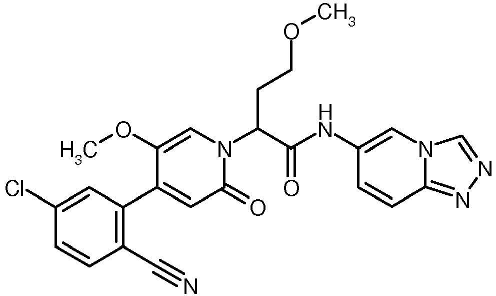

Nach der allgemeinen Methode 1 wurden 150 mg (0.398 mmol) 2-[4-(5-Chlor-2-cyanophenyl)-5-methoxy-2-oxopyridin-1(2*H*)-yl]-4-methoxybutansäure (Racemat) mit 59 mg (0.44 mmol, 1.1 eq.) [1,2,4]Triazolo[4,3-*a*]pyridin-6-amin umgesetzt. Ausbeute: 27 mg (14% d. Th.)
LC/MS [Methode 1]: Rₜ = 0.84 min; MS (ESIpos): m/z = 493 (M+H)⁺,
¹H-NMR (400 MHz, DMSO-d₆): δ [ppm] = 10.8 (s, 1H), 9.44 (d, 1H), 8.45 (s, 1H), 8.02-7.98 (m, 1H), 7.86 (d, 1H), 7.76-7.71 (m, 3H), 7.53 (s, 1H), 6.55 (s, 1H), 5.77 (dd, 1H), 3.70 (s, 3H), 3.43 (dt, 1H), 3.31-3.26 (m, 1H), 3.22 (s, 3H), 2.49-2.36 (m, 2H).

### Beispiel 22

### 2-[4-(5-Chlor-2-cyanophenyl)-5-methoxy-2-oxopyridin-1(2H)-yl]-4-methoxy-N-(3-methyl[1,2,4]triazolo[4,3-a]pyridin-6-yl)butanamid (Racemat)

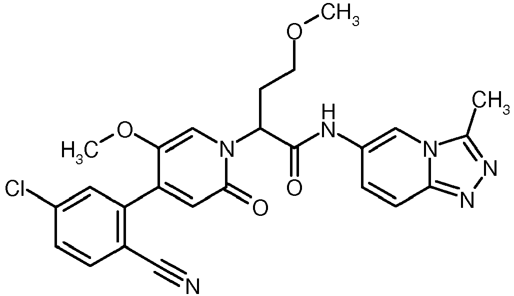

Nach der allgemeinen Methode 1 wurden 130 mg (0.35 mmol) 2-[4-(5-Chlor-2-cyanophenyl)-5-methoxy-2-oxopyridin-1(2*H*)-yl]-4-methoxybutansäure (Racemat) mit 76 mg (74% Reinheit, 0.38 mmol, 1.1 eq.) 3-Methyl[1,2,4]triazolo[4,3-a]pyridin-6-amin umgesetzt. Das Rohprodukt wurde mittels Flash-Chromatographie (Kieselgel-50, Dichlormethan-Methanol-Gradient) aufgereinigt. Ausbeute: 30 mg (90% Reinheit, 15% d. Th.)
LC/MS [Methode 2]: Rₜ = 2.29 min; MS (ESIpos): m/z = 507 (M+H)⁺,
¹H-NMR (400 MHz, DMSO-d₆): δ [ppm] = 10.77 (s, 1H), 8.90 (s, 1H), 8.02-7.98 (m, 1H), 7.77-7.70 (m, 3H), 7.51 (s, 1H), 7.38 (dd, 1H), 6.54 (s, 1H), 5.78 (dd, 1H), 3.70 (s, 3H), 3.46-3.39 (m, 1H), 3.30-3.25 (m, 1H), 3.22 (s, 3H), 2.63 (s, 3H), 2.48-2.39 (m, 2H).

### Beispiel 23

### 2-[4-(5-Chlor-2-cyanophenyl)-5-methoxy-2-oxopyridin-1(2H)-yl]-4-methoxy-N-(3-ethyl[1,2,4]triazolo[4,3-a]pyridin-6-yl)butanamid (Racemat)

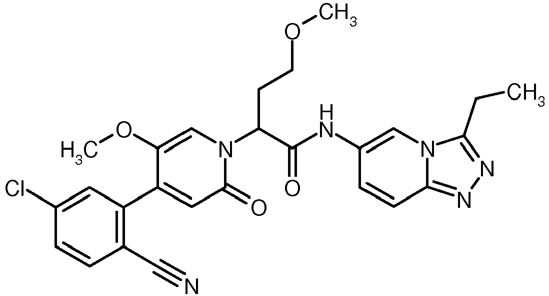

Man legte 50 mg (0.13 mmol) 2-[4-(5-Chlor-2-cyanophenyl)-5-methoxy-2-oxopyridin-1(2*H*)-yl]-4-methoxybutansäure (Racemat) und 26 mg (0.16 mmol, 1.2 eq.) 3-Ethyl[1,2,4]triazolo[4,3-*a*]pyridin-6-amin in 1.5 ml Dimethylformamid vor und gab 0.11 ml (81 mg, 6.0 eq.) Triethylamin hinzu. Dann wurden 237 µl (796 µmol, 3.0 eq.) T3P (50%ig in Essigsäureethylester) zugetropft. Man ließ das Reaktionsgemisch über Nacht bei RT rühren, setzte dann Wasser und Essigsäureethylester hinzu und extrahierte die wässrige Phase zweimal mit Essigsäureethylester. Die vereinten organischen Phasen wurden mit wässriger, gesättigter Natriumchlorid-Lösung gewaschen, über Magnesiumsulfat getrocknet und im Vakuum eingeengt. Das Rohprodukt wurde mittels Flash-Chromatographie (Kieselgel-50, Dichlormethan-Methanol-Gradient) aufgereinigt. Ausbeute: 64 mg (89% d. Th.)
LC/MS [Methode 1]: Rₜ = 0.79 min; MS (ESIpos): m/z = 521 (M+H)⁺,
¹H-NMR (400 MHz, DMSO-d₆): δ [ppm] = 10.76 (s, 1H), 8.92 (s, 1H), 8.03-7.97 (m, 1H), 7.78-7.71 (m, 3H), 7.51 (s, 1H), 7.38 (dd, 1H), 6.54 (s, 1H), 5.77 (dd, 1H), 3.70 (s, 3H), 3.46-3.38 (m, 1H), 3.31-3.25 (m, 1H), 3.22 (s, 3H), 3.03 (d, 2H), 2.44 (d, 2H), 1.36 (t, 3H).

### Beispiel 24

### N-(3-Butyl[1,2,4]triazolo[4,3-a]pyridin-6-yl)-2-[4-(5-chlor-2-cyanophenyl)-5-methoxy-2-oxopyridin-1(2H)-yl]-4-methoxybutanamid (Racemat)

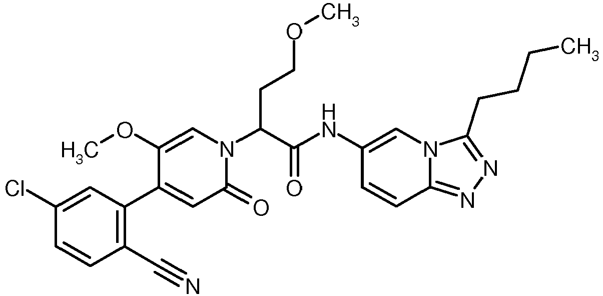

Man legte 100 mg (0.27 mmol) 2-[4-(5-Chlor-2-cyanophenyl)-5-methoxy-2-oxopyridin-1(2*H*)-yl]-4-methoxybutansäure (Racemat) und 71 mg (85% Reinheit, 0.32 mmol, 1.2 eq.) 3-Butyl[1,2,4]triazolo[4,3-a]pyridin-6-amin in 3.0 ml Dimethylformamid vor und gab 0.22 ml (161 mg, 6.0 eq.) Triethylamin hinzu. Dann wurden 474 µl (796 µmol, 3.0 eq.) T3P (50%ig in Essigsäureethylester) zugetropft. Man ließ das Reaktionsgemisch über Nacht bei RT rühren, setzte dann Wasser und Essigsäureethylester hinzu und extrahierte die wässrige Phase zweimal mit Essigsäureethylester. Die vereinten organischen Phasen wurden mit wässriger, gesättigter Natriumchlorid-Lösung gewaschen, über Magnesiumsulfat getrocknet und im Vakuum eingeengt. Das Rohprodukt wurde mittels Flash-Chromatographie (Kieselgel-50, Dichlormethan-Methanol-Gradient) und anschließender präparativer HPLC (Chromatorex 125 mm x 30 mm, 10 µm, Eluent: Wasser/Acetonitril, Gradient 10-90% Acetonitril) aufgereinigt. Ausbeute: 17 mg (12% d. Th.)
LC/MS [Methode 1]: Rₜ = 1.01 min; MS (ESIpos): m/z = 549 (M+H)⁺,
¹H-NMR (400 MHz, DMSO-*d₆*): δ [ppm] = 10.77 (s, 1H), 9.31 (dd, 1H), 8.02-7.98 (m, 1H), 7.76-7.70 (m, 3H), 7.66 (dd, 1H), 7.53 (s, 1H), 6.54 (s, 1H), 5.76 (dd, 1H), 3.69 (s, 3H), 3.45-3.38 (m, 1H), 3.28-3.25 (m, 1H), 3.21 (s, 3H), 2.78 (t, 2H), 2.47-2.37 (m, 2H), 1.77-1.68 (m, 2H), 1.36 (sxt, 2H), 0.91 (t, 3H).

### Beispiel 25

### 2-[4-(5-Chlor-2-cyanophenyl)-5-methoxy-2-oxopyridin-1(2H)-yl]-N-{3-[(dimethylamino)-methyl][1,2,4]triazolo[4,3-a]pyridin-6-yl}-4-methoxybutanamid (Racemat)

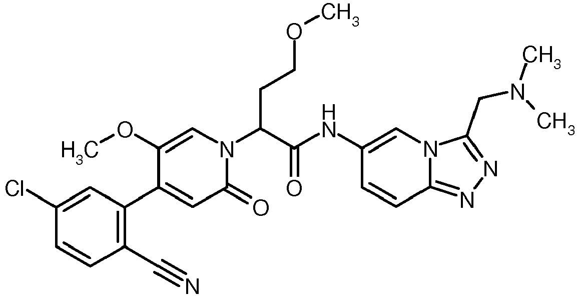

Nach der allgemeinen Methode 1 wurden 75 mg (0.20 mmol) 2-[4-(5-Chlor-2-cyanophenyl)-5-methoxy-2-oxopyridin-1(2*H*)-yl]-4-methoxybutansäure (Racemat) und 46 mg (0.24 mmol, 1.2 eq.) 3-[(Dimethylamino)methyl][1,2,4]triazolo[4,3-*a*]pyridin-6-amin umgesetzt. Das Rohprodukt wurde mittels präparativer HPLC (Chromatorex 125 mm x 30 mm, 10 µm, Eluent: Wasser/Acetonitril, Gradient 10-90% Acetonitril) aufgereinigt. Ausbeute: 96 mg (87% d. Th.)
LC/MS [Methode 1]: Rₜ = 1.01 min; MS (ESIpos): m/z = 550 (M+H)⁺,
¹H-NMR (400 MHz, DMSO-d₆): δ [ppm] = 10.91 (s, 1H), 10.22 (br. s, 1H), 9.47 (dd, 1H), 8.02-7.98 (m, 1H), 7.93 (dd, 1H), 7.81 (dd, 1H), 7.73 (s, 2H), 7.51 (s, 1H), 6.55 (s, 1H), 5.74 (dd, 1H), 4.60 (s, 2H), 3.70 (s, 3H), 3.47-3.39 (m, 1H), 3.30-3.26 (m, 1H), 3.22 (s, 3H), 2.88 (s, 6H), 2.48-2.38 (m, 2H).

### Beispiel 26

### 2-[4-(5-Chlor-2-cyanophenyl)-5-methoxy-2-oxopyridin-1(2H)-yl]-4-methoxy-N-[3-(morpholin-4-ylmethyl)[1,2,4]triazolo[4,3-a]pyridin-6-yl]butanamid (Racemat)

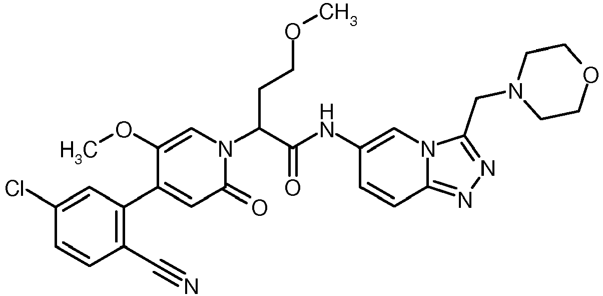

Nach der allgemeinen Methode 6 wurden 75 mg (0.20 mmol) 2-[4-(5-Chlor-2-cyanophenyl)-5-methoxy-2-oxopyridin-1(2*H*)-yl]-4-methoxybutansäure (Racemat) und 91 mg (77% Reinheit, 0.30 mmol, 1.5 eq.) 3-(Morpholin-4-ylmethyl)[1,2,4]triazolo[4,3-a]pyridin-6-amin in Pyridin bei 60°C vorgelegt und durch Zugabe von T3P miteinander umgesetzt. Das Rohprodukt wurde mittels Flash-Chromatographie (Kieselgel-50, Eluent: Dichlormethan-Methanol-Gemische) aufgereinigt. Ausbeute: 31 mg (92% Reinheit, 24% d. Th.)
LC/MS [Methode 1]: Rₜ = 0.68 min; MS (ESIpos): m/z = 592 (M+H)⁺,
¹H-NMR (400 MHz, DMSO-d₆): δ [ppm] = 10.81 (s, 1H), 9.37-9.35 (m, 1H), 8.00 (d, 1H), 7.80-7.68 (m, 4H), 7.53 (s, 1H), 6.54 (s, 1H), 5.76 (dd, 1H), 3.76-3.71 (m, 1H), 3.69 (s, 3H), 3.68-3.62 (m, 1H), 3.59-3.53 (m, 4H), 3.45-3.38 (m, 1H), 3.30-3.24 (m, 1H), 3.21 (s, 3H), 2.48-2.35 (m, 2H).

### Beispiel 27

### 2-[5-Chlor-4-(5-chlor-2-cyanophenyl)-2-oxopyridin-1(2H)-yl]-N-(imidazo[1,5-a]pyridin-6-yl)-4-methoxybutanamid (Racemat)

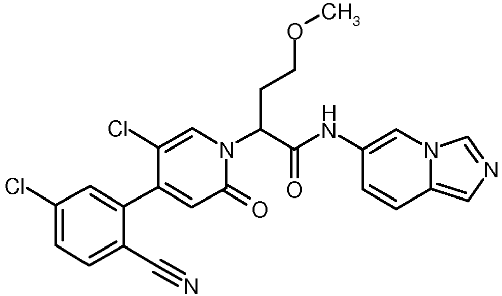

Nach der allgemeinen Methode 1 wurden 50 mg (90% Reinheit, 0.12 mmol) 2-[5-Chlor-4-(5-chlor-2-cyanophenyl)-2-oxopyridin-1(2*H*)-yl]-4-methoxybutansäure (Racemat) und 33 mg (69% Reinheit, 0.18 mmol, 1.5 eq.) Imidazo[1,5-a]pyridin-6-amin umgesetzt. Das Rohprodukt wurde mittels präparativer HPLC (Wasser-Acetonitril-0.1% Ameisensäure-Gradient) aufgereinigt. Ausbeute: 7.9 mg (13% d. Th.)
LC/MS [Methode 1]: Rₜ = 0.77 min; MS (ESIpos): m/z = 496 (M+H)⁺,
¹H-NMR (400 MHz, DMSO-d₆): δ [ppm] = 10.57 (s, 1H), 9.07 (s, 1H), 8.37 (s, 1H), 8.23 (s, 1H), 8.07 (d, 1H), 7.86-7.76 (m, 2H), 7.55 (d, 1H), 7.32 (s, 1H), 6.83-6.76 (m, 1H), 6.68 (s, 1H), 5.85-5.71 (m, 1H), 3.42 (dt, 2H), 3.30-3.25 (m, 1H), 3.20 (s, 3H), 2.42 (q, 2H).

### Beispiel 28

### 2-[4-(5-Chlor-2-cyanophenyl)-5-methoxy-2-oxopyridin-1(2H)-yl]-N-(imidazo[1,2-a]pyridin-6-yl)-3-(pyridin-2-yl)propanamid (Racemat)

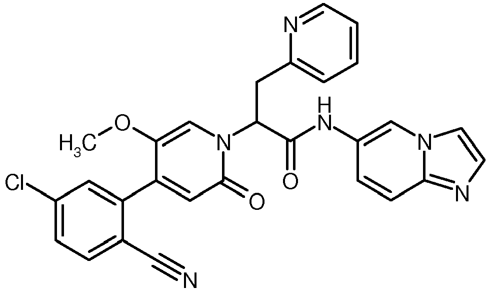

Nach der allgemeinen Methode 6 wurden 30 mg (93% Reinheit, 0.068 mmol) 2-[4-(5-Chlor-2-cyanophenyl)-5-methoxy-2-oxopyridin-1(2*H*)-yl]-3-(pyridin-2-yl)propansäure (Racemat) und 15.0 mg (90% Reinheit, 0.102 mmol, 1.5 eq) Imidazo[1,2-a]pyridin-6-amin umgesetzt. Das Rohprodukt wurde mittels präparativer HPLC (Wasser-Acetonitril-0.1% Ameisensäure-Gradient) aufgereinigt. Das Produkt wurde anschließend in Acetonitril gelöst und über eine Festphasenextraktions-Kartusche (StratoSpheres SPE PL-HCO₃ MP-Resin) filtriert. Das Filtrat wurde lyophilisiert. Ausbeute: 11 mg (31% d. Th.)
LC/MS [Methode 1]: Rₜ = 0.66 min; MS (ESIneg): m/z = 523 (M-H)⁻,
¹H-NMR (400 MHz, DMSO-d₆): δ [ppm] = 10.68 (s, 1H), 9.23 (s, 1H), 8.49 (d, 1H), 8.05-7.92 (m, 2H), 7.76-7.63 (m, 3H), 7.62-7.47 (m, 3H), 7.34 (d, 1H), 7.28-7.17 (m, 2H), 6.43 (s, 1H), 6.16 (t, 1H), 3.69 (d, 2H), 3.64 (s, 3H).

### Beispiel 29

### 2-[4-(5-Chlor-2-cyanophenyl)-5-methoxy-2-oxopyridin-1(2H)-yl]-N-(imidazo[1,2-a]pyridin-6-yl)-3-(1,3-oxazol-5-yl)propanamid (Racemat)

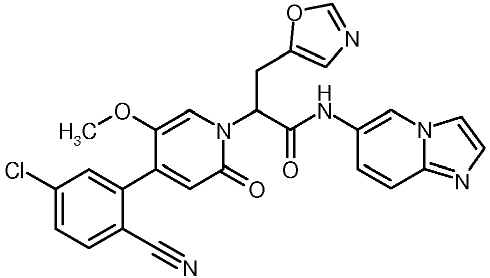

Nach der allgemeinen Methode 6 wurden 50 mg (80% Reinheit, 0.10 mmol) 2-[4-(5-Chlor-2-cyanophenyl)-5-methoxy-2-oxopyridin-1(2*H*)-yl]-3-(1,3-oxazol-5-yl)propansäure (Racemat) und 26.6 mg (90% Reinheit, 0.18 mmol, 1.8 eq.) Imidazo[1,2-a]pyridin-6-amin umgesetzt. Das Rohprodukt wurde mittels präparativer HPLC (Wasser-Acetonitril-0.1% Ameisensäure-Gradient) aufgereinigt. Ausbeute: 35 mg (68% d. Th.)
LC/MS [Methode 8]: Rₜ = 0.82 min; MS (ESIpos): m/z = 515 (M+H)⁺,
¹H-NMR (400 MHz, DMSO-d₆): δ [ppm] = 10.66 (s, 1H), 9.32-9.14 (m, 1H), 8.24 (s, 1H), 8.05-7.93 (m, 2H), 7.77-7.65 (m, 2H), 7.64-7.50 (m, 3H), 7.23 (dd, 1H), 6.92 (s, 1H), 6.51 (s, 1H), 5.99 (dd, 1H), 3.82-3.70 (m, 1H), 3.70-3.59 (m, 4H).

### Beispiel 30

### 2-[5-Chlor-4-(5-chlor-2-cyanophenyl)-2-oxopyridin-1(2H)-yl]-N-(imidazo[1,2-a]pyridin-6-yl)-3-(1,3-oxazol-5-yl)propanamid (Racemat)

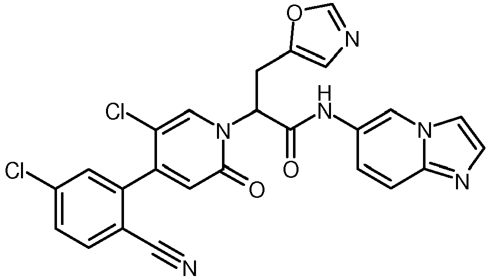

Nach der allgemeinen Methode 6 wurden 50 mg (85% Reinheit, 0.105 mmol) 2-[5-Chlor-4-(5-chlor-2-cyanophenyl)-2-oxopyridin-1(2*H*)-yl]-3-(1,3-oxazo1-5-yl)propansäure (Racemat) und 26.5 mg (90% Reinheit, 0.179 mmol, 1.7 eq.) Imidazo[1,2-a]pyridin-6-amin umgesetzt. Das Rohprodukt wurde mittels präparativer HPLC (Wasser-Acetonitril-0.1% Ameisensäure-Gradient) aufgereinigt. Ausbeute: 21 mg (83% Reinheit, 32% d. Th.)
LC/MS [Methode 2]: Rₜ = 1.87 min; MS (ESIpos): m/z = 519 (M+H)⁺,
¹H-NMR (400 MHz, DMSO-d₆): δ [ppm] = 10.73 (s, 1H), 9.22 (s, 1H), 8.35 (s, 1H), 8.25 (s, 1H), 8.08-8.03 (m, 1H), 8.00 (s, 1H), 7.82-7.75 (m, 2H), 7.59-7.53 (m, 2H), 7.24-7.15 (m, 1H), 6.92 (s, 1H), 6.66 (s, 1H), 5.98 (dd, 1H), 3.78 (dd, 1H), 3.66 (dd, 1H).

### Beispiel 31

### 2-[4-(5-Chlor-2-cyanophenyl)-5-(difluormethoxy)-2-oxopyridin-1(2H)-yl]-N-(imidazo[1,2-a]pyridin-6-yl)-3-(1,3-oxazol-5-yl)propanamid (Racemat)

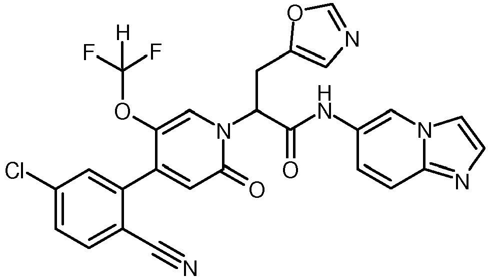

Nach der allgemeinen Methode 6 wurden 40 mg (0.092 mmol) 2-[4-(5-Chlor-2-cyanophenyl)-5-(difluormethoxy)-2-oxopyridin-1(2*H*)-yl]-3-(1,3-oxazol-5-yl)propansäure (Racemat) und 20.4 mg (90% Reinheit, 0.138 mmol, 1.5 eq) Imidazo[1,2-a]pyridin-6-amin umgesetzt. Das Rohprodukt wurde mittels präparativer HPLC (Wasser-Acetonitril-0.1% Ameisensäure-Gradient) aufgereinigt. Ausbeute: 20 mg (40% d. Th.)
LC/MS [Methode 1]: Rₜ = 0.67 min; MS (ESIpos): m/z = 551 (M+H)⁺,
¹H-NMR (400 MHz, DMSO-d₆): δ [ppm] = 10.79 (s, 1H), 9.24 (s, 1H), 8.25 (s, 1H), 8.19-8.11 (m, 1H), 8.09-7.99 (m, 2H), 7.81-7.71 (m, 2H), 7.65-7.54 (m, 2H), 7.30-7.20 (m, 1H), 6.92 (s, 1H), 6.84 (t, 1H), 6.63 (s, 1H), 5.99 (dd, 1H), 3.73 (dd, 1H), 3.64 (dd, 1H).

### Beispiel 32

### 2-[4-(5-Chlor-2-cyanophenyl)-5-methoxy-2-oxopyridin-1(2H)-yl]-N-(imidazo[1,2-a]pyridin-6-yl)-3-(1,3-oxazol-4-yl)propanamid (Racemat)

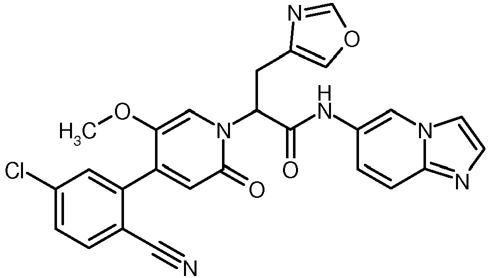

Nach der allgemeinen Methode 6 wurden 40 mg (0.1 mmol) 2-[4-(5-Chlor-2-cyanophenyl)-5-methoxy-2-oxopyridin-1(2*H*)-yl]-3-(1,3-oxazol-4-yl)propansäure (Racemat) und 19.7 mg (0.15 mmol, 1.5 eq) Imidazo[1,2-a]pyridin-6-amin umgesetzt. Das Rohprodukt wurde mittels präparativer HPLC (Wasser-Acetonitril-0.1% Ameisensäure-Gradient) aufgereinigt. Ausbeute: 43.8 mg (84% d. Th.)
LC/MS [Methode 1]: Rₜ = 0.65 min; MS (ESIpos): m/z = 515 (M+H)⁺,
¹H-NMR (400 MHz, DMSO-d₆): δ [ppm] = 10.69 (s, 1H), 9.25 (s, 1H), 8.30 (s, 1H), 8.06-7.91 (m, 2H), 7.82 (s, 1H), 7.76-7.65 (m, 2H), 7.63-7.50 (m, 3H), 7.27 (dd, 1H), 6.48 (s, 1H), 6.00 (dd, 1H), 3.68 (s, 3H), 3.54 (dd, 1H), 3.42 (dd, 1H).

### Beispiel 33

### 2-[5-Chlor-4-(5-chlor-2-cyanophenyl)-2-oxopyridin-1(2H)-yl]-N-(imidazo[1,2-a]pyridin-6-yl)-3-(1,3-oxazol-4-yl)propanamid (Racemat)

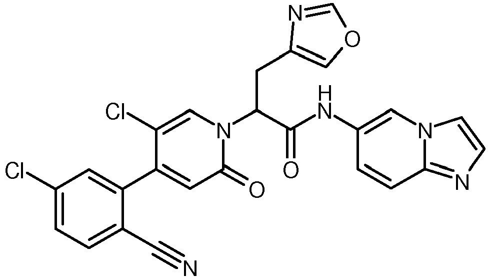

Nach der allgemeinen Methode 6 wurden 40 mg (0.1 mmol) 2-[5-Chlor-4-(5-chlor-2-cyanophenyl)-2-oxopyridin-1(2*H*)-yl]-3-(1,3-oxazol-4-yl)propansäure (Racemat) und 19.8 mg (0.15 mmol, 1.5 eq) Imidazo[1,2-a]pyridin-6-amin umgesetzt. Das Rohprodukt wurde mittels präparativer HPLC (Wasser-Acetonitril-0.1% Ameisensäure-Gradient) aufgereinigt. Ausbeute: 41 mg (79% d. Th.)
LC/MS [Methode 1]: Rₜ = 0.70 min; MS (ESIpos): m/z = 519 (M+H)⁺,
¹H-NMR (400 MHz, DMSO-d₆): δ [ppm] = 10.78 (s, 1H), 9.25 (s, 1H), 8.35-8.19 (m, 2H), 8.10-7.94 (m, 2H), 7.89-7.67 (m, 3H), 7.66-7.47 (m, 2H), 7.26 (d, 1H), 6.62 (s, 1H), 5.99 (dd, 1H), 3.57 (dd, 1H), 3.42 (dd, 1H).

### Beispiel 34

### 2-[4-(5-Chlor-2-cyanophenyl)-5-methoxy-2-oxopyridin-1(2H)-yl]-N-(pyrazolo[1,5-a]pyridin-5-yl)butanamid (Racemat)

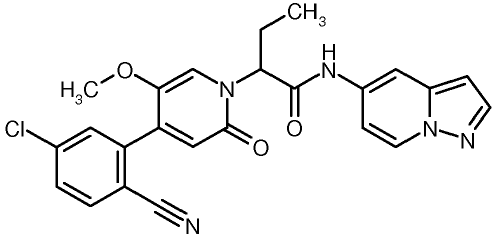

Nach der allgemeinen Methode 5 wurden 138 mg (0.39 mmol) 2-[4-(5-Chlor-2-cyanophenyl)-5-methoxy-2-oxopyridin-1(2*H*)-yl]butansäure (Racemat) mit 47 mg (0.35 mmol) Pyrazolo[1,5-a]pyridin-5-amin [B.C. Baguley et al. Bioorganic and Medicinal Chemistry, 2012, 20, 69-85] umgesetzt. Nach wässriger Aufarbeitung wurde das Rohprodukt mittels Flash-Chromatograhie (Kieselgel-50, Cyclohexan-Essigsäureethylester-Gradient) aufgereinigt. Ausbeute: 82 mg (51% d. Th.)
LC/MS [Methode 1]: Rₜ = 0.97 min; MS (ESIpos): m/z = 462 (M+H)⁺,
¹H-NMR (400 MHz, DMSO-d₆): δ [ppm] =10.75 (s, 1H), 8.62 (d, 1H), 8.14 (d, 1H), 8.00 (d, 1H), 7.92 (d, 1H), 7.70-7.70 (m, 2H), 7.51 (s, 1H), 6.97 (dd, 1H), 6.55 (s, 1H), 6.50 (d, 1H), 5.64 (dd, 1H), 3.70 (s, 3H), 2.27-2.09 (m, 2H), 0.92 (t, 3H).

### Beispiel 35

### 2-[4-(5-Chlor-2-cyanophenyl)-5-methoxy-2-oxopyridin-1(2H)-yl]-4-methoxy-N-(pyrazolo[1,5-a]pyridin-5-yl)butanamid (Racemat)

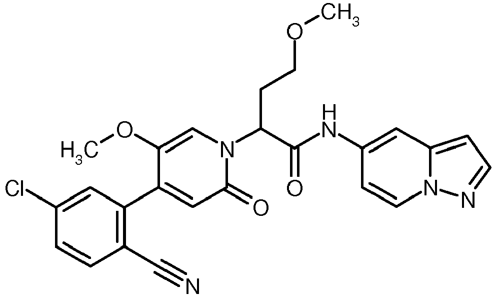

Nach der allgemeinen Methode 1 wurden 800 mg (2.12 mmol) 2-[4-(5-Chlor-2-cyanophenyl)-5-methoxy-2-oxopyridin-1(2*H*)-yl]-4-methoxybutansäure (Racemat) mit 424 mg (3.19 mmol, 1.5 eq.) Pyrazolo[1,5-*a*]pyridin-5-amin umgesetzt. Das Rohprodukt wurde mittels Flash-Chromatographie (Kieselgel-50, Dichlormethan-Methanol-Gradient) aufgereinigt und das Produkt mit Acetonitril verrührt und abgesaugt. Ausbeute: 550 mg (53% d. Th.)
LC/MS [Methode 1]: Rₜ = 0.94 min; MS (ESIpos): m/z = 492 (M+H)⁺,
¹H-NMR (400 MHz, DMSO-d₆): δ [ppm] = 10.70 (s, 1H), 8.61 (d, 1H), 8.14 (d, 1H), 8.02-7.98 (m, 1H), 7.92 (d, 1H), 7.76-7.72 (m, 2H), 7.53 (s, 1H), 7.00 (dd, 1H), 6.54 (s, 1H), 6.50 (dd, 1H), 5.77 (dd, 1H), 3.69 (s, 3H), 3.45-3.38 (m, 1H), 3.31-3.25 (m, 1H), 3.21 (s, 3H), 2.48-2.36 (m, 2H).

### Beispiel 36

### 2-[4-(5-Chlor-2-cyanophenyl)-5-methoxy-2-oxopyridin-1(2H)-yl]-4-methoxy-N-(pyrazolo[1,5-a]pyridin-5-yl)butanamid (Enantiomer 1)

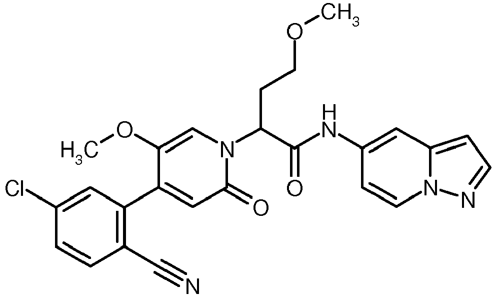

Enantiomerentrennung von 45.3 mg des Racemates aus Beispiel 35 ergab 12.3 mg der Titelverbindung Beispiel 36 (Enantiomer 1) neben 14.4 mg Enantiomer 2.
Chirale HPLC: Enantiomer 1: Rₜ = 2.75 min; 100% ee [Vergleich: Enantiomer 2: Rₜ = 1.71 min; 100% ee]
Trennmethode: Säule: Daicel IF 5 µm 250 mm x 20 mm; Eluent: 40% iso-Hexan, 60% Ethanol; Temperatur: 23°C; Fluss: 20 ml/min; UV-Detektion: 220 nm.
Analytik: Säule: Daicel Chiralpak IF 3 µm 50 mm x 4.6 mm; Eluent: 50% iso-Hexan, 50% Ethanol; Fluss: 1 ml/min; UV-Detektion: 220 nm.
¹H-NMR (400 MHz, DMSO-d₆): δ [ppm] = 10.70 (s, 1H), 8.61 (d, 1H), 8.14 (d, 1H), 8.02-7.87 (m, 1H), 7.92 (d, 1H), 7.76-7.72 (m, 2H), 7.53 (s, 1H), 7.00 (dd, 1H), 6.54 (s, 1H), 6.51-6.49 (m, 1H), 5.77 (dd, 1H), 3.69 (s, 3H), 3.45-3.38 (m, 1H), 3.31-3.25 (m, 1H), 3.21 (s, 3H), 2.48-2.36 (m, 2H).

### Beispiel 37

### 2-[5-Chlor-4-(5-chlor-2-cyanophenyl)-2-oxopyridin-1(2H)-yl]-4-methoxy-N-(pyrazolo[1,5-a]pyridin-5-yl)butanamid (Racemat)

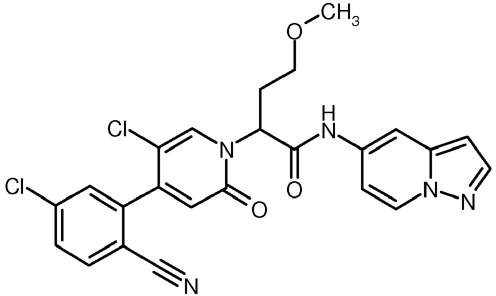

Nach der allgemeinen Methode 6 wurden 110 mg (0.26 mmol) 2-[5-Chlor-4-(5-chlor-2-cyanophenyl)-2-oxopyridin-1(2*H*)-yl]-4-methoxybutansäure (Racemat) und 51 mg (0.39 mmol, 1.5 eq.) Pyrazolo[1,5-a]pyridin-5-amin umgesetzt. Das Rohprodukt wurde mittels präparativer HPLC (Wasser-Acetonitril-0.1% Ameisensäure-Gradient) aufgereinigt. Ausbeute: 95 mg (74% d. Th.)
LC/MS [Methode 1]: Rₜ = 1.00 min; MS (ESIpos): m/z = 496 (M+H)⁺,
¹H-NMR (400 MHz, DMSO-d₆): δ [ppm] = 10.76 (br. s, 1H), 8.62 (d, 1H), 8.23 (s, 1H), 8.11 (d, 1H), 8.07 (d, 1H), 7.92 (d, 1H), 7.84-7.76 (m, 2H), 6.98 (dd, 1H), 6.68 (s, 1H), 6.50 (d, 1H), 5.85-5.67 (m, 1H), 3.42 (dt, 1H), 3.27 (dt, 1H), 3.20 (s, 3H), 2.47-2.38 (m, 2H).

### Beispiel 38

### 2-[5-Chlor-4-(5-chlor-2-cyanophenyl)-2-oxopyridin-1 (2H)-yl]-4-methoxy-N-(pyrazolo[1,5-a]pyridin-5-yl)butanamid (Enantiomer 1)

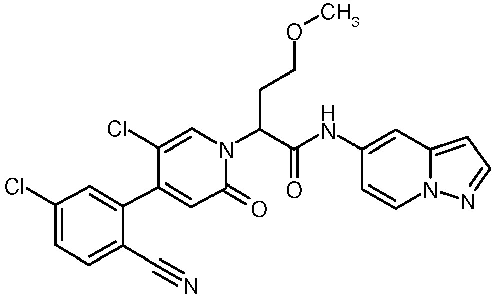

Enantiomerentrennung von 90 mg des Racemates aus Beispiel 37 ergab 34 mg der Titelverbindung Beispiel 38 (Enantiomer 1) neben 33 mg Enantiomer 2.
Chirale HPLC: Enantiomer 1: Rₜ = 7.88 min; 100% ee [Vergleich: Enantiomer 2: Rₜ = 4.37 min; 100% ee]
Trennmethode (SFC): Säule: Daicel Chiralpak AZ-H 5 µm 250 mm x 20 mm; Eluent: 70% Kohlendioxid, 30% 2-Propanol; Temperatur: 40°C; Fluss: 80 ml/min; UV-Detektion: 210 nm.
Analytik (SFC): Säule: Daicel Chiralpak AZ-H 250 mm x 4.6 mm; Eluent: 60% Kohlendioxid, 40% 2-Propanol; Fluss: 3 ml/min; Temperatur: 30°C; UV-Detektion: 210 nm.

### Beispiel 39

### 4-(5-Chlor-2-cyanophenyl)-6-oxo-1-[1-oxo-1-(pyrazol[1,5-a]pyridin-5-ylamino)butan-2-yl]-1,6-dihydropyridin-3-carbonsäure (Racemat)

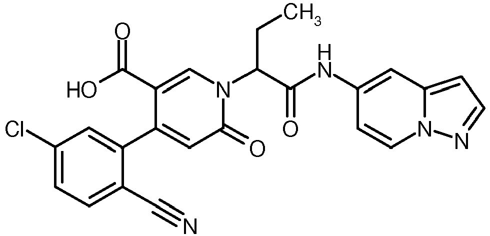

147 mg (260 µmol) 4-(5-Chlor-2-cyanophenyl)-6-oxo-1-[1-oxo-1-(pyrazol[1,5-*a*]pyridin-5-ylamino)butan-2-yl]-1,6-dihydropyridin-3-carbonsäurebenzylester (Racemat) wurden in 3 ml Tetrahydrofuran vorgelegt und mit 14 mg (13 µmol) Palladium (10% auf Kohle) versetzt. Die Reaktionsmischung wurde für 3 h bei Normaldruck hydriert. Anschließend wurde die Reaktionsmischung filtriert und das Lösungsmittel unter reduziertem Druck entfernt. Der Rückstand wurde mittels präparativer HPLC [Säule: Chromatorex C18, 10 µm, 125 mm x 30 mm, Eluent: Acetonitril/0.05% Ameisensäure-Gradient (0 bis 3 min 10% Acetonitril, bis 35 min 90% Acetonitril und weitere 3 min 90% Acetonitril)] gereinigt und die Titelverbindung erhalten. Ausbeute: 36 mg (29% d. Th.)
LC/MS [Methode 1]: Rₜ = 0.81 min; MS (ESIpos): m/z = 476 (M+H)⁺,
¹H-NMR (400 MHz, DMSO-d₆): δ [ppm] = 12.9 (br. s, 1H), 10.9 (s, 1H), 8.62 (d, 1H), 8.59 (s, 1H), 8.12 (s, 1H), 7.95-7.90 (m, 2H), 7.69-7.64 (m, 2H), 6.95 (dd, 1H), 6.50 (d, 1H), 6.47 (s, 1H), 5.64 (dd, 1H), 2.29-2.19 (m, 1H), 2.14-2.01 (m, 1H), 0.94 (t, 3H).

### Beispiel 40

### 2-[4-(5-Chlor-2-cyanophenyl)-5-methoxy-2-oxopyridin-1(2H)-yl]-N-(3-chlorpyrazolo[1,5-a]pyridin-5-yl)butanamid (Racemat)

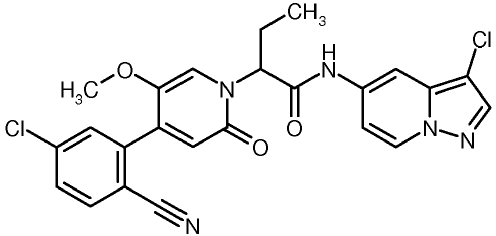

Eine Lösung von 71 mg (0.15 mmol) 2-[4-(5-Chlor-2-cyanophenyl)-5-methoxy-2-oxopyridin-1(2*H*)-yl]-*N*-(pyrazolo[1,5-a]pyridin-5-yl)butanamid (Racemat) in 2 ml Ethanol wurde bei RT mit insgesamt 30 mg (0.22 mmol, 1.45 eq.) *N*-Chlorsuccinimid versetzt und über Nacht bei RT gerührt. Das Reaktionsgemisch wurde im Vakuum eingeengt. Nach Zugabe von Wasser/Essigsäureethylester und Phasentrennung wurde die wässrige Phase mit Essigsäureethylester extrahiert. Die vereinigten, organischen Phasen wurden mit gesättigter, wässriger Natriumchlorid-Lösung gewaschen, getrocknet (Natriumsulfat), filtriert und im Vakuum eingeengt und getrocknet. Ausbeute: 80 mg (quant.)
LC/MS [Methode 1]: Rₜ = 1.12 min; MS (ESIpos): m/z = 496 (M+H)⁺,
¹H-NMR (400 MHz, DMSO-d₆): δ [ppm] = 10.91 (s, 1H), 8.66 (d, 1H), 8.14 (d, 1H), 8.09 (s, 1H), 8.00 (d, 1H), 7.77-7.71 (m, 2H), 7.50 (s, 1H), 7.01 (dd, 1H), 6.56 (s, 1H), 5.62 (dd, 1H), 3.71 (s, 3H), 2.29-2.12 (m, 2H), 0.92 (t, 3H).

### Beispiel 41

### 2-[4-(5-Chlor-2-cyanophenyl)-5-methoxy-2-oxopyridin-1(2H)-yl]-N-(3-chlorpyrazolo[1,5-a]pyridin-5-yl)-4-methoxybutanamid (Racemat)

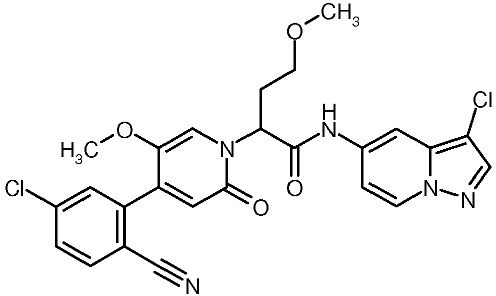

Man löste 100 mg (90% Reinheit, 0.18 mmol) 2-[4-(5-Chlor-2-cyanophenyl)-5-methoxy-2-oxopyridin-1(2*H*)-yl]-4-methoxy-*N*-(pyrazolo[1,5-*a*]pyridin-5-yl)butanamid (Racemat) in 3.0 ml Ethanol, setzte 29 mg (0.20 mmol, 1.1 eq.) *N*-Chlorsuccinimid hinzu und ließ über Nacht bei RT rühren. Danach setzte man einen Tropfen Dimethylformamid und weitere 4.9 mg (37 µmol, 0.2 eq.) *N*-Chlorsuccinimid zu und ließ weitere 4 h rühren. Danach wurde die Reaktionslösung durch präparative HPLC (Chromatorex 125 mm x 30 mm, 10 µm, Eluens: Wasser/Acetonitril, Gradient 10% Acetonitril bis 90% Acetonitril) aufgereinigt. Ausbeute: 16 mg (17% d. Th.)
LC/MS [Methode 1]: Rₜ = 1.02 min; MS (ESIpos): m/z = 526/528 (M+H)⁺,
¹H-NMR (400 MHz, DMSO-d₆): δ [ppm] = 10.85 (s, 1H), 8.66 (dd, 1H), 8.14 (d, 1H), 8.09 (s, 1H), 8.02-7.99 (m, 1H), 7.76-7.72 (m, 2H), 7.52 (s, 1H), 7.05 (dd, 1H), 6.54 (s, 1H), 5.75 (dd, 1H), 3.70 (s, 3H), 3.46-3.38 (m, 1H), 3.30-3.25 (m, 1H), 3.21 (s, 3H), 2.48-2.40 (m, 2H).

### B) Bewertung der physiologischen Wirksamkeit

Die Eignung der erfindungsgemäßen Verbindungen zur Behandlung von thromboembolischen Erkrankungen kann in folgenden Assaysystemen gezeigt werden:

### a) Testbeschreibungen (in vitro)

### a.1) Messung der FXIa-Hemmung

Zur Bestimmung der Faktor XIa-Hemmung der erfindungsgemäßen Substanzen wird ein biochemisches Testsystem verwendet, in dem die Umsetzung eines peptidischen Faktor XIa-Substrates zur Ermittlung der enzymatischen Aktivität von humanem Faktor XIa benutzt wird. Dabei spaltet Faktor XIa von dem peptischen Faktor XIa-Substrat das C-terminale Aminomethylcoumarin (AMC) ab, dessen Fluoreszenz gemessen wird. Die Bestimmungen werden in Mikrotiterplatten durchgeführt.

Prüfsubstanzen werden in Dimethylsulfoxid aufgelöst und seriell in Dimethylsulfoxid verdünnt (3000 µM bis 0.0078 µM; resultierende Endkonzentrationen im Test: 50 µM bis 0.00013 µM). Jeweils 1 µl der verdünnten Substanzlösungen werden in die Vertiefungen von weißen Mikrotiterplatten der Firma Greiner (384 Vertiefungen) vorgelegt. Anschließend werden nacheinander 20 µl Assaypuffer (50 mM Tris/HCl pH 7.4; 100 mM Natriumchlorid; 5 mM Calciumchlorid; 0,1 % bovines Serumalbumin) und 20 µl Faktor XIa der Firma Kordia (0.45 nM in Assaypuffer) hinzugefügt. Nach 15 min Inkubation wird die Enzymreaktion durch Zugabe von 20 µl des in Assaypuffer gelösten Faktor XIa Substrates Boc-Glu(OBzl)-Ala-Arg-AMC (10 µM in Assaypuffer) der Firma Bachem gestartet, für 30 min bei Raumtemperatur (22°C) inkubiert und anschließend eine Fluoreszensmessung durchgeführt (Anregung: 360 nM, Emission: 460 nM). Die gemessenen Emissionen der Testansätze mit Prüfsubstanz werden mit denen von Kontrollansätzen ohne Prüfsubstanz (ausschließlich Dimethylsulfoxid anstatt Prüfsubstanz in Dimethylsulfoxid) verglichen und aus den Konzentrations-Wirkungs-Beziehungen IC₅₀-Werte berechnet. Wirkdaten aus diesem Test sind in der folgenden Tabelle A aufgeführt:

**Tabelle A**

| **Beispiel-Nr.** | **IC₅₀ [nM]** | | **Beispiel-Nr.** | **IC₅₀ [nM)]** |
|---|---|---|---|---|
| 1 | 32 | | 2 | 2.3 |
| 3 | 6.6 | | 4 | 65 |
| 5 | 1.0 | | 6 | 11 |
| 7 | 12 | | 8 | 380 |
| 9 | 0.5 | | 10 | 36 |
| 11 | 20 | | 12 | 3.1 |
| 13 | 16 | | 14 | 31 |
| 15 | 41 | | 16 | 11 |
| 17 | 65 | | 18 | 220 |
| 19 | 33 | | 20 | 86 |
| 21 | 21 | | 22 | 28 |
| 23 | 53 | | 24 | 63 |
| 25 | 20 | | 26 | 24 |
| 27 | 96 | | 28 | 12 |
| 29 | 16 | | 30 | 96 |
| 31 | 500 | | 32 | 92 |
| 33 | 310 | | 34 | 21 |
| 35 | 4.0 | | 36 | 3.6 |
| 37 | 29 | | 38 | 15 |
| 39 | 180 | | 40 | 140 |
| 41 | 26 | | | |

### a.2) Bestimmung der Selektivität

Zum Nachweis der Selektivität der Substanzen bezüglich FXIa-Hemmung werden die Prüfsubstanzen auf ihre Hemmung anderer humaner Serinproteasen wie Faktor Xa, Trypsin und Plasmin hin untersucht. Zur Bestimmung der enzymatischen Aktivität von Faktor Xa (1.3 nmol/l von Kordia), Trypsin (83 mU/ml von Sigma) und Plasmin (0.1 µg/ml von Kordia) werden diese Enzyme gelöst (50 mmol/l Tris-Puffer [C,C,C-Tris(hydroxymethyl)-aminomethan], 100 mmol/l NaCl, 0.1% BSA [bovines Serumalbumin], 5 mmol/l Calciumchlorid, pH 7.4) und für 15 min mit Prüfsubstanz in verschiedenen Konzentrationen in Dimethylsulfoxid sowie mit Dimethylsulfoxid ohne Prüfsubstanz inkubiert. Anschließend wird die enzymatische Reaktion durch Zugabe der entsprechenden Substrate gestartet (5 µmol/l Boc-Ile-Glu-Gly-Arg-AMC von Bachem für Faktor Xa und Trypsin, 5 50 µmol/l MeOSuc-Ala-Phe-Lys-AMC von Bachem für Plasmin). Nach einer Inkubationszeit von 30 min bei 22°C wird die Fluoreszenz gemessen (Anregung: 360 nm, Emission: 460 nm). Die gemessenen Emissionen der Testansätze mit Prüfsubstanz werden mit den Kontrollansätzen ohne Prüfsubstanz (ausschließlich Dimethylsulfoxid anstatt Prüfsubstanz in Dimethylsulfoxid) verglichen und aus den Konzentrations-Wirkungs-Beziehungen IC₅₀-Werte berechnet.

### a.3) Thrombin Generation Assay (Thrombogram)

Die Wirkung der Prüfsubstanzen auf das Thrombogram (Thrombin Generation Assay nach Hemker) wird in vitro in Humanplasma (Octaplas® von der Firma Octapharma) bestimmt.

Im Thrombin Generation Assay nach Hemker wird die Aktivität von Thrombin in gerinnendem Plasma durch die Messung der fluoreszenten Spaltprodukte des Substrats I-1140 (Z-Gly-Gly-Arg-AMC, Bachem) bestimmt. Die Reaktionen werden in Gegenwart variierender Konzentrationen an Prüfsubstanz oder dem entsprechenden Lösungsmittel durchgeführt. Um die Reaktion zu starten werden Reagenzien der Firma Thrombinoscope verwendet (30 pM or 0.1 pM recombinant tissue factor, 24 µM phospholipids in HEPES). Außerdem wird ein Thrombin Calibrator der Firma Thrombinoscope verwendet, dessen amidolytische Aktivität zur Berechnung der Thrombinaktivität in einer Probe mit unbekannter Menge an Thrombin benötigt wird. Die Testdurchführung erfolgt nach Herstellerangaben (Thrombionsocpe BV): 4 µl der Prüfsubstanz oder des Lösungsmittels, 76 µl Plasma und 20 µl PPP-Reagenz oder Thrombin Calibrator werden 5 min bei 37°C inkubiert. Nach Zugabe von 20 µl 2.5 mM Thrombinsubstrat in 20 mM Hepes, 60 mg/ml BSA, 102 mM Calciumchlorid wird die Thrombin Generierung 120 min alle 20 s gemessen. Die Messung wird mit einem Fluorometer (Fluoroskan Ascent) der Firma Thermo Electron durchgeführt, der mit einem 390/460 nM Filterpaar und einem Dispenser ausgestattet ist.

Durch die Verwendung der "thrombinoscope software" wird das Thrombogramm berechnet und graphisch dargestellt. Die folgenden Parameter werden berechnet: lag time, time to Peak, Peak, ETP (endogenous thrombin potential) und start tail.

### a.4) Bestimmung der antikoagulatorischen Wirkung

Die antikoagulatorische Wirkung der Prüfsubstanzen wird in vitro in Human- und Rattenplasma bestimmt. Dazu wird Blut unter Verwendung einer 0.11 molaren Natriumcitrat-Lösung als Vorlage in einem Mischungsverhältnis Natriumcitrat/Blut 1/9 abgenommen. Das Blut wird unmittelbar nach der Abnahme gut gemischt und 15 Minuten bei ca. 4000 g zentrifugiert. Der Überstand wird abpipettiert.

Die Prothrombinzeit (PT, Synonyme: Thromboplastinzeit, Quick-Test) wird in Gegenwart variierender Konzentrationen an Prüfsubstanz oder dem entsprechenden Lösungsmittel mit einem handelsüblichen Testkit (Neoplastin® von der Firma Boehringer Mannheim oder Hemoliance® RecombiPlastin von der Firma Instrumentation Laboratory) bestimmt. Die Testverbindungen werden 3 Minuten bei 37°C mit dem Plasma inkubiert. Anschließend wird durch Zugabe von Thromboplastin die Gerinnung ausgelöst und der Zeitpunkt des Gerinnungseintritts bestimmt. Es wird die Konzentration an Prüfsubstanz ermittelt, die eine Verdoppelung der Prothrombinzeit bewirkt.

Die aktivierte partielle Thromboplastinzeit (APTT) wird in Gegenwart variierender Konzentrationen an Prüfsubstanz oder dem entsprechenden Lösungsmittel mit einem handelsüblichen Testkit (PTT Reagent von der Firma Roche) bestimmt. Die Testverbindungen werden 3 Minuten bei 37°C mit dem Plasma und dem PTT Reagenz (Cephalin, Kaolin) inkubiert. Anschließend wird durch Zugabe von 25 mM Calciumchlorid die Gerinnung ausgelöst und der Zeitpunkt des Gerinnungseintritts bestimmt. Es wird die Konzentration an Prüfsubstanz ermittelt, die eine 50%ige Verlängerung beziehungsweise ein Verdoppelung der APTT bewirkt.

### a.5) Bestimmung der Plasma-Kallikrein Aktivität

Zur Bestimmung der Plasma Kallikrein-Hemmung der erfindungsgemäßen Substanzen wird ein biochemisches Testsystem verwendet, in dem die Umsetzung eines peptidischen Plasma Kallikrein-Substrates zur Ermittlung der enzymatischen Aktivität von humanem Plasma Kallikrein benutzt wird. Dabei spaltet Plasma Kallikrein von dem peptischen Plasma Kallikrein-Substrat das C-terminale Aminomethylcoumarin (AMC) ab, dessen Fluoreszenz gemessen wird. Die Bestimmungen werden in Mikrotiterplatten durchgeführt.

Prüfsubstanzen werden in Dimethylsulfoxid aufgelöst und seriell in Dimethylsulfoxid verdünnt (3000 µM bis 0.0078 µM; resultierende Endkonzentrationen im Test: 50 µM bis 0.00013 µM). Jeweils 1 µl der verdünnten Substanzlösungen werden in die Vertiefungen von weißen Mikrotiterplatten der Firma Greiner (384 Vertiefungen) vorgelegt. Anschließend werden nacheinander 20 µl Assaypuffer (50 mM Tris/HCl pH 7,4; 100 mM Natriumchlorid-Lösung; 5 mM Calciumchlorid-Lösung; 0.1% bovines Serumalbumin) und 20 µl Plasma-Kallikrein der Firma Kordia (0.6 nM in Assaypuffer) hinzugefügt. Nach 15 min Inkubation wird die Enzymreaktion durch Zugabe von 20 µl des in Assaypuffer gelösten Substrates H-Pro-Phe-Arg-AMC (10 µM in Assaypuffer) der Firma Bachem gestartet, für 30 min bei Raumtemperatur (22°C) inkubiert und anschließend eine Fluoreszensmessung durchgeführt (Anregung: 360 nM, Emission: 460 nM). Die gemessenen Emissionen der Testansätze mit Prüfsubstanz werden mit denen von Kontrollansätzen ohne Prüfsubstanz (ausschließlich Dimethylsulfoxid anstatt Prüfsubstanz in Dimethylsulfoxid) verglichen und aus den Konzentrations-Wirkungs-Beziehungen IC₅₀-Werte berechnet.

**Tabelle B**

| **Beispiel-Nr.** | **IC₅₀ [nM]** | | **Beispiel-Nr.** | **IC₅₀ [nM]** |
|---|---|---|---|---|
| 1 | 140 | | 2 | 16 |
| 3 | 13 | | 4 | 44 |
| 5 | 7.1 | | 6 | 61 |
| 7 | 150 | | 9 | 4.8 |
| 10 | 7.0 | | 11 | 5.0 |
| 12 | 4.9 | | 13 | 13 |
| 14 | 65 | | 15 | 31 |
| 16 | 17 | | 17 | 24 |
| 18 | 130 | | 19 | 25 |
| 20 | 23 | | 21 | 8.0 |
| 22 | 8.3 | | 23 | 13 |
| 24 | 16 | | 25 | 23 |
| 26 | 12 | | 27 | 50 |
| 28 | 8.8 | | 29 | 11 |
| 30 | 120 | | 31 | 970 |
| 32 | 36 | | 33 | 250 |
| 34 | 17 | | 35 | 4.3 |
| 36 | 3.2 | | 37 | 65 |
| 38 | 29 | | 39 | 1800 |
| 40 | 51 | | 41 | 11 |

### a.6) Bestimmung der Endothel Integrität

Die Aktivität der erfindungsgemäßen Verbindungen werden mittels eines in-vitro Permeabilitäts-Assays auf "human umbilical venous cells" (HUVEC) charakterisiert. Mittels des EOS Apparatus (EC IS: Electric Cell-substrate Impedance Sensing; Applied Biophysics Inc; Troy, NY) können Unterschiede des transendothelialen elektrischen Widerstandes (TEER) über einer endothelialen Zell-Monoschicht, die über Gold-Elektroden plattiert wurde, kontinuierlich gemessen werden. HUVECs werden auf einer 96-well sensor Elektrodenplatte (96W1 E, Ibidi GmbH, Martinsried) ausgesäht. Eine Hyperpermeabilität der entstandenen konfluenten Zell-Monoschicht wird mittels Stimulation mit Kininogen, Prekallikrein und Faktor XII (je 100 nM) induziert. Die erfindungsgemäßen Verbindungen werden vor der Zugabe der oben angegebenen Substanzen zugegeben. Die üblichen Konzentrationen der Verbindungen sind 1 x 10⁻¹⁰ bis 1 x 10⁻⁶ M.

### a.7) Bestimmung der in-vitro Permeabilität von Endothelzellen

In einem weiteren Hyperpermeabilitäts-Modell wird die Aktivität der Substanzen auf die Modulation der makromolekularen Permeabilität bestimmt. HUVECs werden auf einer Fibronektin-überzogenen Transwell Filter Membran (24-well plates, 6.5 mm-Einsatz mit 0.4 µM Polykarbonat Membran; Costar #3413) ausgesäht. Die Filtermembran trennt den oberen von dem unteren Zellkulturraum mit der konfluenten Endothelzellschicht auf dem Boden des oberen Zellkulturraumes. Dem Medium des oberen Raumes wird 250 g/ml 40 kDa FITC-Dextan (Invitrogen, D1844) zugesetzt. Die Hyperpermeabilität der Monolayer-Schicht wird mittels Stimulation mit Kininogen, Prekallikrein und Faktor XII (je 100 nM) induziert. Medium Proben werden alle 30 min aus der unteren Kammer entnommen und die relative Fluoreszenz, als Parameter für die Veränderungen der makromolekularen Permeabilität in Abhängigkeit von der Zeit, mit einem Fluorimeter bestimmt. Die erfindungsgemäßen Verbindungen werden vor der Zugabe der oben angegebenen Substanzen zugegeben. Die üblichen Konzentationen der Verbindungen sind 1 x 10⁻¹⁰ bis 1 x 10⁻⁶ M.

### b) Bestimmung der antithrombotischen Wirkung (in vivo)

### b.1) Arterielles Thrombose-Modell (Eisen(II)chlorid-induzierte Thrombose) in Kombination mit Ohrblutungszeit im Kaninchen

Die antithrombotische Aktivität der FXIa-Inhibitoren wird in einem arteriellen Thrombose-Modell getestet. Dabei wird die Thrombusbildung durch chemische Beschädigung eines Bereichs der Arteria carotis im Kaninchen ausgelöst. Simultan wird die Ohrblutungszeit bestimmt.

Männliche Kaninchen (Crl:KBL (NZW)BR, Charles River) unter normaler Diät mit einem Gewicht von 2.2 - 2.5 kg Körpergewicht werden durch intramuskuläre Applikation von Xylazin und Ketamin (Rompun, Bayer, 5 mg/kg und Ketavet, Pharmacia & Upjohn GmbH, 40 mg/kg Körpergewicht) anästhesiert. Die Anästhesie wird weiterhin durch intravenöse Gabe derselben Präparate (bolus: Dauerinfusion) über die rechte Ohrvene unterstützt.

Nach Freipräparation der rechten Arteria carotis wird der Gefäßschaden dadurch erzeugt, dass ein Stück Filterpapier (10 mm x 10 mm) auf einem Streifen Parafilm® (25 mm x 12 mm) um die A. carotis gewickelt wird, ohne den Blutfluß dadurch zu beeinträchtigen. Das Filterpapier enthält 100 µL einer 13%igen Lösung von Eisen(II)chlorid (Sigma) in Wasser. Nach 5 min wird das Filterpapier entfernt und das Gefäß zweimal mit wässriger 0.9%iger Natriumchlorid-Lösung gespült. 30 min nach der Verletzung wird die Arteria carotis im Bereich der Schädigung herauspräpariert und eventuell vorhandenes thrombotisches Material entnommen und gewogen.

Die Prüfsubstanzen werden entweder intravenös über die Vena femoralis den anästhesierten oder oral mittels Schlundsonde den wachen Tieren jeweils 5 min beziehungsweise 2 h vor Schädigung verabreicht.

Die Ohrblutungszeit wird 2 min nach der Schädigung der Arteria carotis bestimmt. Hierzu wird das linke Ohr rasiert und ein definierter Schnitt von 3 mm Länge (Klinge Art.Nummer 10-150-10, Martin, Tuttlingen, Germany) parallel zur Ohrlängsachse gesetzt. Dabei wird darauf geachtet, kein sichtbares Gefäß zu verletzen. Eventuell austretendes Blut wird in 15 Sekunden-Intervallen mit genau gewogenen Filterpapierstücken aufgenommen, ohne die Wunde direkt zu berühren. Die Blutungszeit wird berechnet als die Zeitspanne vom Setzen des Schnitts bis zu dem Zeitpunkt, an dem am Filterpapier kein Blut mehr nachweisbar ist. Das ausgetretene Blutvolumen wird nach Wiegen der Filterpapierstücke berechnet.

### c) Bestimmung der Wirkung auf die Extravasation/Ödembildung und/oder Neovaskularisierung im Auge (in vivo)

### c.1) Testung der Wirksamkeit von Substanzen im Laser-induzierten choroidalen Neovaskularisierunes-Modell

Diese Studie dient dem Zweck, die Wirksamkeit einer Testsubstanz auf die Reduktion der Extravasation/Ödembildung und/oder der choroidalen Neovaskularisierung im Rattenmodell der Laser-induzierten choroidalen Neovaskularisierung zu untersuchen.

Dafür werden pigmentierte Ratten vom Stamm Brown-Norway, die keine Anzeichen ophthalmologischer Erkrankungen aufweisen, ausgewählt und nach dem Zufallsprinzip Behandlungsgruppen zugeordnet. Am Tag 0 werden die Tiere mittels intraperitonealer Injektion narkotisiert (15 mg/kg Xylazin und 80 mg/kg Ketamin). Nach Instillation eines Tropfens einer 0.5%igen Tropicamid-Lösung zur Weitstellung der Pupillen wird die choroidale Neovaskularisierung mittels eines 532 nm Argon Laser Photokoagulators an sechs definierten Stellen um den Nervus opticus herum ausgelöst (50-75 µm Durchmesser, 150 mW Intensität, 100 ms Dauer). Die Testsubstanz und das entsprechende Vehikel (z.B. PBS, isotone Natriumchlorid Lösung) werden entweder systemisch oral beziehungsweise intraperitonal appliziert oder lokal am Auge durch mehrfache Gabe als Augentropfen beziehungsweise intravitreale Injektion verabreicht. Das Körpergewicht aller Tiere wird vor Beginn der Studie, und anschließend täglich während der Studie bestimmt.

An Tag 21 wird eine Angiographie mittels einer Fluoreszenz-Funduskammera (z.B. Kowe, HRA) durchgeführt. In Narkose und nach erneuter Pupillenerweiterung wird ein 10%iger Natrium-Fluorescein-Farbstoff subkutan (s.c.) injiziert. 2-10 min später werden Bilder des Augenhintergrundes aufgenommen. Die Stärke der Extravasation/des Ödems, dargestellt durch die Leckage von Fluorescein, wird von zwei bis drei verblindeten Beobachtern beurteilt und in Schweregrade von 0 (keine Extravasation) bis 3 (starke Einfärbung über die eigentliche Läsion hinaus) eingeteilt.

Nach Abtöten der Tiere an Tag 23 werden die Augen entnommen und in 4%iger Paraformaldehyd-Lösung für eine Stunde bei Raumtemperatur fixiert. Nach einem Waschdurchgang wird die Retina vorsichtig herausgeschält, und der Sklera-Choroidea-Komplex wird mit einem FITC-Isolektin B4 Antikörper angefärbt und anschließend flach auf einen Objetträger aufgebracht. Die so erhaltenen Präparate werden mittels eines Fluoreszenz-Mikroskops (Apotom, Zeiss) bei einer Anregungs-Wellenlänge von 488 nm ausgewertet. Die Fläche beziehungsweise das Volumen der choroidalen Neovaskularisierung (in [µm² bzw. µm³) wird durch morphometrische Analyse mittels Axiovision 4.6 Software berechnet.

### c.2) Testung der Wirksamkeit von Substanzen im Sauerstoff-induzierten Retinopathie Modell

Es wurde gezeigt, dass eine durch Sauerstoff induzierte Retinopathie ein wertvolles Tiermodell für die Untersuchung der pathologischen retinalen Angiogenese darstellt. Dieses Modell basiert auf der Beobachtung, dass eine Hyperoxie während der frühen postnatalen Entwicklung in der Retina zum Anhalten oder zur Verlangsamung des Wachstums von normalen retinalen Blutgefäßen führt. Sobald die Tiere nach einer 7-tägigen Hyperoxiephase zur normoxischen Raumluft zurückkehren, ist dieses gleichbedeutend einer relativen Hypoxie, da in der Retina die normalen Gefäße fehlen, welche erforderlich sind, um eine ausreichende Versorgung des neuralen Gewebes unter normoxischen Bedingungen zu gewährleisten. Die dadurch erzeugte ischämische Situation führt zur abnormen Neovaskularisation, die einige Ähnlichkeiten mit der pathophysiologischen Neovaskularisation in Augenerkrankungen wie der feuchten AMD aufzeigt. Darüber hinaus ist die hervorgerufene Neovaskularisierung sehr reproduzierbar, quantifizierbar und ein wichtiger Parameter für die Untersuchung der Krankheitsmechanismen und möglichen Behandlungen für verschiedenste Formen von Netzhauterkrankungen.

Das Ziel dieser Studie ist es, die Wirksamkeit von täglich systemisch verabreichten Dosen der Testverbindung auf das Wachstum der retinalen Gefäße im Sauerstoff-induzierten Retinopathie-Modell zu untersuchen. Neugeborene von C57B1 / 6 Mäusen und ihre Mütter werden am postnatalen Tag 7 (PD7) einer Hyperoxie (70% Sauerstoff) für 5 Tage ausgesetzt. Ab PD12, werden die Mäuse unter normoxischen Bedingungen (Raumluft, 21% Sauerstoff) bis zum PD17 gehalten. Von Tag 12 bis Tag 17 werden die Mäuse täglich mit der Testsubstanz oder dem entsprechenden Vehikel behandelt. Am Tag 17 werden alle Mäuse mit Isofluran narkotisiert und anschließend durch Genickbruch abgetötet. Die Augen werden entnommen und in 4% Formalin fixiert. Nach dem Waschen in Phosphat-gepufferter Natriumchlorid-Lösung wird die Netzhaut präpariert, ein Flachpräperat davon erzeugt und dieses mit Isolectin B4 Antikörper gefärbt. Die Quantifizierung der neugewachsenen Gefäße wird unter Verwendung eines Zeiss ApoTome durchgeführt.

### C) Ausführungsbeispiele für pharmazeutische Zusammensetzungen

Die erfindungsgemäßen Substanzen können folgendermaßen in pharmazeutische Zubereitungen überführt werden:

### Tablette:

### Zusammensetzung:

100 mg der Verbindung des Beispiels 1, 50 mg Lactose (Monohydrat), 50 mg Maisstärke, 10 mg Polyvinylpyrolidon (PVP 25) (Fa. BASF, Deutschland) und 2 mg Magnesiumstearat.

Tablettengewicht 212 mg. Durchmesser 8 mm, Wölbungsradius 12 mm.

### Herstellung:

Die Mischung aus der Verbindung des Beispiels 1, Lactose und Stärke wird mit einer 5%-igen Lösung (m/m) des PVPs in Wasser granuliert. Das Granulat wird nach dem Trocknen mit dem Magnesiumstearat für 5 min. gemischt. Diese Mischung wird mit einer üblichen Tablettenpresse verpresst (Format der Tablette siehe oben).

### Orale Suspension:

### Zusammensetzung:

1000 mg der Verbindung des Beispiels 1, 1000 mg Ethanol (96%), 400 mg Rhodigel (Xanthan gum) (Fa. FMC, USA) und 99 g Wasser.

Einer Einzeldosis von 100 mg der erfindungsgemäßen Verbindung entsprechen 10 ml orale Suspension.

### Herstellung:

Das Rhodigel wird in Ethanol suspendiert, die Verbindung des Beispiels 1 wird der Suspension zugefügt. Unter Rühren erfolgt die Zugabe des Wassers. Bis zum Abschluss der Quellung des Rhodigels wird ca. 6 h gerührt.

### Lösung oder Suspension zur topischen Anwendung am Auge (Augentropfen):

Eine sterile pharmazeutische Zubereitung zur topischen Anwendung am Auge kann durch Rekonstitution eines Lyophilisats der erfindungsgemäßen Verbindung in steriler Natriumchlorid-Lösung hergestellt werden. Als Konservierungsmittel für eine solche Lösung oder Suspension ist beispielsweise Benzalkoniumchlorid, Thiomersal oder Phenylquecksilbernitrat in einem Konzentrationsbereich von 0.001 bis 1 Gewichtsprozent geeignet.

## Patentansprüche

1. Verbindung der Formel 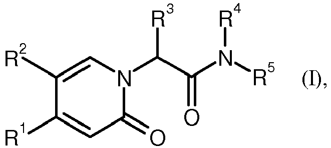 in welcher
R¹ für eine Gruppe der Formel 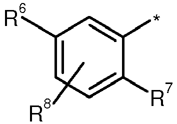 steht,
wobei * die Anknüpfstelle an den Oxopyridinring ist,
R⁶ für Brom, Chlor, Fluor, Methyl, Difluormethyl, Trifluormethyl, Methoxy, Difluormethoxy oder Trifluormethoxy steht,
R⁷ für Brom, Chlor, Fluor, Cyano, Nitro, Hydroxy, Methyl, Difluormethyl, Trifluormethyl, Methoxy, Ethoxy, Difluormethoxy, Trifluormethoxy, Ethinyl, 3,3,3-Trifluorprop-1-in-1-yl oder Cyclopropyl steht,
R⁸ für Wasserstoff, Chlor oder Fluor steht,
R² für Wasserstoff, Brom, Chlor, Fluor, Cyano, C₁-C₃-Alkyl, Difluormethyl, Trifluormethyl, 1,1-Difluorethyl, 2,2-Difluorethyl, 2,2,2-Trifluorethyl, C₁-C₃-Alkoxy, Difluormethoxy, Trifluormethoxy, 1,1-Difluorethoxy, 2,2-Difluorethoxy, 2,2,2-Trifluorethoxy, Hydroxycarbonyl, Methylcarbonyl oder Cyclopropyl steht,
R³ für Wasserstoff, C₁-C₅-Alkyl, C₁-C₄-Alkoxy, Difluormethyl, Trifluormethyl, 1,1-Difluorethyl, 3,3,3-Trifluor-2-hydroxyprop-1-yl, 3,3,3-Trifluor-2-methoxyprop-1-yl, 3,3,3-Trifluor-2-ethoxyprop-1-yl, Prop-2-in-1-yl, Cyclopropyloxy oder Cyclobutyloxy steht,
wobei Alkyl substituiert sein kann mit einem Substituenten ausgewählt aus der Gruppe bestehend aus Fluor, Cyano, Hydroxy, Difluormethyl, Trifluormethyl, Methoxy, Ethoxy, Difluormethoxy, Trifluormethoxy, C₃-C₆-Cycloalkyl, 4- bis 6-gliedriges Oxo- Heterocyclyl, 1,4-Dioxanyl, Oxazolyl, Phenyl und Pyridyl,
worin Cycloalkyl substituiert sein kann mit 1 bis 2 Substituenten unabhängig voneinander ausgewählt aus der Gruppe bestehend aus Fluor, Hydroxy, Methyl, Ethyl, Methoxy, Ethoxy, Difluormethyl, Trifluormethyl, Difluormethoxy und Trifluormethoxy,
R⁴ für Wasserstoff steht,
R⁵ für eine Gruppe der Formel 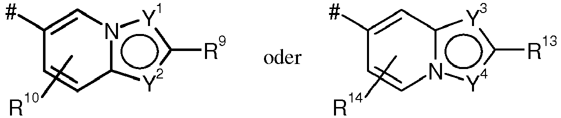 oder 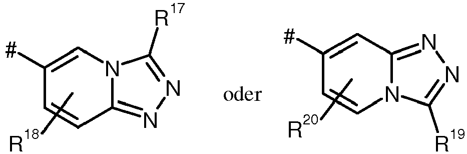 oder 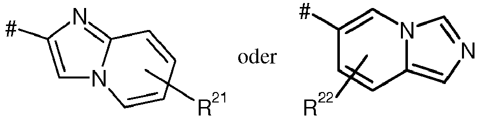 steht,
wobei # die Anknüpfstelle an das Stickstoffatom ist,
Y¹ für ein Stickstoffatom oder C-R¹¹ steht,
worin
R¹¹ für Wasserstoff, Chlor, Hydroxy, Methoxy oder C₁-C₃-Alkoxycarbonyl steht,
Y² für ein Stickstoffatom oder C-R¹² steht,
worin
R¹² für Wasserstoff, Chlor, Hydroxy oder Methoxy steht,
R⁹ für Wasserstoff, Hydroxycarbonyl, Hydroxycarbonylmethyl oder Phenyl steht,
worin Phenyl substituiert sein kann mit 1 bis 2 Substituenten Fluor,
R¹⁰ für Wasserstoff, Chlor, Fluor oder Methyl steht,
Y³ für ein Stickstoffatom oder C-R¹⁵ steht,
worin
R¹⁵ für Wasserstoff, Chlor, Hydroxy oder Methoxy steht,
Y⁴ für ein Stickstoffatom oder C-R¹⁶ steht,
worin
R¹⁶ für Wasserstoff, Chlor, Hydroxy oder Methoxy steht,
R¹³ für Wasserstoff, Hydroxycarbonyl, Hydroxycarbonylmethyl, C₁-C₃-Alkoxycarbonyl oder Aminocarbonyl steht,
R¹⁴ für Wasserstoff, Chlor, Fluor oder Methyl steht,
R¹⁷ für Wasserstoff, Chlor, Hydroxy, C₁-C₄-Alkyl, Methoxy, C₁-C₃-Alkylaminomethyl oder Morpholinylmethyl steht,
R¹⁸ für Wasserstoff, Chlor, Fluor oder Methyl steht,
R¹⁹ für Wasserstoff, Chlor, Hydroxy oder Methoxy steht,
R²⁰ für Wasserstoff, Chlor, Fluor oder Methyl steht,
R²¹ für Wasserstoff, Hydroxycarbonyl oder Hydroxycarbonylmethyl steht,
R²² für Wasserstoff, Chlor, Fluor oder Methyl steht,
oder eines ihrer Salze, ihrer Solvate oder der Solvate ihrer Salze.

2. Verbindung nach Anspruch 1, **dadurch gekennzeichnet, dass**
R¹ für eine Gruppe der Formel 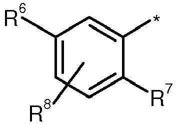 steht,
wobei * die Anknüpfstelle an den Oxopyridinring ist,
R⁶ für Chlor steht,
R⁷ für Cyano, Difluormethyl, Trifluormethyl, Difluormethoxy oder Trifluormethoxy steht,
R⁸ für Wasserstoff steht,
R² für Chlor, Cyano, Methoxy, Ethoxy oder Difluormethoxy steht,
R³ für Wasserstoff, Methyl, Ethyl, n-Propyl, 2-Methyl-prop-1-yl, n-Butyl oder Ethoxy steht,
wobei Methyl substituiert sein kann mit einem Substituenten ausgewählt aus der Gruppe bestehend aus Difluormethyl, Trifluormethyl, Cyclopropyl, Cyclobutyl, Cyclohexyl, Oxetanyl, Tetrahydrofuranyl, Tetrahydro-2H-pyranyl und 1,4-Dioxanyl,
worin Cyclopropyl, Cyclobutyl, Cyclohexyl und Oxetanyl substituiert sein können mit 1 bis 2 Substituenten unabhängig voneinander ausgewählt aus der Gruppe bestehend aus Fluor, Hydroxy, Methyl, Ethyl und Methoxy,
und
wobei Ethyl, n-Propyl und n-Butyl substituiert sein können mit einem Substituenten ausgewählt aus der Gruppe bestehend aus Fluor, Methoxy und Trifluormethoxy,
R⁴ für Wasserstoff steht,
R⁵ für eine Gruppe der Formel 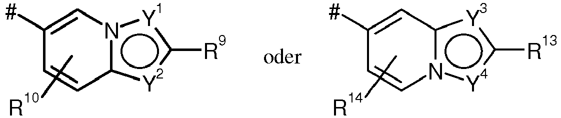 oder 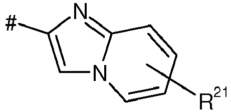 steht,
wobei # die Anknüpfstelle an das Stickstoffatom ist,
Y¹ für ein Stickstoffatom oder C-R¹¹ steht,
worin
R¹¹ für Wasserstoff, Chlor, Hydroxy oder Methoxy steht,
Y² für ein Stickstoffatom oder C-R¹² steht,
worin
R¹² für Wasserstoff, Chlor, Hydroxy oder Methoxy steht,
R⁹ für Wasserstoff oder Hydroxycarbonyl steht,
R¹⁰ für Wasserstoff oder Fluor steht,
Y³ für ein Stickstoffatom oder C-R¹⁵ steht,
worin
R¹⁵ für Wasserstoff, Chlor, Hydroxy oder Methoxy steht,
Y⁴ für ein Stickstoffatom oder C-R¹⁶ steht,
worin
R¹⁶ für Wasserstoff, Chlor, Hydroxy oder Methoxy steht,
R¹³ für Wasserstoff oder Hydroxycarbonyl steht,
R¹⁴ für Wasserstoff oder Fluor steht,
R²¹ für Wasserstoff oder Hydroxycarbonyl steht,
oder eines ihrer Salze, ihrer Solvate oder der Solvate ihrer Salze.

3. Verbindung nach einem der Ansprüche 1 oder 2, **dadurch gekennzeichnet, dass**
R¹ für eine Gruppe der Formel 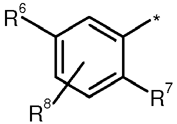 steht,
wobei * die Anknüpfstelle an den Oxopyridinring ist,
R⁶ für Chlor steht,
R⁷ für Cyano oder Difluormethoxy steht,
R⁸ für Wasserstoff steht,
R² für Methoxy steht,
R³ für Methyl oder Ethyl steht,
wobei Methyl substituiert sein kann mit einem Substituenten ausgewählt aus der Gruppe bestehend aus Cyclobutyl und Tetrahydro-2H-pyranyl,
und
wobei Ethyl substituiert sein kann mit einem Substituenten Methoxy,
R⁴ für Wasserstoff steht,
R⁵ für eine Gruppe der Formel 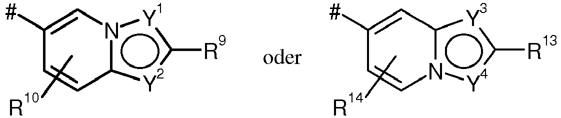 steht,
wobei # die Anknüpfstelle an das Stickstoffatom ist,
Y¹ für ein C-R¹¹ steht,
worin
R¹¹ für Wasserstoff oder Chlor steht,
Y² für ein Stickstoffatom steht,
R⁹ für Wasserstoff oder Hydroxycarbonyl steht,
R¹⁰ für Wasserstoff steht,
Y³ für ein Stickstoffatom steht,
und
Y⁴ für C-R¹⁶ steht,
worin
R¹⁶ für Wasserstoff steht,
oder
Y³ für C-R¹⁵ steht,
worin
R¹⁵ für Wasserstoff oder Chlor steht,
und
Y⁴ für ein Stickstoffatom steht,
R¹³ für Wasserstoff oder Hydroxycarbonyl steht,
R¹⁴ für Wasserstoff steht,
oder eines ihrer Salze, ihrer Solvate oder der Solvate ihrer Salze.

4. Verfahren zur Herstellung einer Verbindung der Formel (I) oder eines ihrer Salze, ihrer Solvate oder der Solvate ihrer Salze nach Anspruch 1, **dadurch gekennzeichnet, dass** entweder
[A] eine Verbindung der Formel 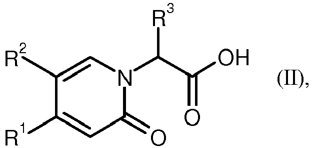 in welcher
R¹, R² und R³ die in Anspruch 1 angegebene Bedeutung haben,
in der ersten Stufe mit einer Verbindung der Formel 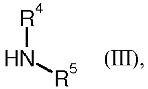 in welcher
R⁴ und R⁵ die in Anspruch 1 angegebene Bedeutung haben,
in Gegenwart eines Dehydratisierungsreagenzes umgesetzt wird, und
gegebenfalls in einer zweiten Stufe durch saure oder basische Esterspaltung zu einer Verbindung der Formel (I) umgesetzt wird,
oder
[B] eine Verbindung der Formel 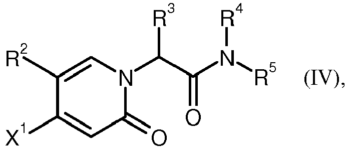 in welcher
R², R³, R⁴ und R⁵ die in Anspruch 1 angegebene Bedeutung haben, und
X¹ für Chlor, Brom oder Iod steht,
mit einer Verbindung der Formel
R¹-Q (V),
in welcher
R¹ die in Anspruch 1 angegebene Bedeutung hat, und
Q für -B(OH)₂, einen Boronsäure-Ester, bevorzugt Boronsäurepinakolester, oder - BF₃⁻K⁺ steht,
unter Suzuki-Kupplungsbedingungen zu einer Verbindung der Formel (I) umgesetzt wird.

5. Verbindung nach einem der Ansprüche 1 bis 3 zur Anwendung in der Behandlung und/oder Prophylaxe von Krankheiten.

6. Verwendung einer Verbindung nach einem der Ansprüche 1 bis 3 zur Herstellung eines Arzneimittels zur Behandlung und/oder Prophylaxe von Krankheiten.

7. Verwendung einer Verbindung nach einem der Ansprüche 1 bis 3 zur Herstellung eines Arzneimittels zur Behandlung und/oder Prophylaxe von thrombotischen beziehungsweise thromboembolischen Erkrankungen.

8. Verwendung einer Verbindung nach einem der Ansprüche 1 bis 3 zur Herstellung eines Arzneimittels zur Behandlung und/oder Prophylaxe von ophthalmologischen Erkrankungen.

9. Verwendung einer Verbindung nach einem der Ansprüche 1 bis 3 zur Herstellung eines Arzneimittels zur Behandlung und/oder Prophylaxe von hereditärem Angioödem oder entzündlichen Erkrankungen des Darms, wie Morbus Crohn oder Colitis ulcerosa.

10. Arzneimittel enthaltend eine Verbindung nach einem der Ansprüche 1 bis 3 in Kombination mit einem inerten, nichttoxischen, pharmazeutisch geeigneten Hilfsstoff.

11. Arzneimittel nach Anspruch 10 zur Anwendung in der Behandlung und/oder Prophylaxe von thrombotischen beziehungsweise thromboembolischen Erkrankungen.

12. Arzneimittel nach Anspruch 10 zur Anwendung in der Behandlung und/oder Prophylaxe von ophthalmologischen Erkrankungen.

13. Arzneimittel nach Anspruch 10 zur Anwendung in der Behandlung und/oder Prophylaxe von hereditärem Angioödem oder entzündlichen Erkrankungen des Darms, wie Morbus Crohn oder Colitis ulcerosa.

## Claims

1. Compound of the formula 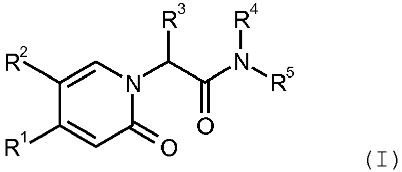 in which
R¹ represents a group of the formula 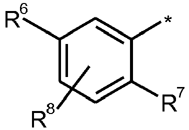 where * is the point of attachment to the oxopyridine ring,
R⁶ represents bromine, chlorine, fluorine, methyl, difluoromethyl, trifluoromethyl, methoxy, difluoromethoxy or trifluoromethoxy,
R⁷ represents bromine, chlorine, fluorine, cyano, nitro, hydroxy, methyl, difluoromethyl, trifluoromethyl, methoxy, ethoxy, difluoromethoxy, trifluoromethoxy, ethynyl, 3,3,3-trifluoroprop-1-yn-1-yl or cyclopropyl,
R⁸ represents hydrogen, chlorine or fluorine,
R² represents hydrogen, bromine, chlorine, fluorine, cyano, C₁-C₃-alkyl, difluoromethyl, trifluoromethyl, 1,1-difluoroethyl, 2,2-difluoroethyl, 2,2,2-trifluoroethyl, C₁-C₃-alkoxy, difluoromethoxy, trifluoromethoxy, 1,1-difluoroethoxy, 2,2-difluoroethoxy, 2,2,2-trifluoroethoxy, hydroxycarbonyl, methylcarbonyl or cyclopropyl,
R³ represents hydrogen, C₁-C₅-alkyl, C₁-C₄-alkoxy, difluoromethyl, trifluoromethyl, 1,1-difluoroethyl, 3,3,3-trifluoro-2-hydroxyprop-1-yl, 3,3,3-trifluoro-2-methoxyprop-1-yl, 3,3,3-trifluoro-2-ethoxyprop-1-yl, prop-2-yn-1-yl, cyclopropyloxy or cyclobutyloxy,
where alkyl may be substituted by a substituent selected from the group consisting of fluorine, cyano, hydroxy, difluoromethyl, trifluoromethyl, methoxy, ethoxy, difluoromethoxy, trifluoromethoxy, C₃-C₆-cycloalkyl, 4- to 6-membered oxoheterocyclyl, 1,4-dioxanyl, oxazolyl, phenyl and pyridyl,
where cycloalkyl may be substituted by 1 to 2 substituents independently of one another selected from the group consisting of fluorine, hydroxy, methyl, ethyl, methoxy, ethoxy, difluoromethyl, trifluoromethyl, difluoromethoxy and trifluoromethoxy,
R⁴ represents hydrogen,
R⁵ represents a group of the formula 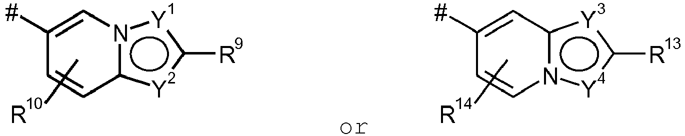 or 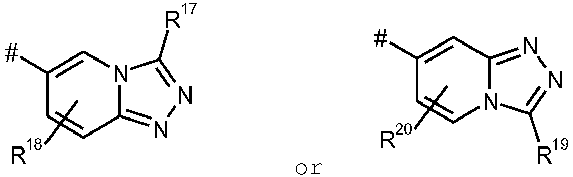 or 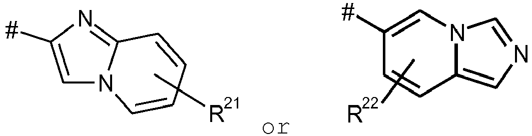 where # is the point of attachment to the nitrogen atom,
Y¹ represents a nitrogen atom or C-R¹¹,
where
R¹¹ represents hydrogen, chlorine, hydroxy, methoxy or C₁-C₃-alkoxycarbonyl,
Y² represents a nitrogen atom or C-R¹²,
where
R¹² represents hydrogen, chlorine, hydroxy or methoxy,
R⁹ represents hydrogen, hydroxycarbonyl, hydroxycarbonylmethyl or phenyl,
where phenyl may be substituted by 1 to 2 fluorine substituents,
R¹⁰ represents hydrogen, chlorine, fluorine or methyl,
Y³ represents a nitrogen atom or C-R¹⁵,
where
R¹⁵ represents hydrogen, chlorine, hydroxy or methoxy,
Y⁴ represents a nitrogen atom or C-R¹⁶,
where
R¹⁶ represents hydrogen, chlorine, hydroxy or methoxy,
R¹³ represents hydrogen, hydroxycarbonyl, hydroxycarbonylmethyl, C₁-C₃-alkoxycarbonyl or aminocarbonyl,
R¹⁴ represents hydrogen, chlorine, fluorine or methyl,
R¹⁷ represents hydrogen, chlorine, hydroxy, C₁-C₄-alkyl, methoxy, C₁-C₃-alkylaminomethyl or morpholinylmethyl, R¹⁸ represents hydrogen, chlorine, fluorine or methyl,
R¹⁹ represents hydrogen, chlorine, hydroxy or methoxy,
R²⁰ represents hydrogen, chlorine, fluorine or methyl,
R²¹ represents hydrogen, hydroxycarbonyl or hydroxycarbonylmethyl,
R²² represents hydrogen, chlorine, fluorine or methyl,
or one of the salts thereof, solvates thereof or solvates of the salts thereof.

2. Compound according to Claim 1, **characterized in that**
R¹ represents a group of the formula 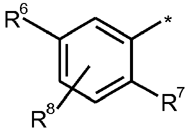 where * is the point of attachment to the oxopyridine ring,
R⁶ represents chlorine,
R⁷ represents cyano, difluoromethyl, trifluoromethyl, difluoromethoxy or trifluoromethoxy,
R⁸ represents hydrogen,
R² represents chlorine, cyano, methoxy, ethoxy or difluoromethoxy,
R³ represents hydrogen, methyl, ethyl, n-propyl, 2-methylprop-1-yl, n-butyl or ethoxy,
where methyl may be substituted by a substituent selected from the group consisting of difluoromethyl, trifluoromethyl, cyclopropyl, cyclobutyl, cyclohexyl, oxetanyl, tetrahydrofuranyl, tetrahydro-2H-pyranyl and 1,4-dioxanyl,
where cyclopropyl, cyclobutyl, cyclohexyl and oxetanyl may be substituted by 1 to 2 substituents independently of one another selected from the group consisting of fluorine, hydroxy, methyl, ethyl and methoxy, and
where ethyl, n-propyl and n-butyl may be substituted by a substituent selected from the group consisting of fluorine, methoxy and trifluoromethoxy,
R⁴ represents hydrogen,
R⁵ represents a group of the formula 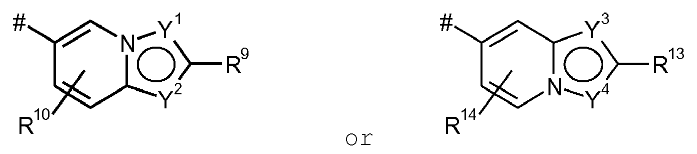 or 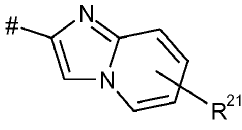 where # is the point of attachment to the nitrogen atom,
Y¹ represents a nitrogen atom or C-R¹¹,
where
R¹¹ represents hydrogen, chlorine, hydroxy or methoxy,
Y² represents a nitrogen atom or C-R¹²,
where
R¹² represents hydrogen, chlorine, hydroxy or methoxy,
R⁹ represents hydrogen or hydroxycarbonyl,
R¹⁰ represents hydrogen or fluorine,
Y³ represents a nitrogen atom or C-R¹⁵,
where
R¹⁵ represents hydrogen, chlorine, hydroxy or methoxy,
Y⁴ represents a nitrogen atom or C-R¹⁶,
where
R¹⁶ represents hydrogen, chlorine, hydroxy or methoxy,
R¹³ represents hydrogen or hydroxycarbonyl,
R¹⁴ represents hydrogen or fluorine,
R²¹ represents hydrogen or hydroxycarbonyl,
or one of the salts thereof, solvates thereof or solvates of the salts thereof.

3. Compound according to either of Claims 1 and 2, **characterized in that**
R¹ represents a group of the formula 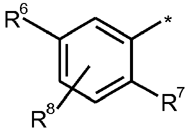 where * is the point of attachment to the oxopyridine ring,
R⁶ represents chlorine,
R⁷ represents cyano or difluoromethoxy,
R⁸ represents hydrogen,
R² represents methoxy,
R³ represents methyl or ethyl
where methyl may be substituted by a substituent selected from the group consisting of cyclobutyl and tetrahydro-2H-pyranyl,
and
where ethyl may be substituted by a methoxy substituent,
R⁴ represents hydrogen,
R⁵ represents a group of the formula 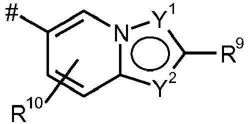 or 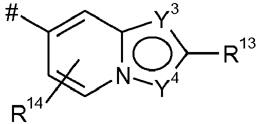 where # is the point of attachment to the nitrogen atom,
Y¹ represents C-R¹¹,
where
R¹¹ represents hydrogen or chlorine,
Y² represents a nitrogen atom,
R⁹ represents hydrogen or hydroxycarbonyl,
R¹⁰ represents hydrogen,
Y³ represents a nitrogen atom,
and
Y⁴ represents C-R¹⁶,
where
R¹⁶ represents hydrogen,
or
Y³ represents C-R¹⁵,
where
R¹⁵ represents hydrogen or chlorine,
and
Y⁴ represents a nitrogen atom,
R¹³ represents hydrogen or hydroxycarbonyl,
R¹⁴ represents hydrogen,
or one of the salts thereof, solvates thereof or solvates of the salts thereof.

4. Process for preparing a compound of the formula (I) or one of the salts thereof, solvates thereof or solvates of the salts thereof according to Claim 1, **characterized in that** either
[A] a compound of the formula 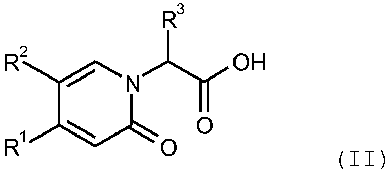 in which
R¹, R² and R³ are each as defined in Claim 1,
is reacted in the first step with a compound of the formula 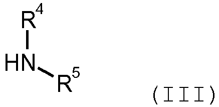 in which
R⁴ and R⁵ are each as defined in Claim 1,
in the presence of a dehydrating agent, and
optionally in a second step converted by acidic or basic ester hydrolysis into a compound of the formula (I),
or
[B] a compound of the formula 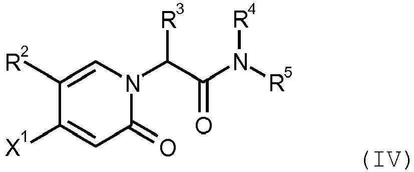 in which
R², R³, R⁴ and R⁵ have the meaning given in Claim 1, and
X¹ represents chlorine, bromine or iodine,
is reacted with a compound of the formula
R¹-Q (V)
in which
R¹ has the meaning given in Claim 1, and
Q represents -B(OH)₂, a boronic ester, preferably boronic acid pinacol ester, or -BF₃⁻K⁺,
under Suzuki coupling conditions to give a compound of the formula (I).

5. Compound according to any of Claims 1 to 3 for use in the treatment and/or prophylaxis of diseases.

6. Use of a compound according to any of Claims 1 to 3 for producing a medicament for the treatment and/or prophylaxis of diseases.

7. Use of a compound according to any of Claims 1 to 3 for producing a medicament for the treatment and/or prophylaxis of thrombotic or thromboembolic disorders.

8. Use of a compound according to any of Claims 1 to 3 for producing a medicament for the treatment and/or prophylaxis of ophthalmic disorders.

9. Use of a compound according to any of Claims 1 to 3 for producing a medicament for the treatment and/or prophylaxis of hereditary angiooedema or inflammatory disorders of the intestine, such as Crohn's disease or ulcerative colitis.

10. Medicament comprising a compound according to any of Claims 1 to 3 in combination with an inert, nontoxic, pharmaceutically suitable excipient.

11. Medicament according to Claim 10 for use in the treatment and/or prophylaxis of thrombotic or thromboembolic disorders.

12. Medicament according to Claim 10 for use in the treatment and/or prophylaxis of ophthalmic disorders.

13. Medicament according to Claim 10 for use in the treatment and/or prophylaxis of hereditary angiooedema or inflammatory disorders of the intestine, such as Crohn's disease or ulcerative colitis.

## Revendications

1. Composé de formule 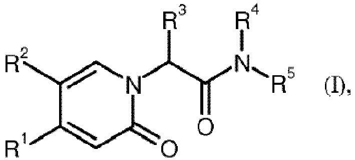 dans laquelle
R¹ représente un groupe de formule 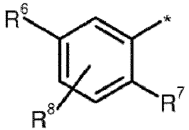 dans laquelle * est le point d'attachement au cycle oxopyridine,
R⁶ représente un atome de brome, chlore, fluor, un groupe méthyle, difluorométhyle, trifluorométhyle, méthoxy, difluorométhoxy ou trifluorométhoxy,
R⁷ représente un atome de brome, chlore, fluor, un groupe cyano, nitro, hydroxy, méthyle, difluorométhyle, trifluorométhyle, méthoxy, éthoxy, difluorométhoxy, trifluorométhoxy, éthynyle, 3,3,3-trifluoroprop-1-yn-1-yle ou cyclopropyle,
R⁸ représente un atome d'hydrogène, de chlore ou de fluor,
R² représente un atome d'hydrogène, de brome, chlore, fluor, un groupe cyano, alkyle en C₁-C₃, difluorométhyle, trifluorométhyle, 1,1-difluoro-éthyle, 2,2-difluoroéthyle, 2,2,2-trifluoroéthyle, alcoxy en C₁-C₃, difluorométhoxy, trifluorométhoxy, 1,1-difluoroéthoxy, 2,2-difluoroéthoxy, 2,2,2-trifluoroéthoxy, hydroxycarbonyle, méthylcarbonyle ou cyclopropyle,
R³ représente un atome d'hydrogène, un groupe alkyle en C₁-C₅, alcoxy en C₁-C₄, difluorométhyle, trifluorométhyle, 1,1-difluoroéthyle, 3,3,3-trifluoro-2-hydroxyprop-1-yle, 3,3,3-trifluoro-2-méthoxyprop-1-yle, 3,3,3-trifluoro-2-éthoxyprop-1-yle, prop-2-yn-1-yle, cyclopropyloxy ou cyclo-butyloxy,
le groupe alkyle pouvant être substitué par un substituant choisi dans le groupe constitué par fluoro, cyano, hydroxy, difluorométhyle, trifluorométhyle, méthoxy, éthoxy, difluoro-méthoxy, trifluorométhoxy, cycloalkyle en C₃-C₆, oxo- hétérocyclyle à 4 à 6 chaînons, 1,4-dioxanyle, oxazolyle, phényle et pyridyle,
le groupe cycloalkyle pouvant être substitué par 1 ou 2 substituants choisis indépendamment l'un de l'autre dans le groupe constitué par fluoro, hydroxy, méthyle, éthyle, méthoxy, éthoxy, difluorométhyle, trifluorométhyle, difluorométhoxy et trifluorométhoxy,
R⁴ représente un atome d'hydrogène,
R⁵ représente un groupe de formule 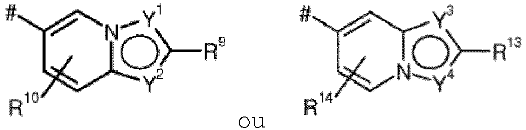 ou 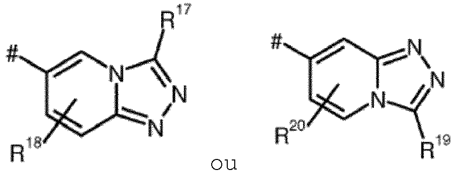 ou 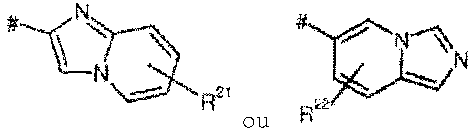 # étant le point d'attachement à l'atome d'azote,
Y¹ représentant un atome d'azote ou C-R¹¹,
où
R¹¹ représente un atome d'hydrogène ou de chlore, un groupe hydroxy, méthoxy ou alcoxy (C₁-C₃) carbonyle,
Y² représentant un atome d'azote ou C-R¹²,
où
R¹² représente un atome d'hydrogène ou de chlore, un groupe hydroxy ou méthoxy,
R⁹ représentant un atome d'hydrogène, un groupe hydroxycarbonyle, hydroxycarbonylméthyle ou phényle,
le groupe phényle pouvant être substitué par 1 ou 2 substituants fluoro,
R¹⁰ représentant un atome d'hydrogène, un atome de chlore ou de fluor ou un groupe méthyle,
Y³ représentant un atome d'azote ou C-R¹⁵,
où
R¹⁵ représente un atome d'hydrogène ou de chlore, un groupe hydroxy ou méthoxy,
Y⁴ représentant un atome d'azote ou C-R¹⁶,
où
R¹⁶ représente un atome d'hydrogène ou de chlore, un groupe hydroxy ou méthoxy,
R¹³ représentant un atome d'hydrogène, un groupe hydroxycarbonyle, hydroxycarbonylméthyle, alcoxy(C₁-C₃)carbonyle ou aminocarbonyle,
R¹⁴ représentant un atome d'hydrogène, de chlore ou de fluor ou un groupe méthyle,
R¹⁷ représentant un atome d'hydrogène ou de chlore, un groupe hydroxy, alkyle en C₁-C₄, méthoxy, alkyl(C₁-C₃)aminométhyle ou morpholinométhyle,
R¹⁸ représentant un atome d'hydrogène, de chlore ou de fluor ou un groupe méthyle,
R¹⁹ représentant un atome d'hydrogène ou de chlore ou un groupe hydroxy ou méthoxy,
R²⁰ représentant un atome d'hydrogène, de chlore ou de fluor ou un groupe méthyle,
R²¹ représentant un atome d'hydrogène, un groupe hydroxycarbonyle ou hydroxycarbonylméthyle,
R²² représentant un atome d'hydrogène, de chlore ou de fluor ou un groupe méthyle,
ou un de ses sels, de ses produits de solvatation ou des produits de solvatation de ses sels.

2. Composé selon la revendication 1, **caractérisé en ce que**
R¹ représente un groupe de formule 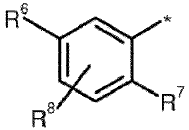 dans laquelle * est le point d'attachement au cycle oxopyridine,
R⁶ représente un atome de chlore,
R⁷ représente un groupe cyano, difluorométhyle, trifluorométhyle, difluorométhoxy ou trifluorométhoxy,
R⁸ représente un atome d'hydrogène,
R² représente un atome de chlore, un groupe cyano, méthoxy, éthoxy ou difluorométhoxy,
R³ représente un atome d'hydrogène, un groupe méthyle, éthyle, n-propyle, 2-méthyl-prop-1-ynyle, n-butyle ou éthoxy,
le groupe méthyle pouvant être substitué par un substituant choisi dans le groupe constitué par diflorométhyle, trifluorométhyle, cyclopropyle, cyclobutyle, cyclohexyle, oxétanyle, tétrahydrofuranyle, tétrahydro-2H-pyranyle et 1,4-dioxanyle,
les groupes cyclopropyle, cyclobutyle, cyclohexyle et oxétanyle pouvant être substitués par 1 ou 2 substituants choisis indépendamment l'un de l'autre dans le groupe constitué par fluoro, hydroxy, méthyle, éthyle et méthoxy,
et
les groupes éthyle, n-propyle et n-butyle pouvant être substitués par un substituant choisi dans le groupe constitué par fluoro, méthoxy et trifluorométhoxy,
R⁴ représente un atome d'hydrogène,
R⁵ représente un groupe de formule 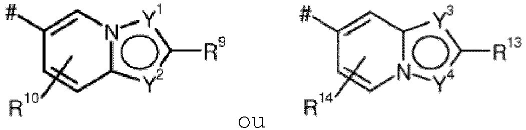 ou 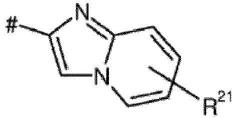 # étant le point d'attachement à l'atome d'azote,
Y¹ représentant un atome d'azote ou C-R¹¹,
où
R¹¹ représente un atome d'hydrogène ou de chlore, un groupe hydroxy ou méthoxy,
Y² représentant un atome d'azote ou C-R¹²,
où
R¹² représente un atome d'hydrogène ou de chlore, un groupe hydroxy ou méthoxy,
R⁹ représentant un atome d'hydrogène ou un groupe hydroxycarbonyle,
R¹⁰ représentant un atome d'hydrogène ou de fluor,
Y³ représentant un atome d'azote ou C-R¹⁵,
où
R¹⁵ représente un atome d'hydrogène ou de chlore, un groupe hydroxy ou méthoxy,
Y⁴ représentant un atome d'azote ou C-R¹⁶,
où
R¹⁶ représente un atome d'hydrogène ou de chlore, un groupe hydroxy ou méthoxy,
R¹³ représentant un atome d'hydrogène ou un groupe hydroxycarbonyle,
R¹⁴ représentant un atome d'hydrogène ou de fluor,
R²¹ représentant un atome d'hydrogène ou un groupe hydroxycarbonyle,
ou un de ses sels, de ses produits de solvatation ou des produits de solvatation de ses sels.

3. Composé selon l'une quelconque des revendications 1 et 2, **caractérisé en ce que**
R¹ représente un groupe de formule 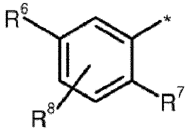 dans laquelle * est le point d'attachement au cycle oxopyridine,
R⁶ représente un atome de chlore,
R⁷ représente un groupe cyano ou difluorométhoxy,
R⁸ représente un atome d'hydrogène,
R² représente un groupe méthoxy,
R³ représente un groupe méthyle ou éthyle,
le groupe méthyle pouvant être substitué par un substituant choisi dans le groupe constitué par cyclobutyle et tétrahydro-2H-pyranyle,
et
le groupe éthyle pouvant être substitué par un substituant méthoxy,
R⁴ représente un atome d'hydrogène,
R⁵ représente un groupe de formule 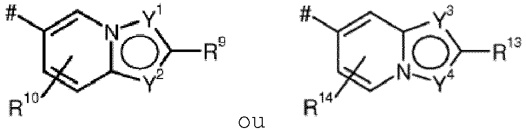 # étant le point d'attachement à l'atome d'azote,
Y¹ représentant un groupe C-R¹¹,
où
R¹¹ représente un atome d'hydrogène ou de chlore,
Y² représentant un atome d'azote,
R⁹ représentant un atome d'hydrogène ou un groupe hydroxycarbonyle,
R¹⁰ représentant un atome d'hydrogène,
Y³ représentant un atome d'azote,
et
Y⁴ représentant C-R¹⁶,
où
R¹⁶ représente un atome d'hydrogène,
ou
Y³ représentant C-R¹⁵,
où
R¹⁵ représente un atome d'hydrogène ou de chlore,
et
Y⁴ représentant un atome d'azote,
R¹³ représentant un atome d'hydrogène ou un groupe hydroxycarbonyle,
R¹⁴ représentant un atome d'hydrogène,
ou un de ses sels, de ses produits de solvatation ou des produits de solvatation de ses sels.

4. Procédé pour la préparation d'un composé de formule (I) ou d'un de ses sels, de ses produits de solvatation ou des produits de solvatation de ses sels selon la revendication 1, **caractérisé en ce que** soit
[A] on fait réagir un composé de formule 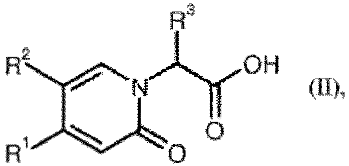 dans laquelle
R¹, R² et R³ ont la signification indiquée dans la revendication 1,
dans la première étape avec un composé de formule 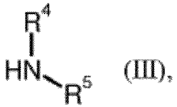 dans laquelle
R⁴ et R⁵ ont la signification indiquée dans la revendication 1,
en présence d'un réactif de déshydratation, et
éventuellement dans une deuxième étape on le convertit en un composé de formule (I) par coupure acide ou basique d'ester,
ou
[B] on fait réagir un composé de formule 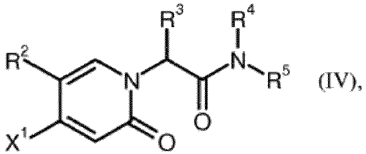 dans laquelle
R², R³, R⁴ et R⁵ ont la signification indiquée dans la revendication 1 et
X¹ représente un atome de chlore, de brome ou d'iode,
avec un composé de formule
R¹-Q (V)
dans laquelle
R¹ a la signification indiquée dans la revendication 1, et
Q représente -B(OH)₂, un ester d'acide boronique, de préférence le boronate de pinacol, ou -BF₃⁻K⁺,
dans des conditions de couplage de Suzuki, pour aboutir à un composé de formule (I).

5. Composé selon l'une quelconque des revendications 1 à 3, destiné à l'utilisation dans le traitement et/ou la prophylaxie de maladies.

6. Utilisation d'un composé selon l'une quelconque des revendications 1 à 3, pour la fabrication d'un médicament destiné au traitement et/ou à la prophylaxie de maladies.

7. Utilisation d'un composé selon l'une quelconque des revendications 1 à 3, pour la fabrication d'un médicament destiné au traitement et/ou à la prophylaxie de maladies thrombotiques ou thromboemboliques.

8. Utilisation d'un composé selon l'une quelconque des revendications 1 à 3, pour la fabrication d'un médicament destiné au traitement et/ou à la prophylaxie de maladies ophtalmologiques.

9. Utilisation d'un composé selon l'une quelconque des revendications 1 à 3, pour la fabrication d'un médicament destiné au traitement et/ou à la prophylaxie d'angio-oedèmes héréditaires ou de maladies inflammatoires de l'intestin, telles que la maladie de Crohn ou la colite ulcéreuse.

10. Médicament contenant un composé selon l'une quelconque des revendications 1 à 3 en association avec un adjuvant inerte, non toxique, pharmaceutiquement convenable.

11. Médicament selon la revendication 10, destiné à l'utilisation dans le traitement et/ou la prophylaxie de maladies thrombotiques ou thromboemboliques.

12. Médicament selon la revendication 10, destiné à l'utilisation dans le traitement et/ou la prophylaxie de maladies ophtalmologiques.

13. Médicament selon la revendication 10, destiné à l'utilisation dans le traitement et/ou la prophylaxie d'angio-oedèmes héréditaires ou de maladies inflammatoires de l'intestin, telles que la maladie de Crohn ou la colite ulcéreuse.
